# EUROPEAN PATENT APPLICATION

(11) **EP 4 745 159 A1**
(43) Date of publication of application: **20.05.2026**
(21) Application number: 24838827.4
(22) Date of filing: 10.07.2024
(51) Int. Cl.: C07K 16/30, A61K 35/17, A61K 39/00, C12N 15/113, A61P 35/00

(54) **COMPOSITION FOR CELLULAR IMMUNOTHERAPY, AND METHOD**

(30) Priority: 10.07.2023 CN 202310842080; 21.07.2023 CN 202310905232; 16.08.2023 CN 202311035421
(71) Applicant: Carsgen Life Sciences Co., Ltd., Shanghai 200131 (CN)
(72) Inventor: LI, Zonghai, Shanghai 200231 (CN); JIANG, Hua, Shanghai 200231 (CN); LIAO, Zhaohui, Shanghai 200231 (CN); WANG, Yi, Shanghai 200231 (CN); ZHANG, Honghong, Shanghai 200231 (CN)
(74) Representative: Lavoix
(86) International application number: PCT/CN2024/104682
(87) International publication number: WO 2025/011575

(57) **Abstract**

A chimeric receptor having an anti-transplant rejection function, an engineered cell expressing the chimeric receptor, an anti-tumor and anti-transplant immune rejection method, and in particular an anti-NK cell immune rejection method. Also provide is a cell therapy.

## Description

### Technical Field

The present application relates to a chimeric receptor, an engineered cell expressing the chimeric receptor, and further relates to a method for applying the chimeric receptor and the engineered cell in immune cell therapy.

### Cross-references

This application claims the benefit of Chinese patent application CN2023108420804 filed on July 10, 2023, Chinese patent application CN2023109052320 filed on July 21, 2023, and Chinese patent application CN2023110354213 filed on August 16, 2023, which is hereby incorporated by reference in its entirety into the present invention.

### Simultaneously Submitted Sequence listing

The entire contents of the following XML file are incorporated herein by reference in their entirety: Sequence Listing FH01069 PCT-Sequence listing in Computer Readable Format (CRF) (created on July 10, 2024, size: 159 KB).

### Background

Due to the immunogenetic differences between the donor and the recipient, during transplantation of an exogenous donor graft, as an exogenous graft, the donor graft may also be recognized and attacked by the immune cells of a recipient, thereby be suppressed or eliminated, and resulting in a host-versus-graft reaction (HVGR). Knocking out the MHC molecules in the graft cells can effectively resist the rejection on the graft by host T cell, however rejections by other immune cells in the host may be triggered. For example, in allogeneic cell transplantation, the absence of MHC-I molecules on allogeneic cells will induce the rejection by NK cells in the host, thereby enhancing the clearance of the allogeneic cells. Therefore, how to effectively prevent the immune rejection by NK cells in the host is crucial for the development of allogeneic cell transplantation therapy.

CD38 is a glycoprotein located on the membrane and has been shown to be widely expressed in various malignant hematologic tumors (e.g., MM and AML), and is a potential therapeutic target. CD38 is also strongly expressed on immunosuppressive cells, and can serve as a potential target for these cells. Since CD38 is also expressed on T cells, fratricide occurs during the culture and expansion of CD38-CAR-T cells, leading to culture failure and poor efficacy of CD38-CAR-T in treating hematologic tumors.

Because activated T cells express NKG2D ligands on their surface, NKG2D-CAR-T cells may undergo fratricide during *in vitro* and *in vivo* culture, resulting in difficulties in culturing and expanding NKG2D-CAR-T cells *in vitro* and poor survival of CAR-T cell *in vivo,* thus affecting the production and clinical application of NKG2D-CAR-T cells.

Therefore, it is crucial to effectively prevent the fratricide during the culture of CD38-CAR-T or NKG2D-CAR-T cells and improve the role of CAR-T in anti-tumor and anti-immune transplantation.

### Summary of the Invention

It should be understood that within the scope of this application, the above-mentioned technical features of this application and the technical features described in detail below (such as in the embodiments) can be combined with each other to form new or preferred technical solutions. Due to space limitations, they are not all listed here.
(I) One aspect of this application comprises the following technical solutions and combinations thereof:
   The first part of the present application provides a chimeric receptor, wherein the chimeric receptor comprises an antigen binding domain that binds to CD38, a transmembrane domain and an intracellular domain, and the antigen binding domain is connected to the transmembrane domain via an IgG4 hinge region or a fragment thereof.

Preferably, the antigen binding domain also binds to a tumor antigen and/or a pathogen antigen.

The second part of the present application provides a polynucleotide, wherein the polynucleotide encodes the chimeric receptor described in the first part.

The third part of the present application provides a polynucleotide, wherein the polynucleotide encodes a chimeric receptor that binds to CD38 and a tumor antigen, or encodes a first chimeric receptor that binds to CD38 and a second chimeric receptor that binds to a tumor antigen;

The polynucleotide component encoding the chimeric receptor that binds to CD38 and a tumor antigen is represented by any of the following formulas:
L-VL1-VH2-I-VL2-VH1-H-TM-C-CD3ζ; L-VL1-VL2-I-VH2-VH1-H-TM-C-CD3ζ;
L-VH1-VH2-I-VL2-VL1-H-TM-C-CD3ζ; L-VH1-VL2-I-VH2-VL1-H-TM-C-CD3ζ;
L-VL2-VH1-I-VL1-VH2-H-TM-C-CD3ζ; L-VH2-VH1-I-VL1-VL2-H-TM-C-CD3ζ;
L-VL2-VL1-I-VH1-VH2-H-TM-C-CD3ζ; L-VH2-VL1-I-VH1-VL2-H-TM-C-CD3ζ;

The polynucleotide components encoding the first chimeric receptor that binds to CD38 and the second chimeric receptor that binds to a tumor antigen are represented by any of the following formulae:
(1) L-VL1-VH1-H-TM-C-CD3ζ-2A-L-VH2-VL2-H-TM-C-CD3ζ
(2) L-VL1-VH1-H-TM-C-CD3ζ-2A-L-VL2-VH2-H-TM-C-CD3ζ
(3) L-VH1-VL1-H-TM-C-CD3ζ-2A-L-VH2-VL2-H-TM-C-CD3ζ
(4) L-VH1-VL1-H-TM-C-CD3ζ-2A-L-VL2-VH2-H-TM-C-CD3ζ
(5) L-VL2-VH2-H-TM-C-CD3ζ-2A-L-VH1-VL1-H-TM-C-CD3ζ
(6) L-VL2-VH2-H-TM-C-CD3ζ-2A-L-VL1-VH1-H-TM-C-CD3ζ
(7) L-VH2-VL2-H-TM-C-CD3ζ-2A-L-VH1-VL1-H-TM-C-CD3ζ
(8) L-VH2-VL2-H-TM-C-CD3ζ-2A-L-VL1-VH1-H-TM-C-CD3ζ

In the formula, each "-" is independently a linker peptide or peptide bond component; L is an optional nucleic acid component encoding a signal peptide; I is a nucleic acid component encoding a flexible linker; H is an optional hinge region nucleic acid component; TM is a transmembrane domain nucleic acid component; C is a co-stimulatory signal molecule nucleic acid component; CD3ζ is a cytoplasmic signal transduction sequence derived from CD3ζ; 2A is an optional 2A self-cleaving peptide nucleic acid component; VH1 is a CD38 antibody heavy chain variable region; VL1 is a CD38 antibody light chain variable region; VL2 is an antibody light chain variable region that binds to a tumor antigen; VH2 is an antibody heavy chain variable region that binds to a tumor antigen; "-" is a linker peptide or peptide bond.

Preferably, the polynucleotide is codon-optimized for expression in human cells.

Preferably, the nucleotide sequences encoding the same component are codon-divergent.

Preferably, the 2A peptide is selected from T2A, P2A, E2A or F2A.

Preferably, the hinge region comprises an IgG4 hinge region or a fragment thereof.

The fourth part of this application provides a vector, which comprises the polynucleotides described in the second or third parts.

The fifth part of the present application provides an engineered cell, wherein the engineered cell comprises the polynucleotide described in the second or third parts, or expresses the chimeric receptor described in the first part.

Preferably, the engineered cells can enhance the survival and proliferation of a second T cell and/or a second CAR-T cell targeting a tumor antigen that is introduced into the subject before, concurrently with, or after the engineered cell; as well as the killing of tumor cells by the second T cell and/or the second CAR-T cell.

Preferably, it can be used to eliminate allogeneic immune cells.

Preferably, the engineered cell is an immune cell; preferably, the immune cell is selected from: a T cell, an NK cell, a cytotoxic T cell, an NKT cell, a dendritic cell, a macrophage, a CIK cell, and a stem cell-derived immune cell, or a combination thereof. Preferably, the engineered cell is an autologous or allogeneic cell. Preferably, the engineered cell has low or no expression of endogenous TCR, B2M, HLA-II, and/or NKG2A.

(II) Another aspect of this application comprises the following technical solutions and combinations thereof:
The first part of the present application provides a chimeric receptor, wherein the chimeric receptor comprises:
(i) a target molecule binding domain; (ii) a membrane-bound receptor or a fragment thereof associated with a degradation pathway;
Wherein, (i) is connected to (ii) directly or via a linker fragment; the target molecule binding domain comprises: an antibody or a fragment thereof, a receptor or a fragment thereof, an antigen or a fragment thereof; and the membrane-bound receptor associated with the degradation pathway comprises a lysosomal shuttle receptor.

Preferably, the chimeric receptor comprises:
(i) the extracellular domain, transmembrane domain, and/or intracellular domain of a membrane-bound receptor associated with a degradation pathway, or a combination of fragments thereof; or, (ii) the transmembrane domain and intracellular domain of a membrane-bound receptor associated with a degradation pathway.

Preferably, the membrane-bound receptor comprises mannose-6-phosphate receptor (M6PR), cation-independent mannose-6-phosphate receptor (CI-M6PR), ASGPR, insulin-like growth factor 2 receptor (IGF2R), or a combination thereof.

Preferably, the membrane-bound receptor is ASGPR1 and/or ASGPR2.

Preferably, the target molecule comprises: a cell marker or its ligand, a tissue marker or its ligand, an immune checkpoint protein or its ligand, a pathogenic autoantibody or a fragment thereof, a pathogenic target, a non-protein substance, or a combination thereof.

Preferably, the target molecule is: an immune cell antigen, a tumor antigen, a pathogen antigen or a combination thereof.

Preferably, the target molecule is: a T cell antigen and/or an NK cell antigen.

Preferably, the target molecule is selected from: CD2, CD3, CD4, CD5, CD7, CD8, CD16a, CD16b, CD25, CD27, CD28, CD30, CD38, CD45, CD48, CD50, CD52, CD56, CD57, CD62L, CD69, CD94, CD100, CD102, CD122, CD127, CD132, CD137, CD160, CD161, CD178, CD218, CD226, CD244, NKG2A, NKG2C, NKG2E, CD279, NKG2D, CD305, NKP46, CD337, CS1, TCRα, TCRβ, SLAMF7, or a ligand thereof.

Preferably, the target molecule is an NKG2D ligand. Preferably, the target molecule binding domain is the full-length NKG2D, the extracellular domain of NKG2D or a fragment thereof, an antibody that recognizes NKG2D or a fragment thereof, or an antibody that recognizes an NKG2D ligand or a fragment thereof; and preferably, the full-length NKG2D or a fragment thereof is located at the C-terminus of the chimeric receptor.

Preferably, the target molecule binding domain comprises: Fab, Fab', F(ab')2, F(ab)2, variable fragment (Fv), domain antibody (dAb), single domain antibody, or single chain variable fragment (scFv).

Preferably, the linker fragment is a (G4S)n linker, wherein n is an integer from 1 to 20.

Preferably, the chimeric receptor, from N-terminus to C-terminus, sequentially comprises SEQ ID NOs: 80 and 76, or comprises SEQ ID NOs: 81 and 76.

The second part provides a chimeric receptor that recognizes NKG2D, wherein the chimeric receptor comprises:
(i) an antibody that recognizes NKG2D or a fragment thereof; (ii) a membrane-bound receptor associated with a degradation pathway or a fragment thereof.

The third part provides a chimeric receptor that recognizes an NKG2D ligand, wherein the chimeric receptor comprises:
(i) an antibody that recognizes an NKG2D ligand or a fragment thereof; (ii) a membrane-bound receptor associated with a degradation pathway or a fragment thereof.

The fourth part provides a chimeric receptor that recognizes an NKG2D ligand, wherein the chimeric receptor sequentially comprises:
(i) a membrane-bound receptor associated with a degradation pathway or a fragment thereof; (ii) full-length NKG2D, the extracellular domain of NKG2D or a fragment thereof.

Preferably, the membrane-bound receptor comprises M6PR, CI-M6PR, ASGPR, IGF2R, or a combination thereof.

Preferably, the membrane-bound receptor is ASGPR1 and/or ASGPR2.

Preferably, (i) and (ii) are connected via a linker fragment, preferably, the linker fragment is a (G4S)n linker, wherein n is an integer from 1 to 20.

The fifth part provides an engineered cell comprising the chimeric receptor described in the first, second, third, or fourth part.

Preferably, the cell comprises an immune cell, a neuron, an epithelial cell, an endothelial cell, a stem cell, or a combination thereof;

Preferably, the cell is selected from: a B cell, a monocyte, a natural killer cell, a basophil, an eosinophil, a neutrophil, a dendritic cell, a macrophage, a T cell, an NKT cell, a peripheral mononuclear cell (PBMC), a stem cell-derived immune cell, or combinations thereof.

Preferably, the cell is an autologous cell or allogeneic cell.

Preferably, the cell further expresses a chimeric receptor 2;

Preferably, the chimeric receptor 2 recognizes the same target molecule as the chimeric receptor;

Preferably, the chimeric receptor 2 is a CAR and/or a recombinant TCR.

Preferably, the chimeric receptor 2 recognizes a tumor antigen and/or a pathogen antigen.

Preferably, the tumor antigen is selected from: CD19, CD20, CD22, CD38, BCMA, GPRC5D, B7H3, GPC3, Claudin 6, Claudin18.2, FAP, Mesothelin, NKG2D, NKG2A, CD94, or a combination thereof.

Preferably, the engineered cell comprises:
a) low or no expression of endogenous MHC-I molecules; b) low or no expression of endogenous TCR molecules;
c) low or no expression of endogenous MHC-II molecules; d) low or no expression of endogenous NKG2A molecules;
e) low or no expression of endogenous FAS and/or CD58 molecules; and/or
f) genetically engineered to increase the expression, activity and/or signaling of a GRM polypeptide in the cell.

Preferably, the engineered cell further comprises NKG2D-CAR.

Preferably, the extracellular domain of the NKG2D-CAR comprises an antibody that recognizes NKG2D or a fragment thereof.

Preferably, the extracellular domain of the NKG2D-CAR comprises full-length NKG2D, the extracellular domain of NKG2D or a fragment thereof, an antibody that recognizes an NKG2D ligand or a fragment thereof.

Preferably, the intracellular domain of the NKG2D-CAR comprises a CD28 intracellular domain, a CD137 intracellular domain, a CD3Z intracellular domain or a combination thereof; preferably, the intracellular domain of the NKG2D-CAR comprises a CD3Z intracellular domain.

Preferably, the engineered cell further comprises a second CAR, which does not recognize NKG2D or its ligand.

Preferably, the second CAR recognizes a tumor antigen and/or a pathogen antigen.

Preferably, the tumor antigen is selected from: CD19, CD20, CD22, CD38, BCMA, GPRC5D, B7H3, GPC3, Claudin 6, Claudin18.2, FAP, Mesothelin, NKG2D, NKG2A, CD94, or a combination thereof.

Preferably, the engineered cells comprise:
a) low or no expression of endogenous MHC-I molecules; b) low or no expression of endogenous TCR molecules;
c) low or no expression of endogenous MHC-II molecules; d) low or no expression of endogenous NKG2A molecules;
e) low or no expression of endogenous FAS and/or CD58 molecules; and/or
f) genetically engineered to increase the expression, activity and/or signaling of a GRM polypeptide in the cell.

Preferably, the engineered cell is a T cells an NK cell, an NKT cell, or a combination thereof.

Preferably, the engineered cell is an autologous cells or an allogeneic cell.

The sixth part provides a composition comprising the engineered cell as described in the fifth part, and further comprising another engineered cell. Preferably, the another engineered cell comprises the chimeric receptor described in the first, second, third or fourth part.

Preferably, the another engineered cell expresses a chimeric receptor 3 that recognizes a tumor antigen and/or a pathogen antigen, and the chimeric receptor 3 does not recognize NKG2D or its ligand; preferably, the chimeric receptor 3 is a CAR or a recombinant TCR.

Preferably, the tumor antigen is selected from: CD19, CD20, CD22, CD38, BCMA, GPRC5D, B7H3, GPC3, Claudin 6, Claudin18.2, FAP, Mesothelin, NKG2D, NKG2A, CD94, or a combination thereof.

Preferably, the another engineered cell comprises:
a) low or no expression of endogenous MHC-I molecules; b) low or no expression of endogenous TCR molecules;
c) low or no expression of endogenous MHC-II molecules; d) low or no expression of endogenous NKG2A molecules;
e) low or no expression of endogenous FAS and/or CD58 molecules; and/or
f) genetically engineered to increase the expression, activity and/or signaling of a GRM polypeptide in the cell.

The seventh part provides a method for treating a disease in a subject, comprising administering the engineered cell described in the fifth part or the composition described in the sixth part to the subject.

Preferably, the method improves the survival and/or expansion of the engineered cell and/or another engineered cell.

Preferably, the method reduces fratricide among the engineered cells.

Preferably, the method reduces killing of the engineered cell and/or another engineered cell by the subject's immune cells, and optionally further comprises depleting the subject's immune cells.

The eighth part provides a method for improving cell survival and/or expansion during *in vivo* and/or *in vitro* culture, comprising genetically engineering the cell to express the chimeric receptor described in the first to fourth parts.

The ninth part provides a method for improving the survival and/or expansion of CAR-T cell *in vivo,* comprising administering the engineered cell described in the fifth part, wherein the engineered cell recognizes an immune cell antigen and the CAR-T cell recognizes a tumor antigen and/or a pathogen antigen.

The tenth part provides a method for reducing the level and/or activity of a membrane protein in an engineered cell, comprising causing the engineered cell to express the chimeric receptor described in the first to fourth parts, wherein the chimeric receptor recognizes the membrane protein.

Preferably, the level and/or activity of the membrane protein is reduced by about 55%, 60%, 65%, 70%, 75%, 80%, 85%, 90%, 91%, 92%, 93%, 94%, 95%, 96%, 97%, 98%, 99% or more relative to a reference.

Preferably, wherein the method is performed *in vivo, ex vivo* or *in vitro.*

Preferably, the engineered cell is from a subject in need or a healthy individual.

The eleventh part provides a method for reducing the level and/or activity of NKG2D or its ligand in engineered cells, comprising causing the engineered cell to express the chimeric receptor described in the second, third, or fourth parts.

Preferably, the level and/or activity of NKG2D or its ligand is reduced by about 55%, 60%, 65%, 70%, 75%, 80%, 85%, 90%, 91%, 92%, 93%, 94%, 95%, 96%, 97%, 98%, 99% or more relative to a reference.

Preferably, wherein the method is performed *in vivo, ex vivo* or *in vitro.*

Preferably, the engineered cell is from a subject in need or a healthy individual.

The twelfth part provides a method for improving the survival of an immune cell comprising an NKG2D-CAR, comprising genetically engineering the immune cell to express the chimeric receptor described in the second, third, or fourth parts, wherein the NKG2D-CAR recognizes NKG2D or its ligand.

Preferably, the method reduces the level and/or activity of NKG2D or its ligand in the immune cell.

Preferably, the level and/or activity of NKG2D or its ligand in the immune cells is reduced by about 55%, 60%, 65%, 70%, 75%, 80%, 85%, 90%, 91%, 92%, 93%, 94%, 95%, 96%, 97%, 98%, 99% or more relative to a reference.

Preferably, the immune cell is from a subject in need or a healthy individual.

The chimeric receptor described in the first, second, third, or fourth parts, the engineered cell described in the fifth part, the composition described in the sixth part, or the method described in the seventh to twelfth parts, for treating tumors.

The chimeric receptor described in the first, second, third, or fourth parts, the engineered cell described in the fifth part, the composition described in the sixth part, and the methods described in the seventh to twelfth parts, for treating, preventing, or ameliorating autoimmune diseases or inflammatory diseases.

The thirteenth part provides a method for increasing the survival time and/or expansion capacity of an engineered cell targeting a tumor and/or a pathogen in the presence of a host immune cell, which comprises administering the engineered cell described in the fifth part, the composition described in the sixth part, and the method described in the seventh to twelfth parts to a subject in need.

Preferably, the subject is a human; preferably, the engineered cell is an autologous T cell, an NK cell, an NKT cell; or an allogeneic T cell, an NK cell, an NKT cell.

The fourteenth part provides a pharmaceutical composition, comprising the engineered cell described in the fifth part, and/or the cell composition described in the sixth part, and a pharmaceutically acceptable excipient.

(III) Yet another aspect of this application comprises the following technical solutions and combinations thereof:
(1) The present application provides a chimeric receptor, comprising a first antigen binding domain and a second antigen binding domain, wherein the first antigen binding domain binds to GPRC5D, comprises a heavy chain variable region (VH) and a light chain variable region (VL), and comprises HCDR1, HCDR2, HCDR3, LCDR1, LCDR2, and LCDR3 comprising the amino acid sequences shown in SEQ ID NOs: 104, 105, 106, 107, 108, and 109, respectively; and the second antigen binding domain binds to a multiple myeloma-associated antigen different from GPRC5D, or an immune cell marker;
(2) The chimeric receptor according to (1), wherein a VH and a VL comprised in the first antigen binding domain comprise amino acid sequences having at least about 80%, 85%, 90%, 95%, 96%, 97%, 98%, 99% or 100% sequence identity to the amino acid sequence shown in SEQ ID NOs: 99 and 102, respectively;

Preferably, an scFv comprised in the first antigen binding domain comprises an amino acid sequence having at least about 80%, 85%, 90%, 95%, 96%, 97%, 98%, 99% or 100% sequence identity to the amino acid sequence shown in SEQ ID NO:103.

Preferably, the second antigen is selected from: BCMA, CD3, CD19, CD20, CD22, CD123, CD38, CD138, CS-1, BAFF-R, TACI, FcRH5, NKG2A, TIGIT, FasL, CD94, CD300A, or a combination thereof.

(3) The chimeric receptor according to (1) or (2), wherein the structure of the chimeric receptor from N-terminus to C-terminus is shown in the following formula, selected from any one of the following formulas (1) to (4): (1) VL1-VH2-VL2-VH1-H-TM-C; (2) VL2-VH1-VL1-VH2-H-TM-C; (3) VH1-VL2-VH2-VL1-H-TM-C; (4) VH2-VL1-VH1-VL2-H-TM-C; wherein each "-" is independently a linker peptide or peptide bond; H is an optional hinge region; TM is a transmembrane domain; C is an intracellular signaling domain; VH1 is the heavy chain variable region of a GPRC5D antibody; VL1 is the light chain variable region of a GPRC5D antibody; VH2 is the heavy chain variable region of an antibody against the second antigen; VL2 is the light chain variable region of an antibody against the second antigen;

(4) The chimeric receptor according to (3), wherein the VH2 comprises HCDR1, HCDR2, and HCDR3, and the VL2 comprises LCDR1, LCDR2, and LCDR3, comprising amino acid sequences shown in SEQ ID NOs: 110, 111, 112, 113, 114, and 115, respectively;

Preferably, the VH2 and VL2 comprise amino acid sequences having at least about 80%, 85%, 90%, 95%, 96%, 97%, 98%, 99% or 100% sequence identity to the amino acid sequences shown in SEQ ID NOs: 100 and 101, respectively, or comprise amino acid sequences having at least about 80%, 85%, 90%, 95%, 96%, 97%, 98%, 99% or 100% sequence identity to the amino acid sequences shown in SEQ ID NOs: 116 and 117, respectively;

(5) The chimeric receptor according to (3) or (4), wherein the transmembrane domain is selected from the transmembrane domain of CD3ε, CD3δ, CD3ζ, CD8, CD28, CD134, CD137, CD150, CD152, DAP10, FcRα, FeRβ, FcRγ, or Zap70; preferably, the transmembrane domain comprises an amino acid sequence shown in SEQ ID NO: 7, 8 or 9.

(6) The chimeric receptor according to any one of (1) to (5), wherein the intracellular signaling domain is selected from the intracellular signaling domain of CD3, CD28, CD137, OX40, DAP10 or ICOS, or a combination thereof; preferably, the intracellular signaling domain comprises the amino acid sequence shown in SEQ ID NO: 10, 11, 12, or 13, or a combination thereof;

(7) The chimeric receptor according to any one of (1) to (6), wherein the hinge region comprises a CD8 hinge, a CD28 hinge, or an IgG4 hinge.

(8) The chimeric receptor according to any one of (1) to (7), wherein the chimeric receptor comprises an amino acid sequence having at least about 80%, 85%, 90%, 95%, 96%, 97%, 98%, 99% or 100% sequence identity to the amino acid sequence encoded by the nucleic acid shown in SEQ ID NO:37; or comprises an amino acid sequence having at least about 80%, 85%, 90%, 95%, 96%, 97%, 98%, 99% or 100% sequence identity to the amino acid sequence shown in SEQ ID NO:84, 96, or 97.

(9). The present application provides a polynucleotide encoding the chimeric receptor as described in any one of (1) to (8).

(10) The polynucleotide according to (9), wherein the polynucleotide comprises a nucleic acid sequence having at least about 80%, 85%, 90%, 95%, 96%, 97%, 98%, 99% or 100% identity to the nucleic acid sequence shown in SEQ ID NO: 37, 90, 91, 92, 93, 94, or 95; preferably, the polynucleotide further comprises a nucleic acid sequence having at least about 80%, 85%, 90%, 95%, 96%, 97%, 98%, 99% or 100% identity to the nucleic acid sequence shown in SEQ ID NO: 4, 18, 23, 25, 29, 30, 31, 32, 33, 34, 35, 36, 37, 38, 78, or 79.

(11). The present application provides a vector comprising the polynucleotide described in (9) or (10).

(12) The present application provides an engineered cell comprising the chimeric receptor described in any one of (1) to (8), the polynucleotide described in (9) or (10), or the vector described in (11);

(13) The engineered cell according to (12), wherein the engineered cell is selected from: a T cell, a natural killer cell, a natural killer T cell, an NK92 cell, a cytotoxic T cell, a dendritic cell, a macrophage, a CIK cell, a stem cell-derived immune cell or a combination thereof; preferably, the immune cell comprises a natural T cell and/or a T cell induced from a pluripotent stem cell; preferably, the T cell comprises an autologous T cell and/or an allogeneic T cell; preferably, the T cell is a primary T cell; preferably, the T cell is derived from a human autologous T cell.

(14). The engineered cells according to (12) or (13), wherein the engineered cell further comprises: low or no expression of endogenous TCR, B2M, HLA-I, HLA-II, NKG2A, FAS and/or CD58; preferably, the engineered cell further comprises: no expression of endogenous TCR/B2M, no expression of endogenous TCR/B2M/FAS, no expression of endogenous TCR/B2M/NKG2A, or no expression of endogenous TCR/B2M/NKG2A/FAS.

(15) The engineered cell according to any one of (12) to (14), wherein the immune cell is prepared by a rapid preparation method; preferably, the total activation and viral transduction time of the immune cell does not exceed 24 hours; preferably, the immune cell does not undergo significant *ex vivo* expansion after transduction.

(16) The present application provides a pharmaceutical composition, comprising an effective amount of the chimeric receptor described in any one of (1) to (8), the polynucleotide described in (9) or (10), the vector described in (11), or the engineered cell described in any one of (12) to (15), and a pharmaceutically acceptable carrier, diluent or excipient.

(17) The present application provides a medicament for preventing or treating a disease, the medicament comprising the chimeric receptor described in any one of (1)-(8), the polynucleotide described in (9) or (10), the vector described in (11), or the engineered cell described in any one of (12)-(15).

(18). The present application provides the chimeric receptor as described in any one of (1) to (8), or the engineered cell as described in any one of (12) to (15), for killing or inhibiting GPRC5D-positive pathological cells; preferably, for use in the manufacture of a medicament for treating an autoimmune disease or inflammatory disease; preferably, for treating, preventing or ameliorating an autoimmune disease or inflammatory disease in subjects in need; preferably, the autoimmune disease or inflammatory disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis or dermatomyositis; preferably, for use in the manufacture of a medicament for treating a tumor; preferably, for treating, preventing or ameliorating a tumor in subjects in need; preferably, the tumor comprises: leukemia, lymphoma, or myeloma.

(19) The present application provides a method for treating, preventing or ameliorating an autoimmune disease, inflammatory disease or a tumor in a subject in need, comprising administering to the subject the chimeric receptor described in any one of (1)-(8), the polynucleotide described in (9) or (10), the vector described in (11), the engineered cell described in any one of (12)-(15), or the pharmaceutical composition described in (16).

Preferably, the autoimmune disease or inflammatory disease is selected from t: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis, or dermatomyositis. The tumor comprises: leukemia, lymphoma, or myeloma.

(IV) Yet another aspect of this application comprises the following technical solutions and combinations thereof:
(1). The present application provides a chimeric receptor, wherein the chimeric receptor comprises: (i) a target molecule binding domain, and (ii) a transmembrane protein or a fragment thereof; wherein (i) is connected to (ii) directly or via a linker fragment; the target molecule binding domain comprises: an antibody or a fragment thereof, a receptor or a fragment thereof, an antigen or a fragment thereof.
(2) The chimeric receptor according to (1), wherein the target molecule binding domain is a type II membrane protein.
(3) The chimeric receptor according to (1) or (2), wherein the transmembrane protein or a fragment thereof is a type II membrane protein or a fragment thereof.
(4) The chimeric receptor according to any one of (1) to (3), wherein the transmembrane protein is selected from ASGPR1, ASGPR2, CD137L, or CD40L.
(5) A chimeric receptor according to any one of (1) to (4), wherein the target molecule comprises: a cell marker or its ligand, a tissue marker or its ligand, an immune checkpoint protein or its ligand, a pathogenic autoantibody or a fragment thereof, a pathogenic target, a non-protein substance, or a combination thereof.
(6) The chimeric receptor according to any one of (1) to (5), wherein the target molecule is: an immune cell antigen, a tumor antigen, a pathogen antigen, or a combination thereof.
(7) The chimeric receptor according to any one of (1) to (6), wherein the target molecule is a T cell antigen and/or a NK cell antigen.
(8) The chimeric receptor according to any one of (1) to (7), wherein the target molecule is selected from: CD1b, CD1c, CD1d, CD3d, CD3e, CD4, CD7, CD8, CD11, CD13, CD16b, CD20, CD22, CD25, CD26, CD27, CD28, CD30, CD33, CD34, CD38, CD39, CD40, CD44, CD45, CD45RA, CD45RO, CD48, CD52, CD56, CD58, CD59, CD62L, CD66a, CD69, CD70, CD80, CD83, CD85, CD86, CD93, CD94, CD95, CD95L, CD96, CD99, CD100, CD102, CD103, CD107a, CD107b, CD111, CD112, CD117, CD119, CD122, CD123, CD126, CD127, CD132, CD134, CD137, CD150, CD152, CD153, CD154, CD155, CD158, CD159a, CD160, CD161, CD178, CD183, CD185, CD191, CD192, CD196, CD212, CD215, CD218, CD223, CD226, CD244, CD252, CD253, CD275, CD278, CD279, CD281, CD282, CD283, CD284, CD286, CD300, CD305, CD314, CD319, CD336, CD337, CD355, CD360, CD366, CD371, B2MG, NKG2DL, or a combination thereof.

Preferably, the target molecule is selected from: CD1d, CD7, CD13, CD20, CD22, CD25, CD26, CD30, CD33, CD38, CD45, CD52, CD56, CD70, CD80, CD83, CD86, CD93, CD94, CD95L, CD99, CD112, CD117, CD123, CD155, CD159a, CD319, CD366, CD371, NKG2DL or a combination thereof.

Preferably, the target molecule is NKG2D ligand (NKG2DL).

(9) The chimeric receptor according to any one of (1) to (8), wherein the target molecule binding domain is the full-length NKG2D, the extracellular domain of NKG2D or a fragment thereof, an antibody that recognizes NKG2D or a fragment thereof, or an antibody that recognizes an NKG2D ligand or a fragment thereof.

(10) The chimeric receptor according to (9), wherein the full-length NKG2D, the extracellular domain of NKG2D or a fragment thereof, the antibody that recognizes NKG2D or a fragment thereof, or the antibody that recognizes an NKG2D ligand or a fragment thereof is located at the C-terminus of the chimeric receptor.

(11). A chimeric receptor according to any one of (1) to (10), wherein the target molecule binding domain comprises: Fab, Fab', F(ab')2, F(ab)2, variable fragment (Fv), domain antibody (dAb), single domain antibody or single chain variable fragment (scFv).

(12) The chimeric receptor according to any one of (1) to (11), wherein the linker fragment is (G4S)n, wherein n is an integer from 1 to 20.

(13) A chimeric receptor according to any one of (1) to (12), wherein the chimeric receptor, from N-terminus to C-terminus, sequentially comprises an amino acid sequence encoded by the nucleic acid sequence shown in SEQ ID NO: 80 and an amino acid sequence encoded by the nucleic acid sequence shown in SEQ ID NO: 76, or comprises an amino acid sequence encoded by the nucleic acid sequence shown in SEQ ID NO: 81 and an amino acid sequence encoded by the nucleic acid sequence shown in SEQ ID NO: 76. Preferably, the chimeric receptor, from N-terminus to C-terminus, sequentially comprises an amino acid sequence encoded by the nucleic acid sequence shown in SEQ ID NO: 80, an amino acid sequence encoded by the nucleic acid sequence shown in SEQ ID NO: 16, and an amino acid sequence encoded by the nucleic acid sequence shown in SEQ ID NO: 76, or an amino acid sequence encoded by the nucleic acid sequence shown in SEQ ID NO: 81, an amino acid sequence encoded by the nucleic acid sequence shown in SEQ ID NO: 16, and an amino acid sequence encoded by the nucleic acid sequence shown in SEQ ID NO: 76.

(14). The present application provides a chimeric receptor composition, comprising a first and a second chimeric receptor that recognize the same target molecule, wherein the first chimeric receptor comprises a target molecule binding domain 1, a transmembrane domain, and an intracellular domain, and the first chimeric receptor mediates the killing of target cells expressing the target molecule; the second chimeric receptor comprises a target molecule binding domain 2, a transmembrane protein or a fragment thereof, and the second chimeric receptor does not mediate the killing of target cells expressing the target molecule.

(15) The chimeric receptor composition according to (14), wherein the target molecule binding domain 2 is connected to the transmembrane protein or a fragment thereof directly or via a linker fragment.

(16) The chimeric receptor composition according to (14) or (15), wherein the second chimeric receptor comprises a type II membrane protein or a fragment thereof.

(17) The chimeric receptor composition according to any one of (14) to (16), wherein the second chimeric receptor comprises ASGPR1 or a fragment thereof, ASGPR2 or a fragment thereof, CD137L or a fragment thereof, or CD40L or a fragment thereof.

(18) A chimeric receptor composition according to any one of (14) to (17), wherein the target molecule comprises: a cell marker or its ligand, a tissue marker or its ligand, an immune checkpoint protein or its ligand, a pathogenic autoantibody or a fragment thereof, a pathogenic target, a non-protein substance, or a combination thereof.

(19) The chimeric receptor composition according to any one of (14) to (18), wherein the target molecule is: an immune cell antigen, a tumor antigen, a pathogen antigen, a T cell antigen, an NK cell antigen, or a combination thereof.

(20) The chimeric receptor composition according to any one of (14) to (19), wherein the target molecule is expressed on tumor cells or inflammatory cells, and also on T cells or NK cells.

(21). A chimeric receptor composition according to any one of (14) to (20), wherein the target molecule binding domain 1 or the target molecule binding domain 2 comprises: Fab, Fab', F(ab')2, F(ab)2, variable fragment (Fv), domain antibody (dAb), single domain antibody or single chain variable fragment (scFv).

(22). A chimeric receptor composition according to any one of (14) to (21), wherein the target molecule is selected from: CD2, CD3, CD4, CD5, CD7, CD8, CD16a, CD16b, CD25, CD27, CD28, CD30, CD38, CD45, CD48, CD50, CD52, CD56, CD57, CD62L, CD69, CD94, CD100, CD102, CD122, CD127, CD132, CD137, CD160, CD161, CD178, CD218, CD226, CD244, NKG2A, NKG2C, NKG2E, CD279, NKG2D, CD305, NKP46, CD337, CS1, TCRα, TCRβ, SLAMF7, or a ligand thereof.

Preferably, the target molecule is an NKG2D ligand.

(23) A chimeric receptor composition according to any one of (14) to (22), wherein the target molecule binding domain 1 or 2 is selected from: full-length NKG2D, the extracellular domain of NKG2D or a fragment thereof, an antibody that recognizes NKG2D or a fragment thereof, an antibody that recognizes NKG2D ligand or a fragment thereof; preferably, the target molecule binding domains 1 and 2 both comprise full-length NKG2D.

(24) The chimeric receptor composition according to any one of (14) to (23), wherein the intracellular domain of the first chimeric receptor comprises a functional signaling domain of a stimulatory molecule and/or a co-stimulatory molecule.

(25). A chimeric receptor composition according to any one of (14) to (24), wherein the linker fragment is (G4S)n, wherein n is an integer from 1 to 20, or the linker fragment comprises an amino acid sequence having at least 80%, 85%, 90%, 95%, 96%, 97%, 98%, 99% or 100% identity to the amino acid sequence shown in SEQ ID NO: 14 or 15.

(26) The chimeric receptor composition according to any one of (14) to (25), wherein the first chimeric receptor is inducible expressed.

(27) A chimeric receptor composition according to any one of (14) to (26), wherein the composition further comprises a third chimeric receptor, wherein the third chimeric receptor recognizes another target molecule different from the target molecule, and after the third chimeric receptor recognizes the another target molecule, it induces the expression of the first chimeric receptor; preferably, after the third chimeric receptor binds to the another target molecule, it induces the cleavage of the third chimeric receptor, releasing a transcription factor which regulates the expression of the first chimeric receptor; preferably, the second chimeric receptor and the third chimeric receptor are connected by a cleavable peptide; preferably, the second chimeric receptor and the third chimeric receptor are connected by a 2A peptide; preferably, the 2A peptide comprises: P2A, T2A, E2A or F2A.

(28) The chimeric receptor composition according to (27), wherein the third chimeric receptor and the first chimeric receptor are located in the same expression vector; preferably, the first, second and third chimeric receptors are located in the same expression vector.

(29) The chimeric receptor composition according to (27) or (28), wherein the another target molecule is selected from: a tumor antigen, a tissue-specific marker, and/or a pathogen antigen.

Preferably, the tissue-specific antigen comprises: brain tissue-specific marker MOG, liver tissue-specific marker ASGR1, prostate tissue marker PSA, or a combination thereof.

Preferably, the tumor antigen is selected from: ALPPL2, ALPI, Axl, B7H3, BCMA, CD 117, CD123, CD171, CD179a, CD19, CD213A2, CD20, CD22, CD24, CD246, CD272, CD30, CD33, CD38, CD44v6, CD46, CD7, CD71, CD94, CD97, CEA, Claudin18.2, CLDN6, CLECL1, CLL1, cMet, CS-1, EGFR, EGFRvIII, ELF2M, EpCAM, EphA2, FAP, FCRH5, FLT3, GD2, GD3, GM3, GPC3, GPRC5D, HER2 (ERBB2), IGLL1, IL llRa, IL13Ra2, Mesothelin, MUC1, NCAM, NKG2D-Ligand, PAP, PDGFR-b, PRSS21, PSCA, PSMA, ROR1, SIRPa, SSEA-4, TAG72, TEM1/CD248, TEM7R, TSHR, VEGFR2 or WT1.

(30) The chimeric receptor composition according to any one of (27) to (29), wherein

The first chimeric receptor comprises an amino acid sequence having at least 80%, 85%, 90%, 95%, 96%, 97%, 98%, 99% or 100% identity to the amino acid sequence encoded by the nucleic acid sequence shown in SEQ ID NO: 78 or 79; or

The second chimeric receptor, from N-terminus to C-terminus, sequentially comprises the amino acid sequence encoded by the nucleic acid sequence of SEQ ID NO: 80 and the amino acid sequence encoded by the nucleic acid sequence of SEQ ID NO: 76. Preferably, the second chimeric receptor, from N-terminus to C-terminus, sequentially comprises the amino acid sequence encoded by the nucleic acid sequence of SEQ ID NO: 80, the amino acid sequence encoded by the nucleic acid sequence of SEQ ID NO: 16, and the amino acid sequence encoded by the nucleic acid sequence of SEQ ID NO: 76; or

The third chimeric receptor comprises an amino acid sequence having at least 80%, 85%, 90%, 95%, 96%, 97%, 98%, 99% or 100% identity to the amino acid sequence encoded by the nucleic acid sequence shown in SEQ ID NO:82.

(31). The present application provides an engineered cell comprising the chimeric receptor described in any one of (1) to (13).

(32) The present application provides an engineered cell comprising a chimeric receptor composition as described in any one of (14) to (30)

(33) The engineered cells according to (31) or (32), wherein the engineered cell has low or no expression of endogenous TCR/B2M, TCR/B2M/FAS, TCR/B2M/NKG2A, or TCR/B2M/FAS/NKG2A.

Preferably, the engineered cell is an autologous or allogeneic cell, selected from: an immune cell, a neuron, an epithelial cell, an endothelial cell, a stem cell, or a combination thereof;

Preferably, the engineered cells is selected from: a B cell, a monocyte, a natural killer cell, a basophil, a eosinophil, a neutrophil, a dendritic cell, a macrophage, a T cell, an NKT cell, a stem cell-derived immune effector cell or a combination thereof; preferably, the engineered cell is an allogeneic T cell.

Preferably, the engineered cell significantly blocks target antigens on its own cells.

Preferably, the engineered cell has prolonged survival time or enhanced expansion capacity in the presence of host immune cells;

Preferably, the host immune cell is an NK cell.

Preferably, the engineered cell can enhance the survival, proliferation, and killing of pathological cells of another engineered cell that is introduced into the subject before, concurrently with, or after the engineered cell.

(34). The present application provides a polynucleotide, encoding the construction of the chimeric receptor as described in any one of (1) to (13), or the first, second, and third chimeric receptors in the chimeric receptor composition described in any one of (14) to (30).

Preferably, the polynucleotide encoding the first chimeric receptor is linked to the polynucleotide encoding the second chimeric receptor via a 2A segment; or the polynucleotide encoding the second chimeric receptor is linked to the polynucleotide encoding the third chimeric receptor via a 2A segment.

(35). The present application provides a vector comprising the polynucleotide described in (34).

(36). The present application provides a virus comprising the vector described in (35).

(37). The present application provides a pharmaceutical composition comprising an effective amount of the chimeric receptor as described in any one of (1) to (13), the chimeric receptor composition as described in any one of (14) to (30), the engineered cell as described in any one of (31) to (33), the polynucleotide as described in (34), the vector as described in (35), or the virus as described in (36), and a pharmaceutically acceptable carrier.

(38) The chimeric receptor described in any one of (1) to (13), the chimeric receptor composition described in any one of (14) to (30), or the engineered cell described in any one of (31) to (33), for use in the manufacture of a medicament for preventing or resisting transplant immune rejection, or for preventing or resisting transplant immune rejection, or for use in the manufacture of a medicament for treating an autoimmune disease or inflammatory disease, or for treating, preventing or ameliorating an autoimmune disease or inflammatory disease in a subject in need, or for use in the manufacture of a medicament for treating a tumor, or for use in treating, preventing or ameliorating a tumor in a subject in need.

Preferably, the autoimmune disease or inflammatory disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis or dermatomyositis, Waldenström's macroglobulinemia, primary systemic amyloidosis, membranous glomerulonephritis, idiopathic thrombocytopenic purpura, myasthenia gravis.

Preferably, the tumor is a solid tumor or a hematological tumor;

Preferably, the tumor is selected from: esophageal cancer, gastric cancer, liver cancer, biliary tract tumor, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, prostate cancer, triple-negative breast tumor, or sarcoma; the hematological tumor is selected from: leukemia, lymphoma, myeloma, chronic lymphatic leukemia (CLL), mantle cell lymphoma (MCL), acute myeloid leukemia (AML), acute lymphoblastic leukemia (ALL), NK cell leukemia, NK/T-cell lymphoma (NKTCL), or myelodysplastic syndrome.

(39). The present application provides a method for treating, preventing or ameliorating an autoimmune disease, inflammatory disease or tumor in a subject in need, or a method for preventing or resisting transplant immune rejection, comprising administering to the subject the chimeric receptor as described in any one of (1) to (13), the chimeric receptor composition as described in any one of (14) to (30), the engineered cell as described in any one of (31) to (33), the polynucleotide as described in (34), the vector as described in (35), the virus as described in (36), or the pharmaceutical composition as described in (37).

Preferably, the autoimmune disease or inflammatory disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis or dermatomyositis, Waldenström's macroglobulinemia, primary systemic amyloidosis, membranous glomerulonephritis, idiopathic thrombocytopenic purpura, myasthenia gravis. Preferably, the tumor is a solid tumor or a hematological tumor; preferably, the tumor is selected from: esophageal cancer, gastric cancer, liver cancer, biliary tract tumor, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, prostate cancer, triple-negative breast tumor, sarcoma; the hematological tumor is selected from: leukemia, lymphoma, myeloma, chronic lymphocytic leukemia (CLL), mantle cell lymphoma (MCL), acute myeloid leukemia (AML), acute lymphoblastic leukemia (ALL), NK cell leukemia, NK/T-cell lymphoma (NKTCL), myelodysplastic syndrome.

### BRIEF DESCRIPTION OF THE DRAWINGS

Figures 1A-1E show the CAR positivity rates of CD38-CAR-T cells constructed with different CD38 antibodies (top of Figures 1A, 1B, and 1C; left of Figures 1D and 1E) , wherein CD38-sBBZ and CD38-s28Z constructed with different antibodies can significantly block the CD38 antigen (bottom of Figures 1A, 1B, and 1C; right of Figures 1D and 1E).
Figure 2 shows that the *in vitro* expansion fold of CD38-sBBZ cells constructed with CD38 antibody 1 was significantly increased (left of Figure 2); the *in vitro* expansion fold of CD38-s28Z cells constructed with CD38 antibody 1 was significantly increased (right of Figure 2); and the *in vitro* expansion fold of CD38-sBBZ cells constructed with CD38 antibodies 2 and 3, respectively, was significantly increased (bottom of Figure 2).
Figures 3A-3B show the killing of tumor cells or NK cells by CD38-CAR-T1, 2, 3, 4, 6, 9, 10 (Figure 3A); and secretion of high levels of IL-2, TNF-α, and IFN-γ after co-incubation with tumor cells(Figure 3B).
Figures 4A-4B show that CD38-CAR-T6, 10 significantly inhibit THP-1 orthotopic tumors in NPG mice.
Figures 5A-5B show that CD38-UCAR-T6, 10 significantly kill rNK, aNK (Figure 5A); and secrete high levels of IL-2, TNF-α, and IFN-γ after co-incubation with NK cells (Figure 5B).
Figure 6 shows that CD38-UCAR-T6 significantly inhibit THP-1 orthotopic tumors in NPG mice.
Figures 7A-7B show that CD38-UCAR-T6 cells expressing HLA-E fragments 1, 2, 3, or 4 significantly kill rNK and aNK (Figure 7A); and secrete high levels of IL-2, TNF-α, and IFN-y after co-incubation with NK cells (Figure 7B).
Figures 8A-8B show that bispecific UCAR-T targeting NKG2A and CD38 kills tumor cells *in vitro* (Figure 8A); and secretes high levels of IL-2, TNF-α, and IFN-y after co-incubation with tumor cells (Figure 8B).
Figures 9A-9B show that bispecific UCAR-T targeting NKG2A and CD38 kills NK cells *in vitro* (Figure 9A); and secretes high levels of IL-2, TNF-α, and IFN-y after co-incubation with tumor cells (Figure 9B).
Figure 10 shows that bispecific UCAR-T targeting NKG2A and CD38 significantly inhibit THP-1 orthotopic tumors in NPG mice.
Figure 11 shows that in the presence of NK cells, bispecific UCAR-T targeting NKG2A and CD38 significantly inhibit THP-1 orthotopic tumors in NPG mice.
Figures 12A-12B show that trispecific UCAR-T cells targeting NKG2A, CD38, and the tumor antigen GPRC5D significantly kill tumor cells (left of Figure 12A) and NK cells (right of Figure 12A); trispecific UCAR-T targeting NKG2A, CD38, and the tumor antigen BCMA, and bispecific UCAR-T cells targeting CD94 and CD38 significantly kill NK cells (Figure 12B).
Figures 13A-13B show that compared to NKG2Dz cells, NKG2Dz cells expressing a polypeptide of full-length ASGPR1-NKG2D extracellular domain, or a polypeptide of full-length ASGPR1-linker peptide-NKG2D extracellular domain have significantly improved expansion and survival capacity (Figure 13A); after co-incubation with target cells THP1, NKG2Dz cells expressing a polypeptide of full-length ASGPR1-NKG2D extracellular domain, or a polypeptide of full-length ASGPR1-linker peptide-NKG2D extracellular domain have significantly improved expansion capacity (Figure 13B).
Figure 14 shows that NKG2Dz cells expressing a polypeptide of full-length ASGPR1-NKG2D extracellular domain, or a polypeptide of full-length ASGPR1-linker peptide-NKG2D extracellular domain can significantly kill tumor cells BxPC3, U251.
Figure 15 shows that NKG2Dz cells comprising a polypeptide of full-length ASGPR1-linker peptide-NKG2D extracellular domain, or comprising a polypeptide of full-length CD137L-NKG2D extracellular domain significantly kill THP-1 orthotopic tumors in NPG mice (left of Figure 15); 7 days after CAR-T infusion, the *in vivo* survival number of NKG2Dz cells comprising a polypeptide of full-length ASGPR1-linker peptide-NKG2D extracellular domain, or comprising a polypeptide of full-length CD137L-NKG2D extracellular domain is significantly increased (right of Figure 15).
Figure 16 shows that compared to inducible NKG2Dz or NKG2D-BBZ cells, inducible NKG2Dz or NKG2D-BBZ cells constitutively expressing a polypeptide of full-length ASGPR1-linker peptide-NKG2D extracellular domain have significantly increased expansion fold upon stimulation with target cells THP1.
Figures 17A-17B show that inducible NKG2Dz or NKG2D-BBZ cells comprising constitutively expressing a polypeptide of full-length ASGPR1-linker peptide-NKG2D extracellular domain significantly kill tumor cells U251, PLC/PRF5 *in vitro* (Figure 17A); after co-incubation with tumor cells THP-1, they secrete high levels of IFN-y and TNF cytokines (Figure 17B).
Figures 18A-18B show that inducible NKG2D-BBZ cells comprising constitutively expressing a polypeptide of full-length ASGPR1-linker peptide-NKG2D extracellular domain significantly inhibit THP1 orthotopic tumors in NPG mice (Figure 18A); the *in vivo* survival number is increased on days 8 and 14 after CAR-T infusion (Figure 18B).
Figures 19A-19B-19C show that BG-BBZ, BGp-BBZ, GB-BBZ, BLG-BBZ, BLGp-BBZ, and GLBp-BBZ cells significantly kill BCMA and GPRC5D double-positive, BCMA single-positive, or GPRC5D single-positive tumor cells (Figure 19A), and mixed tumor cells of BCMA single-positive and GPRC5D single-positive (Figure 19B); after co-incubation with tumor cells, they secrete high levels of IL-2, TNF-α, and IFN-γ (Figure 19C).
Figure 20 shows that the BG-BBZ, GB-BBZ, BGp-BBZ, BLG-BBZ, BLGp-BBZ, and GPRC5D-CART groups significantly inhibit tumors growth.
Figures 21A-21B-21C show that the rapidly prepared R-BGp-BBZ and R-BLGp-BBZ cells significantly kill BCMA-positive, GPRC5D-positive, BCMA and GPRC5D double-positive tumor cells (Figure 21A); significantly kill MM.1S-BCMA KO, MM.1S-GPRC5D KO cells in mixed cells comprising BCMA-positive and GPRC5D-positive cells (Figure 21B); after incubation with tumor cells, they secrete high levels of IL-2, TNF-α, and IFN-γ (Figure 21C).
Figure 22 shows that the *in vivo* CAR-T expansion of rapidly prepared R-BGp-BBZ and R-BLGp-BBZ groups has significant advantage.

### DETAILED DESCRIPTION

This application also provides a chimeric receptor targeting CD38, unexpectedly obtained through the design and screening of various structures. The chimeric receptor can significantly block the CD38 antigen on immune cells expressing the chimeric receptor. This application also discloses an engineered cell comprising the chimeric receptor. During *in vitro* and *in vivo* culture, the expansion fold of the engineered cell is significantly increased. The engineered cell has significant effects in killing NK cells and CD38-positive tumor cells *in vitro* and *in vivo.*

The present application provides a second chimeric receptor that can reduce the fratricide phenomenon of engineered cells expressing a first chimeric receptor during *in vitro* and *in vivo* culture. Engineered cells expressing the first and second chimeric receptors have significantly improved survival and/or expansion capacity during *in vitro* and *in vivo* culture. Engineered cells expressing the first and second chimeric receptors significantly kill target cells recognized by the first chimeric receptor.

Unless specifically defined, all technical and scientific terms used herein have the same meaning as commonly understood by those skilled in the art in the fields of gene therapy, biochemistry, genetics and molecular biology. All methods and materials similar or equivalent to those described herein can be used in the practice or testing of this application, wherein suitable methods and materials are described herein. All publications, patent applications, patents and other references mentioned herein are incorporated herein by reference in their entirety. In case of conflict, this specification, including definitions, shall prevail. In addition, unless otherwise specified, the materials, methods and examples of this application are illustrative only and are not intended to be limiting. Based on the content of this application, it will be understood by those skilled in the art that many changes or variations can be made in the disclosed specific embodiments and still obtain the same or similar results without departing from the spirit and scope of this application. This application is not limited in scope to the specific embodiments described herein (which are only intended to be illustrative of various aspects of this application), and functionally equivalent methods and components are within the scope of this application. This application includes modifications and alterations to the subject matter of this application for various uses and conditions.

Unless otherwise indicated, the practice of this application will employ conventional techniques of cell biology, cell culture, molecular biology, transgenic biology, microbiology, recombinant DNA, and immunology, which are within the skill of the art and are fully explained in the literature.

In this application, the description in range format is only for convenience and brevity and should not be regarded as an unchangeable limitation on the scope of this application. Therefore, the description of a range should be considered to have specifically disclosed all possible subranges and individual numerical values within the range.

### DEFINITIONS

The term "about" refers to the usual error range for each value easily known to those skilled in the art. Reference herein to an "about" value or parameter comprises embodiments referring to the value or parameter itself. For example, a description of "about X" comprises a description of "X." As used herein, "about" can refer to an acceptable error range within the technical field. For example, a value or parameter can refer to a value or parameter within a range of ±10% of an "about" value or parameter, e.g., about 5 µM can comprise any number between 4.5 µM and 5.5 µM.

The term "NKG2A": NKG2A (Gene ID: 3821). NKG2A forms a dimer with CD94 on NK cells and acts as an inhibitory receptor for NK, and its ligand is HLA-E.

The term "CD38" refers to CD38 (Gene ID: 952), which is expressed on the surface of T lymphocytes, B lymphocytes and NK cells.

The term "NKG2D ligand (NKG2DL)" refers to a ligand that binds to NKG2D, comprising MICA, MICB, ULBP-1, ULBP-2, ULBP-3, ULBP-4, ULBP-5, and ULBP-6.

The term "NKG2D": Gene ID: 22914, is a C-type lectin family receptor on the surface of NK cells. The NKG2D described herein comprises the sequence shown in SEQ ID NO: 77, or comprises the nucleic acid sequence shown in SEQ ID NO: 76 or the amino acid sequence encoded thereby.

The term "ASGPR" refers to asialoglycoprotein receptor, Gene ID: 432, comprising two subunits, ASGPR1 (Uniprot: P07306) and ASGPR2 (Uniprot: P07307). The ASGPR1 described herein has the nucleic acid sequence shown in SEQ ID NO: 80 or the amino acid sequence encoded thereby.

The term "CD137L": Gene ID: 8744, a ligand of the CD137 protein, mediates T cell co-stimulatory signal activation. The CD137L described in this application has the nucleic acid sequence shown in SEQ ID NO: 81 or the amino acid sequence encoded thereby.

The term "CD40L": Gene ID: 959, a ligand of the CD40 protein, expressed on the surface of T cells.

The term "GPRC5D" refers to G protein-coupled receptor class C group 5 member D, expressed on malignant bone marrow plasma cells. "GPRC5D" refers to any variant, derivative, or isoform of the GPRC5D gene or the protein it encodes. A GPRC5D polypeptide has an amino acid sequence having at least about 80%, 85%, 90%, 95%, 96%, 97%, 98%, 99%, or 100% homology or identity to the amino acid sequence encoded by the transcript expressed by the gene with NCBI GenBank Gene ID: 55507, or a fragment thereof, and/or may optionally comprise up to one, two, or three conservative amino acid substitutions.

The term "BCMA" generally refers to the BCMA polypeptide, which is specifically and highly expressed on plasma cells and multiple myeloma cells. "BCMA" refers to any variant, derivative, or isoform of the BCMA gene or the protein it encodes. A BCMA polypeptide has an amino acid sequence having at least about 80%, 85%, 90%, 95%, 96%, 97%, 98%, 99%, or 100% homology or identity to the amino acid sequence encoded by the transcript expressed by the gene with NCBI GenBank Gene ID: 608, or fragment thereof, and/or may optionally comprise up to one, two, or three conservative amino acid substitutions.

The term "Host-Versus-Graft Reaction (HVGR)" generally refers to: due to the immunogenetic differences between the donor and the recipient (or host), during transplantation of an exogenous donor graft, as an exogenous graft, the donor graft will be recognized and attacked by the host's immune cells (such as NK cells), thereby be suppressed or eliminated by the host's immune cells.

The term "Graft-Versus-Host Disease (GVHD)" generally refers to: due to the diversity of TCRs on exogenous donor T lymphocytes and their incompatibility with the host's HLA molecules, donor T lymphocytes recognize antigens on the host's normal tissues, proliferate, and release a series of cytokines, attacking host cells.

The term "antibody" is used in the broadest sense herein and comprises various antibody structures, including but not limited to monoclonal antibodies, polyclonal antibodies, multispecific antibodies (e.g., bispecific antibodies), domain antibodies, and antibody fragments thereof that can specifically bind to an antigen or antigenic determinant, as long as they exhibit the desired antigen binding activity. The term "antibody fragment" refers to a molecule other than an intact antibody, which comprises a portion of an intact antibody that binds to the antigen to which the intact antibody binds. Examples of antibody fragment include, but are not limited to, (i) Fab fragment composed of the VL, VH, CL, and CH1 domains, comprising Fab' and Fab'-SH, (ii) Fd fragment composed of the VH and CH1 domains, (iii) Fv fragment composed of the VL and VH domains of a single antibody; (iv) dAb fragment composed of a single variable region; (v) F(ab')2 fragment, which is a bivalent fragment comprising two linked Fab fragments; (vi) antigen binding site of a single-chain Fv molecule; (vii) bispecific single-chain Fv dimer; (viii) "diabody" or "tribody," multivalent or multispecific fragment constructed by gene fusion; and (ix) scFv genetically fused with the same or different antibody. For example, the antibody is selected from: a full antibody, scFv, a single-domain antibody, a Fab fragment, a Fab' fragment, a Fv fragment, a F(ab')2 fragment, a Fd fragment, an sdAb, a multifunctional antibody, a scFv-Fc antibody, or an IgG4 antibody. In one example, the antibodies of the present application comprise typical antibodies, scFv, and combinations thereof, wherein, for example, a DDpp is covalently linked (e.g., via a peptide bond or via a chemical linker) to the N-terminus of the heavy chain and/or light chain of a typical intact (full-length) antibody, or inserted into the heavy chain (H-chain) and/or light chain (L chain) of a full-length antibody. DDpp antibodies that recognize CD38 or NKG2DL are prepared according to the preparation method described in CN111727250A.

The terms "recognize," "bind," and "target" are used interchangeably to refer to selective binding to a target antigen. For example, binding to a target cell refers to binding to a target antigen (e.g., a target molecule) on the target cell.

The term "scFv" refers to a chimeric receptor comprising at least one antibody fragment comprising a light chain variable region and at least one antibody fragment comprising a heavy chain variable region, wherein the light chain variable regions and heavy chain variable region are adjacent (e.g., connected by a synthetic linker, such as a short flexible polypeptide linker) and can be expressed as a single-chain polypeptide, and wherein the scFv retains the specificity of the intact antibody comprising the scFv. Unless otherwise specified, as used herein, scFv can have the VL and VH variable regions in any order (e.g., relative to the N-terminus and C-terminus of the polypeptide), and scFv can comprise VL-linker-VH or VH-linker-VL. The antigen binding function of an antibody can be performed by fragments of naturally occurring antibodies. These fragments are collectively referred to as "antigen binding units." The term "antigen binding unit" also comprises any molecular structure comprising a polypeptide chain with a specific shape that is suitable for recognizing an epitope, wherein one or more non-covalent binding interactions stabilize the complex between the molecular structure and the epitope.

The term "DDpp" refers to a target binding D-domain (DD) polypeptide based on a nonconventional antibody structural scaffold. The D-domain polypeptide (DDpp) is characterized by a high target binding affinity and a non-antibody structural scaffold. DDpp can be monovalent or multivalent. For example, a DDpp is monospecific or multispecific. For example, a DDpp is monospecific and multivalent. For example, a DDpp is multispecific and multivalent. For more information, see CN111727250A.

The term "variable region or variable domain" refers to the domain of an antibody heavy chain or light chain that is involved in antigen binding. The heavy chain variable region (VH) and light chain variable region (VL) of a natural antibody generally have similar structures, each comprises four conserved FRs and three CDRs. A single VH or VL domain can confer antigen binding specificity. In addition, antibodies binding to a specific antigen can be isolated by screening a library of complementary VL or VH domains using VH or VL domains from antibodies that bind to the antigen, respectively. The heavy chain variable regions and light chain variable regions are each composed of four framework regions (FRs) and three complementarity determining regions (CDRs) (also known as "hypervariable regions"). The CDRs in each chain are held together tightly by the framework regions and together with the CDRs from the other chain, promote the formation of the antigen binding site of the antibody.

The term "hypervariable region" or "complementarity determining region" or "CDR" refers to regions within the variable domain of an antibody that are highly variable in sequence, and/or form structurally defined loops ("hypervariable loops"), and/or comprise residues that contact the antigen ("antigen contact site"). Typically, an antibody comprises six CDRs: three in VH (HCDR1, HCDR2, HCDR3) and three in VL (LCDR1, LCDR2, LCDR3).

The term "Fc region" or "Fc" is used to define the C-terminal region of an immunoglobulin heavy chain that comprises at least a portion of the constant region. The term comprises both native sequence Fc regions and variant Fc regions.

"Framework region (FR)" refers to variable domain residues other than the hypervariable region (CDR) residues. The FR of a variable domain typically consists of four FR domains: FR1, FR2, FR3, and FR4. In VH (or VL), the CDR and FR sequences typically appear in the following order: FR1-HCDR1 (LCDR1)-FR2-HCDR2 (LCDR2)-FR3-HCDR3 (LCDR3)-FR4.

Unless otherwise indicated, CDR residues and other residues in the variable domain (eg, FR residues) are numbered herein according to Kabat et al.

The term "natural antibody" refers to an immunoglobulin molecule that exists naturally with a variety of structures. For example, natural IgG antibody is a heterotetrameric glycoprotein of about 150,000 Daltons, composed of two identical light chains and two identical heavy chains linked by a disulfide bond. From N-terminal to C-terminal, each heavy chain has a variable region (VH), which is also referred to as a variable heavy chain region or a heavy chain variable region, followed by three constant domains (CH1, CH2 and CH3). Similarly, from N-terminal to C-terminal, each light chain has a variable region (VL), which is also referred to as a variable light chain region or a light chain variable region, followed by a light chain constant (CL) domain. The light chain of an antibody can be classed based on the amino acid sequence of its constant domain into two types, referred to as kappa (κ) and lambda (λ).

The terms "full antibody," "full-length antibody," and "intact antibody" are used interchangeably to refer to an intact full-length antibody having a structure substantially similar to that of a native antibody or having a heavy chain of an Fc region as defined herein or comprising an antigen binding region.

The term "single-domain antibody (sdAb)", also known as "VHH" or "VHH polypeptide", comprises a variable VHH domain responsible for antigen recognition. The antigen binding of the VHH domain is mediated by three CDRs, flanked by four relatively constant framework regions (FRs). It refers to a type of antibody that lacks the light chain and only has the heavy chain variable region. Due to their small molecular weight, it is also known as a nanobody. For example, a VHH can be truncated at the N-terminus or C-terminus, so that it only comprises a portion of FR1 and/or FR4, or lacks one or two of those framework regions, as long as the VHH substantially maintains antigen binding and specificity.

The term "single domain antibody" refers to an antibody comprising all or part of the heavy chain variable region, or all or part of the light chain variable region. The single domain antibody can be a human single domain antibody.

The terms "monoclonal antibody" and "mAb" refer to an antibody obtained from a population of substantially homologous antibodies, that is, the antibody molecules comprising the population are identical and/or bind to the same epitope, except for possible variant antibodies, for example, comprising naturally occurring mutations or arising during the preparation of the monoclonal antibody preparation, such variants are typically present in small quantities. Compared to polyclonal antibody preparations, which typically include different antibodies targeting different determinants (epitopes), each monoclonal antibody in a monoclonal antibody preparation is targeting a single determinant on the antigen. Thus, the term "monoclonal" indicates that the antibody is obtained from a substantially homologous population of antibodies, and is not to be construed as requiring preparation of the antibody by any particular method. For example, it can be prepared by a variety of techniques, including but not limited to the hybridoma method, recombinant DNA methods, phage display methods, and methods using a transgenic animal comprising all or part of the human immunoglobulin gene loci.

The term "fully human antibody (or human antibody)" is an antibody having an amino acid sequence corresponding to the amino acid sequence of an antibody produced by a human or human cell, or is obtained from the amino acid sequence of an antibody from a non-human source using a human antibody library or other human antibody-encoding sequence. The definition of a fully human antibody explicitly excludes humanized antibodies that comprise non-human antigen binding residues. A fully human antibody can be generated by phage display technology. A fully human antibody can be produced by an engineered strain and/or an engineered cell.

The term "variant" refers to a protein or polypeptide having a specific sequence characteristic ("control protein" or "reference polypeptide"), refers to a different protein or polypeptide which has one or more (e.g., about 1 to about 25, about 1 to about 20, about 1 to about 15, about 1 to about 10, or about 1 to about 5) amino acid substitutions, deletions, and/or additions compared to the control protein or reference polypeptide. The change in the amino acid sequence can be an amino acid substitution. The change in the amino acid sequence can be a conservative amino acid substitution. A functional fragment or functional variant of a protein or polypeptide maintains the basic structure and functional properties of the control protein or polypeptide.

The term "cell marker" also known as a cell surface molecule or a cell surface protein, and preferably refers to a molecule present on the membrane surface of an immune cell. For example, " T cell and/or NK cell markers" refer to markers present on T cells or NK cells, or on both T cells and NK cells, including but not limited to: CD2, CD3, CD4, CD5, CD7, CD8, CD16a, CD16b, CD25, CD27, CD28, CD38, CD45, CD48, CD50, CD52, CD56, CD57, CD62L, CD69, CD94, CD100, CD102, CD122, CD127, CD132, CD160, CD161 CD178, CD218, CD226, CD244, CD159a (NKG2A), CD159c (NKG2C), NKG2E, CD314 (NKG2D), CD305, CD335 (NKP46), CD337, SLAMF7, TIGIT, and FasL. For example, NK cell markers are selected from: NKG2 receptor family, killer immunoglobulin-like receptor (KIR) family, natural cytotoxicity receptor (NCR), and/or other NK cell-specifically expressed antigens. The NKG2 receptor family comprises NKG2A, NKG2D, and NKG2C. The KIR family comprises KIR2DL1, KIR2DL2/3, KIR2DL4, KIR2DL5, KIR3DL1, KIR3DL2, KIR2DS1, KIR2DS2/S3, KIR2DS4, KIR2DS5, and KIR3DS1. NCR comprises NKP30, NKP44, NKP46, and NKp80. Other NK cell-specifically expressed antigens comprises CD159a, CD159c, CD94, CD158, CD56, LIR/ILT2, CD244, CD226, CD2, CD16, CD161, TIGIT, CS1, IL-15R, and FasL.

The term "NK inhibitory receptor (NKIR)" refers to a class of receptors on NK cells that can transduce killing inhibitory signals and inhibit the killing function of NK cells, comprising HLA-specific and non-HLA-specific inhibitory receptors. NKIR includes, but is not limited to, having an immunoreceptor tyrosine-based inhibitory motif (ITIM). For example, NKIR comprises: NKG2/CD94 components, KIR family members, LIR family members, NKR-P1 family members, immune checkpoint receptors, immune checkpoint inhibitors, SIGLEC family members, Ly49 family members, or combinations thereof. For example, the NKG2/CD94 component is selected from NKG2A, NKG2C and CD94. For example, the KIR family member is selected from KIR2DL1, KIR2DL2/3, KIR2DL5A, KIR2DL5B, KIR3DL1, KIR3DL2 and KIR3DL3. For example, LIR family members are selected from LIR1, LIR2, LIR3, LIR5 and LIR8. For example, NKR-P1 family members are selected from NKR-P1B and NKR-P1D. For example, immune checkpoint inhibitors comprise: (a) one or more antagonists of checkpoint molecules, which comprise PD-1, PDL-1, TIM-3, TIGIT, LAG-3, CTLA-4, 2B4, 4-1BB, 4-1BBL, A2aR, BATE, BTLA, CD39, CD47, CD73, CD94, CD96, CD160, CD200, CD200R, CD274, CEACAM1, CSF-1R, Foxpl, GARP, HVEM, IDO, EDO, TDO, LAIR-1, M ICA/B, NR4A2, MAFB, OCT-2, Rara (retinoic acid receptor α), TLR3, VISTA, NKG2A/HLA-E or inhibitory KIR; (b) one or more of atezolizumab, avelumab, durvalumab, ipilimumab, IPH4102, IPH43, IPH33, lirimumab, monalizumab, nivolumab, pembrolizumab and derivatives or functional equivalents thereof; or (c) at least one of atezolizumab, nivolumab and pembrolizumab, or one or more of venetoclax, azacitidine, and pomalidomide. For example, the immune checkpoint receptor is selected from PD-1, TIGIT, CD96, TIM3 and LAG3. For example, the SIGLEC family member is selected from SIGLEC7 and SIGLEC9. For example, the Ly49 family member is selected from Ly49A, Ly49C, Ly49F, Ly49G1 and Ly49G4.

The term "pathological cell" generally refers to any type of cell present in a patient that is considered to cause deterioration of health, or refers to a malignant or infected cell that needs to be reduced or eliminated to achieve patient remission. This application relates to methods for novel adoptive immunotherapy strategies for treating diseases associated with the development of pathological cells, such as cancer (tumors), infections, and autoimmune diseases.

The term "tumor antigen" refers to an antigen that newly appears or is overexpressed during the onset and progression of a hyperproliferative disease. In certain aspects, the hyperproliferative disorder herein refers to cancer. The hyperproliferative disorder refers to cancer or tumor. Tumor antigens comprise solid tumor antigens and hematologic tumor antigens. The tumors described in this application can be a solid tumor or a hematologic tumor (liquid tumor). Non-limiting examples of tumors include hematologic tumors (e.g., leukemia, lymphoma, and myeloma), ovarian cancer, breast cancer, bladder cancer, brain cancer, colon cancer, intestinal cancer, liver cancer, lung cancer, pancreatic cancer, prostate cancer, skin cancer, gastric cancer, glioblastoma, laryngeal cancer, melanoma, neuroblastoma, adenocarcinoma, glioma, soft tissue sarcoma and various carcinomas (including prostate cancer and small cell lung cancer), astrocytoma, fibrosarcoma, myxosarcoma, liposarcoma, oligodendroglioma, ependymoma, medulloblastoma, primitive neuroectodermal tumor (PNET), chondrosarcoma, osteosarcoma, pancreatic ductal adenocarcinoma, small cell and large cell lung adenocarcinoma, chordoma, angiosarcoma, endothelial sarcoma, squamous cell carcinoma, bronchoalveolar carcinoma, epithelial adenocarcinoma and its liver metastases, lymphangiosarcoma, cholangiocarcinoma, gallbladder carcinoma, synovioma, mesothelioma, Ewing's tumor, rhabdomyosarcoma, basal cell carcinoma, bronchogenic carcinoma, renal cell carcinoma, choriocarcinoma, seminoma, embryonal carcinoma, Wilms' tumor, testicular tumors, medulloblastoma, craniopharyngioma, ependymoma, pinealoma, hemangioblastoma, acoustic neuroma, oligodendroglioma, meningioma, neuroblastoma, retinoblastoma, multiple myeloma, Waldenstrom's macroglobulinemia and heavy chain disease, breast tumors such as ductal and lobular adenocarcinoma, cervical cancer, ovarian cancer, bladder cancer, plasmacytoma, acute and chronic leukemia.

The term "chimeric receptor" refers to a fusion molecule formed by connecting DNA fragments or corresponding cDNAs of proteins from different sources using genetic recombination technology, which comprising an extracellular domain, a transmembrane domain, and an intracellular domain. Chimeric receptors include, but are not limited to, a chimeric antigen receptor (CAR), recombinant TCR receptor.

The term "chimeric antigen receptor" (CAR) comprises at least one extracellular antigen binding domain, a transmembrane domain, and an intracellular signaling domain. The intracellular signaling domain comprises a functional signaling domain of a stimulatory molecule and/or a co-stimulatory molecule. For example, the stimulatory molecule is a ζ chain (such as CD3Z) that binds to the T cell receptor complex. For example, the intracellular signaling domain further comprises a functional signaling domain of one or more co-stimulatory molecules, such as 4-1BB (i.e., CD137), CD27, and/or CD28. For example, the polypeptide groups are connected to each other. For example, the intracellular signaling domain (or region) can be selected from the intracellular co-stimulatory domain of any one or more of the following polypeptides: CD27, CD28, TNFRSF9, TNFRSF4, TNFRSF8, TNFRSF14, TNFRSF18, CD40LG, ICOS, ITGB2, CD2, CD7, KLRC2, HAVCR1, LGALS9, CD83.

The term "recombinant T cell receptor (recombinant TCR)" comprises a chimeric receptor derived from one or more TCR subunits. For example, a recombinant TCR comprises at least a portion of the extracellular domain of TCR subunits, a transmembrane domain, and a TCR intracellular domain, and the TCR subunits are effectively linked to an antigen binding domain. For example, the TCR subunits in a recombinant TCR comprises CD3ζ, CD3ε, CD3y, CD3δ, TCRα, TCRβ, TCRy, and/or TCRδ subunits. For example, a recombinant TCR can be integrated into a TCR/CD3 complex expressed on a T cell. For example, a recombinant TCR comprises the constant regions and intracellular domains of TCRα and TCRβ subunits, and the constant regions of these subunits are effectively linked to the antigen binding domain. For example, the recombinant TCR comprises the constant regions and intracellular domains of TCRγ and TCRδ subunits, and the constant regions of these subunits are effectively linked to the antigen binding domain. For example, the recombinant TCR comprises a CD3ζ, CD3ε, CD3γ, or CD3δ subunit, and the extracellular domain of the subunit is effectively linked to an antigen binding domain.

The term "primary signaling domain" or "primary signaling region" regulates the initial activation of the TCR complex in a stimulating manner. In one aspect, the primary signaling is triggered by, for example, the binding of the TCR/CD3 complex to a peptide-loaded MHC molecule, thereby mediating T cell responses (including but not limited to, proliferation, activation, differentiation, etc.). The primary signaling domain that acts in a stimulating manner can comprise an immunoreceptor tyrosine-based activation motif, or a signaling motif of an ITAM. For example, a fragment of the primary signaling domain comprising an ITAM includes but is not limited to, an intracellular signaling domain derived from CD3γ, CD3δ, CD3ε, CD3ζ, CD5, CD79a, CD79b, CD278 and CD66d.

The term "signaling domain" or "signaling transduction domain" refers to the functional portion of a protein that acts by transmitting information within a cell to regulate the activity of the cell via a defined signaling pathway by generating a second messenger or by acting as an effector in response to such a messenger. The intracellular signaling domain can comprise the entire intracellular portion of the molecule, or the entire native intracellular signaling domain, or a functional fragment or derivative thereof.

The term "costimulatory signaling domain" or "costimulatory molecule" generally refers to the intracellular domain of a costimulatory molecule that can combine with a cell stimulatory signaling molecule, such as TCR/CD3, to result in T cell proliferation and/or upregulation or downregulation of key molecules. Costimulatory molecules are typically cognate binding partners on T cells that specifically bind to costimulatory ligands and mediate co-stimulatory responses of T cells, including but not limited to proliferation. Costimulatory molecules are cell surface molecules other than antigen receptors or their ligands required for an effective immune response. Costimulatory molecules include, but are not limited to, MHC class I molecules, BTLA and Toll ligand receptors, as well as OX40, CD2, CD27, CD28, CDS, ICAM-1, LFA-1 (CD11a/CD18) and 4-1BB (CD137).

The term "immunosuppressive signaling domain" or "immunosuppressive receptor molecule" generally refers to a molecule that negatively regulates and suppresses immune cell activation signals, such as a classical receptor comprising ITIM domains. This also comprises non-classical ITIM domains, such as the CD300A receptor. It also comprises other non-ITIM dependent inhibitory receptors or enzyme molecules, such as CTLA-4, IDO1, and IDO2.

The term "CD3ζ (also known as CD3 Zeta)" comprises the protein provided by GenBank Accession No. BAG36664.1, or equivalent residues from non-human species such as mice, rodents, monkeys, apes, etc. "CD3ζ" is used interchangeably with "CD3z" and "CD3Z" in this application.

The term "T cell receptor (TCR)" mediates T cell recognition of specific major histocompatibility complex (MHC)-restricted peptide antigens, comprising classical TCR receptors and optimized TCR receptors. The classical TCR receptor is composed of two peptide chains, α and β, each of which can be divided into a variable region (V region), a constant region (C region), a transmembrane domain, and a cytoplasmic region. Its antigen specificity exists in the V region (Vα, Vβ), each of which has three hypervariable regions, CDR1, CDR2, and CDR3. In one aspect, T cells expressing a classical TCR can be induced to specifically respond to a target antigen by methods such as antigen stimulation of the T cells.

The term "signal peptide (SP)" refers to a short peptide chain (about 5-30 amino acids in length) that directs newly synthesized proteins or polypeptides to the secretory pathway. SP is a short peptide located at the N-terminus of a protein.

The term "cell" refers to a cell of a human or non-human, or an animal.

The term "host" or "subject" refers to a recipient receiving a graft transplant, for example, an individual receiving exogenous cells implantation, such as a human. For example, a "subject" can be a clinical patient, a clinical trial volunteer, an experimental animal, etc. The subject may be suspected of having a disease characterized by cell proliferation or of having a disease characterized by cell proliferation, may be diagnosed with a disease characterized by cell proliferation. For example, the subject may have or be suspected of having an immune disease such as an autoimmune disease, or a tumor, or an inflammatory disease.

The term "engineered" or "engineering" refers to the application of the principles and methods of cell biology and molecular biology, via certain engineering means, to change the genetic material within a cell or obtain a cell product at the whole cell or organelle level according to human wishes. For example, "engineered" refers to one or more changes in nucleic acids (such as nucleic acids within the genome of an organism). "Engineered" can refer to gene alteration, addition and/or deletion. "Engineered cells" can also refer to cells with added, deleted and/or altered genes.

In one example, the engineered cell is an immune cell, a neuron, an epithelial cell, an endothelial cell or a stem cell. Stem cells comprise human pluripotent stem cells (comprising human induced pluripotent stem cells (iPSC) and human embryonic stem cells). For example, the engineered cell is an immune cell. For example, the engineered cell is a primary cell. For example, the engineered cell is a B cell, a monocyte, a natural killer cell, a basophil, an eosinophil, a neutrophil, a dendritic cell, a macrophage, a T cell, a NKT cell or a combination thereof. The engineered cell can be an autologous cell or an allogeneic cell. For example, the engineered cell is obtained by genetic engineering operations on T cells isolated from human PBMC cells.

The term "immune cell" refers to cells involved in the immune response and produce immune effects, such as T cells, B cells, natural killer (NK) cells, natural killer T (NKT) cells, dendritic cells, CIK cells, macrophages, mast cells, etc. For example, the immune cell is a T cell, an NK cell, or an NKT cell. For example, the T cells can be an autologous T cell, a xenogeneic T cell, or an allogeneic T cell. For example, the NK cell can be an autologous NK cell or an allogeneic NK cell. For example, the immune cell can be obtained by sorting peripheral blood mononuclear cells (PBMC) from a donor.

The term "T cell" may refers to a natural T cell obtained from PBMC, bone marrow, lymph node tissue, cord blood, thymus tissue, or from infection sites, ascites, pleural effusion, spleen tissue, tumor tissue, or refer to a cell population with specific phenotypic characteristics obtained by sorting, or a mixed cell population with different phenotypic characteristics, such as "T cell" may comprise a cell population comprising at least one T cell subset: stem cell-like memory T cells (Tscm cells), central memory T cells (Tcm), effector T cells (Tef, Teff), regulatory T cells (Tregs), and/or effector memory T cells (Tem). For example, "T cell" can be a specific subtype of T cell, such as αβT cells, γδT cells. In some cases, T cells can be obtained from blood collected from an individual using any techniques known to those skilled in the art, such as Ficoll^{™} separation and/or apheresis. For example, T cells are obtained by inducing differentiation of pluripotent stem cells. For example, cells of an individual's circulating blood are obtained by apheresis. Apheresis products typically contain lymphocytes, comprising T cells, monocytes, granulocytes, B cells, other nucleated white blood cells, red blood cells and platelets. For example, the cells collected by apheresis can be washed to remove plasma molecules and the cells can be placed in a suitable buffer or culture medium for subsequent processing steps. The T cells can be derived from healthy donors, or from cells derived from individuals diagnosed with cancer. The T cell can be an autologous T cell or an allogeneic T cell. The T cell can be a primary T cell. For example, "T cell" can also be a T cell carrying a chimeric receptor that binds CD38. For example, a CAR T cell, or a recombinant TCR-T cell.

The term "cell composition" generally refers to a combination of at least two types of cells, wherein the first type of cell can at least bind to CD38; and the second type of cell binds to an NK cell marker and/or a target antigen on pathological cells (e.g., tumor cells, pathological cells of autoimmune diseases). For example, each type of cell can be present in different containers and can be formulated into a desired preparation with a suitable adjuvant simultaneously or separately as needed; each type of cell can be from a different source (e.g., prepared, produced, or sold by different manufacturers; for example, naturally occurring T cells isolated from a donor and T cells derived from stem cells, respectively); each type of cell can be prepared as an independent preparation (solid, liquid, gel, etc.); each type of cell can be present in a mixed form. The cell composition can also comprise an effective amount of antibody, immunoconjugate, chimeric receptor, nucleic acid, or host cell, and can also comprise a pharmaceutically acceptable carrier.

The term "MHC" refers to the major histocompatibility complex. In human cells, MHC is referred to as HLA antigen and plays an important role in transplantation reactions, thereby mediating the rejection by T cells responding to the histocompatibility antigens on the surface of the transplanted tissue.

The term "human leukocyte antigen" (HLA) is the gene encoding the human major histocompatibility complex and is closely related to the function of the human immune system. HLA comprises class I, class II and class III gene segments. HLA class I is a heterodimer composed of a heavy chain (α chain) and a light chain β2 microglobulin (B2M). The term "B2M" refers to β-2 microglobulin, also known as B2M, which is the light chain of the MHC class I molecule. HLA-II class genes comprise the HLA-D family, mainly HLA-DP, HLA-DQ and HLA-DR, which are mainly distributed on the surface of professional antigen-presenting cells such as B lymphocytes, macrophages and dendritic cells.

The term "exogenous" refers to a nucleic acid molecule, polypeptide, cell, tissue, etc. that is not endogenously expressed in the organism itself, or whose expression level is insufficient to achieve the function of overexpressed.

The term "endogenous" refers to a nucleic acid molecule or polypeptide, etc., originating from the organism itself.

The terms "activation" , "activated" and " activating" are used interchangeably and refer to the process by which a cell transitions from a resting state to an active state. This process comprises a response to antigen, and a response to phenotypic or genetic changes in migration, and/or functional activity status. For example, the term "activation" refers to the stepwise activation process of T cells. This activation process is jointly regulated by a primary stimulation signal and a co-stimulation signal. The activation of T cells is a dynamic process, with its duration and degree of activation are influenced by external stimuli. "T cell activation" or "T cell activation" refers to the state of T cells that are stimulated to induce detectable cell proliferation, cytokine production and/or detectable effector function. The use of CD3/CD28 magnetic beads, *in vitro* antigen stimulation or *in vivo* antigen stimulation will affect the degree and duration of T cell activation. For example, the engineered T cells are activated after co-incubated with tumor cells comprising specific target antigens or after viral infection.

The term "genome editing" or ""gene editing" refers to a genetic engineering technology that uses site-specific nucleases to insert, knock out, modify or replace DNA at a specific location in the genome of an organism to change the DNA sequence. Gene editing can be used to achieve precise and efficient gene knockout or gene knock-in. Gene knockout technology using nucleases comprises CRISPR/Cas technology, ZFN technology, TALEN technology and TALEN-CRISPR/Cas technology, Base Editor technology, Prime Editor technology, and Meganuclease technology. The guide sequence (gRNA) is a polynucleotide sequence that has sufficient complementarity with the target polynucleotide sequence to hybridize with the target sequence, and the gRNA can guide the CRISPR complex to specifically bind to the target sequence. In this application, whenever a gRNA sequence is involved, it can be a targeted DNA sequence, or it can be a complete Cas9 guide sequence formed by the ribonucleotide corresponding to the DNA along with crRNA and TracrRNA. gRNA is used to guide, bind to or recognize the Cas enzyme. For example, when using a suitable alignment algorithm for optimal alignment, the degree of complementarity between the guide sequence and its corresponding target sequence is about or more than about 80%, 85%, 90%, 95%, 97.5%, 99%, or more. For example, engineered cells with endogenous TCR/B2M, TCR/B2M/FAS, TCR/B2M/CD38, TCR/B2M/CD38/NKG2A, TCR/B2M/NKG2A, TCR/B2M/FAS/CD38, or TCR/B2M/FAS/NKG2A knockout can be constructed using CRISPR technology. The gRNA sequences targeting TCR, B2M, NKG2A, and CD38 are shown in SEQ ID NOs: 39, 40, 41, and 42, respectively.

The "low expression" refers to a state in which the level of protein and/or RNA expressed by a target gene in the engineered cell is lower than its expression level before engineering. For example, low expression of B2M, TCR, CD38, FAS, NKG2A refers to a reduction in the expression of B2M, TCR, CD38, FAS, NKG2A in the cell by at least 1%, at least 5%, at least 10%, at least 20%, at least 30%, at least 40%, at least 50%, at least 60%, at least 70%, at least 80%, at least 90%, at least 95%, at least 99% or 100%. The expression or content of a specific protein in the cell can be determined by any suitable method known in the art, such as ELISA, immunohistochemistry, immunoblotting or flow cytometry using specific antibodies. CRISPR library screening technology has been widely used for screening various biological problems, including screening for tumor medicament resistance regulatory genes, screening for function enhancement genes in cell therapy product, etc.

The term "transfection" refers to the introduction of exogenous nucleic acid into eukaryotic cells. Transfection can be achieved by various means known in the art, comprising calcium phosphate-DNA coprecipitation, DEAE-dextran-mediated transfection, polybrene-mediated transfection, electroporation, microinjection, liposome fusion, lipid transfection, protoplast fusion, retroviral infection, and biolistics.

The terms "nucleic acid molecule encoding," "encoding DNA sequence," and "encoding DNA" refer to the sequence or order of deoxyribonucleotides along a deoxyribonucleic acid strand. For example, a nucleic acid sequence encodes an amino acid sequence. When referring to a nucleotide sequence, "sequence" can comprise DNA or RNA, and can be single-stranded or double-stranded.

The term "subject" refers to any animal, such as a mammal or marsupial. The subject of the present application includes, but is not limited to, humans, non-human primates (e.g., rhesus monkeys or other types of macaques), mice, pigs, horses, donkeys, cattle, sheep, rats, and any kind of poultry.

The term "peripheral blood mononuclear cell" (PBMC) refers to cells in peripheral blood with a single nucleus, comprising lymphocytes, monocytes, etc. PBMC can be obtained by density-based cell separation methods, for example, lysing red blood cells or without lysing red blood cells and subjecting peripheral blood, apheresis samples or leukapheresis samples by Percoll/Ficoll gradient centrifugation.

The term "effective amount" or "therapeutically effective amount" refers to a dose sufficient to prevent or treat a disease (tumor or cancer) in an individual. The effective dose for therapeutic or prophylactic use depends on the stage and severity of the disease being treated, the age, weight and general health status of the subject, and the judgment of the prescribing physician. The magnitude of the dose also depends on the selected active substance, the method of administration, the time and frequency of administration, the presence, nature and extent of any adverse side effects that may accompany the administration of a particular active substance, and the desired physiological effect. Based on the judgment from a prescribing physician or those skilled in the art, one or more rounds or multiple administrations of the engineered cells of the present application may be required.

The term "expression vector" refers to a vector comprising a recombinant polynucleotide, which comprises expression regulatory sequences effectively linked to the nucleotide sequence to be expressed. An expression vector contains sufficient cis-acting elements for expression; other elements for expression can be provided by the host cell or an *in vitro* expression system. Expression vectors comprise plasmids and viruses (e.g., lentivirus, retrovirus, adenovirus, and adeno-associated virus).

The term "vector" refers to a composition that comprises an isolated nucleic acid and usable for delivering the isolated nucleic acid into the interior of cells. This includes, but is not limited to, linear polynucleotides, polynucleotides associated with ions or amphiphilic compounds, plasmids, and viruses. For example, it comprises autonomously replicating plasmid or virus. It Also comprises non-plasmid and non-viral compounds that facilitate the transfer of nucleic acid into cells, such as polylysine compounds, liposomes, etc.

The term "regulate" or "modulate" refers to a positive or negative change. Examples of regulation include a 1%, 2%, 10%, 25%, 50%, 75%, or 100% change. In one embodiment, it refers to a negative change.

The term "homology" or "identity" refers to the subunit sequence identity between two polymer molecules, for example, between two nucleic acid molecules such as two DNA molecules or two RNA molecules, or between two polypeptide molecules. The term "substantial identity" or "substantial homology" refers to a polypeptide or nucleic acid molecule that exhibits at least about 50% homology or identity to a reference amino acid sequence or nucleic acid sequence. In one embodiment, such a sequence is at least about 60%, 65%, 70%, 75%, 80%, 85%, 90%, 95%, 99% or 100% homology or identity to the amino acid or nucleic acid sequence used for comparison. Polypeptides that have been modified and/or substituted with one or several amino acids, and/or deleted and/or added with one or several amino acids based on the amino acid sequences provided herein, and have an amino acid sequence that is 60%, 65%, 70%, 75%, 80%, 85%, 90%, or 95% or more identity to the amino acid sequences provided herein and have the same function are also within the scope of protection of this application.

The term "treatment" refers to interventions that intended to alter the course of a disease, which can be preventative or or may intervene during the clinical pathological process. Treatment effects include, but are not limited to, preventing the occurrence or recurrence of a disease, alleviating symptoms, reducing any direct or indirect pathological consequences of the disease, preventing metastasis, slowing the rate of disease progression, improving or alleviating the condition, and alleviating or improving prognosis, etc.

The term "prevention" refers to interventions attempted to be performed before the occurrence of a disease (such as rejection caused by cell transplantation).

The term "transplant rejection" refers to the immune response in which the host's immune system recognizes the exogenous graft as an "foreign component" after receives a transplant of allogeneic tissue, organ, or cells, and initiates an immune attack, destruction, and clearance against the graft. This application provides cells and methods for resisting transplant immune rejection.

The term "graft" refers to a biological material or preparation separated from an individual other than the host, intended for implantation into a host. The graft can be isolated and obtained from any animal, such as a mammalian source, preferably from humans. The graft can be isolated from a host itself, such as cells isolated from a host, cultured *in vitro* or modified and then re-implanted into the host. The graft can be isolated and obtained from other allogeneic individuals, such as cells isolated from other humans cultured *in vitro* or modified and then implanted into the host. The graft can be isolated and obtained from a xenogeneic individual, such as an organ from another species (such as mice, pigs, monkeys) that are implanted into a human. Xenotransplantation includes, but is not limited to, vascularized xenotransplantation, partially vascularized xenotransplantation, non-vascularized xenotransplantation, xeno dressings, xeno bandages, and xeno structures.

The term "autologous" refers to originating from the same organism. For example, a sample (e.g., a cell) can be taken from a subject, processed, and returned to the same subject (e.g., a patient) at a later time. The autologous process is distinct from allogeneic process, in which the donor and recipient are different subjects.

The term "autologous transplantation" comprises any procedure involving the transplantation, implantation, or infusion of cells, tissues, or organs into a recipient, wherein the subject and the donor are the same individual. Transplantation of cells, organs, and/or tissues as described herein can be used for autologous transplantation into humans. Autologous transplantation includes, but is not limited to, vascularized autologous transplantation, partially vascularized autologous transplantation, non-vascularized autologous transplantation, autologous dressings, autologous bandages, and autologous structures.

The term "allogeneic transplantation" comprises any procedure involving the transplantation, implantation, or infusion of cells, tissues, or organs into a subject, wherein the subject and the donor are different individuals of the same species. Transplantation of cells, organs, and/or tissues described herein can be used for allogeneic transplantation into humans. Allogeneic transplantation includes, but is not limited to, vascularized allogeneic transplantation, partially vascularized allogeneic transplantation, non-vascularized allogeneic transplantation, allodressing, allobandages, and allogenic structures.

The term "block" or "blocking" refers to preventing the recognition of a target molecule on the cell surface, thereby preventing the biological reaction triggered by the recognition of the target molecule.

The statistical analysis used in this application, the t-test (also known as the Student t-test), is a tool for evaluating the mean of one or two populations using hypothesis testing. The p-value refers to the probability that a more extreme result than the observed result of the sample will occur when the null hypothesis is true. A very small p-value indicates that the probability of the observed result under the null hypothesis is low . In this application, *: P-value ≤ 0.05; **: P-value ≤ 0.01; ***: P-value ≤ 0.001.

### Chimeric receptor

The present application also provides a chimeric receptor of the present invention, from the N-terminus to the C-terminus, comprising an extracellular domain, a transmembrane domain (also referred to transmembrane structural domain, or transmembrane region), and an intracellular signal transduction domain (also referred to intracellular signaling domain, or intracellular domain). When the intracellular signal transduction domain comprises one or more signal transduction domains or motifs, such as immunoreceptor tyrosine-based activation motifs (ITAMs), kinase domains, costimulatory domains, etc., it can directly promote cellular responses. The intracellular signal transduction domain will indirectly promote cellular responses by associating with one or more other proteins, which in turn directly promote cellular responses. The intracellular signaling domain or a functional fragment thereof can be from CD3ε, CD3δ, CD3ζ, CD25, CD27, CD28, CD40, CD47, CD79A, CD79B, CD134 (OX40), CD137 (4-1BB), CD150 (SLAMF1), CD278 (ICOS), CD357 (GITR), CARD11, DAP10, DAP12, FcRα, FcRβ, FcRγ, Fyn, Lck, LAT, LRP, NKG2D, NOTCH1, NOTCH2, NOTCH3, NOTCH4, ROR2, Ryk, Slp76, pTα, TCRα, TCRβ, TRIM, Zap70, PTCH2 or any combination thereof. The chimeric receptor provided by the present invention comprises an intracellular signaling domain. In one example, the intracellular signal transduction domain comprises an immunoreceptor tyrosine-based activation motif or a signaling motif of an ITAM.

In one example, the intracellular signal transduction domain comprises an intracellular signal transduction domain selected from: TCRα, TCRβ, TCRγ, TCRδ, CD3γ, CD3δ, CD3ε, CD3ζ, CD5, CD22, CD79a, CD79b, CD278, or CD66d, or a combination thereof. In one example, the intracellular signal transduction domain comprises a CD3ζ intracellular signaling domain (SEQ ID NO: 12 or 13).

In one example, the intracellular signal transduction domain comprised in the chimeric receptor provided by the present invention further comprises a costimulatory domain. For example, the costimulatory domain is selected from the intracellular signal transduction domain of CD137, CD28, CD27, TNFRSF9, TNFRSF4, TNFRSF8, TNFRSF14, TNFRSF18, CD40LG, ICOS, ITGB2, CD2, CD7, KLRC2, HAVCR1, LGALS9, or CD83, or a combination thereof. For example, the costimulatory structure is the intracellular signal transduction domain of CD137 (SEQ ID NO: 11). For example, the costimulatory structure is the intracellular signal transduction domain of CD28 (SEQ ID NO: 10).

In one embodiment, the chimeric receptor further comprises a spacer (also referred to as a hinge or hinge region) located between the antigen binding domain and the transmembrane domain. For example, the spacer comprises a portion of an immunoglobulin. For example, the spacer comprises sequences of a hinge region, a CH2 region, and a CH3 region. For example, the hinge region is selected from: a CD28 hinge region, a CD8 hinge region, an IgG4 hinge region or a fragment thereof, or a combination thereof. For example, the hinge region is a CD8 hinge region (SEQ ID NO: 3) or an IgG4 spacer region (SEQ ID NO: 5 or 6).

The transmembrane domain can anchor the CAR to the cell membrane. The transmembrane domain of the chimeric receptor of the present invention comprises a transmembrane domain selected from the following: a transmembrane domain of the α, β, or ζ of the T cell receptor, CD28, CD3ε, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS(CD278), 4-1BB(CD137), GITR, CD40, BAFFR, HVEM(LIGHTR), SLAMF7, NKp80(KLRF1), CD160, CD19, IL2Rβ, IL2Ry, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, the transmembrane domain of NKG2D and/or NKG2C.

In one embodiment, the transmembrane domain is selected from: CD2, CD3ε, CD3δ, CD3ζ, CD8, CD25, CD27, CD28, CD40, CD79A, CD79B, CD80, CD86, CD95 (Fas), CD134 (OX40), CD137, CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5 or Zap70. For example, the transmembrane domain is the transmembrane domain of CD28 (SEQ ID NO: 8 or 9). For example, the transmembrane domain is a variant of the transmembrane domain of CD28 (SEQ ID NO: 8 or 9) with no more than three amino acid mutations. For example, the transmembrane domain is the CD8 transmembrane domain (SEQ ID NO: 7). For example, the transmembrane domain is a variant of the CD8 transmembrane domain (SEQ ID NO: 7) with no more than three amino acid mutations.

In one embodiment, the chimeric receptor comprises a signal peptide, for example, the signal peptide is a CD8 signal peptide (SEQ ID NO: 1) or a GMCSFRα signal peptide (SEQ ID NO: 2).

In one embodiment, the chimeric receptor of the present invention targets a target antigen on a pathological cell. For example, the pathological cell comprises a malignant cell or an infected cell. For example, the pathological cell is a tumor cell or a pathological cell of an autoimmune disease. For example, the pathological cell comprises any of the diseases disclosed in this application. For example, the tumor comprises solid tumors and hematological tumors. For example, the solid tumor is selected from: esophageal cancer, gastric cancer, liver cancer, biliary tract tumors, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, and sarcoma; the hematological tumor is selected from: leukemia, lymphoma, and myeloma; and the autoimmune disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), and polymyositis/dermatomyositis.

In one embodiment, the target antigen is selected from: thyroid stimulating hormone receptor (TSHR); CD171; CS-1; C-type lectin-like molecule-1; ganglioside GD3; Tn antigen; CD19; CD20; CD22; CD30; CD70; CD123; CD138; CD33; CD44; CD44v7/8; CD38; CD44v6; B7H3 (CD276), B7H6; KIT (CD117); interleukin-13 receptor subunit alpha (IL-13Rα); interleukin-11 receptor alpha (IL-11Rα); prostate stem cell antigen (PSCA); prostate-specific membrane antigen (PSMA); carcinoembryonic antigen (CEA); NY-ESO-1; HIV-1 Gag; MART-1; gp100; tyrosinase; mesothelin; EpCAM; proteinase serine 21 (PRSS21); vascular endothelial growth factor receptor, vascular endothelial growth factor receptor 2 (VEGFR2); Lewis (Y) antigen; CD24; platelet-derived growth factor receptor beta (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin type A receptor 2 (EphA2); fucosyl GM1; sialyl Lewis adhesion molecule (sLe); ganglioside GM3; TGS5; high molecular weight melanoma-associated antigen (HMWMAA); o-acetyl GD2 ganglioside (OAcGD2); folate receptor; tumor endothelial marker 1 (TEM1/CD248); tumor endothelial marker 7-related (TEM7R); claudin 6, Claudin18.2, Claudin18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AChR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variants in the necrotic area of tumors; G protein-coupled receptor class C group 5, member D (GPRC5D); X chromosome open reading frame 61 (CXORF61); CD97; CD179a; anaplastic lymphoma kinase (ALK); polysialic acid; placenta-specific 1 (PLAC1); the hexose portion of globoH glycoceramide (GloboH); breast cancer differentiation antigen (NY-BR-1); uroplakin 2 (UPK2); hepatitis A virus cellular receptor 1 (HAVCR1); adrenergic receptor beta 3 (ADRB3); pannexin 3 (PANX3); G protein-coupled receptor 20 (GPR20); lymphocyte antigen 6 complex locus K9 (LY6K); olfactory receptor 51E2 (OR51E2); TCR gamma alternate reading frame protein (TARP); Wilms tumor protein (WT1); ETS translocation variant 6 (ETV6-AML); sperm protein 17 (SPA17); X antigen family member 1A (XAGE1); angiopoietin-binding cell surface receptor 2 (Tie2); melanoma cancer testis antigen-1 (MAD-CT-1); melanoma cancer testis Antigen-2 (MAD-CT-2); Fos-related antigen 1; p53 mutant; human telomerase reverse transcriptase (hTERT); sarcoma translocation breakpoints; melanoma inhibitor of apoptosis (ML-IAP); ERG (transmembrane protease serine 2 (TMPRSS2) ETS fusion gene); N-acetylglucosaminyltransferase V (NA17); paired box protein Pax-3 (PAX3); androgen receptor; cyclin B1; V-myc avian myelocytomatosis viral oncogene neuroblastoma-derived homolog (MYCN); Ras homolog family member C (RhoC); cytochrome P4501B1 (CYP1B1); CCCTC-binding factor (zinc finger protein)-like (BORIS); squamous cell carcinoma antigen recognized by T cells 3 (SART3); paired box protein Pax-5 (PAX5); proacrosin-binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte associated immunoglobulin-like receptor 1 (LAIR1); Fc fragment of the IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module containing mucin-like hormone receptor-like 2 (EMR2); lymphocyte antigen 75 (LY75); glypican-3 (GPC3); Fc receptor-like 5 (FCRL5);Immunoglobulin lambda-like polypeptide 1 (IGLL1). In one embodiment, the target antigen is selected from: CD19, CD20, CD22, CD38, BCMA, GPRC5D, B7H3, GPC3, Claudin 6, Claudin18.2, FAP, Mesothelin, NKG2D ligand, NKG2A, CD94, FCRH5, EGFR and its mutants, or a combination thereof.

For example, the target antigen is a pathogen. For example, the target antigen is selected from: an antigen of viruses, bacteria, fungi, protozoa, or parasites. For example, the target antigen is a viral antigen. The viral antigen is selected from: cytomegalovirus antigen, Epstein-Barr virus antigen, human immunodeficiency virus antigen, or influenza virus antigen.

### Engineered cells

In one example, the engineered cell is an immune cell, a neuron, an epithelial cell, an endothelial cell, a stem cell, or a combination thereof. For example, the engineered cell is an immune cell. For example, the immune cell is an autologous cell. For example, the immune cell is an allogeneic cell. For example, the immune cell is selected from: a B cell, a monocyte, a natural killer cell, a basophil, an eosinophil, a neutrophil, a dendritic cell, a macrophage, a T cell, an NKT cell, a stem cell-derived immune effector cell, or a combination thereof. For example, the engineered cell is a T cell. For example, the engineered cell is an allogeneic T cell. For example, the engineered cell is a stem cell-derived T cell. The T cell can be cytotoxic T cell, helper T cell, or αβT, γδT, CD4+/CD8+ double positive T cell, CD4+T cell, CD8+T cells CD4/CD8 double negative T cell, CD3+T cell, naive T cell, effector T cell, cytotoxic T cell, helper T cell, memory T cell, regulatory T cell, Th0 cell, Th1 cell, Th2 cell, Th3 (Treg) cell, Th9 cell, Th17 cell, Thαβ helper cell, Tfh cell, stem cell-like central memory TSCM cell, central memory TCM cell, effector memory TEM cell, effector memory TEMRA cell or γδT cell. For example, the T cell is a cytotoxic T cell. In some embodiments, the genetically engineered T cells provided herein are isolated. For example, the genetically engineered T cells provided herein are substantially purified. For example, the engineered cells provided herein are obtained by genetically engineering cells isolated from a subject. As used herein, the engineered cells of the present application are obtained by genetically manipulating cells isolated from a subject. Alternatively, gene manipulation disclosed herein can be performed on cells isolated from a subject that have been passaged *in vitro* or subjected to other genetic manipulations, so as to obtain the engineered cells of this disclosure.

In one example, the genetically engineered cells provided herein are obtained by genetic modifications of cells isolated from the human body. Immune effector cells (e.g., T cells) can be obtained from many sources, including peripheral blood mononuclear cells, bone marrow, lymph node tissue, umbilical cord blood, thymus tissue, infected tissue, ascites, pleural effusion, spleen tissue and tumor. For example, T cell lines available in the art can be used. For example, the genetically engineered cells provided herein are obtained by genetic modifications of cells isolated from peripheral blood. For example, the genetically engineered cells provided herein are obtained by genetic modifications on cells isolated from bone marrow. For example, the genetically engineered cells provided herein are obtained by genetic modifications on cells isolated from peripheral blood mononuclear cells (PBMC).

In one embodiment, the engineered cells provided herein are obtained by genetically modifications of cells differentiated from stem or progenitor cells *in vitro.* For example, the stem or progenitor cells are selected from the group consisting of T cell progenitors, hematopoietic stem/progenitor cells, hematopoietic multipotent progenitor cells, embryonic stem cells, and induced pluripotent cells. For example, the genetically engineered cells provided herein are obtained by genetically modifications of cells differentiated from T cell progenitor cells *in vitro.* For example, the genetically engineered cells provided herein are obtained by genetically modifications of cells differentiated from hematopoietic stem/progenitor cells *in vitro.* For example, the genetically engineered cells provided herein are obtained by genetically modifications of cells differentiated from hematopoietic multipotent progenitor cells *in vitro.* For example, the genetically engineered cells provided herein are obtained by genetically modifications of cells differentiated from embryonic stem cells *in vitro.* For example, the genetically engineered cells provided herein are obtained by genetically modifications of cells differentiated from induced pluripotent cells *in vitro.*

Due to the immunogenetic differences between the donor and the recipient (or host), during transplantation of an exogenous donor graft, as an exogenous graft, the donor graft may be recognized and attacked by the immune cells(e.g., NK cells) of a recipient, thereby be suppressed or eliminated, and resulting in a Host-Versus-Graft Reaction (HVGR). For example, in allogeneic cell transplantation, the absence of HLA-I molecules on the allogeneic cells can reduce the host's CD8+ T cells mediated cellular immune rejection. For example, the absence of HLA-I molecules on the allogeneic cells can enhance the host NK cell-mediated cellular immune rejection. For example, the present application provides engineered cells with low or no expression of endogenous TCR and/or B2M.

Graft-Versus-Host Disease (GVHD) is caused by the diversity of T lymphocyte TCR in exogenous transplanted from the donor, as well as incompatibility with host HLA molecules. T lymphocytes from the donor recognize antigens on normal host tissues, proliferate and release a series of cytokines, enhancing the immune response of the graft against host antigens and attacking host cells. Knocking out the TCR of the exogenous transplanted donor (e.g., T cells) can avoid Graft-Versus-Host Disease. For example, engineered cells with low or no expression of endogenous HLA-I/TCR are provided. For example, the present application uses a CRISPR system to knock out the TRAC gene of the endogenous TCR α chain to prepare cells with low or no expression of endogenous TCR. For example, the CRISPR system is used to knock out endogenous B2M to prepare cells lacking endogenous HLA-I. For example, cells with low or no expression of HLA-A and HLA-B and/or TCR are provided. For example, engineered cells with low or no expression of endogenous HLA-I/TCR and/ or HLA-II are provided. For example, engineered cells with low or no expression of endogenous HLA-I/TCR are provided. For example, the engineered cells have low or no expression of endogenous TCR/B2M, TCR/B2M/CD38, TCR/B2M/FAS, TCR/B2M/NKG2A, TCR/B2M/CD38/NKG2A, or TCR/B2M/FAS/NKG2A.

In one example, the present application provides a method for genetic engineering cell by transferring the polynucleotides provided herein into cells using gene editing. If desired, techniques such as nucleases, transcription activator-like effector nucleases (TALENs), zinc finger nucleases (ZFNs), clustered regularly interspaced short palindromic repeats (CRISPRs), homologous recombination, nonhomologous end joining, microhomology-mediated end joining, homology-mediated end joining, etc. can be used for site-directed integration.

In one embodiment, endogenous TCR/B2M, TCR/B2M/CD38, TCR/B2M/NKG2A, or TCR/B2M/CD38/NKG2A are knocked out using CRISPR/Cas9 technology. For example, the sgRNA sequences targeting TRAC, B2M, NKG2A, and CD38 are shown in SEQ ID NOs: 39, 40, 41, and 42, respectively.

In one embodiment, T cells expressing a specific CAR is first constructed, and then the endogenous TCR/B2M, TCR/B2M/CD38, TCR/B2M/NKG2A or TCR/B2M/CD38/NKG2A in the CAR-T cells are knocked out using the CRISPER/Cas9 technology to construct the corresponding UCAR-T cells. For example, universal T cells are first constructed by knocking out the endogenous TCR/B2M, TCR/B2M/CD38, TCR/B2M/NKG2A or TCR/B2M/CD38/NKG2A using CRISPER/Cas9 technology, and then a specific CAR is expressed to construct the UCAR-T cells. For example, knocking out the endogenous TCR/B2M, TCR/B2M/CD38, TCR/B2M/NKG2A or TCR/B2M/CD38/NKG2A using the CRISPER/Cas9 technology and expressing a specific CAR are performed simultaneously to construct the UCAR-T cells.

For example, the present application provides an engineered T cell expressing a chimeric receptor, the engineered T cell comprises no expression of endogenous TCR/B2M; the chimeric receptor comprises, from N-terminus to C-terminus, an extracellular domain, a transmembrane domain, and optionally, an intracellular signal transduction domain; wherein the extracellular domain comprises an anti-CD38 antibody disclosed herein, the transmembrane domain comprises, for example, a transmembrane domain of CD28 or CD8, wherein the intracellular signal transduction domain comprises, for example, a CD3ζ intracellular signaling domain and a CD28 intracellular signaling domain and/or a CD137 intracellular signaling domain. Its extracellular domain and transmembrane domain may be connected by, for example, a CD8 hinge region, a CD28 hinge region, or an IgG spacer region or a fragment thereof. The chimeric receptor may further comprise a domain that binds to an immune cell marker other than CD38 and/or a domain that binds to a target antigen on a pathological cell.

In one example, the engineered cells expressing the chimeric receptor of the present application can bind to target cells expressing CD38. For example, the engineered cells of the present application can bind to NK cells. For example, the engineered cells of the present application can bind to target cells expressing CD38 and immune cell markers. For example, the engineered cells of the present application can bind to target cells expressing CD38 and NK cell markers. For example, the engineered cells of the present application can bind to target cells expressing CD38 and NK inhibitory receptors.

In one example, the engineered cells expressing chimeric receptors of the present application do not cause host rejection of the graft. For example, in the presence of host immune cells (e.g., NK cells), the engineered cells expressing chimeric receptors of the present application have a longer survival time and/or enhanced expansion capacity. For example, the engineered cells can kill the host's immune cells. For example, the engineered cells can kill the host's NK cells. For example, the engineered cells can kill allogeneic NK cells. For example, the engineered cells can resist killing by host NK cells. For example, the engineered cells can resist killing by allogeneic NK cells. For example, compared to cells not expressing the chimeric receptor of this application, the engineered cells expressing the chimeric receptors of the present application have a significantly improved ability to kill pathological cells.

In one embodiment, the engineered cells have a prolonged survival time or enhanced expansion ability in the presence of host immune cells. For example, the engineered cells have an inhibitory or killing function against the host's immune cells. For example, the engineered cells have an inhibitory or killing function against the host's T cells or NK cells. For example, the engineered cells have an inhibitory or killing function against the host's NK cells. For example, the engineered cells can enhance the survival, proliferation, and killing of pathological cells of another engineered cell introduced into the subject before, concurrently with, or after the engineered cell.

In one example, in the presence of host immune cells (e.g., NK cells), the engineered cells of the present application that bind to CD38 have a longer survival time and/or enhanced expansion capacity. For example, the engineered cells have a stronger ability to kill host NK cells. For example, the engineered cells have a stronger ability to kill allogeneic NK cells. For example, the engineered cells have a stronger ability to resist the killing by host NK cells. For example, the engineered cells have a stronger ability to resist the killing by allogeneic NK cells.

In one example, in the presence of host immune cells (e.g., NK cells), the dual-target engineered cells that bind to both CD38 and a target antigen on a pathological cell (e.g., a tumor cell or a pathological cell of autoimmune disease) have a longer survival time and/or enhanced expansion capacity. For example, the engineered cells exhibit a stronger cytotoxic effect on pathological cells carrying the target antigen both *in vivo* and *in vitro.*

In one example, the engineered cells expressing the chimeric receptor also express an immune checkpoint inhibitor. The immune checkpoint inhibitor comprises any agent that blocks, inhibits, or reduces the activity or function of an inhibitory pathway of the immune system. Such inhibitors may include small molecule inhibitors or may include antibodies or antigen binding fragments thereof that bind to and block or inhibit immune checkpoint receptors, ligands, and/or receptor-ligand interactions. For example, regulation, enhancement, and/or stimulation of a specific receptor can override immune checkpoint pathway components. Exemplary immune checkpoint molecules that can be targeted for blocking, inhibition, regulation, enhancement and/or stimulation include, but are not limited to: PD-1 (CD279), PD-L1 (CD274, B7-H1), PDL2 (CD273, B7-DC), CTLA-4, LAG-3 (CD223), TIM-3, 4-1BB (CD137), 4-1BBL (CD137L), GITR (TNFRSF18, AITR), CD40, OX40 (CD134, TNFRSF4), CXCR2, tumor associated antigen (TAA), B7-H3, B7-H4, BTLA, HVEM, GAL9, B7H3, B7H4, VISTA, KIR, 2B4 (belonging to the CD2 family of molecules and expressed on all NK, γδ and memory CD8+ (αβ) T cells), CD160 (also known as BY55), CGEN-15049, CEACAM (e.g., CEACAM-1, CEACAM-3 and/or CEACAM-5), TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine and transforming growth factor receptor (TGFR; e.g., TGFRβ). Immune checkpoint inhibitors comprise antibodies antigen binding fragments thereof or other binding proteins that bind to and block or inhibit and/or enhance or stimulate the activity of one or more of any of the molecules described.

The present application discloses engineered cells comprising a chimeric receptor targeting CD38. The present application discloses engineered cells comprising a chimeric receptor targeting NKG2A and CD38. The present application discloses engineered cells comprising a chimeric receptor targeting CD94 and CD38. The present application discloses engineered cells comprising a chimeric receptor targeting BCMA, NKG2A, and CD38. The present application discloses engineered cells comprising a chimeric receptor targeting GPRC5D, NKG2A, and CD38.

Provided herein is an autologous or allogeneic immune cell (e.g., T cell), which is genetically engineered to express NKG2D-CAR for resisting NK cell killing, thereby providing a method for increasing the persistence and/or engraftment survival rate of an autologous or allogeneic first immune cell in the presence of a second immune cell of a host. To clarify, the "host" is the recipient of a "first immune cell", such as a subject, a patient, etc.; after the "first immune cell" is engineered and transplanted into the host, any immune cell in the host other than the "first immune cell" is termed the "second immune cell". The "first immune cell" and the "second immune cell" can be cells from the same individual, or can be allogeneic cells.

In one example, the first immune cell is genetically engineered to express NKG2D-CAR. For example, the first immune cell is genetically engineered to express NKG2D-CAR, and endogenous NKG2D ligands are knocked out using gene editing technology. For example, the first immune cell is genetically engineered to express NKG2D-CAR, and endogenous B2M and TCR are knocked out using gene editing technology. For example, the first immune cell is genetically engineered to express NKG2D-CAR, and endogenous NKG2D ligands, B2M and TCR are knocked out using gene editing technology. For example, the first immune cell is genetically engineered to express NKG2D-CAR, and endogenous B2M and TCR are knocked out using gene editing technology, and an ASGPR chimeric receptor is expressed. For example, the first immune cell is genetically engineered to express a CAR or TCR that recognizes CD19, CD20, CD22, CD38, BCMA, GPRC5D, B7H3, GPC3, Claudin 6, Claudin18.2, FAP, Mesothelin, NKG2D, NKG2A or CD94, or a combination thereof, and also expresses an ASGPR1 chimeric receptor, an ASGPR2 chimeric receptor, an M6PR chimeric receptor, or an IGF2R chimeric receptor. For example, the first immune cell is genetically engineered to express a CAR or TCR that recognizes CD19, CD20, CD22, CD38, BCMA, GPRC5D, B7H3, GPC3, Claudin 6, Claudin18.2, FAP, Mesothelin, NKG2D, NKG2A or CD94 or a combination thereof, and endogenous B2M and TCR are knock out using gene editing technology , and also express an ASGPR1 chimeric receptor, an ASGPR2 chimeric receptor, an M6PR chimeric receptor, or an IGF2R chimeric receptor. For example, the NKG2D-CAR described herein comprises the nucleic acid sequence shown in SEQ ID NO: 78, or 79, or the amino acid sequence encoded thereof.

### Composition

The present application also provides pharmaceutical compositions comprising the chimeric receptors disclosed herein. The present application also provides cell compositions comprising the engineered cells disclosed herein. The present application also provides pharmaceutical compositions comprising the engineered cells disclosed herein. For example, the pharmaceutical composition comprises an effective amount of the chimeric receptor disclosed herein and a pharmaceutically acceptable carrier. For example, the pharmaceutical composition comprises an effective amount of the engineered cells disclosed herein and a pharmaceutically acceptable carrier. For example, the pharmaceutical composition is used for inhibiting host rejection of a graft. For example, the pharmaceutical composition is used in immunotherapy. For example, the pharmaceutical composition is used in oncology. For example, the pharmaceutical composition is used for inhibiting tumor growth in a subject (e.g., a human patient). For example, the pharmaceutical composition is used for treating viral infections. For example, the pharmaceutical composition is used for treating autoimmune diseases.

In one example, the cell composition provided herein comprises a first engineered cell and a second engineered cell. For example, the first engineered cell in the cell composition comprises a chimeric receptor comprising the CD38 binding domain disclosed herein, and the second engineered cell binds to a target antigen (e.g., CD19, BCMA, CD20, GPRC5D, CLD18.2, GPC3, CLDN6, B7H3, FAP, Mesothelin, FcRH5, EGFR and its mutants, ASGPR1, IL13RA2, WT1, CLL1, or a combination thereof) on a pathological cell (e.g., a tumor cell, a pathological cell of an autoimmune disease). The second engineered cell is an engineered immune cell known in the art for tumor treatment. For example, the second engineered cell is a CAR-T cell known in the art.

In one embodiment, the types of cells in the cell composition provided herein can be the same or different. For example, the first engineered cell or the second engineered cell can be any known suitable immune cell, and the type of the first engineered cell does not affect the type of the second engineered cell. For example, the first engineered cell can be a T, NK, or NKT cell, and the second engineered cell can be a T, NK, or NKT cell, or any other suitable immune cell.

In one example, the present application provides a pharmaceutical composition comprising chimeric receptors or cells provided herein. For example, the composition is suitable for intravenous, intramuscular, subcutaneous, parenteral, spinal or epidermal administration (e.g., by injection or infusion). For example, the composition is suitable for local administration. For example, local administration includes intratumoral injection, peritumoral injection, juxtatumoral injection, intralesional injection and/or injection into tumor-draining lymph nodes, or essentially any tumor-targeted injection, wherein the anti-tumor agent is expected to leak into the primary lymph nodes adjacent to the targeted solid tumor.

According to the route of administration, an active ingredient (i.e. chimeric receptor or engineered cell) can be coated in a material to protect it from the effect of acid and other natural conditions that can inactivate it. Pharmaceutically acceptable carrier that can be used for the compositions or formulations provided herein comprises any and all solvents, dispersion media, coatings, antibacterial and antifungal agents, isotonic and absorption delay agents and other materials with physiological compatibility. For example, the carrier is suitable for intravenous, intramuscular, subcutaneous, parenteral, spinal or epidermal administration (for example, by injection or infusion). According to the route of administration, an active ingredient (i.e. the genetically engineered chimeric receptor or cell) can be coated in a material to protect it from the effect of acid and other natural conditions that can inactivate it.

The present application provides a kit for preparing pharmaceutical compositions comprising the chimeric receptors disclosed herein. For example, the kit comprises the chimeric receptors disclosed herein, and pharmaceutically acceptable excipients present in one or more containers. For example, the kit can comprise the chimeric receptors disclosed herein for administration to a subject. For example, the kit can comprise instructions for the preparation and/or administration of the chimeric receptors.

The present application provides a kit for preparing the cells disclosed herein. For example, the kit comprises one or more vectors for generating engineered cells (e.g., T cells) expressing the chimeric receptors disclosed herein. The kit can be used to generate genetically engineered cells from autologous or non-autologous cells. For example, the kit can comprise the cells disclosed herein for administration to a subject. For example, the kit comprises the cells disclosed herein in one or more containers. For example, the kit comprises instructions for the preparation and/or administration of the genetically engineered cells.

The present application provides a kit for inducing and/or enhancing an immune response and/or treating and/or preventing tumors or pathogen infections, autoimmune diseases, or inflammatory diseases in a subject. For example, the kit comprises an effective amount of the composition and pharmaceutical composition of the chimeric receptor of this application and/or a chimeric receptor binding pathological cells. For example, the kit comprises a sterile container; such a container can be a box, an ampoule, a bottle, a vial, a tube, a bag, a pouch, a blister pack, or other suitable container forms known in the art. Such a container can be made of plastic, glass, laminated paper, metal foil, or other materials suitable for containing medicaments. For example, the kit comprises molecules encoding the chimeric receptor, recombinant TCR, exogenous cytokine, and/or therapeutic monoclonal antibody of the present application, optionally comprised in one or more vectors.

### Methods and uses

In one embodiment, the present application provides the use of the chimeric receptors or engineered cells disclosed herein. For example, the chimeric receptors or engineered cells is used for preparing a medicament for preventing or resisting transplant immune rejection. For example, the chimeric receptors or engineered cells is used for preventing or resisting transplant immune rejection.

In one embodiment, the present application provides a method for preventing, alleviating, and/or treating tumors, autoimmune diseases, and inflammatory diseases, comprising administering the chimeric receptor, engineered cell, or pharmaceutical composition to a subject in need thereof. The pharmaceutical composition provided herein has been described above, and the method for preventing, alleviating, and/or treating tumors, autoimmune diseases, or inflammatory diseases provided herein comprises all technical solutions thereof.

In one example, the chimeric receptors or engineered cells disclosed in the present application can induce and/or increase an immune response in a subject. The present application provides a method for inhibiting the activity of pathological cells in a subject, the method comprising: administering to a subject a therapeutically effective amount of engineered cells expressing the chimeric receptors of the present application, wherein the engineered cells inhibit the activity of pathological cells and/or kill pathological cells in the subject. For example, the pathological cells are tumor cells. For example, the pathological cells include, but are not limited to, acute myeloma leukemia cells, anaplastic lymphoma cells, astrocytoma cells, B cell cancer cells, breast cancer cells, colon cancer cells, ependymoma cells, esophageal cancer cells, glioblastoma cells, glioma cells, leiomyosarcoma cells, liposarcoma cells, liver cancer cells, lung cancer cells, mantle cell lymphoma cells, melanoma cells, neuroblastoma cells, non-small cell lung cancer cells, oligodendroglioma cells, ovarian cancer cells, pancreatic cancer cells, peripheral T cell lymphoma cells, renal cancer cells, sarcoma cells, gastric cancer cells, hepatocellular carcinoma cells, mesothelioma cells, or sarcoma cells. For example, the target cell is an immune cell.

In one example, the chimeric receptors or engineered cells disclosed herein can be used for treating and/or preventing tumors in a subject. For example, the chimeric receptors or engineered cells are used for prolonging the survival of a subject suffering from a tumor. For example, the chimeric receptors or engineered cells are used for treating and/or preventing pathogen infections or other infectious diseases in human subjects with immunocompromised. This method comprises administering an effective amount of the composition of the present application to achieve the desired effect, whether it is alleviating existing conditions or preventing recurrence. For treatment, the amount administered is an amount effective to produce the desired effect. The effective amount can be provided in one or multiple administrations. The effective amount can be provided in large doses or by continuous infusion. The tumor can be any of those disclosed herein.

In one embodiment, the chimeric receptors or engineered cells disclosed herein are used for preparing a medicament for treating an autoimmune disease (AID) or an inflammatory disease. For example, the chimeric receptors or engineered cells are used for treating, preventing, or ameliorating an autoimmune disease or inflammatory disease in a subject in need thereof. The autoimmune disease or inflammatory disease can be any of those disclosed herein.

In one example, autoimmune disease-related inflammatory disease is selected from, for example, arthritis (e.g., rheumatoid arthritis, arthritis chronica progrediente and deformin arthritis) and rheumatic diseases, including inflammatory conditions and rheumatic diseases involving bone loss, inflammatory pain, spondyloarthropathies (including ankylosing spondylitis), Reiter's syndrome, reactive arthritis, psoriatic arthritis, juvenile idiopathic arthritis and enteropathic arthritis, enthesitis, hypersensitivity reactions (including airway hypersensitivity reactions and skin hypersensitivity reactions) and allergies. The engineered T cells provided herein are useful for treating and preventing autoimmune hematological disorders (including, for example, hemolytic anemia, aplastic anemia, pure red cell anemia, and idiopathic thrombocytopenia), systemic lupus erythematosus (SLE), lupus nephritis, inflammatory muscle disease (dermatomyositis), periodontitis, polychondritis, scleroderma, Wegener's granulomatosis, dermatomyositis, chronic active hepatitis, myasthenia gravis, psoriasis, Stevens Johnson syndrome, spontaneous sprue, autoimmune inflammatory bowel disease (including, for example, ulcerative colitis, Crohn's disease, and irritable bowel syndrome), endocrine ophthalmopathy, Graves' disease, sarcoidosis, multiple sclerosis, systemic sclerosis, fibrotic diseases, primary biliary cirrhosis, juvenile diabetes (type I diabetes), uveitis, keratoconjunctivitis sicca and vernal keratoconjunctivitis, interstitial pulmonary fibrosis, periprosthetic osteolysis, glomerulonephritis (with and without nephrotic syndrome, including, for example, idiopathic nephrotic syndrome or minimal change disease), multiple myeloma, inflammatory diseases of the skin and cornea, myositis, loosening of bone implants, metabolic disorders (such as obesity, atherosclerosis and other cardiovascular diseases, including dilated cardiomyopathy, myocarditis, type II diabetes and dyslipidemia) and autoimmune thyroid diseases (including Hashimoto's thyroiditis), primary vasculitis of small and medium vessels, large vessel vasculitis including giant cell arteritis, hidradenitis suppurativa, neuromyelitis optica, Sjögren's syndrome, Behçet's disease, atopic and contact dermatitis, bronchiolitis, inflammatory muscle diseases, autoimmune peripheral neuropathies, immune kidney, liver and thyroid diseases, inflammation and atherosclerosis, auto inflammatory febrile syndrome, immune hematological disorders and bullous diseases of the skin and mucous membranes. For example, AID refers to a group of diseases that invade multiple tissues, organs or systems, including systemic lupus erythematosus (SLE), rheumatoid arthritis (RA), ankylosing spondylitis (AS), Sjögren's syndrome (SS), polymyositis or dermatomyositis, etc.

In one example, the chimeric receptors or engineered cells disclosed herein can be used for treating, preventing or ameliorating asthma, bronchitis, bronchiolitis, idiopathic interstitial pneumonia, pneumoconiosis, emphysema and other obstructive or inflammatory diseases of the airways.

In one example, the present application provides a method for treating a disease in a subject suffering from a tumor, an immune disease (e.g., an autoimmune disease), or an inflammatory disease, the method comprising: i) genetically modifying T cells, NK cells, and/or NKT cells obtained from an autologous or allogeneic source with a vector comprising a nucleic acid encoding the chimeric receptor of this application, wherein the chimeric receptor can specifically bind to immune cells (e.g., NK cells) and pathological cells (e.g., tumor cells, pathological cells of autoimmune diseases, pathological cells of inflammatory diseases) in the subject; ii) introducing the genetically modified T cells, NK cells, and/or NKT cells into the subject, wherein the genetically modified T cells, NK cells, and/or NKT cells recognize and kill tumor cells, and the genetically modified cells can resist attack by the subject's immune cells.

In one example, the methods described in this application can also be interpreted as therapeutic uses, that is, the methods described in this application can be considered as therapeutic uses of the compositions, nucleic acid molecules or engineered cells of this application or pharmaceutical uses for preparing the corresponding therapeutic uses. For example, this application relates to the use of the above-mentioned engineered cells or nucleic acid molecules for regulating the activity of engineered cells or the use of the above-mentioned engineered cells for preparing medicaments for regulating the activity of engineered cells; the use of the above-mentioned engineered cells or nucleic acid molecules for activating engineered cells or the use of the above-mentioned engineered cells for preparing medicaments for activating engineered cells; this application relates to the use of the above-mentioned engineered cells or nucleic acid molecules for inhibiting the activity of target cells in a subject or for preparing medicaments for inhibiting the activity of target cells in a subject; this application relates to the use of the above-mentioned engineered cells or nucleic acid molecules for improving or treating the health status of subjects in need or for preparing medicaments for improving or treating the health status of subjects in need. All the contents described in the above-mentioned application can also be applied to therapeutic uses or pharmaceutical uses.

The engineered cells of the present application can be used as the sole active ingredient or in combination with other medicaments such as immunosuppressants or immunomodulators or other anti-inflammatory agents or other cytotoxic agents or anticancer agents (for example, as adjuvants or in combination therewith), for example, to treat or prevent immune disorder-related diseases. For example, the antibodies of the present application can be used in combination with the following medicaments: DMARDs, e.g., gold salts, sulfasalazine, antimalarials, methotrexate, D-penicillamine, azathioprine, mycophenolic acid, tacrolimus, sirolimus, minocycline, leflunomide, glucocorticoids; calcineurin inhibitors, e.g., cyclosporine A or FK 506; regulators of lymphocyte recirculation, such as FTY720 and FTY720 analogs; mTOR inhibitors, such as rapamycin, 40-O-(2-hydroxyethyl)-rapamycin, CCI779, ABT578, AP23573 or TAFA-93; ascomycins with immunosuppressive properties, such as ABT-281, ASM981, etc.; corticosteroids; cyclophosphamide; azathioprine; leflunomide; mizoribine; mycophenolate mofetil; 15-deoxyspergualin or its immunosuppressive homologs, analogs or derivatives; immunosuppressive monoclonal antibodies, such as monoclonal antibodies against leukocyte receptors, e.g., MHC, CD2, CD3, CD4, CD7, CD8, CD25, CD28, CD40, CD45, CD58, CD80, CD86 or their ligands; other immunomodulatory compounds.

CD38 expression was detected in T cells, B cells, and NK cells collected from peripheral blood of different healthy human donors. In one example, the chimeric receptor targeting CD38 and the engineered cells expressing the chimeric receptor disclosed in this application can be used to treat: chronic lymphocytic leukemia (CLL), Waldenström's macroglobulinemia, primary systemic amyloidosis, mantle cell lymphoma (MCL), acute myeloid leukemia, acute lymphoblastic leukemia, NK cell leukemia, NK/T-cell lymphoma (NKTCL), multiple myeloma, melanoma, glioma, esophageal cancer, cervical cancer, lung cancer, myelodysplastic syndrome, lymphoma, membranous glomerulonephritis, non-small cell lung cancer, prostate cancer, triple-negative breast cancer, head and neck cancer, idiopathic thrombocytopenic purpura, myasthenia gravis, systemic lupus erythematosus.

### Transduction

The present application also provides methods for constructing engineered cells comprising any of the chimeric receptors disclosed herein. For example, a method for transducing a viral vector into a cell (e.g., an immune effector cell) involves activation and transduction of cells to be transduced, and the activation and transduction of the cell can be performed simultaneously, i.e., co-incubating an input composition comprising cells to be transduced, a stimulator for the cell to be transduced, and a viral vector particle carrying a recombinant nucleic acid encoding the chimeric receptor of the present invention, or activation first followed by transduction, such as co-incubating an input composition comprising cells to be transduced, and a stimulator for the cell to be transduced for activation, and then adding a viral vector particle carrying a recombinant nucleic acid encoding the chimeric receptor of the present invention for incubation, with the total time for activation and transduction of the recombinant nucleic acid is controlled to be completed within 72 hours, preferably within 48 hours, or within 36 hours, or within 24 hours. For example, the provided methods involve incubating and/or contacting retroviral vector particles (e.g., lentiviral vectors) with a population of cells (e.g., immune cells, such as T cells) , and activating and/or stimulating the T cells with an *ex vivo* cell activation reagent (e.g., an anti-CD3/anti-CD28 reagent) before, and/or simultaneously with, and/or after contacting or incubating the cells with the viral particles. Preferably, the cells are activated first, followed by viral transduction. For example, when the input composition comprising cells to be transduced, a stimulator for the cells to be transduced, and the viral vector particles carrying a recombinant nucleic acid encoding the chimeric receptor of the present invention are incubated together, the incubation time does not exceed 72 hours before harvesting to obtain an output composition, wherein the output composition comprises cells transduced with the recombinant nucleic acid; preferably, the incubation time is 1 hour to 72 hours; more preferably, the incubation time is 2 hours to 48 hours; more preferably, the incubation time is 2 hours to 36 hours; more preferably, the incubation time is 12 hours to 36 hours; more preferably, the incubation time is 12 hours to 24 hours; more preferably, the incubation time is 15 hours to 24 hours. For example, after purification treatment such as washing, centrifugation, the output composition can be used for preparing a pharmaceutical formulation without further *in vitro* expansion and culture, that is, the medicament prepared using the output composition does not require *in vitro* expansion before use in a subject (or patient).

### Chimeric receptor targeting CD38 and engineered cells expressing the chimeric receptor

The present application provides a chimeric receptor targeting CD38, the chimeric receptor having an extracellular domain, a transmembrane domain and an intracellular domain, the extracellular domain comprising a binding domain that binds to CD38, and the extracellular domain is connected to the transmembrane domain via a spacer. For example, the spacer comprises an IgG4 hinge region (or hinge), or a CD8 hinge region (or hinge). The chimeric receptor has anti-immune rejection and anti-tumor effects. For example, the CD38 positivity rate on the engineered cells expressing the chimeric receptor is low, or CD38 is almost undetectable. For example, the engineered cells expressing the chimeric receptor exhibit less fratricide. For example, the engineered cells expressing the chimeric receptor have strong expansion capacity during both *in vitro* and *in vivo* cultures. For example, the engineered cells expressing the chimeric receptor have strong survival ability during both *in vitro* and *in vivo* cultures. For example, the engineered cells expressing the chimeric receptor can significantly kill CD38-positive tumor cells both *in vitro* and *in vivo.* For example, the engineered cells expressing the chimeric receptor can significantly kill NK cells both *in vitro* and *in vivo.* For example, the engineered cells expressing the chimeric receptor can resist NK cell killing both *in vitro* and *in vivo.*

The chimeric receptor targeting CD38 disclosed in the present application, from N-terminus to C-terminus, sequentially comprises: an antigen binding unit that binds to CD38, an IgG4 hinge region or a fragment thereof, a transmembrane domain, and an intracellular domain (intracellular signal transduction domain).

In one embodiment, the antigen binding unit that binds to CD38 comprises the extracellular segment of a natural CD38 ligand or a fragment or variant thereof that can bind to CD38, an anti-CD38 antibody or fragment thereof, or a synthetic binding domain that binds to CD38. Any antibody described in this application or known in the art that has a high affinity for CD38 can be used as the CD38 binding domain in the chimeric receptor targeting CD38 in this application. For example, the anti-CD38 antibody or fragment thereof is selected from: a full antibody, an scFv, a single domain antibody, a Fab fragment, a Fab' fragment, an Fv fragment, a F(ab')2 fragment, a Fd fragment, an sdAb, a multifunctional antibody, a DDPP antibody, an scFv-Fc antibody, or an IgG4 antibody. For example, the anti-CD38 antibody is an scFv. For example, the anti-CD38 antibody is a single domain antibody. For example, the anti-CD38 antibody is a DDPP antibody.

In one embodiment, the anti-CD38 antibody or fragment thereof comprises an scFv. For example, the scFv has an amino acid sequence shown in SEQ ID NO: 19, 20, 21, or 22, or an amino acid sequence having at least 80%, 85%, 90%, 95%, 98%, 99%, or 100% identity thereto. For example, the anti-CD38 antibody or fragment thereof comprises a heavy chain variable region (VH) and a light chain variable region (VL). For example, the VH and VL have amino acid sequences shown in SEQ ID NOs: 49, and 50, respectively; or amino acid sequences shown in SEQ ID NOs: 57, and 58, respectively; or amino acid sequences shown in SEQ ID NOs: 65, and 66, respectively; or amino acid sequences shown in SEQ ID NOs: 73, and 74, respectively, or amino acid sequences having at least 80%, 85%, 90%, 95%, 98%, 99%, or 100% identity thereto. For example, the VH comprises HCDR1, HCDR2, and HCDR3, and the VL comprises LCDR1, LCDR2, and LCDR3, wherein the HCDR1, HCDR2, HCDR3, LCDR1, LCDR2, and LCDR3 have amino acid sequences having at least 95%, 96%, 97%, 98% or 99% sequence identity to the amino acid sequences shown in SEQ ID NOs:43, 44, 45, 46, 47, and 48, respectively; or amino acid sequences shown in SEQ ID NOs:51, 52, 53, 54, 55, and 56, respectively; or amino acid sequences shown in SEQ ID NOs:59, 60, 61, 62, 63, and 64, respectively; or amino acid sequences shown in SEQ ID NOs:67, 68, 69, 70, 71, and 72, respectively.

It is well known in the art that the sequences of VH and VL regions can undergo some amino acid substitutions while retaining their affinity for the target in the case while the CDR sequence remains unchanged; and the CDR sequences can also undergo some amino acid substitutions while retaining its affinity for the target in the case while keeping the amino acids in contact with the target unchanged.

In one embodiment, the anti-CD38 antibody or fragment thereof is an scFv. The VH (heavy chain variable region) and VL (light chain variable region) in the scFv can be connected by a linker peptide (or linker fragment), and their positions can be interchanged. For example, the anti-CD38 antibody comprises, from the N-terminus to the C-terminus, VH, a linker peptide, and VL. For example, the anti-CD38 antibody comprises, from the N-terminus to the C-terminus, VL, a linker peptide, and VH. Any linker peptide that connects VH and VL together to form a functionally complete single-chain antibody can be used. For example, the linker peptide is a GS linker peptide, for example, GGGGS, (GGGGS)3, or (GGGGS)4. For example, the scFv in the chimeric receptor sequentially comprises, from the N-terminus to the C-terminus, VH and VL. For example, the scFv in the chimeric receptor sequentially comprises, from the N-terminus to the C-terminus, VL and VH.

In one example, the CD38 binding domain in the extracellular domain of the chimeric receptor targeting CD38 comprises the extracellular segment of a CD38 ligand or a variant thereof that can bind to CD38.

In one example, the spacer of the chimeric receptor targeting CD38 is derived entirely or partially from an IgG4 hinge, preferably a human IgG4 hinge; the IgG4 hinge comprises a truncated IgG4 sequence; or the IgG4 hinge comprises a modified IgG4 hinge with at least one amino acid substitution; the IgG4 hinge is or comprises the amino acid sequence shown in SEQ ID NO: 5 or 6; or comprises an amino acid sequence having at least 80%, 85%, 90%, 95%, 96%, 97%, 98% or 99% sequence identity to SEQ ID NO: 5 or 6.

In one embodiment, the transmembrane domain of the chimeric receptor targeting CD38 is selected from: the transmembrane domain of CD2, CD3ε, CD3δ, CD3ζ, CD8, CD9, CD16, CD22, CD25, CD27, CD28, CD33, CD37, CD40, CD45, CD64, CD79A, CD79B, CD80, CD86, CD95 (Fas), CD134 (OX40), CD137 (4-1BB), CD150 (SLAMF1), CD152 (CTLA4), CD154, CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5, PDGFR, or Zap70. For example, the transmembrane domain is the transmembrane domain of CD8 (SEQ ID NO: 7), or the transmembrane domain of CD28 (SEQ ID NO: 8, or 9). For example, the transmembrane domain is a variant of the transmembrane domain of CD8 (SEQ ID NO: 7) or the transmembrane domain of CD28 (SEQ ID NO: 8, or 9) with no more than three amino acid mutations.

In one example, the intracellular signaling domain comprises one or more signaling domains or motifs, such as an immunoreceptor tyrosine-based activation motif (ITAM), a kinase domain, a co-stimulatory domain, etc., which can directly promote a cellular response. The intracellular signaling domain can indirectly promote a cellular response by associating with one or more other proteins, which in turn directly promote a cellular response. For example, the intracellular signaling domain or a functional fragment thereof comprises the intracellular signaling domain of CD3ε, CD3δ, CD3ζ, CD25, CD27, CD28, CD40, CD47, CD79A, CD79B, CD134 (OX40), CD137 (4-1BB), CD150 (SLAMF1), CD278 (ICOS), CD357 (GITR), CARD11, DAP10, DAP12, FcRα, FcRβ, FcRγ, Fyn, Lck, LAT, LRP, NKG2D, NOTCH1, NOTCH2, NOTCH3, NOTCH4, ROR2, Ryk, Slp76, pTα, TCRα, TCRβ, TRIM, Zap70, PTCH2, or any combination thereof. For example, the chimeric receptor targeting CD38 provided by the present invention comprises an intracellular signaling domain. For example, the chimeric receptor targeting CD38 provided by the present invention does not comprise an intracellular signaling domain. For example, the intracellular signal transduction domain comprises an immunoreceptor tyrosine-based activation motif or the signaling motif of an ITAM.

In one example, the intracellular signaling domain comprised in the chimeric receptor targeting CD38 comprises an intracellular signaling domain selected from: TCRα, TCRβ, TCRγ, TCRδ, FcRγ, FcRβ, CD3γ, CD3δ, CD3ε, CD3ζ, CD5, CD22, CD79a, CD79b, CD278, or CD66d, or a combination thereof. For example, the intracellular signaling domain comprises the CD3ζ intracellular signaling domain (SEQ ID NO: 12, or 13).

In one example, the intracellular signaling domain comprised in the chimeric receptor targeting CD38 provided herein further comprises a costimulatory domain. For example, the costimulatory domain is selected from the intracellular signaling domain of CD137 (4-1BB), CD28, CD27, TNFRSF9, TNFRSF4, TNFRSF8, TNFRSF14, TNFRSF18, CD40LG, ICOS, ITGB2, CD2, CD7, KLRC2, HAVCR1, LGALS9, CARD11, CD30, CD54, OX40, CD150, CD152, CD223, CD270, PD-L2, PD-L1, DAP10, LAT, NKD2C, SLP76, TRIM, FcεRIγ, MyD88, ICAM-1, LFA-1 (CD11a/CD18), 41BBL, or CD83, or a combination thereof. For example, the costimulatory domain is the intracellular signaling domain of CD137 (4-1BB) (SEQ ID NO: 11). For example, the costimulatory domain is the intracellular signaling domain of CD28 (SEQ ID NO: 10).

In one embodiment, the chimeric receptor targeting CD38 comprises a signal peptide. For example, the signal peptide comprises an amino acid sequence shown in SEQ ID NO: 1 or 2.

In one example, the chimeric receptor targeting CD38 provided by the present application, from N-terminus to C-terminus, sequentially comprises (1) a domain comprising an anti-CD38 antibody (scFv selected from SEQ ID NO: 19, 20, 21, or 22), (2) an IgG4 spacer fragment (SEQ ID NO: 5, or 6), (3) a CD8 transmembrane domain (SEQ ID NO: 7) or a CD28 transmembrane domain (SEQ ID NO: 8, or 9), and (4) a CD28 intracellular signaling domain (SEQ ID NO: 10) and/or a CD137 intracellular signaling domain (SEQ ID NO: 11); and (5) a CD3ζ intracellular signaling domain (SEQ ID NO: 12, or 13). Its extracellular domain can comprise any CD38 binding domain mentioned in the present application, such as the extracellular segment of a CD38 ligand or an antibody that binds to CD38. For example, the chimeric receptor sequentially comprises, an amino acid sequence having at least about 80%, 90%, 95%, 96%, 97%, 98%, 99% or 100% identity to the amino acid sequences shown in: SEQ ID NOs: 19, 6, 7, 10 and 12, or SEQ ID NOs: 19, 6, 7, 10 and 13, or SEQ ID NOs: 19, 6, 7 and 12, or SEQ ID NOs: 19, 6, 7 and 13, or SEQ ID NOs: 19, 6, 7 and 10, or SEQ ID NOs: 19, 6, 8, 10 and 12, or SEQ ID NOs: 19, 6, 8, 10 and 13, or SEQ ID NOs: 19, 6, 8 and 12, or SEQ ID NOs: 19, 6, 8 and 13, or SEQ ID NOs: 19, 6, 8 and 10, or SEQ ID NOs: 19, 6, 9, 10 and 12, or SEQ ID NOs: 19, 6, 9, 10 and 13, or SEQ ID NOs: 19, 6, 9 and 12, or SEQ ID NOs: 19, 6, 9 and 13, or SEQ ID NOs: 19, 6, 9 and 10, or SEQ ID NOs: 19, 5, 7, 10 and 12, or SEQ ID NOs: 19, 5, 7, 10 and 13, or SEQ ID NOs: 19, 5, 7 and 12, or SEQ ID NOs: 19, 5, 7 and 13, or SEQ ID NOs: 19, 5, 7 and 10, or SEQ ID NOs: 19, 5, 8, 10 and 12, or SEQ ID NOs: 19, 5, 8, 10 and 13, or SEQ ID NOs: 19, 5, 8 and 12,or SEQ ID NOs: 19, 5, 8 and 13, or SEQ ID NOs: 19, 5, 8 and 10, or SEQ ID NOs: 19, 5, 9, 10 and 12, or SEQ ID NOs: 19, 5, 9, 10 and 13,or SEQ ID NOs: 19, 5, 9 and 12, or SEQ ID NOs: 19, 5, 9 and 13, or 19, 5, 9 and 10, or SEQ ID NOs: 19, 6, 7, 11 and 12, or SEQ ID NOs: 19, 6, 7, 11 and 13, or 19, 6, 7 and 11, or SEQ ID NOs: 19, 6, 8, 11 and 12, or SEQ ID NOs: 19, 6, 8, 11 and 13, or 19, 6, 8 and 11, or SEQ ID NOs: 19, 6, 9, 11 and 12, or SEQ ID NOs: 19, 6, 9, 11 and 13, or SEQ ID NOs: 19, 6, 9 and 11, or SEQ ID NOs: 19, 5, 7, 11 and 12, or SEQ ID NOs: 19, 5, 7, 11 and 13, or SEQ ID NOs: 19, 5, 7 and 11, or SEQ ID NOs: 19, 5, 8, 11 and 12, or SEQ ID NOs: 19, 5, 8, 11 and 13, or SEQ ID NOs: 19, 5, 8 and 11, or SEQ ID NOs: 19, 5, 9, 11 and 12, or SEQ ID NOs: 19, 5, 9, 11 and 13, or SEQ ID NOs: 19, 5, 9 and 11, or SEQ ID NOs: 20, 6, 7, 10 and 12, or SEQ ID NOs: 20, 6, 7, 10 and 13, or SEQ ID NOs: 20, 6, 7 and 12, or SEQ ID NOs: 20, 6, 7 and 13, or SEQ ID NOs: 20, 6, 7 and 10, or SEQ ID NOs: 20, 6, 8, 10 and 12, or SEQ ID NOs: 20, 6, 8, 10 and 13, or SEQ ID NOs: 20, 6, 8 and 12, or SEQ ID NOs: 20, 6, 8 and 13, or SEQ ID NOs: 20, 6, 8 and 10, or SEQ ID NOs: 20, 6, 9, 10 and 12, or SEQ ID NOs: 20, 6, 9, 10 and 13, or SEQ ID NOs: 20, 6, 9 and 12, or SEQ ID NOs: 20, 6, 9 and 13, or SEQ ID NOs: 20, 6, 9 and 10, or SEQ ID NOs: 20, 5, 7, 10 and 12, or SEQ ID NOs: 20, 5, 7, 10 and 13, or SEQ ID NOs: 20, 5, 7 and 12, or SEQ ID NOs: 20, 5, 7 and 13,or SEQ ID NOs: 20, 5, 7 and 10, or SEQ ID NOs: 20, 5, 8, 10 and 12, or SEQ ID NOs: 20, 5, 8, 10 and 13, or SEQ ID NOs: 20, 5, 8 and 12, or SEQ ID NOs: 20, 5, 8 and 13, or SEQ ID NOs: 20, 5, 8 and 10, or SEQ ID NOs: 20, 5, 9, 10 and 12, or SEQ ID NOs: 20, 5, 9, 10 and 13, or SEQ ID NOs: 20, 5, 9 and 12, or SEQ ID NOs: 20, 5, 9 and 13, or SEQ ID NOs: 20, 5, 9 and 10, or SEQ ID NOs: 20, 6, 7, 11 and 12, or SEQ ID NOs: 20, 6, 7, 11 and 13, or SEQ ID NOs: 20, 6, 7 and 11, or SEQ ID NOs: 20, 6, 8, 11 and 12, or SEQ ID NOs: 20, 6, 8, 11 and 13, or SEQ ID NOs: 20, 6, 8 and 11, or SEQ ID NOs: 20, 6, 9, 11 and 12, or SEQ ID NOs: 20, 6, 9, 11 and 13, or SEQ ID NOs: 20, 6, 9 and 11, or SEQ ID NOs: 20, 5, 7, 11 and 12, or SEQ ID NOs: 20, 5, 7, 11 and 13, or SEQ ID NOs: 20, 5, 7 and 11, or SEQ ID NOs: 20, 5, 8, 11 and 12, or SEQ ID NOs: 20, 5, 8, 11 and 13, or SEQ ID NOs: 20, 5, 8 and 11, or SEQ ID NOs: 20, 5, 9, 11 and 12, or SEQ ID NOs: 20, 5, 9, 11 and 13, or SEQ ID NOs: 20, 5, 9 and 11, or SEQ ID NOs: 21, 6, 7, 10 and 12, or SEQ ID NOs: 21, 6, 7, 10 and 13, or SEQ ID NOs: 21, 6, 7 and 12, or SEQ ID NOs: 21, 6, 7 and 13, or SEQ ID NOs: 21, 6, 7 and 10, or SEQ ID NOs: 21, 6, 8, 10 and 12, or SEQ ID NOs: 21, 6, 8, 10 and 13, or SEQ ID NOs: 21, 6, 8 and 12, or SEQ ID NOs: 21, 6, 8 and 13,or SEQ ID NOs: 21, 6, 8 and 10, or SEQ ID NOs: 21, 6, 9, 10 and 12, or SEQ ID NOs: 21, 6, 9, 10 and 13, or SEQ ID NOs: 21, 6, 9 and 12, or SEQ ID NOs: 21, 6, 9 and 13, or SEQ ID NOs: 21, 6, 9 and 10, or SEQ ID NOs: 21, 5, 7, 10 and 12, or SEQ ID NOs: 21, 5, 7, 10 and 13, or SEQ ID NOs: 21, 5, 7 and 12, or SEQ ID NOs: 21, 5, 7 and 13, or SEQ ID NOs: 21, 5, 7 and 10, or SEQ ID NOs: 21, 5, 8, 10 and 12, or SEQ ID NOs: 21, 5, 8, 10 and 13, or SEQ ID NOs: 21, 5, 8 and 12, or SEQ ID NOs: 21, 5, 8 and 13, or SEQ ID NOs: 21, 5, 8 and 10, or SEQ ID NOs: 21, 5, 9, 10 and 12, or SEQ ID NOs: 21, 5, 9, 10 and 13, or SEQ ID NOs: 21, 5, 9 and 12, or SEQ ID NOs: 21, 5, 9 and 13, or SEQ ID NOs: 21, 5, 9 and 10, or SEQ ID NOs: 21, 6, 7, 11 and 12, or SEQ ID NOs: 21, 6, 7, 11 and 13, or SEQ ID NOs: 21, 6, 7 and 11, or SEQ ID NOs: 21, 6, 8, 11 and 12, or SEQ ID NOs: 21, 6, 8, 11 and 13, or SEQ ID NOs: 21, 6, 8 and 11, or SEQ ID NOs: 21, 6, 9, 11 and 12, or SEQ ID NOs: 21, 6, 9, 11 and 13, or SEQ ID NOs: 21, 6, 9 and 11, or SEQ ID NOs: 21, 5, 7, 11 and 12, or SEQ ID NOs: 21, 5, 7, 11 and 13, or SEQ ID NOs: 21, 5, 7 and 11, or SEQ ID NOs: 21, 5, 8, 11 and 12, or SEQ ID NOs: 21, 5, 8, 11 and 13, or SEQ ID NOs: 21, 5, 8 and 11, or SEQ ID NOs: 21, 5, 9, 11 and 12, or SEQ ID NOs: 21, 5, 9, 11 and 13,or SEQ ID NOs: 21, 5, 9 and 11, or SEQ ID NOs: 22, 6, 7, 10 and 12, or SEQ ID NOs: 22, 6, 7, 10 and 13, or SEQ ID NOs: 22, 6, 7 and 12, or SEQ ID NOs: 22, 6, 7 and 13, or SEQ ID NOs: 22, 6, 7 and 10, or SEQ ID NOs: 22, 6, 8, 10 and 12, or SEQ ID NOs: 22, 6, 8, 10 and 13, or SEQ ID NOs: 22, 6, 8 and 12, or SEQ ID NOs: 22, 6, 8 and 13, or SEQ ID NOs: 22, 6, 8 and 10, or SEQ ID NOs: 22, 6, 9, 10 and 12, or SEQ ID NOs: 22, 6, 9, 10 and 13, or SEQ ID NOs: 22, 6, 9 and 12, or SEQ ID NOs: 22, 6, 9 and 13, or SEQ ID NOs: 22, 6, 9 and 10, or SEQ ID NOs: 22, 5, 7, 10 and 12, or SEQ ID NOs: 22, 5, 7, 10 and 13, or SEQ ID NOs: 22, 5, 7 and 12, or SEQ ID NOs: 22, 5, 7 and 13, or SEQ ID NOs: 22, 5, 7 and 10, or SEQ ID NOs: 22, 5, 8, 10 and 12, or SEQ ID NOs: 22, 5, 8 and 13, or SEQ ID NOs: 22, 5, 8 and 10, or SEQ ID NOs: 22, 5, 9, 10 and 12, or SEQ ID NOs: 22, 5, 9, 10 and 13, or SEQ ID NOs: 22, 5, 9 and 12, or SEQ ID NOs: 22, 5, 9 and 13, or SEQ ID NOs: 22, 5, 9 and 10, or SEQ ID NOs: 22, 6, 7, 11 and 12, or SEQ ID NOs: 22, 6, 7, 11 and 13, or SEQ ID NOs: 22, 6, 7 and 11, or SEQ ID NOs: 22, 6, 8, 11 and 12, or SEQ ID NOs: 22, 6, 8, 11 and 13, or SEQ ID NOs: 22, 6, 8 and 11, or SEQ ID NOs: 22, 6, 9, 11 and 12,or SEQ ID NOs: 22, 6, 9, 11 and 13, or SEQ ID NOs: 22, 6, 9 and 11, or SEQ ID NOs: 22, 5, 7, 11 and 12, or SEQ ID NOs: 22, 5, 7, 11 and 13, or SEQ ID NOs: 22, 5, 7 and 11, or SEQ ID NOs: 22, 5, 8, 11 and 12, or SEQ ID NOs: 22, 5, 8, 11 and 13, or SEQ ID NOs: 22, 5, 8 and 11, or SEQ ID NOs: 22, 5, 9, 11 and 12, or SEQ ID NOs: 22, 5, 9, 11 and 13, or SEQ ID NOs: 22, 5, 9, and 11 .

For example, a chimeric receptor targeting CD38 comprises an amino acid sequence having about 80%, 90%, 95%, 96%, 97%, 98%, 99%, or 100% identity to the amino acid sequence shown in any one of SEQ ID NO: 24, 26, 27, or 28. For example, the chimeric receptor comprises an amino acid sequence encoded by a nucleotide sequence having about 80%, 90%, 95%, 96%, 97%, 98%, 99%, or 100% identity to the nucleotide sequence shown in any one of SEQ ID NO: 18, 23, 25, or 29.

Variants of chimeric receptor targeting CD38 in these examples include, but are not limited to, chimeric receptors obtained by swapping the positions of VH and VL in the scFv structure of the anti-CD38 antibody portion; chimeric receptors obtained by replacing the linker peptide with another linker peptide; chimeric receptors in which the anti-CD38 antibody portion is replaced with another antibody structure (such as Fab, single-domain antibody, etc.); and chimeric receptors in which the sequences of the VH and VL of the anti-CD38 antibody portion can undergo certain changes without affecting the binding of the anti-CD38 antibody to CD38; etc. Those skilled in the art can obtain the above possible variants through common knowledge in the art and routine experiments.

In one embodiment, the chimeric receptor targeting CD38 comprises a domain that binds to an immune cell marker different from CD38, and the domain can together with the CD38 binding domain constitute the extracellular region of the chimeric receptor. For example, the extracellular domain of the chimeric receptor further comprises a domain that binds to an immune cell marker different from CD38.

In one example, the domain that binds to an immune cell marker comprises a ligand, a synthetic binding domain, an antibody or a fragment thereof that binds to the immune cell marker. For example, the extracellular domain comprises an antibody or a fragment thereof that binds to the immune cell marker. For example, the antibody or a fragment thereof that binds to the immune cell marker is selected from: a full antibody, an scFv, a single domain antibody, a Fab fragment, a Fab' fragment, an Fv fragment, a F(ab')2 fragment, a Fd fragment, an sdAb, a multifunctional antibody, a DDPP antibody, an scFv-Fc antibody, or an IgG4 antibody. For example, the antibody or a fragment thereof that binds to the immune cell marker is an scFv. For example, the antibody or a fragment thereof that binds to the immune cell marker is a single domain antibody. For example, the antibody or a fragment thereof that binds to the immune cell marker is a Fab fragment.

In one example, the antibody or a fragment thereof that binds to the immune cell marker comprises a heavy chain variable region (VH2) and a light chain variable region (VL2). For example, the extracellular domain of the chimeric receptor comprises an anti-CD38 antibody or a fragment thereof, and an antibody or a fragment thereof that binds to the immune cell marker; wherein the anti-CD38 antibody or a fragment thereof comprises a heavy chain variable region (VH1) and a light chain variable region (VL1), and the antibody or a fragment thereof binding the immune cell marker comprises a heavy chain variable region (VH2) and a light chain variable region (VL2). For example, the VH1, VL1, VH2 and VL2 are connected to form a tandem structure. The tandem structure is formed as follows: wherein VH1 and VL1 are connected as an scFv, and VH2 and VL2 are connected to both ends of the scFv; or wherein VH2 and VL2 are connected as an scFv, and VH1 and VL1 are connected to both ends of the scFv, such as VL2-VH1-VL1-VH2, VH2-VL1-VH1-VL2, VL1-VH2-VL2-VH1, VH1-VL2-VH2-VL1, VH2-VH1-VL1-VL2, VL2-VL1-VH1-VH2, VH1-VH2-VL2-VL1, or VL1-VL2-VH2-VH1. For example, in the chimeric receptor, the anti-CD38 antibody or a fragment thereof and the antibody or a fragment thereof that binds to an immune cell marker are connected in a tandem manner. The tandem connection is such as VH1-VL1-VH2-VL2, VH2-VL2-VH1-VL1, VL1-VH1-VL2-VH2, VL2-VH2-VL1-VH1, VH1-VL1-VL2-VH2, VH2-VL2-VL1-VH1, VL1-VH1-VH2-VL2 or VL2-VH2-VH1-VL1. The "-" represents an optional linker peptide or peptide bond. Different variable regions are connected by a linker peptide. For example, the linker peptide is a GS linker peptide, for example, (GGGGS)n, wherein n can be any number from 1 to 20. For example, the linker peptide comprises GGGGS, (GGGGS)3 or (GGGGS)4. For example, the linker peptide comprises the amino acid sequence shown in SEQ ID NO: 14 or 15.

In one example, the chimeric receptor targeting CD38 comprises a domain that binds to a target antigen on pathological cells. The domain that binds to a target antigen on pathological cells can together with the CD38 binding domain constitute the extracellular domain of the chimeric receptor.

In one embodiment, the extracellular domain of the chimeric receptor targeting CD38 further comprises a domain that binds to a target antigen on pathological cells. For example, the domain that binds to the target antigen comprises a ligand, a synthetic binding domain, an antibody or a fragment thereof that binds to the target antigen. For example, the extracellular domain comprises an antibody or a fragment thereof that binds to the target antigen. For example, the antibody or a fragment thereof is selected from: a full antibody, an scFv, a single domain antibody, a Fab fragment, a Fab' fragment, an Fv fragment, a F(ab')2 fragment, a Fd fragment, an sdAb, a multifunctional antibody, a DDPP antibody, an scFv-Fc antibody, or an IgG4 antibody.

In one example, the antibody or a fragment thereof that binds to the target antigen on pathological cells comprises a heavy chain variable region (VH3) and a light chain variable region (VL3). For example, the extracellular domain of the chimeric receptor targeting CD38 comprises an anti-CD38 antibody or a fragment thereof, and an antibody or a fragment thereof that binds to the target antigen on pathological cells; wherein the anti-CD38 antibody or a fragment thereof comprises a heavy chain variable region (VH1) and a light chain variable region (VL1), and the antibody or a fragment thereof that binds to the target antigen on pathological cells comprises a heavy chain variable region (VH3) and a light chain variable region (VL3). For example, the VH1, VL1, VH3, and VL3 are connected to form a tandem structure. The tandem structure is formed as follows: wherein VH1 and VL1 are connected as an scFv, and VH3 and VL3 are connected to both ends of the scFv; or wherein VH3 and VL3 are connected as an scFv, and VH1 and VL1 are connected to both ends of the scFv, such as VL3-VH1-VL1-VH3, VH3-VL1-VH1-VL3, VL1-VH3-VL3-VH1, VH1-VL3-VH3-VL1, VH3-VH1-VL1-VL3, VL3-VL1-VH1-VH3, VH1-VH3-VL3-VL1, or VL1-VL3-VH3-VH1. For example, in the chimeric receptor, the anti-CD38 antibody or a fragment thereof and the antibody or a fragment thereof that binds to the target antigen on the pathological cells are connected in a tandem manner. The tandem connection is VH1-VL1-VH3-VL3, VH3-VL3-VH1-VL1, VL1-VH1-VL3-VH3, VL3-VH3-VL1-VH1, VH1-VL1-VL3-VH3, VH3-VL3-VL1-VH1, VL1-VH1-VH3-VL3 or VL3-VH3-VH1-VL1. The "-" represents an optional linker peptide or peptide bond. Different variable regions are connected by a linker peptide. For example, the linker peptide is a GS linker peptide, for example, (GGGGS)n, wherein n can be any number from 1 to 20. For example, the linker peptide comprises GGGGS, (GGGGS)3 or (GGGGS)4. For example, the linker peptide comprises the amino acid sequence shown in SEQ ID NO: 14 or 15.

The present application discloses chimeric receptors targeting CD38 as shown in Table 1. Dual-target chimeric receptors targeting NKG2A and CD38 as shown in Table 2. Chimeric receptors targeting NK cell markers (e.g., NKG2A, CD94, FasL, CD300A, or TIGIT) as shown in Table 3.

**Table 1. Chimeric receptors targeting CD38**

| Vector | Structure |
|---|---|
| CD38-BBZ | αCD38(scFv)-CD8(H)-CD8(TM)-CD137(C)-CD3ζ(C) |
| CD38-sBBZ | αCD38(scFv)-IgG4(H)-CD28(TM)-CD137(C)-CD3ζ(C) |
| CD38-28Z | αCD38(scFv)-CD8(H)-CD28(TM)-CD28(C)-CD3ζ(C) |
| CD38-s28Z | αCD38(scFv)-IgG4(H)-CD28(TM)-CD28(C)-CD3ζ(C) |

**Table 2. Dual-target chimeric receptors targeting NKG2A and CD38**

| Vector | Structure |
|---|---|
| CD38/N-s2 8Z | αCD38_VL-(L)-αNKG2A_VH-(L)-αNKG2A₋VL-(L)-αCD38₋VH-IgG4(H)-CD28(T M)-CD28(C)-CD3ζ |
| N /CD38-s28 Z | αNKG2A₋VL-(L)-αCD38_VH-(L)-αCD38-VL-(L)-αNKG2A₋VH-IgG4(H)-CD28(T M)-CD28(C)-CD3ζ |

**Table 3. Chimeric receptors targeting immune cell markers**

| Vector | Structure |
|---|---|
| NKG2A-s28z | αNKG2A(scFv)-IgG4(H)-CD28(TM)-CD28(C)-CD3ζ |
| NKG2A-28z | αNKG2A(scFv)-CD8(H)-CD28(TM)-CD28(C)-CD3ζ |
| TIGIT-s28z | αTIGIT(scFv)-IgG4(H)-CD28(TM)-CD28(C)-CD3ζ |
| TIGIT-28z | αTIGIT(scFv)-CD8(H)-CD28(TM)-CD28(C)-CD3ζ |
| FasL-s28z | αFasL(scFv)-IgG4(H)-CD28(TM)-CD28(C)-CD3ζ |
| FasL-28z | αFasL(scFv)-CD8(H)-CD28(TM)-CD28(C)-CD3ζ |
| CD300A-PDGFR | αCD300A(scFv)-PDGFR(TM) |

In Tables 1, 2, and 3: αCD38 (scFv): scFv of anti-CD38 antibodies 1, 2, 3, and 4 are SEQ ID NOs: 19, 20, 21, and 22, respectively; αCD38₋VH: heavy chain variable regions of anti-CD38 antibodies 1, 2, 3, and 4 are SEQ ID NOs: 49, 57, 65, and 73, respectively; αCD38₋VL: light chain variable regions of anti-CD38 antibodies 1, 2, 3, and 4 are SEQ ID NOs: 50, 58, 66, and 74, respectively; CD8(H): CD8 hinge region (SEQ ID NO: 3); IgG4(H): IgG4 hinge region (SEQ ID NO: 5); CD8(TM): CD8 transmembrane domain (SEQ ID NO: 7); CD28(TM): CD28 transmembrane domain (SEQ ID NO: 9); CD137(C): CD137 intracellular domain (SEQ ID NO: 11); CD28(C): CD28 intracellular domain (SEQ ID NO: 10); CD3ζ(C): CD3ζ intracellular domain (SEQ ID NO: 12); (L): linker peptide (SEQ ID NO: 14, 15, or 16).

In the embodiments disclosed herein, CD38-CAR1, 2, 3, and 4, respectively comprise CD38-BBZ constructed with CD38 antibody 1 (SEQ ID NO: 19), CD38 antibody 2 (SEQ ID NO: 20), CD38 antibody 3 (SEQ ID NO: 21), and CD38 antibody 4 (SEQ ID NO: 22) as shown in Table 1. CD38-CAR2s comprises CD38-sBBZ constructed with CD38 antibody 2 (SEQ ID NO: 20) as shown in Table 1. CD38-CAR3s comprises CD38-sBBZ constructed with CD38 antibody 3 (SEQ ID NO: 21) as shown in Table 1. CD38-CAR6 comprises CD38-sBBZ constructed with CD38 antibody 1 (SEQ ID NO: 19) as shown in Table 1. CD38-CAR9 comprises CD38-28Z constructed with CD38 antibody 1 (SEQ ID NO: 19) as shown in Table 1. CD38-CAR10,comprises CD38-s28Z constructed with CD38 antibody 1 (SEQ ID NO: 19) as shown in Table 1.

In the embodiment, from the 5' end to the 3' end, the CD38/NKG2A-DCAR1 fragment comprises CD38-sBBZ as shown in Table 1, 2A peptide and NKG2A-28Z as shown in Table 3; the CD38/NKG2A-DCAR2 fragment sequentially comprises CD38-s28Z as shown in Table 1, 2A peptide and NKG2A-28Z as shown in Table 3; the CD38/NKG2A-DCAR3 fragment sequentially comprises NKG2A-28Z as shown in Table 3, 2A peptide and CD38-s28Z as shown in Table 1.

The present application provides an engineered cell comprising a chimeric receptor targeting CD38 as disclosed in the present application.

In one example, the engineered cell further comprises a chimeric receptor 2 that binds to one or more immune cell markers other than CD38. For example, the immune cell marker is selected from: a T cell marker and/or a NK cell marker. For example, the immune cell marker is an NK inhibitory receptor (NKIR). For example, the immune cell marker is selected from: NKG2/CD94, a KIR family member, a LIR family member, a NKR-P1 family member, an immune checkpoint receptor, a SIGLEC family member, a Ly49 family member, or a combination thereof. For example, the NKG2/CD94 component is selected from NKG2A, NKG2C and CD94; the KIR family member is selected from KIR2DL1, KIR2DL2/3, KIR2DL5A, KIR2DL5B, KIR3DL1, KIR3DL2 and KIR3DL3; the LIR family member is selected from LIR1, LIR2, LIR3, LIR5 and LIR8; the NKR-P1 family member is selected from NKR-P1B and NKR-P1D; the immune checkpoint receptor is selected from PD-1, TIGIT, CD96, TIM3 and LAG3; the SIGLEC family member is selected from SIGLEC7 and SIGLEC9; the Ly49 family member is selected from Ly49A, Ly49C, Ly49F, Ly49G1 and Ly49G4. For example, the immune cell marker is selected from: CD2, CD3, CD4, CD5, CD7, CD8, CD16a, CD16b, CD25, CD27, CD28, CD30, CD38, CD45, CD48, CD50, CD52, CD56, CD57, CD62L, CD69, CD94, CD100, CD102, CD122, CD127, CD132, CD137, CD138, CD160, CD161, CD178, CD218, CD226, CD244, CD159a (NKG2A), CD159c (NKG2C), NKG2E, CD279, CD300A, CD314 (NKG2D), CD305, CD335 (NKP46), CD337, CD319 (CS1), TCRα, TCRβ, TIGIT, TRAIL, SLAMF7, NKG2F, NKG2H, NKp30, NKp44, NKp46, NKp80, SLAM family members, L-selectin, natural cytotoxicity receptor NCR1, NCR2, NCR3, or a combination thereof.

In one example, the engineered cell comprises a chimeric receptor 1 that binds to CD38 disclosed in the present invention and a chimeric receptor 2 that binds to one or more immune cell markers other than CD38. For example, the structure of chimeric receptor 2 is as shown in Table 3. For example, the chimeric receptor that binds to CD38 is as shown in Table 1. For example, the chimeric receptor 1 and chimeric receptor 2 are constructed on a single vector. For example, the polypeptide fragment, from the N-terminus to the C-terminus, sequentially comprises chimeric receptor 2, a 2A peptide, and chimeric receptor 1. For example, the 2A peptide is P2A, T2A, E2A, or F2A. For example, the chimeric receptor 1 and chimeric receptor 2 are connected by a cleavable peptide. For example, the polypeptide fragment, from the N-terminus to the C-terminus, sequentially comprises chimeric receptor 1, a 2A peptide, and chimeric receptor 2. For example, the chimeric receptor 1 and chimeric receptor 2 are constructed on different vectors, respectively. For example, chimeric receptor 2 comprises a ligand, a synthetic binding domain, an antibody or a fragment thereof that binds to the immune cell marker. For example, chimeric receptor 2 comprises an antibody or a fragment thereof that binds to the immune cell marker. For example, the antibody or a fragment thereof that binds to the immune cell marker is selected from: a full antibody, an scFv, a single domain antibody, a Fab fragment, a Fab' fragment, an Fv fragment, a F(ab')2 fragment, an Fd fragment, an sdAb, a multifunctional antibody, a DDPP antibody, an scFv-Fc antibody, or an IgG4 antibody. For example, the antibody or a fragment thereof that binds to the immune cell marker is an scFv. For example, the antibody or a fragment thereof that binds to the immune cell marker is a single domain antibody. For example, the antibody or a fragment thereof that binds to the immune cell marker is a Fab fragment. For example, the chimeric receptor 2 also binds to a target antigen on a pathological cell. The chimeric receptor 1 and chimeric receptor 2 can independently select the same or different hinge regions, transmembrane domains, and intracellular domains.

In one example, the engineered cell comprises a chimeric receptor 1 that binds to CD38 disclosed in the present invention and a chimeric receptor 2 that binds to one or more target antigens on pathological cells. For example, the chimeric receptor that binds to CD38 is as shown in Table 1. For example, the chimeric receptor 1 and the chimeric receptor 2 are constructed on a single vector. For example, the polypeptide fragment, from the N-terminus to the C-terminus, sequentially comprises chimeric receptor 2, a 2A peptide, and chimeric receptor 1. For example, the 2A peptide is P2A, T2A, E2A, or F2A. For example, the chimeric receptor 1 and the chimeric receptor 2 are connected by a 2A peptide. For example, the polypeptide fragment, from the N-terminus to the C-terminus, sequentially comprises chimeric receptor 1, a 2A peptide, and chimeric receptor 2. For example, the chimeric receptor 1 and the chimeric receptor 2 are constructed on different vectors, respectively. For example, the chimeric receptor 2 comprises a ligand, a synthetic binding domain, an antibody or a fragment thereof that binds to the target antigen on the pathological cell. For example, the chimeric receptor 2 comprises an antibody or a fragment thereof that binds to the target antigen on the pathological cell. For example, the antibody or a fragment thereof that binds to the target antigen on the pathological cell is selected from : a full antibody, an scFv, a single-domain antibody, a Fab fragment, a Fab' fragment, an Fv fragment, a F(ab')2 fragment, a Fd fragment, an sdAb, a multifunctional antibody, a DDPP antibody, an scFv-Fc antibody, or an IgG4 antibody. For example, the antibody or a fragment thereof that binds to the target antigen on the pathological cell is an scFv. For example, the antibody or a fragment thereof that binds to the target antigen on the pathological cell is a single-domain antibody. For example, the antibody or a fragment thereof that binds to the target antigen on the pathological cell is a Fab fragment. The chimeric receptor 1 and chimeric receptor 2 can independently select the same or different hinge regions, transmembrane domains, and intracellular domains.

In one example, the engineered cell comprises a chimeric receptor 1 that binds to CD38 disclosed herein, and a chimeric receptor 2; the chimeric receptor 2 binds to one or more immune cell markers other than CD38, and one or more target antigens on pathological cells. For example, chimeric receptors that bind to CD38 are shown in Table 1. For example, the chimeric receptor 1 and chimeric receptor 2 are constructed on a single vector. For example, the chimeric receptor 1 and chimeric receptor 2 are connected by a 2A peptide. For example, the 2A peptide is P2A, T2A, E2A, or F2A. For example, the chimeric receptor 1 and chimeric receptor 2 are connected by a 2A peptide. For example, the polypeptide fragment, from the N-terminus to the C-terminus, sequentially comprises chimeric receptor 1, a 2A peptide, and chimeric receptor 2. For example, the chimeric receptor 1 and chimeric receptor 2 are constructed on different vectors, respectively. For example, the chimeric receptor 2 comprises a ligand, a synthetic binding domain, an antibody or a fragment thereof that binds to the immune cell marker and the target antigen on the pathological cell. For example, chimeric receptor 2 comprises an antibody or a fragment thereof that binds to the immune cell marker and the target antigen on the pathological cell. For example, the antibody or a fragment thereof that binds to the immune cell marker and the target antigen on the pathological cell is selected from: a full antibody, an scFv, a single domain antibody, a Fab fragment, a Fab' fragment, an Fv fragment, a F(ab')2 fragment, an Fd fragment, an sdAb, a multifunctional antibody, a DDPP antibody, an scFv-Fc antibody or an IgG4 antibody. For example, the antibody or a fragment thereof that binds to the immune cell marker and the target antigen on the pathological cell is an scFv. For example, the antibody or a fragment thereof that binds to the immune cell marker and the target antigen on the pathological cell is a single domain antibody. For example, the antibody or a fragment thereof that binds to the immune cell marker and the target antigen on the pathological cell is a Fab fragment. For example, in the chimeric receptor 2, the antibody or a fragment thereof that binds to the immune cell marker comprises a heavy chain variable region (VH2) and a light chain variable region (VL2), and the antibody or a fragment thereof that binds to the target antigen on the pathological cell comprises a heavy chain variable region (VH3) and a light chain variable region (VL3). For example, the VH2, VL2, VH3, and VL3 are connected to form a tandem structure. The tandem structure is formed as follows: wherein VH2 and VL2 are connected as an scFv, and VH3 and VL3 are respectively connected to the two ends of the scFv; or wherein VH3 and VL3 are connected as an scFv, and VH2 and VL2 are respectively connected to the two ends of the scFv, such as VL3-VH2-VL2-VH3, VH3-VL2-VH2-VL3, VL2-VH3-VL3-VH2, VH2-VL3-VH3-VL2, VH3-VH2-VL2-VL3, VL3-VL2-VH2-VH3, VH2-VH3-VL3-VL2, or VL2-VL3-VH3-VH2. For example, in the chimeric receptor 2, the antibody or a fragment thereof that binds to an immune cell marker and the antibody or a fragment thereof that binds to a target antigen on a pathological cell are connected in a tandem manner. The tandem connection comprises VH2-VL2-VH3-VL3, VH3-VL3-VH2-VL2, VL2-VH2-VL3-VH3, VL3-VH3-VL2-VH2, VH2-VL2-VL3-VH3, VH3-VL3-VL2-VH2, VL2-VH2-VH3-VL3 or VL3-VH3-VH2-VL2. The "-" represents an optional linker peptide or peptide bond. Different variable regions are connected by a linker peptide. For example, the linker peptide is a GS linker peptide, for example, (GGGGS)n, wherein n can be any number from 1 to 20. For example, the linker peptide comprises GGGGS, (GGGGS)3 or (GGGGS)4. For example, the linker peptide comprises the amino acid sequence shown in SEQ ID NO: 14 or 15. The chimeric receptor 1 and chimeric receptor 2 can independently select the same or different hinge regions, transmembrane domains and intracellular domains.

In one embodiment, the engineered cell comprises a chimeric receptor 1 that binds to CD38, a chimeric receptor 2, and a chimeric receptor 3, wherein the chimeric receptor 2 binds to the immune cell marker, and the chimeric receptor 3 binds to a target antigen on pathological cells. For example, the chimeric receptor 1, chimeric receptor 2, and chimeric receptor 3 are constructed on a single vector. For example, the chimeric receptor 1 and chimeric receptor 3 are constructed on one vector, while the chimeric receptor 2 is constructed on a different vector. For example, the chimeric receptor 2 and chimeric receptor 3 are constructed on one vector, while the chimeric receptor 1 is constructed on a different vector. The chimeric receptors constructed on the same vector are connected by a coding sequence for a 2A peptide. For example, the 2A peptide is P2A, T2A, E2A, or F2A. For example, the chimeric receptor 1, chimeric receptor 2, and chimeric receptor 3 are constructed on different vectors, respectively. The chimeric receptor 1, chimeric receptor 2, and chimeric receptor 3 can independently select the same or different hinge regions, transmembrane domains, and intracellular domains.

The engineered cells targeting CD38 provided herein comprise the chimeric receptors as shown in Table 1. For example, the engineered cells comprise CD38-sBBZ or CD38-s28Z. In one example, the engineered cells comprise CD38-sBBZ or CD38-s28Z, and further comprise another polypeptide, wherein the another polypeptide comprises : a second antigen binding domain or a second chimeric receptor that binds to another NK cell marker, a target antigen on a pathological cell, or a combination thereof, or an HLA-E polypeptide or a fragment thereof, or CD300A-PDGFR. For example, the engineered cells comprise CD38-sBBZ or CD38-s28Z; and further comprise NKG2A-s28Z or NKG2A-28Z. For example, the engineered cells comprise CD38-sBBZ or CD38-s28Z; and further comprise TIGIT-s28Z or TIGIT-28Z. For example, the engineered cells comprise CD38-sBBZ or CD38-s28Z and further comprise FasL-s28Z or FasL-28Z. For example, the engineered cells comprise CD38-sBBZ or CD38-s28Z and further comprise CD300A-PDGFR. For example, the engineered cells comprise CD38-sBBZ or CD38-s28Z and further comprise HLA-E. For example, the engineered cells comprise CD38-sBBZ or CD38-s28Z and further comprise BCMA-CAR. For example, the engineered cells comprise CD38-sBBZ or CD38-s28Z and further comprise CD94-CAR. For example, the engineered cells comprise CD38-sBBZ or CD38-s28Z and further comprise BCMA/NKG2A-CAR. For example, the engineered cells comprise CD38-sBBZ or CD38-s28Z and further comprise GPRC5D/NKG2A-CAR. For example, the engineered cells comprise CD38-sBBZ or CD38-s28Z and further comprise CD19-CAR. For example, the engineered cells comprise CD38-sBBZ or CD38-s28Z and further comprise CD19/CD20-CAR. For example, the engineered cells comprise CD38-sBBZ or CD38-s28Z and further comprise CD19/CD20/NKG2A-CAR. For example, the engineered cells comprise CD38-sBBZ or CD38-s28Z and further comprise an antigen binding domain that recognizes NKG2A, TIGIT, FasL, or CD94. For example, the engineered cells comprise CD38-sBBZ or CD38-s28Z and further comprise an antigen binding domain that recognizes BCMA, GPRC5D, or CD19.

In one example, the engineered cells disclosed herein comprise an amino acid sequence having about 80%, 90%, 95%, 96%, 97%, 98%, 99%, or 100% identity to the sequence shown in any one of SEQ ID NO: 24, 26, 27, or 28. For example, the engineered cells comprise an amino acid sequence encoded by a nucleotide sequence having about 80%, 90%, 95%, 96%, 97%, 98%, 99%, or 100% identity to the nucleotide sequence shown in any one of SEQ ID NO: 18, 23, 25, 29, 30, 31, 32, 33, 34, 35, 36, 37, or 38. For example, the engineered cells comprise an amino acid sequence encoded by the nucleotide sequence shown in SEQ ID NOs: 18 and 30. The engineered cell comprises an amino acid sequence having at least about 80%, 90%, 95%, 96%, 97%, 98%, 99%, or 100% identity to the amino acid sequence encoded by the nucleotide sequences shown in SEQ ID NOs: 23 and 30, or SEQ ID NOs: 25 and 30, or SEQ ID NOs: 29 and 30, or SEQ ID NOs: 18 and 31, or SEQ ID NOs: 23 and 31, or SEQ ID NOs: 25 and 31, or SEQ ID NOs: 29 and 31, or SEQ ID NOs: 18 and 32, or SEQ ID NOs: 23 and 32, or SEQ ID NOs: 25 and 32, or SEQ ID NOs: 29 and 32, or SEQ ID NOs: 18 and 33, or SEQ ID NOs: 23 and 33, or SEQ ID NOs: 25 and 33, or SEQ ID NOs: 29 and 33, or SEQ ID NOs: 18 and 34, or SEQ ID NOs: 23 and 34, or SEQ ID NOs: 25 and 34, or SEQ ID NOs: 29 and 34, or SEQ ID NOs: 18 and 35, or SEQ ID NOs: 23 and 35, or SEQ ID NOs: 25 and 35, or SEQ ID NOs: 29 and 35.

In one example, the engineered cell comprises an amino acid sequence having at least about 80%, 90%, 95%, 96%, 97%, 98%, 99%, or 100% identity to the amino acid sequence shown in SEQ ID NOs: 24 and 84, or SEQ ID NOs: 26 and 84, or SEQ ID NOs: 27 and 84, or SEQ ID NOs: 28 and 84, or SEQ ID NOs: 24 and 85, or SEQ ID NOs: 26 and 85, or SEQ ID NOs: 27 and 85, or SEQ ID NOs: 28 and 85, or SEQ ID NOs: 24 and 86, or SEQ ID NOs: 26 and 86, or SEQ ID NOs: 27 and 86, or SEQ ID NOs: 28 and 86.

In one example, the target antigen is selected from: CD19, CD20, CD22, CD38, BCMA, GPRC5D, B7H3, GPC3, Claudin 6, Claudin18.2, FAP, Mesothelin, NKG2D ligand, NKG2A, CD94, FCRH5, EGFR and its mutants, ASGPR1, IL13RA2, WT1, CLL1 or a combination thereof.

In one embodiment, the pathological cells are selected from malignant cells or infected cells. For example, the pathological cells are selected from pathological cells of solid tumors, hematological tumors, and autoimmune diseases. For example, the solid tumors are selected from: esophageal cancer, gastric cancer, liver cancer, biliary tract tumors, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, or sarcoma; the hematological tumors are selected from: leukemia, lymphoma, or myeloma; and the autoimmune diseases are selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis, or dermatomyositis.

In one example, a CD38-CAR with an IgG4 hinge region significantly reduces the expression of the CD38 antigen on engineered cells. For example, the CD38 antigen on engineered cells expressing a CD38-CAR with an IgG4 hinge region is blocked and almost undetectable. For example, engineered cells expressing a CD38-CAR with an IgG4 hinge region have a high survival rate and strong expansion capacity during *in vitro* and *in vivo* cultures. For example, expressing a CD38-CAR with an IgG4 hinge region enhances the survival and/or expansion of immune cells that recognize tumor antigens in the host. For example, expressing a CD38-CAR with an IgG4 hinge region enhances the anti-tumor activity of immune cells that recognize tumor antigens. For example, expressing a CD38-CAR with an IgG4 hinge region enhances the survival and/or expansion of autologous or allogeneic immune cells that recognize solid tumor antigens in the host. For example, expressing a CD38-CAR with an IgG4 hinge region enhances the anti-tumor activity of autologous or allogeneic immune cells that recognize solid tumor antigens in the host. For example, the endogenous TCR, B2M, TCR/B2M, TCR/B2M/NKG2A, TCR/B2M/CD38, TCR/B2M/FAS, TCR/B2M/NKG2A/CD38, TCR/B2M/NKG2A/FAS or TCR/B2M/NKG2A/CIITA of the autologous or allogeneic immune cells comprising the CD38-CAR of the present invention are lowly expressed or not expressed.

In one example, the anti-tumor activity of an immune cell comprising a CD38-CAR with an IgG4 hinge region is about 10%, 20%, 30%, 40%, 50%, 60%, 70%, 80%, 90% or 100%, 2-fold, 3-fold, 4-fold, 5-fold or more higher than the activity of an immune cell comprising a chimeric receptor that only recognizes a target antigen on a pathological cell in killing or inhibiting target antigen-positive pathological cells. For example, the survival and/or expansion ability of an immune cell comprising a CD38-CAR with an IgG4 hinge region in the host is about 10%, 20%, 30%, 40%, 50%, 60%, 70%, 80%, 90% or 100%, 2-fold, 3-fold, 4-fold, 5-fold or more higher than that of an immune cell comprising a chimeric receptor that only recognizes a target antigen on a pathological cell.

The present application provides a polynucleotide encoding a chimeric receptor targeting CD38 disclosed herein; and/or a second antigen binding domain or a second chimeric receptor that binds to another NK cell marker, a target antigen on a pathological cell, or a combination thereof, or an HLA-E polypeptide or a fragment thereof, or CD300A-PDGFR. The present application provides a vector comprising the polynucleotide disclosed herein. The present application provides a virus comprising the vector disclosed herein.

### Chimeric receptor composition and engineered cells thereof

The present application discloses engineered cells (e.g., NK, T, NKT cells) expressing a first chimeric receptor and a second chimeric receptor, wherein the extracellular domains of both the first and second chimeric receptors recognize a target antigen, and pathological cells (e.g., tumor cells, pathological cells of autoimmune diseases), cells that mediate transplant immune rejection (e.g., NK, T, NKT cells), and the engineered cells all express the target antigen; the first chimeric receptor mediates killing of pathological cells (e.g., tumor cells, pathological cells of autoimmune diseases) that express the target antigen and/or killing of cells that mediate transplant immune rejection (e.g., NK, T, NKT cells), and the second chimeric receptor can block the target antigen expressed on the engineered cells (e.g., NK, T, NKT cells).

The present application discloses engineered cells (e.g., NK, T, NKT cells) expressing a first chimeric receptor and a second chimeric receptor, wherein the extracellular domains of both the first and second chimeric receptors recognize a target antigen, and pathological cells (e.g., tumor cells, pathological cells of autoimmune diseases), cells that mediate transplant immune rejection (e.g., NK, T, NKT cells), and the engineered cells all express the target antigen; the first chimeric receptor mediates killing of pathological cells (e.g., tumor cells, pathological cells of autoimmune diseases) that express the target antigen and/or killing of cells that mediate transplant immune rejection (e.g., NK, T, NKT cells), and the first chimeric receptor does not mediate, or has a significantly reduced ability to mediate, the killing of the engineered cells (e.g., NK, T, NKT cells).

In one example, the extracellular domain of the second chimeric receptor can bind to the target antigen that is targeted by the first chimeric receptor , and the first chimeric receptor mediates killing of target cells expressing the target antigen; the second chimeric receptor further comprises ASGPR, full-length CD137L or a fragment thereof, or further comprises full-length ASGPR1 or a fragment thereof, or further comprises full-length ASGPR2 or a fragment thereof, or further comprises full-length CD137L or a fragment thereof, or further comprises the transmembrane domain and intracellular domain of ASGPR1, or further comprises the transmembrane domain and intracellular domain of ASGPR2, or further comprises the transmembrane domain and intracellular domain of CD137L.

In one embodiment, the chimeric receptor composition comprises:
1. A first chimeric receptor selected from: (1) comprising a full-length natural ligand (or receptor), and an intracellular domain; (2) comprising the extracellular domain of a natural ligand (or receptor), a transmembrane domain and an intracellular domain; (3) comprising an antibody or a fragment thereof, a transmembrane domain and an intracellular domain; and
2. A second chimeric receptor selected from: (1) comprising the same natural ligand (or receptor) extracellular domain as the first chimeric receptor; further comprising full-length ASGPR1 or a fragment thereof, full-length ASGPR2 or a fragment thereof, the transmembrane domain and intracellular domain of ASGPR1, the transmembrane domain and intracellular domain of ASGPR2, full-length CD137L or a fragment thereof, or the transmembrane domain and intracellular domain of CD137L; (2) comprising an antibody or a fragment thereof that recognizes the target antigen recognized by the first chimeric receptor; further comprising full-length ASGPR1 or a fragment thereof, full-length ASGPR2 or a fragment thereof, the transmembrane domain and intracellular domain of ASGPR1, the transmembrane domain and intracellular domain of ASGPR2, full-length CD137L or a fragment thereof, or the transmembrane domain and intracellular domain of CD137L.

Preferably, the first chimeric receptor in the chimeric receptor composition mediates killing of target cells expressing the target antigen.

In one embodiment, the antigen recognized by the first chimeric receptor and the second chimeric receptor are selected from: CD1b, CD1c, CD1d, CD3d, CD3e, CD4, CD7, CD8, CD11, CD13, CD16b, CD20, CD22, CD25, CD26, CD27, CD28, CD30, CD33, CD34, CD38, CD39, CD40, CD44, CD45, CD45RA, CD45RO, CD48, CD52, CD56, CD58, CD59, CD62L, CD66a, CD69, CD70, CD80, CD83, CD85, CD86, CD93, CD94, CD95, CD95L, CD96, CD99, CD100, CD102, CD103, CD107a, CD107b, CD111, CD112, CD117, CD119, CD122, CD123, CD126, CD127, CD132, CD134, CD137, CD150, CD152, CD153, CD154, CD155, CD158, CD159a, CD160, CD161, CD178, CD183, CD185, CD191, CD192, CD196, CD212, CD215, CD218, CD223, CD226, CD244, CD252, CD253, CD275, CD278, CD279, CD281, CD282, CD283, CD284, CD286, CD300, CD305, CD314, CD319, CD336, CD337, CD355, CD360, CD366, CD371, B2MG, NKG2DL, or a combination thereof. For example, the antigen recognized by the first chimeric receptor and the second chimeric receptor is selected from: CD1d, CD7, CD13, CD20, CD22, CD25, CD26, CD30, CD33, CD38, CD45, CD52, CD56, CD70, CD80, CD83, CD86, CD93, CD94, CD95L, CD99, CD112, CD117, CD123, CD155, CD159a, CD319, CD366, CD371, NKG2DL, or a combination thereof.

The present application discloses engineered cells expressing a first chimeric receptor and a second chimeric receptor. For example, the engineered cell is an immune cell, a neuron, an epithelial cell, an endothelial cell, a stem cell, or a combination thereof. For example, the engineered cell is an immune cell. For example, the immune cell is an autologous cell. For example, the immune cell is an allogeneic cell. For example, the immune cell is selected from: a B cell, a monocyte, a natural killer cell, a basophil, an eosinophil, a neutrophil, a dendritic cell, a macrophage, a T cell, an NKT cell, a stem cell-derived immune effector cell, or a combination thereof. For example, the engineered cell is a T cell. In one example, the engineered cell is an allogeneic T cell. For example, the engineered cell is a stem cell-derived T cell. The T cell can be a cytotoxic T cell, a helper T cell, or an αβT, a γδT, a CD4+/CD8+ double positive T cell, a CD4+T cell, a CD8+T cell, a CD4/CD8 double negative T cell, a CD3+T cell, a naive T cell, an effector T cell, a cytotoxic T cell, a helper T cell, a memory T cell, a regulatory T cell, a Th0 cell, a Th1 cell, a Th2 cell, a Th3 (Treg) cell, a Th9 cell, a Th17 cell, a Thαβ helper cell, a Tfh cell, a stem cell-like central memory TSC cell, a central memory TCM cell, an effector memory TEM cell, an effector memory TEMRA cell, or a γδT cell. For example, the T cell is a cytotoxic T cell.

The present application discloses engineered cells (e.g., NK, T, NKT cells) expressing a first chimeric receptor and a second chimeric receptor, wherein the extracellular domains of the first and second chimeric receptors recognize NKG2DL, the first chimeric receptor mediates killing of target cells expressing NKG2DL (e.g., tumor cells, pathological cells of autoimmune diseases, NK, T, NKT cells), and the second chimeric receptor can block the NKG2DL polypeptide on the engineered cells (e.g., NK, T, NKT cells).

In one example, the engineered cells (e.g., NK, T, NKT cells) expressing the first chimeric receptor and the second chimeric receptor, wherein the extracellular domains of the first and second chimeric receptors recognize NKG2DL, the first chimeric receptor mediates killing of target cells expressing NKG2DL (e.g., tumor cells, pathological cells of autoimmune diseases, NK, T, NKT cells), and the first chimeric receptor does not mediate, or exhibits a significantly reduced ability to mediate, the killing of the engineered cells (e.g., NK, T, NKT cells).

In one embodiment, a chimeric receptor composition comprises:
1. A first chimeric receptor, from the N-terminus to the C-terminus, selected from: (1) comprising a CD3 intracellular domain and full-length NKG2D; (2) comprising a CD3Z intracellular domain, a CD137 intracellular domain, and full-length NKG2D; (3) comprising a CD3Z intracellular domain, a CD28 intracellular domain, and full-length NKG2D; (4) comprising the NKG2D extracellular domain, a CD8 or CD28 or IgG1 or IgG4 hinge region, a CD8 or CD28 transmembrane domain, and a CD3Z intracellular domain; (5) comprising the NKG2D extracellular domain, a CD8 or CD28 or IgG1 or IgG4 hinge region, a CD8 or CD28 transmembrane domain, a CD137 intracellular domain, and a CD3Z intracellular domain; (6) comprising a NKG2D extracellular domain, a CD8 or CD28 or IgG1 or IgG4 hinge region, a CD8 or CD28 transmembrane domain, a CD28 intracellular domain, and a CD3Z intracellular domain; and
2. A second chimeric receptor, from the N-terminus to the C-terminus, is selected from: (1) comprising full-length ASGPR1 and a NKG2D extracellular domain; (2) comprising full-length ASGPR2 and a NKG2D extracellular domain; (3) comprising full-length CD137L and a NKG2D extracellular domain; (4) comprising full-length ASGPR1 and an antibody or a fragment thereof that binds to NKG2DL; (5) comprising full-length ASGPR2 and an antibody or a fragment thereof that binds to NKG2DL; (6) comprising full-length CD137L and an antibody or a fragment thereof that binds to NKG2DL; (7) comprising an ASGPR1 intracellular domain and full-length NKG2D; (8) comprising an ASGPR2 intracellular domain and full-length NKG2D; (9) comprising a CD137L intracellular domain and full-length NKG2D; (10) comprising an ASGPR1 intracellular domain, a NKG2D transmembrane domain, and a NKG2D extracellular domain; (11) comprising an ASGPR2 intracellular domain, a NKG2D transmembrane domain, and a NKG2D extracellular domain; (12) comprising a CD137L intracellular domain, a NKG2D transmembrane domain, and a NKG2D extracellular domain; (13) comprising an ASGPR1 intracellular domain, an ASGPR1 transmembrane domain, and a NKG2D extracellular domain; (14) comprising an ASGPR2 intracellular domain, an ASGPR2 transmembrane domain, and a NKG2D extracellular domain; (15) comprising a CD137L intracellular domain, a CD137L transmembrane domain, and a NKG2D extracellular domain; (16) comprising an antibody or a fragment thereof that binds to NKG2DL, a CD8 or CD28 transmembrane domain, and an ASGPR1 intracellular domain; (17) comprising an antibody or a fragment thereof that binds to NKG2DL, a CD8 or CD28 transmembrane domain, and an ASGPR2 intracellular domain; (18) comprising an antibody or a fragment thereof that binds to NKG2DL, a CD8 or CD28 transmembrane domain, and a CD137L intracellular domain.

For example, a chimeric receptor composition, from the N-terminus to the C-terminus, a first chimeric receptor comprises a CD3Z intracellular domain and full-length NKG2D, and a second chimeric receptor comprises full-length ASGPR1 and a NKG2D extracellular domain. For example, a chimeric receptor composition, from the N-terminus to the C-terminus, a first chimeric receptor comprises a CD3Z intracellular domain and full-length NKG2D, and a second chimeric receptor comprises full length CD137L and a NKG2D extracellular domain. For example, a chimeric receptor composition, from the N-terminus to the C-terminus, a first chimeric receptor comprises a CD3Z intracellular domain, a CD137 intracellular domain and full-length NKG2D, and a second chimeric receptor comprises full-length ASGPR1 and a NKG2D extracellular domain. For example, a chimeric receptor composition, from the N-terminus to the C-terminus, a first chimeric receptor comprises a CD3Z intracellular domain, a CD137 intracellular domain and full-length NKG2D, and a second chimeric receptor comprises full-length CD137L and a NKG2D extracellular domain. For example, a chimeric receptor composition, from the N-terminus to the C-terminus, a first chimeric receptor comprises a CD3Z intracellular domain, a CD28 intracellular domain and full-length NKG2D, and a second chimeric receptor comprises full-length ASGPR1 and a NKG2D extracellular domain. For example, a chimeric receptor composition, from the N-terminus to the C-terminus, a first chimeric receptor comprises a CD3Z intracellular domain, a CD28 intracellular domain and full-length NKG2D, and a second chimeric receptor comprises full-length CD137L and a NKG2D extracellular domain.

For example, a chimeric receptor composition, from the N-terminus to the C-terminus, a first chimeric receptor comprises a CD3Z intracellular domain and full-length NKG2D, and a second chimeric receptor comprises full-length ASGPR1, a linker peptide, and a NKG2D extracellular domain. For example, a chimeric receptor composition, from the N-terminus to the C-terminus, a first chimeric receptor comprises a CD3Z intracellular domain and full length NKG2D, and a second chimeric receptor comprises full length CD137L, a linker peptide, and a NKG2D extracellular domain. For example, a chimeric receptor composition, from the N-terminus to the C-terminus, a first chimeric receptor comprises a CD3Z intracellular domain, a CD137 intracellular domain, and full-length NKG2D, and a second chimeric receptor comprises full-length ASGPR1, a linker peptide, and a NKG2D extracellular domain. For example, a chimeric receptor composition, from the N-terminus to the C-terminus, a first chimeric receptor comprises a CD3Z intracellular domain, a CD137 intracellular domain and full-length NKG2D, and a second chimeric receptor comprises full-length CD137L, a linker peptide, and a NKG2D extracellular domain. For example, a chimeric receptor composition, from the N-terminus to the C-terminus, a first chimeric receptor comprises a CD3Z intracellular domain, a CD28 intracellular domain and full-length NKG2D, and a second chimeric receptor comprises full-length ASGPR1, a linker peptide, and a NKG2D extracellular domain. For example, a chimeric receptor composition, from the N-terminus to the C-terminus, a first chimeric receptor comprises a CD3Z intracellular domain, a CD28 intracellular domain and full-length NKG2D, and a second chimeric receptor comprises full-length CD137L, a linker peptide, and a NKG2D extracellular domain. In one example, the linker peptide is a GS linker peptide, for example, (GGGGS)n, wherein n can be any number from 1 to 20. For example, the linker peptide comprises GGGGS, (GGGGS)3, or (GGGGS)4. In one example, the linker peptide comprises the amino acid sequence shown in SEQ ID NO: 14 or 15.

In one embodiment, a chimeric receptor composition comprises: 1. a first chimeric receptor: comprising an amino acid sequence having at least about 80%, 90%, 95%, 96%, 97%, 98%, 99%, or 100% identity to the amino acid sequence encoded by the nucleic acid shown in SEQ ID NO: 78 or 79; 2. A second chimeric receptor: comprising an amino acid sequence having at least about 80%, 90%, 95%, 96%, 97%, 98%, 99%, or 100% identity to the amino acid sequence encoded by the nucleic acid shown in SEQ ID NOs: 80 and 76, or SEQ ID NOs: 80, 16 and 76, or SEQ ID NOs: 81 and 76, or SEQ ID NOs: 81, 16 and 76.

The present application discloses engineered cells with inducible expression of a first chimeric receptor and constitutive expression of a second chimeric receptor, wherein the extracellular domains of both the first and second chimeric receptors recognize a target antigen, and the first chimeric receptor mediates killing of target cells expressing the target antigen (e.g., tumor cells, pathological cells of autoimmune diseases, NK, T, NKT cells, etc., cells mediating transplant immune rejection), and the first chimeric receptor does not mediate, or exhibits a significantly reduced ability to mediate, the killing of the engineered cells (e.g., NK, T, NKT cells) . For example, the second chimeric receptor is constitutively expressed, and the first chimeric receptor is regulated by a synNOTCH receptor. For example, the synNOTCH receptor refers to PCT/CN2022/102395.

The engineered cells comprising the first and second chimeric receptors provided herein can not only resist killing by autologous or allogeneic NK cells, but also can significantly kill pathological cells. For example, the present application provides T cells expressing a CAR recognizing NKG2D ligands and a CAR recognizing a tumor antigen. The present application provides universal T cells expressing a CAR recognizing NKG2D ligands and a CAR recognizing a tumor antigen, with the endogenous TCR and B2M are knocked out, and expressing an ASGPR1 chimeric receptor or an ASGPR2 chimeric receptor.

The present application provides the combined use of T cells that can resist killing by autologous or allogeneic NK cells and T cells expressing a second protein (such as a CAR) that recognizes a tumor antigen. The T cells provided in the present application, which can resist killing by autologous or allogeneic NK cells, can promote the survival of T cells expressing a second protein (such as a CAR) that recognizes a tumor antigen in the presence of autologous or allogeneic immune cells.

The chimeric receptor composition disclosed in the present application comprises any one of the first chimeric receptors targeting NKG2DL as shown in Table 4, and any one of the second chimeric receptors targeting NKG2DL as shown in Table 5.

**Table 4. First chimeric receptors targeting NKG2DL**

| Vector | Structure |
|---|---|
| NKG2Dz | CD3ζ(C)-NKG2D |
| NKG2D-BBZ | CD3ζ(C)- CD137(C)-NKG2D |
| NKG2D-28Z | CD3ζ(C)- CD28(C)-NKG2D |
| NKG2Dz1 | αNKG2DL(scFv)-CD8(H)-CD8(TM)-CD3ζ(C) |
| NKG2D-BBZ1 | αNKG2DL(scFv)-CD8(H)-CD8(TM)-CD137(C)-CD3ζ(C) |
| NKG2D-BBZ2 | αNKG2DL(scFv)-IgG4(H)-CD28(TM)-CD137(C)-CD3ζ(C) |
| NKG2D-28Z1 | αNKG2DL(scFv)-CD8(H)-CD28(TM)-CD28(C)-CD3ζ(C) |
| NKG2D-28Z2 | αNKG2DL(scFv)- IgG4(H)-CD28(TM)-CD28(C)-CD3ζ(C) |

**Table 5. Second chimeric receptors targeting NKG2DL**

| Vector | Structure |
|---|---|
| A-N1 | ASGPR1-NKG2Dex |
| A-N2 | ASGPR1-(L)-NKG2Dex |
| A-N3 | ASGPR1-αNKG2DL(scFv) |
| A-N4 | ASGPR1-(L)-αNKG2DL(scFv) |
| CD137L-N | CD137L-NKG2Dex |
| CD40L-N | CD40L-NKG2Dex |
| CD137L-N1 | CD137L-(L)-NKG2Dex |
| CD40L-N1 | CD40L-(L)-NKG2Dex |
| CD137L-N2 | CD137L-(L)-αNKG2DL(scFv) |
| CD40L-N2 | CD40L-(L)-αNKG2DL(scFv) |

In Tables 4 and 5 above: NKG2D: full-length NKG2D (SEQ ID NO: 77); NKG2Dex: NKG2D extracellular domain (SEQ ID NO: 76); CD8 (H): CD8 hinge region (SEQ ID NO: 3); IgG4 (H): IgG4 hinge region (SEQ ID NO: 5); CD8 (TM): CD8 transmembrane domain (SEQ ID NO: 7); CD28 (TM): CD28 transmembrane domain (SEQ ID NO: 8, or 9); CD137 (C): CD137 intracellular domain (SEQ ID NO: 11); CD28 (C): CD28 intracellular domain (SEQ ID NO: 10); CD3ζ (C): CD3ζ intracellular domain (SEQ ID NO: 12, or 13); ASGPR1: full-length ASGPR1 (SEQ ID NO: 80); (L): linker peptide (SEQ ID NO: 14, 15, or 16); CD137L: full-length CD137L (SEQ ID NO: 81).

The A-N1/NKG2Dz fragment disclosed in this application: from the 5' to 3' direction, sequentially comprises the NKG2Dz fragment, a 2A peptide, and the A-N1 fragment. The A-N2/NKG2Dz fragment: from the 5' to 3' direction, sequentially comprises the NKG2Dz fragment, a 2A peptide, and the A-N2 fragment. The CD137L-N/NKG2Dz fragment: from the 5' to 3' direction, sequentially comprises the NKG2Dz fragment, a 2A peptide, and the CD137L-N fragment. The CD40L-N/NKG2Dz fragment: from the 5' to 3' direction, sequentially comprises the NKG2Dz fragment, a 2A peptide, and the CD40L-N fragment. The A-N1/NKG2D-BBZ fragment: from the 5' to 3' direction, sequentially comprises the NKG2D-BBZ fragment, a 2A peptide, and the A-N1 fragment. The A-N2/NKG2D-BBZ fragment: from the 5' to 3' direction, sequentially comprises the NKG2D-BBZ fragment, a 2A peptide, and the A-N2 fragment. The CD137L-N/NKG2D-BBZ fragment: from the 5' to 3' direction, sequentially comprises the NKG2D-BBZ fragment, a 2A peptide, and the CD137L-N fragment. The CD40L-N/NKG2D-BBZ fragment: from the 5' to 3' direction, sequentially comprises the NKG2D-BBZ fragment, a 2A peptide, and the CD40L-N fragment. For example, the 2A peptide is P2A, T2A, E2A, or F2A.

The present application discloses engineered cells modified with the above-mentioned A-N1/NKG2Dz fragment, A-N2/NKG2Dz fragment, CD137L-N/NKG2Dz fragment, CD40L-N/NKG2Dz fragment, A-N1/NKG2D-BBZ fragment, A-N2/NKG2D-BBZ fragment, CD137L-N/NKG2D-BBZ fragment, or CD40L-N/NKG2D-BBZ fragment.

In order to enhance the protective effect of the second chimeric receptor on the engineered cells, the first chimeric receptor is inducible expressed, and the second chimeric receptor is constitutively expressed. Engineered cells with regulated expression of the first chimeric receptor are prepared according to PCT/CN2022/102395. For example, regulated NKG2D-CAR-T cells: T cells comprising a chimeric polypeptide that binds to a tumor antigen or a tissue-specific antigen (according to PCT/CN2022/102395) and NKG2D-CAR (as shown in Table 4); and a chimeric polypeptide synE or its mutant or truncated form or modified form, synJagged2EC, synE-APLP2 (TM), synNOTCH, or syn-CD8 that binds to a tumor antigen or a tissue-specific antigen can trigger transcriptional regulation of NKG2D-CAR.

Regulated NKG2D-CAR-T cells have one or more of the following characteristics: 1) After incubation with tumor antigen or tissue-specific antigen-positive cells, they can effectively kill NKG2D ligand-positive tumor cells (e.g., MV-4-11, THP-1) or immune cells (e.g., NK cells, T cells, NKT cells), while showing weak killing effects on cells with low NKG2D ligand expression (e.g., KG-1, Molm13); 2) When co-incubated with cells having low or no expression of tumor antigen or tissue-specific antigen, the killing effect on NKG2D ligand-positive cells (e.g., SK-Hep1, K562) is weakened or absent. The killing ability of synE- or synE-del3-regulated NKG2D-CAR-T cells is NKG2D ligand-dependent, while cells with only high expression of tumor antigen or tissue-specific antigen do not cause NKG2D ligand-independent killing. Placing NKG2D-CAR under the regulation of synE or synE-del3 can improve the *in vivo* survival ability and cytotoxicity of regulated NKG2D-CAR-T cells against target cells (pathological cells or immune rejection cells) . Regulated NKG2D-CAR-T cells can recognize target cells (tumor antigens or tissue-specific antigen-positive cells) via synE or synE-del3, and the killing effect of NKG2D-CAR-T cells still requires the initiation by NKG2D ligands on the target cell surface, and the downstream signals of synE or synE-del3 and the downstream signals of NKG2D-CAR do not cross-influence each other.

The present application discloses a CD123-synE fragment that recognizes the CD123 antigen, which, from the 5' to 3' direction, sequentially comprises a CD8 signal peptide, a CD123-scFv, an EphrinB2 extracellular domain, a Notch transmembrane domain, and GAL4-VP64. The CD123-synE-NKG2Dz fragment, from the 5' to 3' direction, sequentially comprises UAS-CMV, an NKG2Dz fragment, an EF1a promoter, and a CD123-synE fragment. The CD123-synE-NKG2D-BBZ fragment, from the 5' to 3' direction, sequentially comprises UAS-CMV, an NKG2D-BBZ fragment, an EF1a promoter, and a CD123-synE fragment.

The A-N1/CD123-synE-NKG2Dz fragment, from the 5' to 3' direction, sequentially comprises UAS-CMV, an NKG2Dz fragment, an EF1a promoter, a CD123-synE fragment, a 2A peptide, and the A-N1 fragment. The A-N1/CD123-synE-NKG2D-BBZ fragment, from the 5' to 3' direction, sequentially comprises UAS-CMV, an NKG2D-BBZ fragment, an EF1a promoter, a CD123-synE fragment, a 2A peptide, and the A-N1 fragment. The A-N2/CD123-synE-NKG2Dz fragment, from the 5' to 3' direction, sequentially comprises UAS-CMV, an NKG2Dz fragment, an EF1a promoter, a CD123-synE fragment, a 2A peptide, and the A-N2 fragment. The A-N2/CD123-synE-NKG2D-BBZ fragment, from the 5' to 3' direction, sequentially comprises UAS-CMV, an NKG2D-BBZ fragment, a EF1a promoter, a CD123-synE fragment, a 2A peptide, and the A-N2 fragment. For example, the 2A peptide is P2A, T2A, E2A, or F2A.

The regulatory NKG2D-CAR-T cells disclosed herein also comprise constitutively expressed a second chimeric receptor. For example, engineered cells comprising a CD123-synE-NKG2Dz fragment and any of the second chimeric receptors described in Table 5. For example, the engineered cells comprise: a CD123-synE-NKG2Dz fragment and an A-N1 fragment, a CD123-synE-NKG2Dz fragment and an A-N2 fragment, a CD123-synE-NKG2Dz fragment and an A-N3 fragment, a CD123-synE-NKG2Dz fragment and an A-N4 fragment, a CD123-synE-NKG2Dz fragment and a CD137L-N fragment, a CD123-synE-NKG2Dz fragment and a CD40L-N fragment, a CD123-synE-NKG2Dz fragment and a CD137L-N1 fragment, a CD123-synE-NKG2Dz fragment and a CD40L-N1 fragment, a CD123-synE-NKG2Dz fragment and a CD137L-N2 fragment, or a CD123-synE-NKG2Dz fragment and a CD40L-N2 fragment. For example, the CD123-synE-NKG2Dz fragment and the second chimeric receptor are constructed on a single vector. For example, the CD123-synE-NKG2Dz fragment and the second chimeric receptor are connected by the coding sequence for a 2A peptide. For example, from the 5' end to the 3' end, it sequentially comprises the CD123-synE-NKG2Dz fragment, the coding sequence for the 2A peptide, and the second chimeric receptor. For example, the 2A peptide is P2A, T2A, E2A, or F2A.

For example, the engineered cells comprise: a CD123-synE-NKG2D-BBZ fragment and an A-N1 fragment, a CD123-synE-NKG2D-BBZ fragment and an A-N2 fragment, a CD123-synE-NKG2D-BBZ fragment and an A-N3 fragment, a CD123-synE-NKG2D-BBZ fragment and an A-N4 fragment, a CD123-synE-NKG2D-BBZ fragment and a CD137L-N fragment, a CD123-synE-NKG2D-BBZ fragment and a CD40L-N fragment, a CD123-synE-NKG2D-BBZ fragment and a CD137L-N1 fragment, a CD123-synE-NKG2D-BBZ fragment and a CD40L-N1 fragment, a CD123-synE-NKG2D-BBZ fragment and a CD137L-N2 fragment, or a CD123-synE-NKG2D-BBZ fragment and a CD40L-N2 fragment. For example, the CD123-synE-NKG2D-BBZ fragment and the second chimeric receptor are connected by a coding sequence for a 2A peptide.For example, from the 5 ' end to the 3 ' end, sequentially comprise, CD123-synE-NKG2D-BBZ fragment, the coding sequence for a 2A peptide, and the second chimeric receptor. For example, the 2A peptide is P2A, T2A, E2A or F2A.

### Bispecific CAR that binds to GPRCSD and BCMA, and bispecific CAR that binds to GPRC5D and NKG2A

The present application discloses a bispecific CAR targeting GPRC5D and another antigen, and an engineered cell comprising the bispecific CAR. For example, the another antigen is a second antigen expressed on or associated with multiple myeloma. For example, the another antigen comprises: BCMA, CD3, CD19, CD20, CD22, CD123, CD38, CD138, CS-1, BAFF-R, TACI and FcRH5. For example, the another antigen is an immune cell marker. For example, the another antigen is any one of the T cell or NK cell markers disclosed in the present application. For example, the another antigen is NKG2A, CD38, TIGIT, FasL, CD94, CD300A or a combination thereof.

The present application provides immune cells that can recognize GPRC5D and a second antigen to enhance the killing effect of immune cells on tumor cells such as multiple myeloma cells or enhance their anti-immune rejection effect.

The present application discloses bispecific CARs targeting GPRC5D and another antigen, comprising those as shown in Table 6. CARs targeting GPRC5D or BCMA, comprising those as shown in Table 7.

**Table 6 Bispecific chimeric receptors targeting GPRC5D and BCMA, targeting GPRC5D and NKG2A**

| Vector | Structure (from N-terminal to C-terminal) |
|---|---|
| BLG-sBBZ | αBCMA_VL-(L) -αGPRC5D_VH-(L)-αGPRC5D_VL-(L) |
| | -αBCMA_VH-IgG4(H)-CD28(TM)-CD137(C)-CD3ζ |
| BLG-BBZ | αBCMA_VL-(L) -αGPRC5D_VH-(L)-αGPRC5D_VL-(L) |
| | -αBCMA_VH-CD8(H)-CD8(TM)-CD137(C)-CD3ζ |
| GLB-sBBZ | αGPRC5D_VL-(L) -αBCMA _VH-(L)-αBCMA_VL-αGPRC5D_VH-IgG4(H)-CD28(TM)-CD 137(C)-CD3ζ |
| GLB-BBZ | αGPRC5D_VL-(L) |
| | -αBCMA_VH-(L)-αBCMA_VL-αGPRC5D_VH-CD8(H)-CD8(TM)-CD137(C) -CD3ζ |
| GLN-BBZ | αGPRC5D_VL-(L) |
| | -αNKG2A_VH-(L)-αNKG2A_VL-αGPRC5D_VH-CD8(H)-CD8(TM)-CD137( C)-CD3ζ |

**Table 7 Chimeric receptors targeting GPRC5D or BCMA**

| Vector | Structure (from N-terminal to C-terminal) |
|---|---|
| BCMA-CAR | αBCMA_VH-(L)-αBCMA_1VL- CD8(H)-CD8(TM)-CD137(C)-CD3ζ |
| GPRC5D-CAR | αGPRC5D_VH-(L)-αGPRC5D_1VL-CD8(H)-CD8(TM)-CD137(C)-CD3ζ |

Tables 6 and 7: αGPRC5D_VH: GPRC5D antibody VH (SEQ ID NO: 99); αGPRC5D_VL: GPRC5D antibody VL (SEQ ID NO: 102); αBCMA_VH: BCMA antibody VH (SEQ ID NO: 100); αBCMA_VL: BCMA antibody VL (SEQ ID NO: 101); αNKG2A_VH: NKG2A antibody VH (SEQ ID NO: 116); αNKG2A _VL: NKG2A antibody VL (SEQ ID NO: 117); (L): linker peptide (SEQ ID NO: 14, 15, or 16); CD8(H): CD8 hinge (SEQ ID NO: 3); CD8(TM): CD8 transmembrane domain (SEQ ID NO: 7); CD28(TM): CD28 transmembrane domain (SEQ ID NO: 9); CD28(C): CD28 intracellular domain (SEQ ID NO: 10); CD137 (C): CD137 intracellular domain (SEQ ID NO: 11); CD3ζ: CD3ζ intracellular domain (SEQ ID NO: 12); IgG4 (H): IgG4-spacer (SEQ ID NO: 5).

The present application provides bispecific chimeric receptors: BCMA-CAR and GPRC5D-CAR as shown in Table 7, or BLG-sBBZ, BLG-BBZ, GLB-sBBZ, GLB-BBZ, GLN-BBZ as shown in Table 6, or GPRC5D-CAR as shown in Table 7 and NKG2A-s28z or NKG2A-28z as shown in Table 3.

The chimeric receptor targeting GPRC5D provided by the present invention comprises a GPRC5D binding domain. For example, the GPRC5D binding domain comprises a heavy chain variable region VH and a light chain variable region VL. For example, GPRC5D VH and VL comprise the amino acid sequences shown in SEQ ID NOs: 99 and 102, respectively, or amino acid sequences having at least about 80%, 85%, 90%, 95%, 96%, 97%, 98%, 99%, or 100% identity thereto. For example, the CDR1, CDR2, and CDR3 comprised in the VH comprise the amino acid sequences shown in SEQ ID NOs: 104, 105, and 106, respectively; the CDR1, CDR2, and CDR3 comprised in the VL comprise the amino acid sequences shown in SEQ ID NOs: 107, 108, and 109, respectively. For example, the GPRC5D scFv comprises an amino acid sequence shown in SEQ ID NO: 103, or an amino acid sequence having at least about 80%, 85%, 90%, 95%, 96%, 97%, 98%, 99%, or 100% identity thereto.

In one example, the bispecific CAR targeting GPRC5D further comprises a BCMA binding domain. For example, the BCMA binding domain comprises VH and VL, comprising an amino acid sequence shown in SEQ ID NOs: 100, and 101, respectively, or an amino acid sequence having at least about 80%, 85%, 90%, 95%, 96%, 97%, 98%, 99%, or 100% identity thereto. For example, the CDR1, CDR2, and CDR3 comprised in the VH comprise the amino acid sequences shown in SEQ ID NOs: 110, 111, and 112, respectively; the CDR1, CDR2, and CDR3 comprised in the VL comprise the amino acid sequences shown in SEQ ID NOs: 113, 114, and 115, respectively.

In one embodiment, the bispecific CAR targeting GPRC5D further comprises an NKG2A binding domain. For example, the NKG2A binding domain comprises VH and VL, comprising an amino acid sequence shown in SEQ ID NOs: 116 and 117, respectively, or an amino acid sequence having at least about 80%, 85%, 90%, 95%, 96%, 97%, 98%, 99%, or 100% identity thereto.

The present application discloses a bispecific chimeric receptor targeting GPRC5D and BCMA and an immune cell thereof, comprising: (1) an anti-GPRC5D antibody or a fragment thereof comprising a heavy chain variable region (VH1) and a light chain variable region (VL1): the HCDR1, HCDR2, HCDR3, LCDR1, LCDR2, and LCDR3, comprising the amino acid sequences shown in SEQ ID NOs: 104, 105, 106, 107, 108, and 109, respectively; (2) an anti-BCMA antibody or a fragment thereof comprising a heavy chain variable region (VH2) and a light chain variable region (VL2): the HCDR1, HCDR2, HCDR3, LCDR1, LCDR2, and LCDR3, comprising the amino acid sequences shown in SEQ ID NOs: 110, 111, 112, 113, 114, and 115, respectively. For example, the immune cell comprises a chimeric receptor targeting GPRC5D and a chimeric receptor targeting BCMA. For example, the immune cell comprises a chimeric receptor that simultaneously targets GPRC5D and BCMA. For example, the immune cell comprises a nucleic acid sequence having at least about 60%, 65%, 70%, 75%, 80%, 85%, 90%, 95%, 99% or 100% homology or identity to the nucleic acid sequence shown in SEQ ID NO: 90, 91, 92, 93, 94, or 95, or an amino acid sequence having at least about 60%, 65%, 70%, 75%, 80%, 85%, 90%, 95%, 99% or 100% homology or identity to the amino acid sequence encoded thereby.

The present application discloses a bispecific chimeric receptor targeting GPRC5D and NKG2A and an immune cell thereof, comprising: (1) an anti-GPRC5D antibody or a fragment thereof comprising a heavy chain variable region (VH1) and a light chain variable region (VL1): the CDR1, CDR2, and CDR3 comprised in the VH1 comprise the amino acid sequences shown in SEQ ID NOs: 104, 105, and 106, respectively; the CDR1, CDR2, and CDR3 comprised in the VL1 comprise the amino acid sequences shown in SEQ ID NOs: 107, 108, and 109, respectively; (2) an anti-NKG2A antibody or a fragment thereof comprising a heavy chain variable region (VH3) and a light chain variable region (VL3), comprising an amino acid sequences having at least about 80%, 85%, 90%, 95%, 96%, 97%, 98%, 99%, and 100% identity to the amino acid sequences shown in SEQ ID NOs: 116 and 117, respectively. For example, the immune cell comprises a chimeric receptor targeting GPRC5D and a chimeric receptor targeting NKG2A. For example, the immune cell comprises a chimeric receptor that targets both GPRC5D and NKG2A. For example, the immune cell comprises an amino acid sequence having at least about 60%, 65%, 70%, 75%, 80%, 85%, 90%, 95%, 99% or 100% homology or identity to the amino acid sequence shown in SEQ ID NO: 84.

The transmembrane domain of the bispecific chimeric receptor targeting GPRC5D and BCMA, or targeting GPRC5D and NKG2A, can be any transmembrane domain disclosed herein. For example, a CD8 or a CD28 transmembrane domain. The hinge of the bispecific chimeric receptor targeting GPRC5D and BCMA, or targeting GPRC5D and NKG2A, can be any hinge disclosed herein. For example, a CD8 hinge, a CD28 hinge, or an IgG4 hinge. The intracellular signaling domain of the chimeric receptor comprises one or more costimulatory signaling domains and/or primary signaling domains; the primary signaling domain is selected from: TCRξ, FcRy, FcRβ, CD3γ, CD3δ, CD3ε, CD5, CD22, CD79a, CD79b, ICOS, CD66d, or CD3ζ; and/or the costimulatory signaling molecule is selected from: the intracellular domain of CD27, CD28, 4-1BB, OX40, CD30, CD40, PD-1, ICOS, CD2, CD5, ICAM-1, LFA-1 (CD11a/CD18), 4-1BBL, MyD88 or a combination thereof. For example, the intracellular domain comprises a costimulatory signal 4-1BB intracellular signaling domain and a primary signal CD3ζ intracellular activation domain.

Compared to CARs targeting a single antigen, affinity is enhanced, T cell activity is improved, and these targets have additive or synergistic effects. CAR-T cells that simultaneously target BCMA and GPRC5D on the surface of tumor cells can reduce the possibility of antigen escape caused by downregulation or loss of a single surface antigen. CAR-T cells that simultaneously target NKG2A and GPRC5D can provide CAR-T cells with anti-immune rejection capability. The antibodies against GPRC5D and BCMA are connected in tandem. The antibodies against GPRC5D and NKG2A are connected in tandem. For example, the GPRC5D antibody VH1 and GPRC5D antibody VL1 are connected to form an antibody scFv1, and the BCMA antibody VH2 and BCMA antibody VL2 are connected to both ends of the antibody scFv1, respectively. For example, the GPRC5D antibody VH1 and VL1 are connected to form an antibody scFv1, and the NKG2A antibody VH3 and VL3 are connected to both ends of the antibody scFv1, respectively. For example, the BCMA antibody VH2 and VL2 are connected to form an antibody scFv2, and the GPRC5D antibody VH1 and VL1 are connected to both ends of the antibody scFv2, respectively. For example, the NKG2A antibody VH3 and VL3 are connected to form an antibody scFv3, and the GPRC5D antibody VH1 and VL1 are connected to both ends of the antibody scFv3, respectively. The antigen binding domains of the chimeric receptors can have different conformations.

For example, the antigen binding domains comprise the GPRC5D antibody VH1 and VL1, and the BCMA antibody VH2 and VL2; from N-terminus to C-terminus, VH1-VL1-VH2-VL2, VL1-VH1-VL2-VH2, VH1-VL1-VL2-VH2, VL1-VH1-VH2-VL2, VH1-VH2-VL2-VL1, VL1-VH2-VL2-VH1, VH1-VL2-VH2-VL1, VL1-VL2-VH2-VH1, VH2-VH1-VL1-VL2, VL2-VH1-VL1-VH2, VH2-VL1-VH1-VL2, or VL2-VL1-VH1-VH2.

For example, the antigen binding domains comprise the GPRC5D antibody VH1 and VL1, and the NKG2A antibody VH3 and VL3; from N-terminus to C-terminus, VH1-VL1-VH3-VL3, VL1-VH1-VL3-VH3, VH1-VL1-VL3-VH3, VL1-VH1-VH3-VL3, VH1-VH3-VL3-VL1, VL1-VH3-VL3-VH1, VH1-VL3-VH3-VL1, VL1-VL3-VH3-VH1, VH3-VH1-VL1-VL3, VL3-VH1-VL1-VH3, VH3-VL1-VH1-VL3, or VL3-VL1-VH1-VH3.

It is well known in the art that the sequences of VH and VL regions can undergo some amino acid substitutions while retaining their affinity for the target in the case while the CDR sequence remains unchanged; and the CDR sequences can also undergo some amino acid substitutions while retaining its affinity for the target in the case while keeping the amino acids in contact with the target unchanged.

In one embodiment, the anti-GPRC5D or BCMA or NKG2A antibody or a fragment thereof is an scFv. The VH (heavy chain variable region) and VL (light chain variable region) in the scFv can be connected by a linker peptide chain (linker), and the positions can be interchanged. For example, the anti-GPRC5D or BCMA or NKG2A antibody comprises, from the N-terminus to the C-terminus, VH, a linker peptide chain, and VL. For example, the anti-GPRC5D or BCMA or NKG2A antibody comprises, from the N-terminus to the C-terminus, VL, a linker peptide chain, and VH. Any linker peptide chain that connects VH and VL together to form a functionally complete single-chain antibody is applicable. For example, the linker peptide chain is a GS linker peptide chain, for example, GGGGS, (GGGGS)3, or (GGGGS)4.

In one example, the chimeric receptor provided by the present invention comprises (1) a domain comprising an anti-GPRC5D antibody (VH/VL selected from SEQ ID NO: 99, and 102), an anti-BCMA antibody (VH/VL selected from SEQ ID NO: 100, and 101), and/or an anti-NKG2A antibody (VH/VL selected from SEQ ID NO: 116, and 117), (2) a CD8 hinge region (SEQ ID NO: 3), or an IgG4 spacer fragment (SEQ ID NO: 5 or 6), (3) a CD28 transmembrane domain (SEQ ID NO: 8 or 9) or a CD8 transmembrane domain (SEQ ID NO: 7), (4) a CD28 intracellular signaling domain (SEQ ID NO: 10) or a CD137 intracellular signaling domain (SEQ ID NO: 11); optionally, further comprising (5) a CD3ζ intracellular signaling domain (SEQ ID NO: 12 or 13).

The present invention provides a chimeric receptor comprising a nucleic acid sequence having at least about 60%, 65%, 70%, 75%, 80%, 85%, 90%, 95%, 99% or 100% homology or identity to the nucleic acid sequences shown in SEQ ID NO: 90, 91, 92, 93, 94, or 95, or an amino acid sequence having at least about 60%, 65%, 70%, 75%, 80%, 85%, 90%, 95%, 99% or 100% homology or identity to the amino acid sequence encoded thereby. The present invention provides a chimeric receptor comprising an amino acid sequence having at least about 60%, 65%, 70%, 75%, 80%, 85%, 90%, 95%, 99% or 100% homology or identity to the amino acid sequence shown in SEQ ID NO: 84 or 96.

The present invention provides a engineered cell comprising: a chimeric receptor 1 that binds to GPRC5D, comprising an amino acid sequence having at least about 60%, 65%, 70%, 75%, 80%, 85%, 90%, 95%, 99% or 100% homology or identity to the amino acid sequence shown in SEQ ID NO: 84 or 96; and optionally, further comprising a chimeric receptor 2, comprising an amino acid sequence having at least about 60%, 65%, 70%, 75%, 80%, 85%, 90%, 95%, 99% or 100% homology or identity to the amino acid sequence shown in SEQ ID NO: 24, 26, 27, 28, 83, 85, 86, 96 or 98. For example, the engineered cell comprises: a chimeric receptor 1 that binds to GPRC5D, comprising an amino acid sequence having at least about 60%, 65%, 70%, 75%, 80%, 85%, 90%, 95%, 99% or 100% homology or identity to the amino acid sequence shown in SEQ ID NO: 84 or 96; further comprising a chimeric receptor 2 comprising an amino acid sequence having at least about 60%, 65%, 70%, 75%, 80%, 85%, 90%, 95%, 99% or 100% homology or identity to the amino acid sequence encoded by the nucleic acid sequence shown in SEQ ID NO: 4, 18, 23, 25, 29, 30, 31, 32, 33, 34, 35, 36, 37, 38, 78, or 79. For example, the engineered cell comprises: a chimeric receptor 1 that binds to GPRC5D, comprising an amino acid sequence having at least about 60%, 65%, 70%, 75%, 80%, 85%, 90%, 95%, 99% or 100% homology or identity to the amino acid sequence shown in SEQ ID NO: 97; further comprising a chimeric receptor 2 comprising an amino acid sequence having at least about 60%, 65%, 70%, 75%, 80%, 85%, 90%, 95%, 99% or 100% homology or identity to the amino acid sequence shown in SEQ ID NO: 24, 26, 27, 28, 83, 85, 86, 96 or 98. For example, the engineered cell comprises: a chimeric receptor 1 that binds to GPRC5D, comprising an amino acid sequence having at least about 60%, 65%, 70%, 75%, 80%, 85%, 90%, 95%, 99%, or 100% homology or identity to the amino acid sequence shown in SEQ ID NO: 97; further comprising a chimeric receptor 2, comprising an amino acid sequence having at least about 60%, 65%, 70%, 75%, 80%, 85%, 90%, 95%, 99%, or 100% homology or identity to the amino acid sequence encoded by the nucleic acid sequence shown in SEQ ID NO: 4, 18, 23, 25, 29, 30, 31, 32, 33, 34, 35, 36, 37, 38, 78, or 79. In one example, the chimeric receptor 1 and chimeric receptor 2 are constructed on a single vector. For example, the polypeptide fragment, from the N-terminus to the C-terminus, sequentially comprises chimeric receptor 2, a 2A peptide, and chimeric receptor 1. For example, the 2A peptide is P2A, T2A, E2A, or F2A. For example, the chimeric receptor 1 and chimeric receptor 2 are connected by a cleavable peptide. For example, the polypeptide fragment, from the N-terminus to the C-terminus, sequentially comprises chimeric receptor 1, a 2A peptide, and chimeric receptor 2. For example, the chimeric receptor 1 and chimeric receptor 2 are constructed on different vectors, respectively.

### Advantages of the Invention

Through the design and screening of various structures, the present invention has screened structures that can effectively block the expression of the CD38 antigen on engineered cells, thereby reducing the phenomenon of fratricidal during *in vitro* and *in vivo* culture of engineered cells comprising chimeric receptors targeting CD38, prolonging the survival of the engineered cells *in vivo* and *in vitro,* and improving their expansion capacity. This can greatly enhance the anti-tumor and anti-immune transplantation effects of CD38-CAR-T cells.

Through the design and screening of various structures, the present invention has screened structures that can effectively block the expression of NKG2DL on engineered cells, thereby reducing the phenomenon of fratricidal during *in vitro* and *in vivo* culture of engineered cells comprising chimeric receptors targeting NKG2DL, prolonging the survival of the engineered cells *in vivo* and *in vitro,* and improving their expansion capacity. This can greatly enhance the anti-tumor and anti-immune transplantation effects of NKG2D-CAR-T cells that recognize NKG2DL.

The present invention discloses CARs that simultaneously target GPRC5D and BCMA; and CARs that simultaneously target GPRC5D and NKG2A. Compared to CARs targeting a single antigen, affinity is enhanced, T cell activity is improved, and these targets have additive or synergistic effects. CAR-T cells that simultaneously target BCMA and GPRC5D on the surface of tumor cells can reduce the possibility of antigen escape caused by downregulation or loss of a single surface antigen. CAR-T cells that simultaneously target the NK cell marker NKG2A and GPRC5D can enhance the anti-immune rejection capability of GPRC5D-CAR-T cells.

The present application is further described below in conjunction with specific examples. It should be understood that these examples are only used to illustrate the present application and not to limit the scope of the present application. The experimental methods without specific conditions noted in the following examples are generally carried out under conventional conditions, such as the conditions described in J. Sambrook et al., Molecular Cloning Laboratory Manual, 3rd edition, Science Press, 2002*,* or according to the conditions recommended by the manufacturer. All publications, patents, and patent applications mentioned in this specification are incorporated herein by reference, as if each individual publication, patent, or patent application was specifically and individually indicated to be incorporated herein by reference.

### Examples

### Example 1. Biological Materials

MM.1S (Cell Bank of Chinese Academy of Sciences, SCSP-5017), THP-1 (Cell Bank of Chinese Academy of Sciences, SCSP-567), and MV-4-11 (Cell Bank of Chinese Academy of Sciences, SCSP-5031) cells were cultured and expanded in vitro, and transfected with lentivirus overexpressing Luciferase-GFP using conventional cell biology techniques to prepare MM.1S-luci-gfp, THP-1-luci-gfp, and MV-4-11-luci-gfp cells. U251 cells were obtained from the Chinese Academy of Sciences Cell Bank, TCHu58. BxPC3 cells were obtained from the ATCC Cell Bank, CRL-1687. MM.1S cells were infected with lentivirus overexpressing Luciferase-GFP and mCherry to prepare MM.1S-GFP/mCherry cells. MM.1S-GFP-GPRC5D KO with endogenous GPRC5D knockout (also known as MM.1S-GPRC5D KO) and MM.1S-mCherry-BCMA KO cells with endogenous BCMA knockout (also known as MM.1S-BCMAKO) were constructed using CRISPR editing technology.

The multiple myeloma cell lines MM.1S, NCI-H929 (ATCC, CRL-9068), RPMI-8226 (Cell Bank of Chinese Academy of Sciences (Shanghai), TCHu234) and the stably GFP-transfected monoclonal cell line MM1S-GFP were analyzed by flow cytometry, and it shows that the expression of BCMA in MM.1S, NCI-H929, RPMI-8226, and MM.1S-GFP cells was approximately 80%, 78%, 90%, and 83%, respectively; and the expression of GPRC5D was approximately 78%, 54%, 38%, and 84%, respectively.

rNK (Resting NK cells): PBMCs were isolated from peripheral blood of healthy human donors, and rNK cells were obtained from PBMCs by negative selection of NK cells (Miltenyi Biotec, 130-092-657). rNK cells were activated and expanded in vitro with cytokines (500 IU/mL IL-2 and 150 IU/mL IL-15) to obtain aNK cells (activated NK cells). NPG mice were obtained from Beijing Vitalstar Biotechnology Co., Ltd., genotype NOD.Cg-Prkdcscid Il2rgtm1VstNst.

### Example 2. Preparation of Vectors

Vectors expressing the chimeric receptor disclosed in this application were constructed using conventional molecular biology techniques. Full-length DNA was synthesized to construct a CAR targeting CD38, which was inserted between the MluI and SalI restriction endonuclease sites of the pRRLSIN lentiviral vector (Addgene) for lentiviral packaging. CD38-CAR1, 2, 3, and 4, comprise CD38-BBZ as shown in Table 1 which was constructed with CD38 antibody 1, CD38 antibody 2, CD38 antibody 3, and CD38 antibody 4, respectively. CD38-CAR9 comprises CD38-28Z as shown in Table 1 which was constructed with CD38 antibody 1. CD38-CAR6, CD38-CAR10, CD38-CAR2s, and CD38-CAR3s comprise the sequences shown in SEQ ID NOs: 23, 25, 18, and 29, respectively; and they were respectively integrated into the pRRLSIN vector (Addgene). The corresponding lentivirus of the above expression vectors was prepared using conventional molecular biology techniques.

### Example 3. Preparation of CAR-T cells

CAR-T cells were prepared using conventional methods known in the art: donor PBMCs were collected, and activated with anti-CD3 and CD28 antibody magnetic beads (Life Technologies, 40203D), and then infected with lentivirus comprising the aforementioned vectors, CD38-CAR1, 2, 3, 4, 6, 9, 10, 2s, and 3s, to prepare CD38-CAR-T1, CD38-CAR-T2, CD38-CAR-T3, CD38-CAR-T4, CD38-CAR-T6, CD38-CAR-T9, CD38-CAR-T10, CD38-CAR-T2s, and CD38-CAR-T3s cells expressing the corresponding chimeric receptors. T cells not transfected with virus were UTD. CAR positive rate was detected by FACS.

### Example 4. Detection of CD38 expression on T, B, and NK cells in PBMCs from different human donors

PBMCs isolated from peripheral blood of different healthy human donors were collected and divided into two groups for staining: the control group was labeled with CD3-percp, CD19 APC-cy7, and CD56 APC; the experimental group (anti-CD38) was stained with CD3-percp, CD19 APC-cy7, CD56 APC, and CD38 BV421; the cells were incubated at 4°C for 30 minutes, and detected by flow cytometry. CD3+, CD19+, and CD56+ cell populations were separated respectively, and CD38 expression was detected. The results showed that CD38 was expressed on T, B, and NK cells.

### Example 5. Detection of the positive rate of CD38-CAR-T and the expression of CD38 on CAR-T cells

CD38-CAR-T cells were cultured *in vitro* for 7 days, washed twice with PBS + 1% FBS, and divided equally into two portions: one was detected for positive rate using Biotin-anti-huF(ab)2 and Streptavidin-PE; and the other was co-incubated with anti-CD38 BV421 to detect CD38 expression. Negative control N.C was T cells not labeled with any antibody.

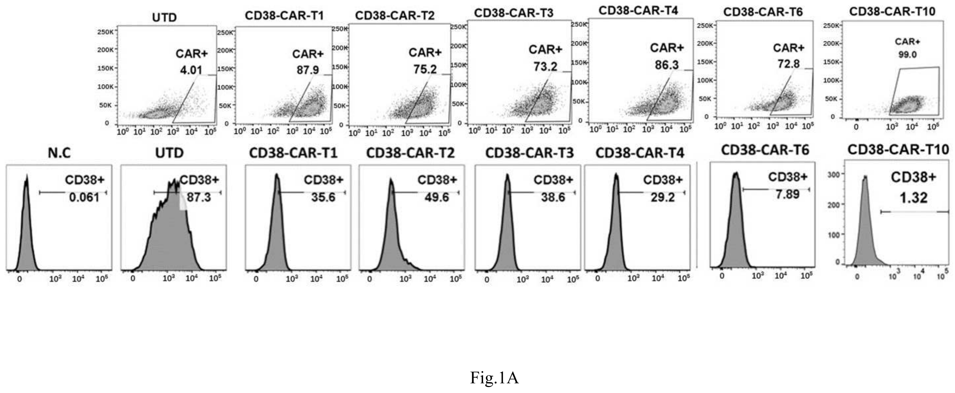

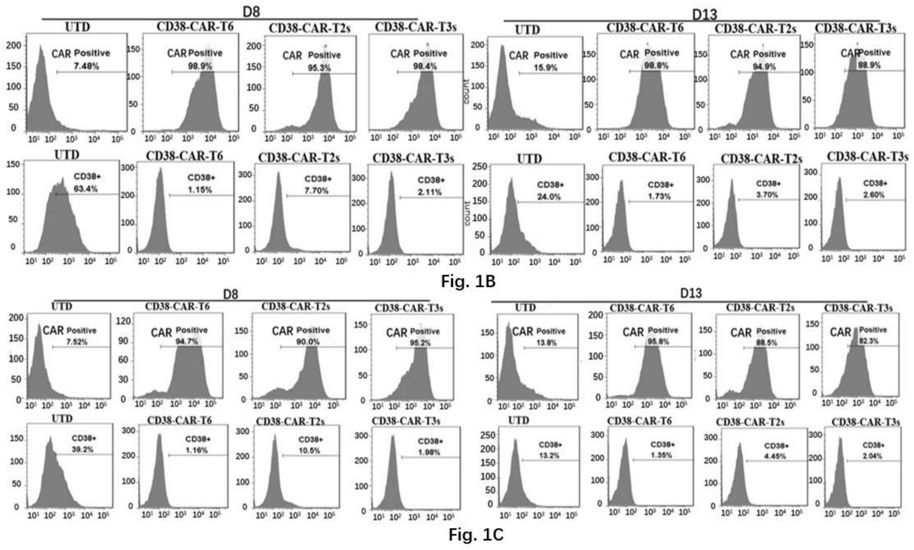

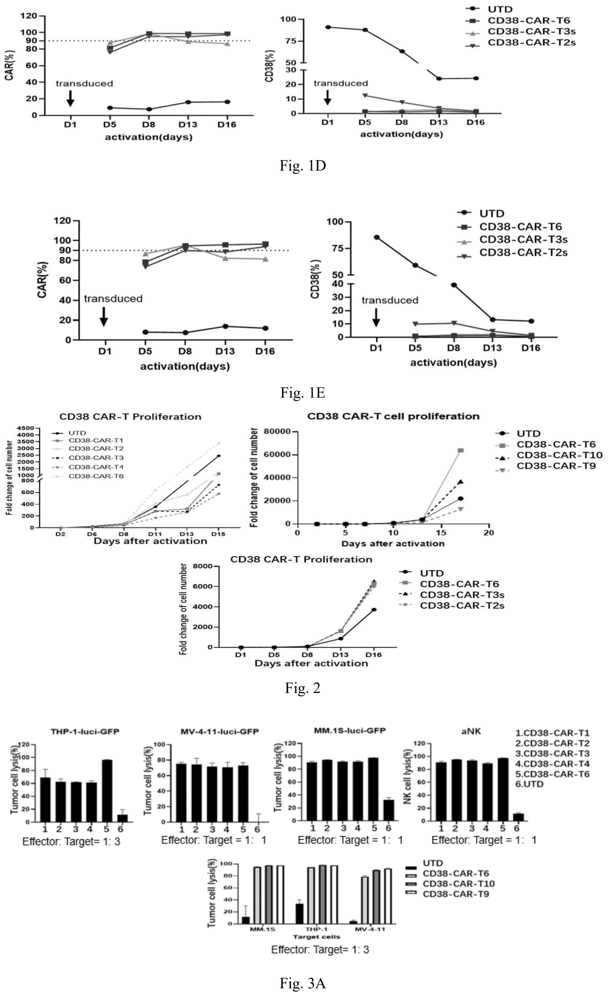

Figure 1A (top) shows that the CAR positive rates of CD38-CAR-T1, 2, 3, 4, 6, and 10 cells were generally above 70%. Figure 1A (bottom) shows that the CD38 positive rate of CD38-CAR-T cells was significantly lower than that of UTD, with CD38-CAR-T6 and 10 showing the most significant reduction. Figure 1B (bottom) shows that, similar to CD38-CAR-T6 constructed with CD38 antibody 1, CD38-sBBZ constructed with CD38 antibody 2 or 3 also significantly blocked the CD38 antigen on its own T cells on D8 or D13. Figure 1D (right) shows that CD38-sBBZ constructed with CD38 antibody 1 and 3 almost completely blocked the CD38 antigen from D5, and CD38-sBBZ constructed with CD38 antibody 2 also almost completely blocked the CD38 antigen from D13.

The above experiment was repeated using PBMCs from another healthy human donor. Figure 1C (lower panel) and Figure 1E (right panel) show that CD38-sBBZ constructed with CD38 antibody 1 and 3 could almost completely block the CD38 antigen from D5, and CD38-sBBZ constructed with CD38 antibody 2 could also almost completely block the CD38 antigen from D13.

### Example 6. Detection of CD38-CAR-T cell proliferation in vitro

48 hours after Human peripheral blood PBMCs were stimulated and activated with CD3/CD28 magnetic beads for 48 hours (recorded as D2), an equal number of T cells were infected with virus comprising CD38-CAR to prepare CD38-CAR-T cells, cultured *in vitro* and counted every 2-3 days. After each counting, 2e5 cells were centrifuged, resuspended at 2e5/mL and seeded into culture plates for continuing culture and counting. Figure 2 (left) shows that starting from D8, the expansion fold of CD38-CAR-T6 cells increased significantly and maintained a high expansion capacity. Figure 2 (right) shows that by D10, the expansion capacity of CD38-CAR-T6 and CD38-CAR-T10 cells was significantly higher than that of CD38-CAR-T9. Figure 2 (bottom) shows that starting from D13, the expansion fold of CD38-sBBZ cells constructed with CD38 antibody 1, 2, or 3 began to increase significantly.

### Example 7. Flow cytometry detection of killing on tumor cells or NK cells and cytokine secretion by CD38-CAR-T cells

CAR-T cells and target cells (THP-1, MV-4-11, MM.1S, aNK cells) were co-cultured at an effector-target ratio of 1:1 or 1:3 for 20 hours. T cells were labeled with anti-CD3-PE, dead cells were stained with 7-AAD, and finally counting beads (Invitrogen) were added. Data was read using FACS, and the number of target cells and T cells were analyzed and counted. The cytotoxic lysis rate was calculated according to the formula: cell lysis% = (1 - experimental group/control group) × 100%. Figure 3A shows that CD38-CAR-T 1, 2, 3, 4, 6, 9, and 10 significantly killed THP-1, MV-4-11, MM.1S, and aNK cells.

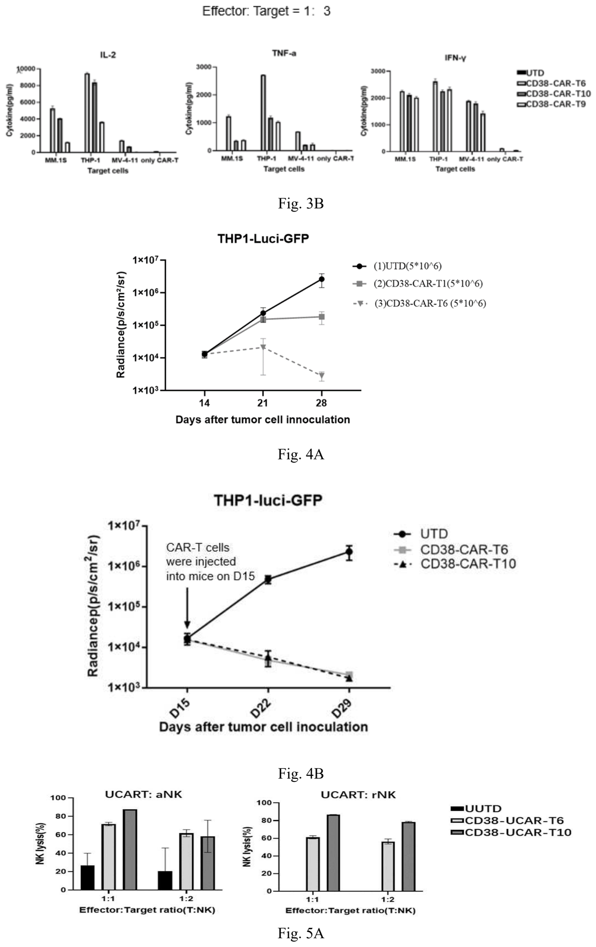

The supernatants were collected after CD38-CAR-T6, 9, and 10 cells were co-cultured with MM.1S, THP-1, or MV-4-11 for 18 hours, and the secretion of IL-2, TNF-α, and IFN-γ cytokines was detected using the CBA method (BD, 558264). As shown in Figure 3B, compared with UTD, the above-mentioned CAR-T cells secreted higher levels of cytokines after co-incubation with tumor cells.

### Example 8. CD38-CAR-T treatment of orthotopic tumors in NPG mice

NPG mice were injected via tail vein with 1 × 10 ⁷ THP-1-Luci cells (denoted as D0), and divided into 3 groups, with 5 mice per group. Tumor photon intensity was observed at various time points using fluorescence imaging (IVIS LUMINA III SYSTEM). On D14 (when the photon intensity reached an average of approximately 1.5×10⁴ p/s/cm ² /sr), 5×10 ⁶ CAR-T cells/mouse were injected via tail vein. As shown in Figure 4A, on D42, one mouse died in the UTD group, while the CD38-CAR-T6 group showed significant tumor suppression, with all mice surviving and in good condition.

NPG mice were injected via tail vein with 1 × 10 ⁷ THP-1-Luci cells (denoted as D0), and divided into 3 groups, with 5 mice per group. Tumor photon intensity was observed at various time points using fluorescence imaging (IVIS LUMINA III SYSTEM). On D15 (when the photon intensity reached an average of approximately 1.5×10 ⁴ p/s/cm ² /sr), 4×10 ⁶ CAR-T cells/mouse were injected via tail vein. As shown in Figure 4B, on D36, both the CD38-CAR-T6 and 10 groups showed significant tumor suppression compared with the control group.

### Example 9. Construction of CD38-UCAR-T cells

sgRNA sequences targeting TRAC or B2M (shown in SEQ ID NO: 39, 40, respectively) (Kaixing Diagnostics) were synthesized *in vitro,* and UCAR-T (or dko) cells with both TRAC and B2M knockout were prepared using CRISPR/Cas9 technology (Cas9 protein, Kactus Biosystems (Shanghai), Cat#CAS-EE109). UTD cells that were not transduced but with TCR/B2M knockout (UUTD) were used as control. The positive rate of CAR expression was detected using Biotin-anti-huF(ab)2 and Streptavidin-PE. The results showed that CAR molecules were successfully expressed in all T cells groups .

### Example 10. Detection of Killing on NK Cells and Cytokine Secretion by CD38-UCAR-T cell

CD38-UCAR-T cells were plated with NK cells at a ratio of 1:1 or 1:2, with 20,000 CD38-UCAR-T cells per well, co-incubated for 24 hours, and stained with anti-HLA-ABC-APC (Thermo, 17-9983-42). HLA-ABC-negative cells were CD38-UCAR-T cells, while HLA-ABC-positive cells were NK cells. Dead cells were labeled with 7-AAD (BD, 559925), and finally counting beads (Invitrogen, C36995) were added. The result was analyzed by FACS. The cytotoxic lysis rate was calculated according to the formula: Specific cell lysis % = (1 - experimental group / control group) × 100%. As shown in Figure 5A, after 24 hours of co-cultured with NK cells, CD38-UCAR-T6 and 10 cells significantly killed both resting and activated NK cells.

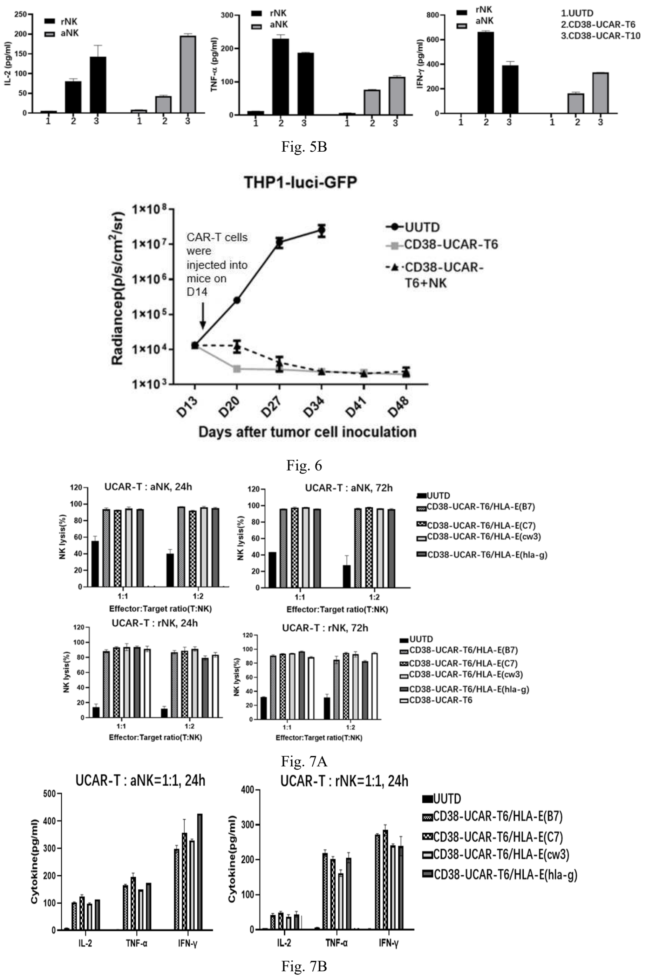

The supernatant was collected after CD38 UCAR-T cells were co-cultured with NK cells at a ratio of 1:1 for 24 hours, and the secretion of IL-2, TNF-α, and IFN-γ cytokines was detected using the CBA method (BD, 558264). As shown in Figure 5B, compared with UUTD, CD38 UCAR-T cells secreted higher levels of cytokines after co-incubation with NK cells.

### Example 11. CD38-UCAR-T cells treatment of orthotopic tumors in NPG mice

NPG mice were injected via tail vein with 1 × 10 ⁷ THP-1-Luci cells/mouse, and divided into 3 groups, 5 mice per group. Tumor photon intensity was observed at various time points using fluorescence imaging (IVIS LUMINA III SYSTEM). In the NK cell injection group, on D13 (when the photon intensity reached an average of approximately 1.5×10 ⁴ p/s/cm ² /sr), 2×10 ⁶ NK cells/mouse were injected via tail vein for 3 consecutive days. On D14, 5×10 ⁶ UCAR-T cells were injected via tail vein. Figure 6 shows that in the presence of NK cells, the CD38-UCAR-T6 group still exhibited a significant tumor suppression effect. By D48, all mice survived and were in good condition, while all mice in the control group died by D48.

### Example 12. Preparation of CD38-HLA-E UCAR-T cells

CD38-HLA-E UCAR-T1, 2, 3, and 4 cells with endogenous TRAC and B2M knockout were constructed according to Example 9. From 5' to 3', the CD38-HLA-E1 fragment sequentially comprises the sequences shown in SEQ ID NOs: 23, 75, and 30; the CD38-HLA-E2 fragment sequentially comprises the sequences shown in SEQ ID NOs: 23, 75, and 31; the CD38-HLA-E3 fragment sequentially comprises the sequences shown in SEQ ID NOs: 23, 75, and 32; and the CD38-HLA-E4 fragment sequentially comprises the sequences shown in SEQ ID NOs: 23, 75, and 33.

### Example 13. Detection of Killing on NK cells and Cytokine Secretion by CD38-HLA-E UCAR-T Cell

According to Example 7, UCAR-T was co-cultured with NK for 24h or 72h. Compared with UTD, CD38-HLA-E-UCAR-T1, 2, 3, and 4 all significantly killed aNK and rNK (Figure 7A). The supernatant was taken after UCAR-T cells were co-cultured with NK cells for 24h, and the secretion of IL-2, TNF-α, and IFN-γ cytokines was detected using the CBA method (BD, 558264). As shown in Figure 7B, compared with UUTD, the above-mentioned UCAR-T secreted higher levels of cytokines after co-incubation with NK cells.

### Example 14. Preparation of NKG2A/CD38-CAR-T and CD94/CD38-CAR-T cells

NKG2A/CD38-CAR-T cells were constructed according to Examples 2 and 3: CD38/N-DCAR1, CD38/N-DCAR2, CD38/N-DCAR3, CD38/N-s28z, N/CD38-s28Z, CD38/N-DCAR4, CD38/N-DCAR5. CD94-CD38-CAR-T cells were constructed. CD300A/CD38-CAR-T cells were constructed. GPRC5D/N/CD38-CAR-T cells targeting the tumor antigen GPRC5D were constructed. BCMA/N/CD38-CAR-T cells targeting the tumor antigen BMCA were constructed.

The structures of CD38/N-s28z and N/CD38-s28Z are shown in Table 2. CD38/N-DCAR2, from N-terminus to C-terminus, sequentially comprises the sequences shown in SEQ ID NO: 26, SEQ ID NO: 87, and SEQ ID NO: 83. CD38/N-DCAR4, from N-terminus to C-terminus, sequentially comprises the sequences shown in SEQ ID NO: 27, SEQ ID NO: 87, and SEQ ID NO: 83. CD38/N-DCAR5, from N-terminus to C-terminus, sequentially comprises the sequences shown in SEQ ID NO: 28, SEQ ID NO: 87, and SEQ ID NO: 83. CD38/N-DCAR1, CD38/N-DCAR3, GPRC5D/N/CD38-CAR, and BCMA/N/CD38-CAR sequentially comprise the sequences shown in SEQ ID NOs: 34, 4, 37, and 38, respectively.

TKO cells with endogenous TRAC, B2M, and NKG2A knockout, and QKO cells with endogenous TRAC, B2M, NKG2A, and CD38 knockout were prepared according to Example 9. The sgRNA sequences targeting TRAC, B2M, NKG2A, and CD38 are shown in SEQ ID NOs: 39, 40, 41, and 42, respectively. The positive rate of CAR expression was detected using Biotin-anti-huF(ab)2 and Streptavidin-PE. The results showed that CAR molecules were successfully expressed in all T cells groups.

### Example 15. Detection of in vitro killing on tumor cell and cytokine secretion by NKG2A/CD38-UCAR-T

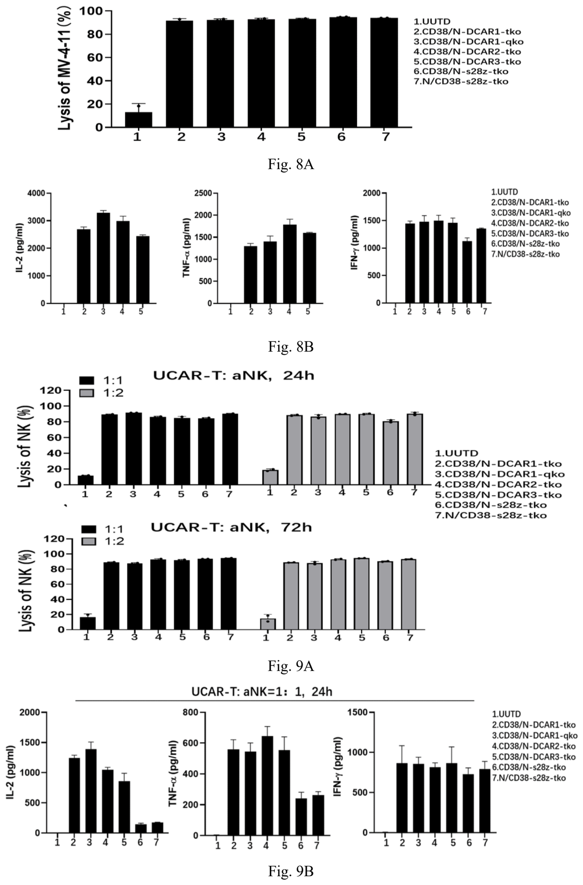

UCAR-T cells were co-cultured with MV-4-11 at a ratio of 1:3 for 16 hours and stained with anti-HLA-ABC APC (Thermo, 17-9983-42). UCAR-T cells are HLA-ABC negative cells, and MV-4-11 cells are HLA-ABC positive cells. Dead cells were labeled with 7-AAD (BD, 559925), and finally counting beads (Invitrogen, C36995) were added. The result was analyzed by FACS. Figure 8A shows that CD38/N-DCAR1-tko, CD38/N-DCAR1-qko, CD38/N-DCAR2-tko, CD38/N-DCAR3-tko, CD38/N-s28z-tko, and N/CD38-s28Z-tko significantly killed MV-4-11 cells.

The supernatant was taken after the above-mentioned UCART were co-incubated with MV-4-11 cells for 16 hours, and the secretion of IL-2, TNF-α, and IFN-γ cytokines was detected using the CBA method (BD, 558264). Figure 8B shows that compared with UUTD, the above-mentioned UCAR-T secreted higher levels of cytokines after co-incubation with tumor cells.

### Example 16. Detection of killing on aNK cells and cytokine secretion by NKG2A/CD38-UCAR-T

UCAR-T was co-cultured with aNK for 24h or 72h according to Example 7.. Compared with the control, CD38/N-DCAR1-tko, CD38/N-DCAR1-qko, CD38/N-DCAR2-tko, CD38/N-DCAR3-tko, CD38/N-s28Z-tko, and N/CD38-s28Z-tko significantly killed NK cells (Figure 9A). After co-cultured with NK for 24h, the secretion of IL-2, TNF-α, and IFN-γ cytokines was detected. As shown in Figure 9B, compared with UUTD, the above-mentioned UCAR-T secreted higher levels of cytokines after co-incubation with NK cells.

### Example 17. NKG2A/CD38-UCAR-T cells treatment of orthotopic tumors in NPG mice

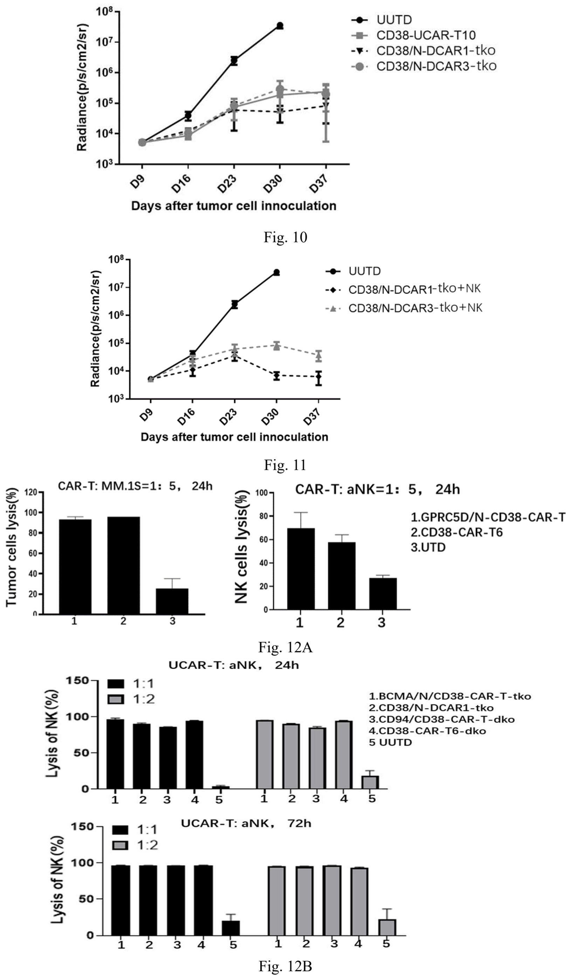

NPG mice were injected via tail vein with 1 × 10 ⁷ THP-1-Luci cells/mouse, and divided into 4 groups, with 6 mice per group. Tumor photon intensity was observed at various time points using fluorescence imaging (IVIS LUMINA III SYSTEM). On D10, mice were injected with 2.5×10 ⁶ UCAR-T cells via tail vein. Figure 10 shows that by D37, all mice in the control group died, while the CD38-UCAR-T10, CD38/N-DCAR1-tko, and CD38/N-DCAR3-tko groups all exhibited significant tumor suppression effects.

### Example 18. NKG2A/CD38-UCAR-T treatment of orthotopic tumors in NPG mice in the presence of NK cells

NPG mice were injected via tail vein with 1 × 10 ⁷ THP-1-Luci cells/mouse, and divided into 3 groups, with 6 mice per group. Tumor photon intensity was observed at various time points using fluorescence imaging (IVIS LUMINA III SYSTEM). In the NK cell injection group, on D9 (when the photon intensity reached an average of approximately 5.3×10 ³ p/s/cm ²/sr), and 1.5×10 ⁶ NK cells/mouse were injected via tail vein for 3 consecutive days. On D10, 2.5×10 ⁶ UCAR-T cells were injected via tail vein. Figure 11 shows that in the presence of NK cells, the CD38/N-DCAR1-tko and CD38/N-DCAR3-tko groups still had significant tumor suppression effects.

### Example 19. Detection of killing on tumor cells or NK cells and cytokine secretion by CAR-T

CAR-T cells were co-cultured with MM.1S cells at a ratio of 1:5 for 24 hours, labeled and stained using Anti-CD3-APC (Biolegend, 300412), CD3+ cells were T cells and CD3- cells were MM.1S cells, dead cells were labeled using 7-AAD (BD, 559925), and finally counting beads (Invitrogen, C36995) were added. The result was analyzed by FACS. Figure 12A (left) shows that GPRC5D/N/CD38-CAR-T cells significantly killed tumor cells.

According to Example 7. Figure 12A (right) and Figure 12B show that after co-cultured with NK cells for 24 or 72 hours, GPRC5D/N/CD38-CAR-T, BCMA/N/CD38-CAR-T-tko, and CD94/CD38-CAR-T-dko cells significantly killed NK cells. After co-cultured with NK cells for 24 hours, BCMA/N/CD38-CAR-T-tko and CD94/CD38-CAR-T-dko cells significantly secreted IL-2, TNF-α, and IFN-γ.

### Example 20. Combination of CD38-CAR-T cells and CAR-T cells targeting tumor antigens

Claudin18.2-CAR-T, GPC3-CAR-T, and Mesothelin-CAR-T cells were constructed according to Examples 2 and 3. Claudin18.2/N-CAR-T cells that bind to Claudin18.2 and NKG2A were constructed.

CD38-CAR-T cells and Claudin18.2/N-CAR-T cells were mixed in a ratio of 1:1, 1:2, or 2:1 and administered to subjects for the treatment of Claudin18.2-positive tumors.

### Example 21. Preparation and expansion of CAR-T cells targeting NKG2DL

Vectors expressing the chimeric receptor disclosed in this application were constructed using conventional molecular biology techniques. Full-length DNA was synthesized to construct chimeric receptors targeting NKG2DL and then integrated into the pRRLSIN lentiviral vector (Addgene).

The NKG2Dz fragment and the NKG2D-BBZ fragment comprise the sequences shown in SEQ ID NOs: 78 and 79, respectively.

A-N1 fragment: sequentially comprises the full-length ASGPR1 (SEQ ID NO: 80) and the extracellular region of NKG2D (SEQ ID NO: 76); A-N2 fragment: sequentially comprises the full-length ASGPR1 (SEQ ID NO: 80), G4S-linker (SEQ ID NO: 16), and the extracellular region of NKG2D (SEQ ID NO: 76); CD137L-N fragment: sequentially comprises the full-length CD137L (SEQ ID NO: 81) and the extracellular region of NKG2D (SEQ ID NO: 76).

From 5' to 3' direction, the A-N1/NKG2Dz fragment sequentially comprises NKG2Dz (SEQ ID NO: 78), P2A (SEQ ID NO: 17), the full-length ASGPR1 (SEQ ID NO: 80), and the extracellular region of NKG2D (SEQ ID NO: 76); the A-N2/NKG2Dz fragment sequentially comprises NKG2Dz (SEQ ID NO: 78), P2A (SEQ ID NO: 17), the full-length ASGPR1 (SEQ ID NO: 80), G4S-linker (SEQ ID NO: 16), and the extracellular region of NKG2D (SEQ ID NO: 76); and the CD137L-N/NKG2Dz fragment sequentially comprises NKG2Dz (SEQ ID NO: 78), P2A (SEQ ID NO: 17), the full-length CD137L (SEQ ID NO: 81), and the extracellular region of NKG2D (SEQ ID NO: 76).

From 5' to 3' direction, the A-N1/NKG2D-BBZ fragment sequentially comprises NKG2D-BBZ (SEQ ID NO: 79), P2A (SEQ ID NO: 17), the full-length ASGPR1 (SEQ ID NO: 80), and the extracellular region of NKG2D (SEQ ID NO: 76). The A-N2/NKG2D-BBZ fragment sequentially comprises NKG2D-BBZ (SEQ ID NO: 79), P2A (SEQ ID NO: 17), the full-length ASGPR1 (SEQ ID NO: 80), G4S-linker (SEQ ID NO: 16), and the extracellular region of NKG2D (SEQ ID NO: 76). The CD137L-N/NKG2D-BBZ fragment sequentially comprises NKG2D-BBZ (SEQ ID NO: 79), P2A (SEQ ID NO: 17), the full-length CD137L (SEQ ID NO: 81), and the extracellular region of NKG2D (SEQ ID NO: 76).

The above sequences were inserted between the MluI and SalI restriction endonuclease sites of the pRRLSIN lentiviral vector (Addgene) for lentiviral packaging.

CAR-T cells were prepared using conventional methods known in the art: donor PBMCs were collected, and activated with anti-CD3 and CD28 antibody magnetic beads (Life Technologies, 40203D). T cells were then infected with lentivirus comprising the aforementioned vectors to prepare A-N1/NKG2Dz, A-N2/NKG2Dz, CD137L-N/NKG2Dz, CD40L-N/NKG2Dz, A-N1/NKG2D-BBZ, A-N2/NKG2D-BBZ, and CD137L-N/NKG2D-BBZ cells expressing the corresponding chimeric receptors. T cells not transfected with the virus were UTD.

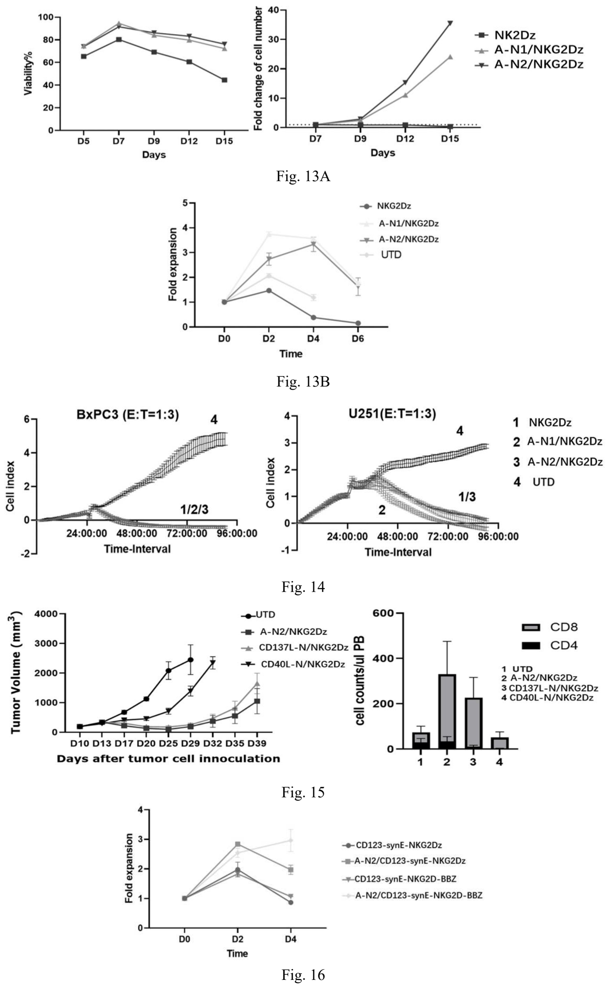

The expression vectors were introduced into T cells via lentivirus, and the cells were cultured and expanded *in vitro* for 2 weeks, with cell counting and viability analysis (PI staining) every 2-3 days. Detection of NKG2D expression on the surface of CD4 T cells (endogenously NKG2D-negative) showed at least about 93% positivity. As shown in Figure 13A, the expansion fold and survival rates of A-N1/NKG2Dz and A-N2/NKG2Dz cells were significantly improved compared to the control group.

### Example 22. Detection of NKG2D-CAR-T cell expansion under target cell stimulation

CAR-T cells were co-incubated with THP1 cells at an effector-to-target ratio of 1:1 (THP1 cells were pre-treated with mitomycin C to stop proliferation), cell counting was performed after 48 hours, and THP1 cells were added again at an effector-to-target ratio of 1:1 for stimulation, repeated for three rounds. As shown in Figure 13B, co-expression of the A-N1 or A-N2 peptide increased the cell expansion fold of NKG2D-CAR-T cells under target cell stimulation compared to the control group.

### Example 23. Detection of in vitro killing on tumor cells by NKG2D-CAR-T

Using the xCELLigence RTCA real-time monitoring method, CAR-T cells were co-incubated with target cells (U251, BxPC3) at an effector-to-target ratio of 1:3 (target cells were plated on the electrode plate 24 hours in advance). As shown in Figure 14, A-N1/NKG2Dz and A-N2/NKG2Dz cells significantly killed U251 and BxPC3 tumor cells.

### Example 24. NKG2D-CAR-T treatment of orthotopic tumors in NPG mice

NPG mice were injected via tail vein with 1 × 10 ⁷ THP1-luci cells/mouse (recorded as D0). On D15, 5×10⁶ CAR-T cells were injected via tail vein. Luciferase substrate was injected weekly for imaging to observe tumor burden. As shown in Figure 15 (left), compared to UTD, CD137L-N/NKG2Dz and A-N2/NKG2Dz cells exhibited significant *in vivo* anti-tumor efficacy.

On day 7 after CAR-T infusion, peripheral blood was collected from mice, and the number of CD3/CD4/CD8 T cells in the peripheral blood was quantitatively analyzed by flow cytometry (BD Trucount Tubes). As shown in Figure 15 (right), compared to UTD, the *in vivo* survival number of CD137L-N/NKG2Dz and A-N2/NKG2Dz cells increased.

### Example 25. Preparation of regulated NKG2D-CAR-T cells

In order to further enhance the protective effect of A-N fragments, T cells with constitutive expression of A-N fragments and regulated expression of NKG2D-CAR were prepared. A chimeric polypeptide comprising CD123-synE fragment recognizing CD123 was prepared according to PCT/CN2022/102395,.

Vectors expressing the chimeric receptor disclosed in this application were constructed using conventional molecular biology techniques. Full-length DNA was synthesized to construct chimeric receptors targeting NKG2DL and then integrated into the pRRLSIN lentiviral vector (Addgene) , respectively.

Full-length DNA was synthesized. From 5' to 3' direction, A-N2/CD123-synE fragment, sequentially comprises CD123-synE fragment (SEQ ID NO: 82), P2A (SEQ ID NO: 17), the full-length ASGPR1 (SEQ ID NO: 80), G4S-linker (SEQ ID NO: 16), and the extracellular region of NKG2D (SEQ ID NO: 76).

Briefly, from 5' to 3' direction, the UAS-CMV promoter (SEQ ID NO: 88), NKG2Dz (SEQ ID NO: 78) or NKG2D-BBZ (SEQ ID NO: 79), EF1a promoter (SEQ ID NO: 89), and CD123-synE fragment (SEQ ID NO: 82) were sequentially connected, and the obtained fragments were inserted between the C1aI and SalI restriction endonuclease sites of the pRRLSIN lentiviral vector (Addgene) to prepare the lentiviral vectors CD123-synE-NKG2Dz and CD123-synE-NKG2D-BBZ.

From the 5' to 3' direction, the UAS-CMV promoter (SEQ ID NO: 88), NKG2Dz (SEQ ID NO: 78) or NKG2D-BBZ (SEQ ID NO: 79), EF1a promoter (SEQ ID NO: 89), and A-N2/CD123-synE fragment were sequentially connected. Finally, the obtained fragments were inserted between the C1aI and SalI restriction endonuclease sites of the pRRLSIN lentiviral vector (Addgene) to prepare the lentiviral vectors A-N2/CD123-synE-NKG2Dz and A-N2/CD123-synE-NKG2D-BBZ.

CAR-T cells were prepared using conventional methods known in the art: donor PBMCs were collected and activated with anti-CD3 and CD28 antibody magnetic beads (Life Technologies, 40203D). T cells were then infected with lentivirus comprising the above-mentioned vectors to prepare T cells expressing the corresponding chimeric receptors: CD123-synE-NKG2Dz cells, CD123-synE-NKG2D-BBZ cells, A-N2/CD123-synE-NKG2Dz cells, and A-N2/CD123-synE-NKG2D-BBZ cells. T cells not transfected with the virus were UTD.

The transduction efficiency was determined by detecting the expression of CD123 single-chain antibody on the surface of T cells, showing that the expression vectors were effectively transduced into T cells.

### Example 26. Cell Expansion of Regulated NKG2D-CAR-T Cells under Target Cell Stimulation

CAR-T cells were co-incubated with THP1 cells at an effector-to-target ratio of 1:1 (THP1 cells were pre-treated with mitomycin C to stop proliferation), cell counting was performed after 48 hours, and THP1 cells were added again at an effector-to-target ratio of 1:1 for stimulation, repeated for two rounds. As shown in Figure 16, the cell proliferation folds of A-N2/CD123-synE-NKG2Dz and A-N2/CD123-synE-NKG2D-BBZ cells were significantly increased under target cell stimulation compared to CD123-synE-NKG2Dz and CD123-synE-NKG2D-BBZ cells, respectively.

### Example 27. Killing function on target cells and cytokine secretion of regulated NKG2D-CAR- T cells

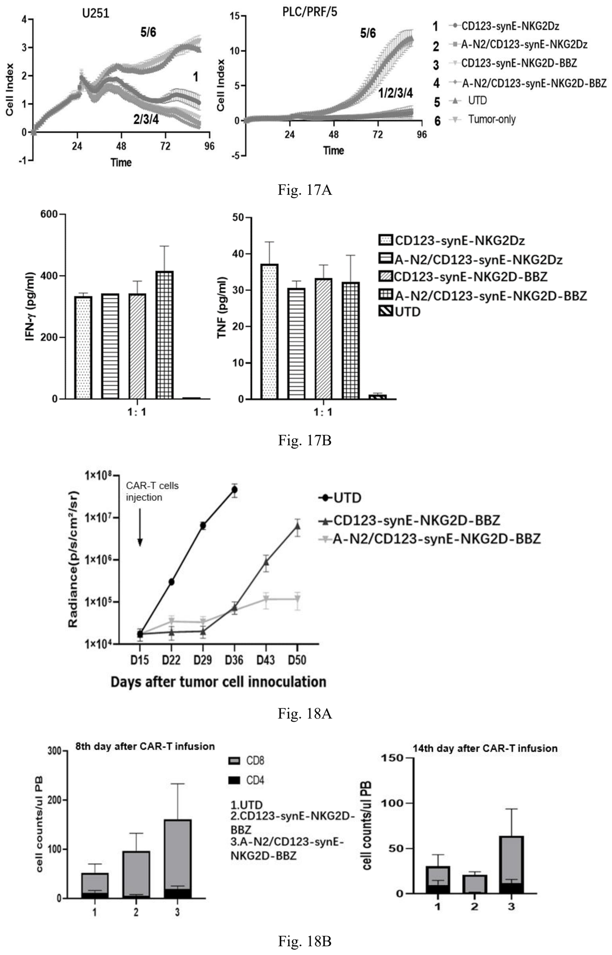

The xCELLigence RTCA real-time monitoring method was used. Target cells were plated onto electrode plates 24 hours in advance, and CAR-T cells were co-incubated with target cells U251 or PLC/PFR/5 at an effector-target ratio of 1:1. As shown in Figure 17A, A-N2/CD123-synE-NKG2Dz and A-N2/CD123-synE-NKG2D-BBZ cells significantly killed tumor cells. As shown in Figure 17B, after A-N2/CD123-synE-NKG2Dz and A-N2/CD123-synE-NKG2D-BBZ cells were co-incubated with THP-1 at an effector-target ratio of 1:1 for 24 hours, significant cytokine secretion was detected in the supernatant.

### Example 28. Regulated NKG2D-CAR-T cells Treatment of orthotopic tumors in NPG mice

NPG mice were injected via tail vein with 1 × 10 ⁷ THP1-luci cells (recorded as D0). On D15, 5×10 ⁶CAR-T cells/mouse were injected via tail vein. Luciferase substrate was injected weekly for imaging to observe tumor burden. As shown in Figure 18A, the A-N2/CD123-synE-NKG2D-BBZ group, which co-expresses the A-N2 peptide, showed better *in vivo* anti-tumor efficacy, with a median survival of 77 days, significantly higher than the 61 days of the CD123-synE-NKG2D-BBZ group. Peripheral blood was collected from mice on day 8 and day 14 after CAR-T infusion, and the number of CD3/CD4/CD8 T cells in the peripheral blood was quantitatively analyzed by flow cytometry (BD Trucount Tubes). As shown in Figure 18B, the number of CAR-T cells surviving *in vivo* increased in the A-N2/CD123-synE-NKG2D-BBZ group .

### Example 29. Preparation of BCMA/GPRC5D-CAR-T cells

According to Examples 2 and 3. Full-length DNA was synthesized to construct CAR targeting BCMA and GPRC5D, which were inserted between the MluI and SalI restriction endonuclease sites of the pRRLSIN lentiviral vector (Addgene) for lentiviral packaging. The BG-BBZ, BGp-BBZ, GB-BBZ, BLG-BBZ, BLGp-BBZ, and GLBp-BBZ fragments comprises the sequences shown in SEQ ID NOs: 90, 91, 92, 94, 93, and 95, respectively. The CAR positive rate in each BCMA/GPRC5D-CAR-T cell was detected by FACS, and the CAR molecule were successfully expressed in each group of T cells.

### Example 30. Detection of in vitro killing on tumor cells and cytokine secretion by BCMA/GPRC5D-CAR-T cells

About 3×10⁴ tumor cells were seeded in 96-well plates, and CAR-T cells were added at an effector-target ratio of 1:1. After 24 hours of culture, the number of GPF and mCherry positive cells were detected by flow cytometry, and the lysis values of CAR-T cells against tumor cells MM.1 S-GFP, MM.1 S-BCMAKO, and MM.1S-GPRC5D KO were calculated.

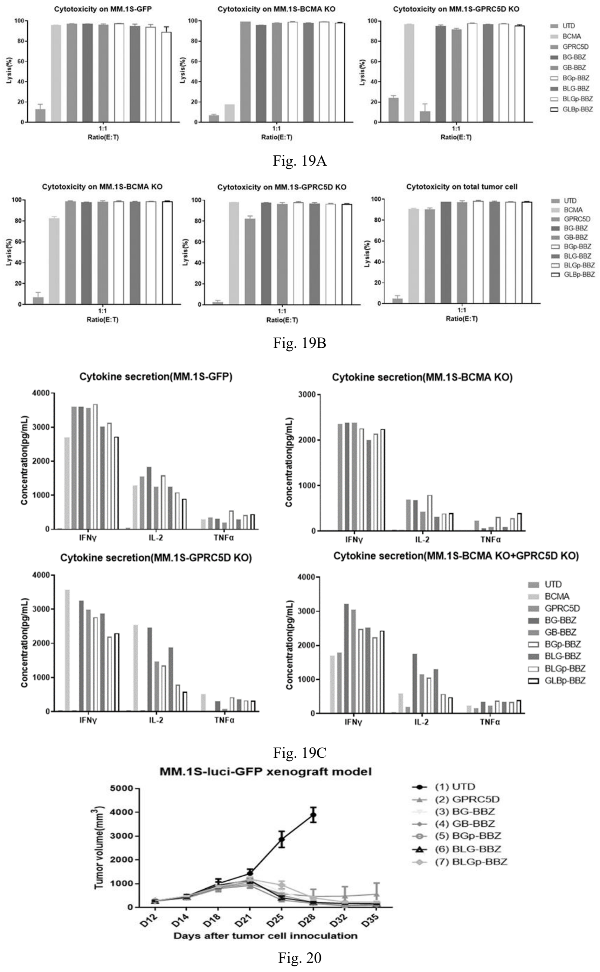

MM.1S-BCMA KO and MM.1 S-GPRC5D KO cells were mixed at a ratio of 1:1, and 1×10 ⁴ of the mixed target cells were seeded into a 96-well plate, and CAR-T cells were added at an effector-target ratio of 1:1. After culturing for 24 hours, the number of GPF and mCherry-positive cells were detected by flow cytometry, and the tumor cell lysis value was calculated. Figures 19A and B show that BG-BBZ, BGp-BBZ, GB-BBZ, BLG-BBZ, BLGp-BBZ, and GLBp-BBZ cells effectively inhibited the growth of BCMA-positive, GPRC5D-positive, BCMA and GPRC5D double-positive, or mixed tumor cells containing both BCMA-positive and GPRC5D-positive cells. The CBA assay showed that the above-mentioned CAR-T cells secreted high levels of cytokines after 24 hours of co-incubation with tumor cells (Figure 19C).

### Example 31. Detection of the anti-tumor effect of BCMA/GPRC5D-CAR-T cells in vivo

3×10 ⁶ MM.1S-luciferase/GFP cells were subcutaneously injected into NPG mice (recorded as D0), and the tumor burden was approximately 300 mm³ on D11, and the mice were divided into 7 groups, with 5 mice per group. On D12, 0.8×10 ⁶ CAR-T cells/mouse were injected via tail vein, and tumor growth was monitored. As shown in Figure 20, compared to the UTD group, the BG-BBZ, GB-BBZ, BGp-BBZ, BLG-BBZ, BLGp-BBZ, and GPRC5D-CAR-T groups significantly inhibited tumor growth. On D28, peripheral blood from mice were collected, and flow cytometry detection shows significant expansion of CAR-T cells in the BCMA/GPRC5D-CAR-T group.

### Example 32. Detection of the anti-tumor effect of BCMA/GPRC5D-CAR-T cells

The hinge and transmembrane domains of BLG-BBZ (SEQ ID NOs: 94, or 96) and GLBp-BBZ (SEQ ID NO: 95) fragments were replaced with IgG4 (H) and CD28 (TM), respectively, BLG-sBBZ and GLB-sBBZ. BLG-sBBZ and GLB-sBBZ cells were constructed according to Examples 2 and 3.

CAR-T cells were plated with tumor cells at a ratio of 3:1, 1:1, or 1:3. After 24 hours of culture, flow cytometry detection showed that BLG-sBBZ and GLB-sBBZ cells effectively inhibited BCMA-positive, GPRC5D-positive, and BCMA and GPRC5D double-positive tumor cells, and secreted high levels of the cytokines IFNγ, IL-2, and TNFα. *In vivo* anti-tumor experiments showed that BLG-sBBZ and GLB-sBBZ cells effectively inhibited the growth of MM.1S subcutaneous tumors in NPG mice.

### Example 33. Rapid preparation of R-CAR-T cells

PBMCs isolated from donor human peripheral blood were taken, thawed to recover for about 24 hours, and then activated with anti-CD3/CD28 TransAct magnetic beads for 22 hours. They were transfected with lentivirus comprising BG-BBZ, GB-BBZ, BLG-BBZ, BGp-BBZ, or BLGp-BBZ, and after two hours of infection, R-BG-BBZ, R-GB-BBZ, R-BLG-BBZ, R-BGp-BBZ, and R-BLGp-BBZ cells were obtained. The obtained R-CAR-T cells were expanded *in vitro* for 4 days, and then the positive rate was detected. R-UTD, R-BCMA-CART, and R-GPRC5D-CART cells were prepared using the same method.

### Example 34. Detection of in vitro killing on tumor cells and cytokine secretion by R-CAR-T cells

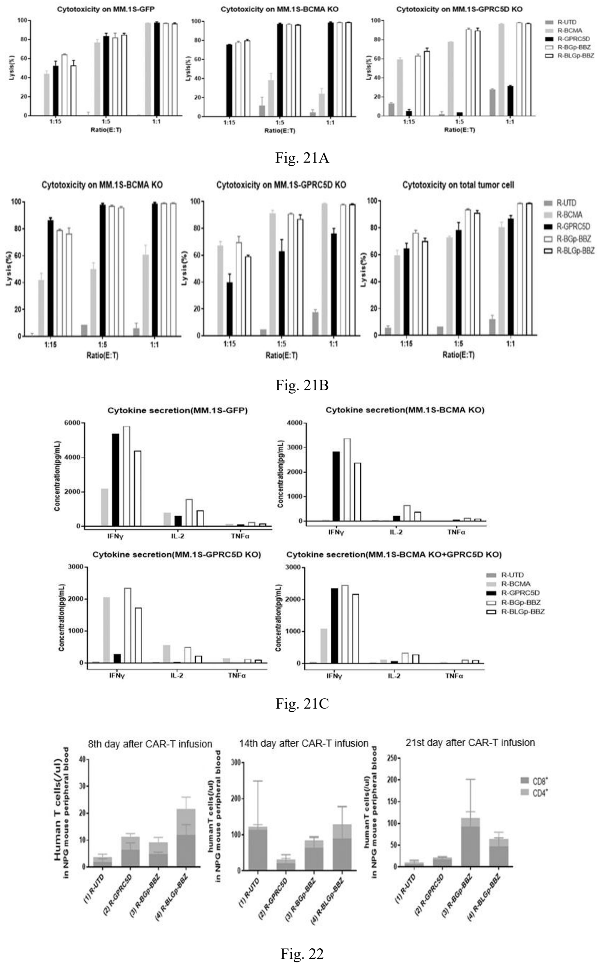

MM.1S-GPF, MM.1S-BCMA KO, MM.1S-GPRC5D KO, and MM.1S-BCMA KO+MM.1S-GPRC5D KO (mixed at 1:1) were used as target cells, respectively, and 3×10⁴ cells were seeded into 96-well plates. R-CAR-T cells expanded *in vitro* to day 5 were used as effector cells, and co-cultured for 24 hours at effector-target ratios of 1:1, 1:5, and 1:15, respectively. The number of tumor cells was detected and quantified by flow cytometry. Figure 21A shows that R-BGp-BBZ and R-BLGp-BBZ cells significantly killed BCMA-positive, GPRC5D-positive, and BCMA-GPRC5D double-positive tumor cells. Figure 21B shows that R-BGp-BBZ and R-BLGp-BBZ cells significantly killed MM.1S-BCMA KO and MM.1S-GPRC5D KO cells in a mixture of BCMA-positive and GPRC5D-positive cells.

R-CAR-T cells and tumor cells were plated at a 1:1 ratio. After 24 hours of culture, the supernatant was collected and the concentrations of INFγ, IL-2, and TNFα were measured using the CBA method. Figure 21C shows that R-BGp-BBZ and R-BLGp-BBZ cells significantly secreted cytokines after tumor cell stimulation.

### Example 35. In vivo Anti-tumor effect of R-CAR-T cells

NPG mice were injected via tail vein with 2 × 10 ⁶ MM.1S-luciferase/GFP cells/mouse (recorded as D0). On D10, tumor burden was about 3-5×10 ⁴ Radiance(p/s/cm ² /sr), and the mice were divided into 4 groups, with 5 mice per group. On D11, 0.1×10 ⁶ CAR-T cells/mouse were injected via tail vein, and the tumor growth was monitored. On D44, the average fluorescence values of tumors in the UTD, R-BGp-BBZ, and R-BLGp-BBZ groups were 3.47×10 ⁶ , 3.36×10 ⁴ , 2.27×10 ³ p/s/cm ²/sr, respectively. The tumor inhibition rates of the R-BGp-BBZ and R-BLGp-BBZ groups relative to the UTD group were 99.90% and 99.93%, respectively. T cell numbers in the peripheral blood of mice were detected by flow cytometry on days 8, 14, and 21 after CAR-T injection. Figure 22 shows that the *in vivo* CAR-T expansion in the R-BGp-BBZ and R-BLGp-BBZ groups exhibited significant advantages.

The embodiments described in this application comprise any embodiment as a single embodiment or in combination with any other embodiment or part thereof. In addition, it should be understood that after reading the above teachings of this application, those skilled in the art can make various changes or modifications to this application, and these equivalent forms also fall within the scope defined by the claims attached hereto.

| | |
|---|---|
| 1 | MALPVTALLLPLALLLHAARP |
| 2 | MLLLVTSLLLCELPHPAFLLIP |
| 3 | TTTPAPRPPTPAPTIASQPLSLRPEACRPAAGGAVHTRGLDFACD |
| 4 | ATGGCCTTACCAGTGACCGCCTTGCTCCTGCCGCTGGCCTTGCTGCTCCACGCCGCCAGGCCGCAGGTGCAGCT GGTGCAGAGCGGCGCCGAGGTGAAGAAGCCCGGCGCCAGCGTGAAGGTGAGCTGCAAGGCCAGCGGCTACAC CTTCACCAGCTACTGGATGAACTGGGTGCGGCAGGCCCCCGGCCAGGGCCTGGAGTGGATGGGCCGGATCGAC CCCTACGACAGCGAGACCCACTACGCCCAGAAGCTGCAGGGCCGGGTGACCATGACCACCGACACCAGCACCA GCACCGCCTACATGGAGCTGCGGAGCCTGCGGAGCGACGACACCGCCGTGTACTACTGCGCCCGGGGCGGCTA CGACTTCGACGTGGGCACCCTGTACTGGTTCTTCGACGTGTGGGGCCAGGGCACCACCGTGACCGTGAGCAGCG GTGGAGGCGGTTCAGGCGGAGGTGGTTCTGGCGGTGGCGGATCGGACATCCAGATGACCCAGAGCCCCAGCAG CCTGAGCGCCAGCGTGGGCGACCGGGTGACCATCACCTGCCGGGCCAGCGAGAACATCTACAGCTACCTGGCC TGGTACCAGCAGAAGCCCGGCAAGGCCCCCAAGCTGCTGATCTACAACGCCAAGACCCTGGCCGAGGGCGTGC CCAGCCGGTTCAGCGGCAGCGGCAGCGGCACCGACTTCACCCTGACCATCAGCAGCCTGCAGCCCGAGGACTT CGCCACCTACTACTGCCAGCACCACTACGGCACCCCCCGGACCTTCGGCGGCGGCACCAAGGTGGAGATCAAG ACCACGACGCCAGCGCCGCGACCACCAACACCGGCGCCCACCATCGCGTCGCAGCCCCTGTCCCTGCGCCCAG AGGCGTGCCGGCCAGCGGCGGGGGGCGCAGTGCACACGAGGGGGCTGGACTTCGCCTGTGATTTTTGGGTGCT GGTGGTGGTTGGTGGAGTCCTGGCTTGCTATAGCTTGCTAGTAACAGTGGCCTTTATTATTTTCTGGGTGAGGAGT AAGAGGAGCAGGCTCCTGCACAGTGACTACATGAACATGACTCCCCGCCGCCCCGGGCCAACCCGCAAGCATT ACCAGCCCTATGCCCCACCACGCGACTTCGCAGCCTATCGCTCCAGAGTGAAGTTCAGCAGGAGCGCAGACGCC CCCGCGTACCAGCAGGGCCAGAACCAGCTCTATAACGAGCTCAATCTAGGACGAAGAGAGGAGTACGATGTTTT GGACAAGAGACGTGGCCGGGACCCTGAGATGGGGGGAAAGCCGCAGAGAAGGAAGAACCCTCAGGAAGGCCT GTACAATGAACTGCAGAAAGATAAGATGGCGGAGGCCTACAGTGAGATTGGGATGAAAGGCGAGCGCCGGAGG GGCAAGGGGCACGATGGCCTTTACCAGGGTCTCAGTACAGCCACCAAGGACACCTACGACGCCCTTCACATGCA GGCCCTGCCCCCTCGCGGAAGCGGAGCTACTAACTTCAGCCTGCTGAAGCAGGCTGGAGACGTGGAGGAGAAC CCTGGGCCCATGGCTCTGCCTGTGACAGCTCTGCTGCTGCCTCTGGCTCTGCTTCTGCATGCCGCTAGACCTGAA GTGCAGCTGCTGGAATCTGGCGGAGGACTGGTTCAACCTGGCGGCTCTCTGAGACTGAGCTGTGCCGTGTCCGG CTTCACCTTCAACAGCTTCGCCATGAGCTGGGTCCGACAGGCCCCTGGAAAAGGCCTTGAATGGGTGTCCGCCA TCTCTGGAAGCGGCGGAGGCACATATTACGCCGACTCTGTGAAGGGCAGATTCACCATCAGCCGGGACAACAGC AAGAACACCCTGTACCTGCAGATGAACTCCCTGAGAGCCGAGGATACCGCCGTGTACTTCTGCGCCAAGGACAA GATCCTTTGGTTCGGCGAGCCCGTGTTCGACTATTGGGGACAGGGAACCCTGGTCACCGTTTCTAGCGGAGGCG GAGGTTCTGGTGGTGGTGGATCAGGTGGCGGCGGATCTGAGATTGTGCTGACACAGTCTCCCGCCACACTGTCT CTTAGCCCTGGCGAAAGAGCCACACTGAGCTGCAGAGCCAGCCAGAGCGTGTCCTCTTACCTGGCCTGGTATCA GCAGAAGCCCGGCCAAGCTCCTCGGCTGCTGATCTACGATGCCAGCAATAGAGCCACAGGCATCCCTGCCAGAT TTTCCGGCTCTGGCAGCGGCACCGATTTCACCCTGACCATAAGCAGCCTGGAACCTGAGGACTTCGCCGTGTATT ACTGCCAGCAGCGGAGCAACTGGCCTCCTACATTTGGCCAGGGAACCAAAGTCGAGATCAAGGAGTCTAAGTAC GGCCCTCCTTGTCCTCCATGTCCTATGTTTTGGGTCCTCGTCGTCGTCGGAGGCGTGCTGGCTTGCTATAGCCTGC TTGTGACAGTGGCCTTTATTATCTTCTGGGTTAGAAGCAAGCGGAGCCGGCTGCTGCACAGCGACTACATGAACA TGACCCCTAGACGGCCCGGACCTACCAGAAAGCACTACCAGCCTTACGCTCCTCCTCGGGATTTCGCCGCCTAC AGATCTCGCGTGAAATTCTCCAGAAGCGCTGACGCCCCTGCTTATCAGCAAGGCCAGAATCAGCTCTATAACGA ACTCAATCTCGGCAGGCGCGAGGAATATGATGTGCTGGATAAGAGGCGCGGCAGGGACCCAGAAATGGGAGGA AAACCTCAGCGGCGGAAAAACCCACAAGAAGGCCTTTACAACGAACTGCAAAAGGATAAGATGGCAGAAGCTT ACTCCGAGATTGGCATGAAGGGCGAACGTCGGAGAGGAAAAGGCCACGACGGACTCTATCAGGGACTGTCTAC AGCCACAAAGGACACATACGACGCACTCCATATGCAGGCTCTCCCACCTAGATAG |
| 5 | ESKYGPPCPPCPAPPVAGPSVFLFPPKPKDTLMISRTPEVTCVVVDVSQEDPEVQFNWYVDGVEVHNAKTKPREEQFQ STYRVVSVLTVLHQDWLNGKEYKCKVSNKGLPSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCSVMHEALHNHYTQKSLSLSLGK↵ |
| 6 | ESKYGPPCPPCP |
| 7 | IYIWAPLAGTCGVLLLSLVITLYC |
| 8 | FWVLVVVGGVLACYSLLVTVAFIIFWV |
| 9 | MFWVLVVVGGVLACYSLLVTVAFIIFWV |
| 10 | RSKRSRLLHSDYMNMTPRRPGPTRKHYQPYAPPRDFAAYRS |
| 11 | KRGRKKLLYIFKQPFMRPVQTTQEEDGCSCRFPEEEEGGCEL |
| 12 | RVKFSRSADAPAYQQGQNQLYNELNLGRREEYDVLDKRRGRDPEMGGKPQRRKNPQEGLYNELQKDKMAEAYSEIG |
| | ← |
| 13 | ↵ RVKFSRSADAPAYQQGQNQLYNELNLGRREEYDVLDKRRGRDPEMGGKPRRKNPQEGLYNELQKDKMAEAYSEIGM KGERRRGKGHDGLYQGLSTATKDTYDALHMQALPPR↵ |
| 14 | GGGGSGGGGSGGGGS |
| 15 | GSTSGSGKPGSGEGSTKG |
| 16 | GGCGGAGGTGGAAGCGGAGGCGGAGGATCGGGCGGCGGAGGAAGC |
| 17 | GCTACTAACTTCAGCCTGCTGAAGCAGGCTGGAGACGTGGAGGAGAACCCTGGGCCC |
| 18 | ATGGCTCTGCCTGTGACAGCTCTGCTGCTGCCTCTGGCTCTGCTTCTGCATGCCGCTAGACCTCAGGTGCAGCTG GTGGAGAGCGGCGGCGGCCTGGTGCAGCCCGGCGGCAGCCTGCGGCTGAGCTGCGCCGCCAGCGGCTTCACCT TCAGCAGCTACTACATGAACTGGGTGCGGCAGGCCCCCGGCAAGGGCCTGGAGTGGGTGAGCGGCATCAGCGG CGACCCCAGCAACACCTACTACGCCGACAGCGTGAAGGGCCGGTTCACCATCAGCCGGGACAACAGCAAGAAC ACCCTGTACCTGCAGATGAACAGCCTGCGGGCCGAGGACACCGCCGTGTACTACTGCGCCCGGGACCTGCCCCT GGTGTACACCGGCTTCGCCTACTGGGGCCAGGGCACCCTGGTGACCGTGAGCAGCGGTGGAGGCGGTTCAGGC GGAGGTGGTTCTGGCGGTGGCGGATCGGACATCGAGCTGACCCAGCCCCCCAGCGTGAGCGTGGCCCCCGGCC AGACCGCCCGGATCAGCTGCAGCGGCGACAACCTGCGGCACTACTACGTGTACTGGTACCAGCAGAAGCCCGG CCAGGCCCCCGTGCTGGTGATCTACGGCGACAGCAAGCGGCCCAGCGGCATCCCCGAGCGGTTCAGCGGCAGC AACAGCGGCAACACCGCCACCCTGACCATCAGCGGCACCCAGGCCGAGGACGAGGCCGACTACTACTGCCAGA CCTACACCGGCGGCGCCAGCCTGGTGTTCGGCGGCGGCACCAAGCTGACCGTGCTGGGCCAGGAGTCTAAGTA CGGCCCTCCTTGTCCTCCATGTCCTATGTTCTGGGTGCTCGTGGTTGTTGGCGGAGTGCTGGCCTGTTACTCTCTG CTGGTGACCGTGGCCTTCATCATCTTTTGGGTCAAGCGGGGCAGAAAGAAGCTGCTGTACATCTTCAAGCAGCCC TTCATGCGGCCCGTGCAGACCACACAAGAGGAAGATGGCTGCTCCTGCAGATTCCCCGAGGAAGAAGAAGGCG GCTGCGAGCTGAGAGTGAAGTTCAGCAGATCCGCCGACGCTCCTGCCTATCAGCAGGGACAGAACCAGCTGTAC AACGAGCTGAACCTCGGGAGAAGAGAAGAGTACGACGTGCTGGACAAGCGGAGAGGCAGAGATCCTGAGATG GGCGGAAAGCCCCAGCGGAGAAAGAATCCTCAAGAGGGCCTGTATAATGAGCTGCAGAAAGACAAGATGGCCG AGGCCTACAGCGAGATCGGAATGAAGGGCGAGCGCAGAAGAGGCAAGGGACACGATGGACTGTACCAGGGCC TGAGCACCGCCACCAAGGATACCTATGATGCCCTGCACATGCAGGCCCTGCCTCCAAGA |
| 19 | EVQLLESGGGLVQPGGSLRLSCAVSGFTFNSFAMSWVRQAPGKGLEWVSAISGSGGGTYYADSVKGRFTISRDNSKNT LYLQMNSLRAEDTAVYFCAKDKILWFGEPVFDYWGQGTLVTVSSGGGGSGGGGSGGGGSEIVLTQSPATLSLSPGERA TLSCRASQSVSSYLAWYQQKPGQAPRLLIYDASNRATGIPARFSGSGSGTDFTLTISSLEPEDFAVYYCQQRSNWPPTFG QGTKVEIK |
| 20 | QVQLVESGGGLVQPGGSLRLSCAASGFTFSSYYMNWVRQAPGKGLEWVSGISGDPSNTYYADSVKGRFTISRDNSKN TLYLQMNSLRAEDTAVYYCARDLPLVYTGFAYWGQGTLVTVSSGGGGSGGGGSGGGGSDIELTQPPSVSVAPGQTARI SCSGDNLRHYYVYWYQQKPGQAPVLVIYGDSKRPSGIPERFSGSNSGNTATLTISGTQAEDEADYYCQTYTGGASLVFG GGTKLTVLGQ |
| 21 | QVQLVQSGAEVAKPGTSVKLSCKASGYTFTDYWMQWVKQRPGQGLEWIGTIYPGDGDTGYAQKFQGKATLTADKSS KTVYMHLSSLASEDSAVYYCARGDYYGSNSLDYWGQGTSVTVSSGGGGSGGGGSGGGGSDIVMTQSHLSMSTSLGD PVSITCKASQDVSTVVAWYQQKPGQSPRRLIYSASYRYIGVPDRFTGSGAGTDFTFTISSVQAEDLAVYYCQQHYSPPYT FGGGTKLEIK |
| 22 | QVQLVQSGAEVKKPGSSVKVSCKAFGGTFSSYAISWVRQAPGQGLEWMGRIIRFLGIANYAQKFQGRVTLIADKSTNT AYMELSSLRSEDTAVYYCAGEPGERDPDAVDIWGQGTMVTVSSGGGGSGGGGSGGGGSDIQMTQSPSSLSASVGDR VTITCRASQGISNYLAWFQQKPGKAPKSLIYAASSLQSGVPSRFSGSGSGTDFTLTISSLQPEDFATYYCQQYNSYPYTFG QGTKLEIK |
| 23 | ATGGCCTTACCAGTGACCGCCTTGCTCCTGCCGCTGGCCTTGCTGCTCCACGCCGCCAGGCCGGAGGTGCAGCT GCTGGAGAGCGGCGGCGGCCTGGTGCAGCCCGGCGGCAGCCTGCGGCTGAGCTGCGCCGTGAGCGGCTTCAC CTTCAACAGCTTCGCCATGAGCTGGGTGCGGCAGGCCCCCGGCAAGGGCCTGGAGTGGGTGAGCGCCATCAGC GGCAGCGGCGGCGGCACCTACTACGCCGACAGCGTGAAGGGCCGGTTCACCATCAGCCGGGACAACAGCAAG AACACCCTGTACCTGCAGATGAACAGCCTGCGGGCCGAGGACACCGCCGTGTACTTCTGCGCCAAGGACAAGAT CCTGTGGTTCGGCGAGCCCGTGTTCGACTACTGGGGCCAGGGCACCCTGGTGACCGTGAGCAGCGGTGGAGGC GGTTCAGGCGGAGGTGGTTCTGGCGGTGGCGGATCGGAGATCGTGCTGACCCAGAGCCCCGCCACCCTGAGCC TGAGCCCCGGCGAGCGGGCCACCCTGAGCTGCCGGGCCAGCCAGAGCGTGAGCAGCTACCTGGCCTGGTACCA GCAGAAGCCCGGCCAGGCCCCCCGGCTGCTGATCTACGACGCCAGCAACCGGGCCACCGGCATCCCCGCCCGG TTCAGCGGCAGCGGCAGCGGCACCGACTTCACCCTGACCATCAGCAGCCTGGAGCCCGAGGACTTCGCCGTGT ACTACTGCCAGCAGCGGAGCAACTGGCCCCCCACCTTCGGCCAGGGCACCAAGGTGGAGATCAAGGAGTCCAA |
| | ATATGGTCCCCCATGCCCACCATGCCCAATGTTTTGGGTGCTGGTGGTGGTTGGTGGAGTCCTGGCTTGCTATAG CTTGCTAGTAACAGTGGCCTTTATTATTTTCTGGGTGAAACGGGGCAGAAAGAAACTCCTGTATATATTCAAACAA CCATTTATGAGACCAGTACAAACTACTCAAGAGGAAGATGGCTGTAGCTGCCGATTTCCAGAAGAAGAAGAAGG AGGATGTGAACTGAGAGTGAAGTTCAGCAGGAGCGCAGACGCCCCCGCGTACCAGCAGGGCCAGAACCAGCTC TATAACGAGCTCAATCTAGGACGAAGAGAGGAGTACGATGTTTTGGACAAGAGACGTGGCCGGGACCCTGAGAT GGGGGGAAAGCCGCAGAGAAGGAAGAACCCTCAGGAAGGCCTGTACAATGAACTGCAGAAAGATAAGATGGC GGAGGCCTACAGTGAGATTGGGATGAAAGGCGAGCGCCGGAGGGGCAAGGGGCACGATGGCCTTTACCAGGG TCTCAGTACAGCCACCAAGGACACCTACGACGCCCTTCACATGCAGGCCCTGCCCCCTCGC |
| 24 | MALPVTALLLPLALLLHAARPEVQLLESGGGLVQPGGSLRLSCAVSGFTFNSFAMSWVRQAPGKGLEWVSAISGSGGG TYYADSVKGRFTISRDNSKNTLYLQMNSLRAEDTAVYFCAKDKILWFGEPVFDYWGQGTLVTVSSGGGGSGGGGSGG GGSEIVLTQSPATLSLSPGERATLSCRASQSVSSYLAWYQQKPGQAPRLLIYDASNRATGIPARFSGSGSGTDFTLTISSLE PEDFAVYYCQQRSNWPPTFGQGTKVEIKESKYGPPCPPCPMFWVLVVVGGVLACYSLLVTVAFIIFWVKRGRKKLLYIFK QPFMRPVQTTQEEDGCSCRFPEEEEGGCELRVKFSRSADAPAYQQGQNQLYNELNLGRREEYDVLDKRRGRDPEMG GKPQRRKNPQEGLYNELQKDKMAEAYSEIGMKGERRRGKGHDGLYQGLSTATKDTYDALHMQALPPR |
| 25 | ATGGCTCTGCCTGTGACAGCTCTGCTGCTGCCTCTGGCTCTGCTTCTGCATGCCGCTAGACCTGAAGTGCAGCTG CTGGAATCTGGCGGAGGACTGGTTCAACCTGGCGGCTCTCTGAGACTGAGCTGTGCCGTGTCCGGCTTCACCTTC AACAGCTTCGCCATGAGCTGGGTCCGACAGGCCCCTGGAAAAGGCCTTGAATGGGTGTCCGCCATCTCTGGAAG CGGCGGAGGCACATATTACGCCGACTCTGTGAAGGGCAGATTCACCATCAGCCGGGACAACAGCAAGAACACC CTGTACCTGCAGATGAACTCCCTGAGAGCCGAGGATACCGCCGTGTACTTCTGCGCCAAGGACAAGATCCTTTG GTTCGGCGAGCCCGTGTTCGACTATTGGGGACAGGGAACCCTGGTCACCGTTTCTAGCGGAGGCGGAGGTTCTG GTGGTGGTGGATCAGGTGGCGGCGGATCTGAGATTGTGCTGACACAGTCTCCCGCCACACTGTCTCTTAGCCCTG GCGAAAGAGCCACACTGAGCTGCAGAGCCAGCCAGAGCGTGTCCTCTTACCTGGCCTGGTATCAGCAGAAGCC CGGCCAAGCTCCTCGGCTGCTGATCTACGATGCCAGCAATAGAGCCACAGGCATCCCTGCCAGATTTTCCGGCT CTGGCAGCGGCACCGATTTCACCCTGACCATAAGCAGCCTGGAACCTGAGGACTTCGCCGTGTATTACTGCCAG CAGCGGAGCAACTGGCCTCCTACATTTGGCCAGGGAACCAAAGTCGAGATCAAGGAGTCTAAGTACGGCCCTCC TTGTCCTCCATGTCCTATGTTTTGGGTCCTCGTCGTCGTCGGAGGCGTGCTGGCTTGCTATAGCCTGCTTGTGACA GTGGCCTTTATTATCTTCTGGGTTAGAAGCAAGCGGAGCCGGCTGCTGCACAGCGACTACATGAACATGACCCCT AGACGGCCCGGACCTACCAGAAAGCACTACCAGCCTTACGCTCCTCCTCGGGATTTCGCCGCCTACAGATCTCG CGTGAAATTCTCCAGAAGCGCTGACGCCCCTGCTTATCAGCAAGGCCAGAATCAGCTCTATAACGAACTCAATCT CGGCAGGCGCGAGGAATATGATGTGCTGGATAAGAGGCGCGGCAGGGACCCAGAAATGGGAGGAAAACCTCA GCGGCGGAAAAACCCACAAGAAGGCCTTTACAACGAACTGCAAAAGGATAAGATGGCAGAAGCTTACTCCGAG ATTGGCATGAAGGGCGAACGTCGGAGAGGAAAAGGCCACGACGGACTCTATCAGGGACTGTCTACAGCCACAA AGGACACATACGACGCACTCCATATGCAGGCTCTCCCACCTAGA |
| 26 | MALPVTALLLPLALLLHAARPEVQLLESGGGLVQPGGSLRLSCAVSGFTFNSFAMSWVRQAPGKGLEWVSAISGSGGG TYYADSVKGRFTISRDNSKNTLYLQMNSLRAEDTAVYFCAKDKILWFGEPVFDYWGQGTLVTVSSGGGGSGGGGSGG GGSEIVLTQSPATLSLSPGERATLSCRASQSVSSYLAWYQQKPGQAPRLLIYDASNRATGIPARFSGSGSGTDFTLTISSLE PEDFAVYYCQQRSNWPPTFGQGTKVEIKESKYGPPCPPCPMFWVLVVVGGVLACYSLLVTVAFIIFWVRSKRSRLLHSD YMNMTPRRPGPTRKHYQPYAPPRDFAAYRSRVKFSRSADAPAYQQGQNQLYNELNLGRREEYDVLDKRRGRDPEMG GKPQRRKNPQEGLYNELQKDKMAEAYSEIGMKGERRRGKGHDGLYQGLSTATKDTYDALHMQALPPR |
| 27 | MALPVTALLLPLALLLHAARPQVQLVESGGGLVQPGGSLRLSCAASGFTFSSYYMNWVRQAPGKGLEWVSGISGDPSN TYYADSVKGRFTISRDNSKNTLYLQMNSLRAEDTAVYYCARDLPLVYTGFAYWGQGTLVTVSSGGGGSGGGGSGGGG SDIELTQPPSVSVAPGQTARISCSGDNLRHYYVYWYQQKPGQAPVLVIYGDSKRPSGIPERFSGSNSGNTATLTISGTQA EDEADYYCQTYTGGASLVFGGGTKLTVLGQESKYGPPCPPCPMFWVLVVVGGVLACYSLLVTVAFIIFWVKRGRKKLLYI FKQPFMRPVQTTQEEDGCSCRFPEEEEGGCELRVKFSRSADAPAYQQGQNQLYNELNLGRREEYDVLDKRRGRDPEM GGKPQRRKNPQEGLYNELQKDKMAEAYSEIGMKGERRRGKGHDGLYQGLSTATKDTYDALHMQALPPR↵ |
| 28 | MALPVTALLLPLALLLHAARPQVQLVQSGAEVAKPGTSVKLSCKASGYTFTDYWMQWVKQRPGQGLEWIGTIYPGDG DTGYAQKFQGKATLTADKSSKTVYMHLSSLASEDSAVYYCARGDYYGSNSLDYWGQGTSVTVSSGGGGSGGGGSGG GGSDIVMTQSHLSMSTSLGDPVSITCKASQDVSTVVAWYQQKPGQSPRRLIYSASYRYIGVPDRFTGSGAGTDFTFTISS VQAEDLAVYYCQQHYSPPYTFGGGTKLEIKESKYGPPCPPCPMFWVLVVVGGVLACYSLLVTVAFIIFWVKRGRKKLLYIF KQPFMRPVQTTQEEDGCSCRFPEEEEGGCELRVKFSRSADAPAYQQGQNQLYNELNLGRREEYDVLDKRRGRDPEMG GKPQRRKNPQEGLYNELQKDKMAEAYSEIGMKGERRRGKGHDGLYQGLSTATKDTYDALHMQALPPR |
| 29 | ATGGCCTTACCAGTGACCGCCTTGCTCCTGCCGCTGGCCTTGCTGCTCCACGCCGCCAGGCCGCAGGTGCAGCT GGTGCAGAGCGGCGCCGAGGTGGCCAAGCCCGGCACCAGCGTGAAGCTGAGCTGCAAGGCCAGCGGCTACAC |
| | CTTCACCGACTACTGGATGCAGTGGGTGAAGCAGCGGCCCGGCCAGGGCCTGGAGTGGATCGGCACCATCTAC CCCGGCGACGGCGACACCGGCTACGCCCAGAAGTTCCAGGGCAAGGCCACCCTGACCGCCGACAAGAGCAGC AAGACCGTGTACATGCACCTGAGCAGCCTGGCCAGCGAGGACAGCGCCGTGTACTACTGCGCCCGGGGCGACT ACTACGGCAGCAACAGCCTGGACTACTGGGGCCAGGGCACCAGCGTGACCGTGAGCAGCGGTGGAGGCGGTTC AGGCGGAGGTGGTTCTGGCGGTGGCGGATCGGACATCGTGATGACCCAGAGCCACCTGAGCATGAGCACCAGC CTGGGCGACCCCGTGAGCATCACCTGCAAGGCCAGCCAGGACGTGAGCACCGTGGTGGCCTGGTACCAGCAGA AGCCCGGCCAGAGCCCCCGGCGGCTGATCTACAGCGCCAGCTACCGGTACATCGGCGTGCCCGACCGGTTCAC CGGCAGCGGCGCCGGCACCGACTTCACCTTCACCATCAGCAGCGTGCAGGCCGAGGACCTGGCCGTGTACTACT GCCAGCAGCACTACAGCCCCCCCTACACCTTCGGCGGCGGCACCAAGCTGGAGATCAAGGAGTCCAAATATGG TCCCCCATGCCCACCATGCCCAATGTTTTGGGTGCTGGTGGTGGTTGGTGGAGTCCTGGCTTGCTATAGCTTGCTA GTAACAGTGGCCTTTATTATTTTCTGGGTGAAACGGGGCAGAAAGAAACTCCTGTATATATTCAAACAACCATTTA TGAGACCAGTACAAACTACTCAAGAGGAAGATGGCTGTAGCTGCCGATTTCCAGAAGAAGAAGAAGGAGGATG TGAACTGAGAGTGAAGTTCAGCAGGAGCGCAGACGCCCCCGCGTACCAGCAGGGCCAGAACCAGCTCTATAAC GAGCTCAATCTAGGACGAAGAGAGGAGTACGATGTTTTGGACAAGAGACGTGGCCGGGACCCTGAGATGGGGG GAAAGCCGCAGAGAAGGAAGAACCCTCAGGAAGGCCTGTACAATGAACTGCAGAAAGATAAGATGGCGGAGG CCTACAGTGAGATTGGGATGAAAGGCGAGCGCCGGAGGGGCAAGGGGCACGATGGCCTTTACCAGGGTCTCAG TACAGCCACCAAGGACACCTACGACGCCCTTCACATGCAGGCCCTGCCCCCTCGC |
| 30 | ATGTCTCGCTCCGTGGCTCTGGCCGTCTTGGCCTTGTTATCTCTTTCTGGCCTGGAGGCTGTCATGGCGCCCCGAA CCGTCCTCCTGGGTGGTGGCGGAAGCGGTGGTGGCGGTTCTGGTGGTGGCGGCTCCATCCAGCGTACTCCAAAG ATTCAGGTTTACTCACGTCATCCAGCAGAGAATGGAAAGTCAAATTTCCTGAATTGCTATGTGTCTGGGTTTCATC CATCCGACATTGAAGTTGACTTACTGAAGAATGGAGAGAGAATTGAAAAAGTGGAGCATTCAGACTTGTCTTTCA GCAAGGACTGGTCTTTCTATCTCTTGTACTACACTGAATTCACCCCCACTGAAAAAGATGAGTATGCCTGCCGTGT GAACCATGTGACTTTGTCACAGCCCAAGATAGTTAAGTGGGATCGCGACATGGGTGGTGGCGGTTCTGGTGGTG GCGGTAGTGGCGGCGGAGGAAGCGGTGGTGGCGGTTCCGGATCTCACTCCTTGAAGTATTTCCACACTTCCGTG TCCCGGCCCGGCCGCGGGGAGCCCCGCTTCATCTCTGTGGGCTACGTGGACGACACCCAGTTCGTGCGCTTCGA CAACGACGCCGCGAGTCCGAGGATGGTGCCGCGGGCGCCGTGGATGGAGCAGGAGGGGTCAGAGTATTGGGA CCGGGAGACACGGAGCGCCAGGGACACCGCACAGATTTTCCGAGTGAACCTGCGGACGCTGCGCGGCTACTAC AATCAGAGCGAGGCCGGGTCTCACACCCTGCAGTGGATGCATGGCTGCGAGCTGGGGCCCGACAGGCGCTTCC TCCGCGGGTATGAACAGTTCGCCTACGACGGCAAGGATTATCTCACCCTGAATGAGGACCTGCGCTCCTGGACC GCGGTGGACACGGCGGCTCAGATCTCCGAGCAAAAGTCAAATGATGCCTCTGAGGCGGAGCACCAGAGAGCCT ACCTGGAAGACACATGCGTGGAGTGGCTCCACAAATACCTGGAGAAGGGGAAGGAGACGCTGCTTCACCTGGA GCCCCCAAAGACACACGTGACTCACCACCCCATCTCTGACCATGAGGCCACCCTGAGGTGCTGGGCCCTGGGCT TCTACCCTGCGGAGATCACACTGACCTGGCAGCAGGATGGGGAGGGCCATACCCAGGACACGGAGCTCGTGGA GACCAGGCCTGCAGGGGATGGAACCTTCCAGAAGTGGGCAGCTGTGGTGGTGCCTTCTGGAGAGGAGCAGAGA TACACGTGCCATGTGCAGCATGAGGGGCTACCCGAGCCCGTCACCCTGAGATGGAAGCCGGCTTCCCAGCCCAC CATCCCCATCGTGGGCATCATTGCTGGCCTGGTTCTCCTTGGATCTGTGGTCTCTGGAGCTGTGGTTGCTGCTGTG ATATGGAGGAAGAAGAGCTCAGGTGGAAAAGGAGGGAGCTACTCTAAGGCTGAGTGGAGCGACAGTGCCCAG GGGTCTGAGTCTCACAGCTTG |
| 31 | ATGTCTCGCTCCGTGGCTCTGGCCGTCTTGGCCTTGTTATCTCTTTCTGGCCTGGAGGCTGTCATGGCGCCCCGAG CCCTCCTCCTGGGTGGTGGCGGAAGCGGTGGTGGCGGTTCTGGTGGTGGCGGCTCCATCCAGCGTACTCCAAAG ATTCAGGTTTACTCACGTCATCCAGCAGAGAATGGAAAGTCAAATTTCCTGAATTGCTATGTGTCTGGGTTTCATC CATCCGACATTGAAGTTGACTTACTGAAGAATGGAGAGAGAATTGAAAAAGTGGAGCATTCAGACTTGTCTTTCA GCAAGGACTGGTCTTTCTATCTCTTGTACTACACTGAATTCACCCCCACTGAAAAAGATGAGTATGCCTGCCGTGT GAACCATGTGACTTTGTCACAGCCCAAGATAGTTAAGTGGGATCGCGACATGGGTGGTGGCGGTTCTGGTGGTG GCGGTAGTGGCGGCGGAGGAAGCGGTGGTGGCGGTTCCGGATCTCACTCCTTGAAGTATTTCCACACTTCCGTG TCCCGGCCCGGCCGCGGGGAGCCCCGCTTCATCTCTGTGGGCTACGTGGACGACACCCAGTTCGTGCGCTTCGA CAACGACGCCGCGAGTCCGAGGATGGTGCCGCGGGCGCCGTGGATGGAGCAGGAGGGGTCAGAGTATTGGGA CCGGGAGACACGGAGCGCCAGGGACACCGCACAGATTTTCCGAGTGAACCTGCGGACGCTGCGCGGCTACTAC AATCAGAGCGAGGCCGGGTCTCACACCCTGCAGTGGATGCATGGCTGCGAGCTGGGGCCCGACAGGCGCTTCC TCCGCGGGTATGAACAGTTCGCCTACGACGGCAAGGATTATCTCACCCTGAATGAGGACCTGCGCTCCTGGACC GCGGTGGACACGGCGGCTCAGATCTCCGAGCAAAAGTCAAATGATGCCTCTGAGGCGGAGCACCAGAGAGCCT ACCTGGAAGACACATGCGTGGAGTGGCTCCACAAATACCTGGAGAAGGGGAAGGAGACGCTGCTTCACCTGGA GCCCCCAAAGACACACGTGACTCACCACCCCATCTCTGACCATGAGGCCACCCTGAGGTGCTGGGCCCTGGGCT |
| | TCTACCCTGCGGAGATCACACTGACCTGGCAGCAGGATGGGGAGGGCCATACCCAGGACACGGAGCTCGTGGA GACCAGGCCTGCAGGGGATGGAACCTTCCAGAAGTGGGCAGCTGTGGTGGTGCCTTCTGGAGAGGAGCAGAGA TACACGTGCCATGTGCAGCATGAGGGGCTACCCGAGCCCGTCACCCTGAGATGGAAGCCGGCTTCCCAGCCCAC CATCCCCATCGTGGGCATCATTGCTGGCCTGGTTCTCCTTGGATCTGTGGTCTCTGGAGCTGTGGTTGCTGCTGTG ATATGGAGGAAGAAGAGCTCAGGTGGAAAAGGAGGGAGCTACTCTAAGGCTGAGTGGAGCGACAGTGCCCAG GGGTCTGAGTCTCACAGCTTG |
| 32 | ATGTCTCGCTCCGTGGCTCTGGCCGTCTTGGCCTTGTTATCTCTTTCTGGCCTGGAGGCTGTTATGGCTCCGCGGA CTTTAATTTTAGGTGGTGGCGGATCCGGTGGTGGCGGTTCTGGTGGTGGCGGCTCCATCCAGCGTACTCCAAAGA TTCAGGTTTACTCACGTCATCCAGCAGAGAATGGAAAGTCAAATTTCCTGAATTGCTATGTGTCTGGGTTTCATCC ATCCGACATTGAAGTTGACTTACTGAAGAATGGAGAGAGAATTGAAAAAGTGGAGCATTCAGACTTGTCTTTCAG CAAGGACTGGTCTTTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGCGACATGGGTGGTGGCGGTTCTGGTGGTGG CGGTAGTGGCGGCGGAGGAAGCGGTGGTGGCGGTTCCGGATCTCACTCCTTGAAGTATTTCCACACTTCCGTGTC CCGGCCCGGCCGCGGGGAGCCCCGCTTCATCTCTGTGGGCTACGTGGACGACACCCAGTTCGTGCGCTTCGACA ACGACGCCGCGAGTCCGAGGATGGTGCCGCGGGCGCCGTGGATGGAGCAGGAGGGGTCAGAGTATTGGGACC GGGAGACACGGAGCGCCAGGGACACCGCACAGATTTTCCGAGTGAACCTGCGGACGCTGCGCGGCTACTACAA TCAGAGCGAGGCCGGGTCTCACACCCTGCAGTGGATGCATGGCTGCGAGCTGGGGCCCGACAGGCGCTTCCTC CGCGGGTATGAACAGTTCGCCTACGACGGCAAGGATTATCTCACCCTGAATGAGGACCTGCGCTCCTGGACCGC GGTGGACACGGCGGCTCAGATCTCCGAGCAAAAGTCAAATGATGCCTCTGAGGCGGAGCACCAGAGAGCCTAC CTGGAAGACACATGCGTGGAGTGGCTCCACAAATACCTGGAGAAGGGGAAGGAGACGCTGCTTCACCTGGAGC CCCCAAAGACACACGTGACTCACCACCCCATCTCTGACCATGAGGCCACCCTGAGGTGCTGGGCCCTGGGCTTC TACCCTGCGGAGATCACACTGACCTGGCAGCAGGATGGGGAGGGCCATACCCAGGACACGGAGCTCGTGGAGA CCAGGCCTGCAGGGGATGGAACCTTCCAGAAGTGGGCAGCTGTGGTGGTGCCTTCTGGAGAGGAGCAGAGATA CACGTGCCATGTGCAGCATGAGGGGCTACCCGAGCCCGTCACCCTGAGATGGAAGCCGGCTTCCCAGCCCACC ATCCCCATCGTGGGCATCATTGCTGGCCTGGTTCTCCTTGGATCTGTGGTCTCTGGAGCTGTGGTTGCTGCTGTGA TATGGAGGAAGAAGAGCTCAGGTGGAAAAGGAGGGAGCTACTCTAAGGCTGAGTGGAGCGACAGTGCCCAGG GGTCTGAGTCTCACAGCTTG |
| 33 | ATGTCTCGCTCCGTGGCTCTGGCCGTCTTGGCCTTGTTATCTCTTTCTGGCCTGGAGGCTGTTATGGCTCCGCGGA CTTTATTTTTAGGTGGTGGCGGATCCGGTGGTGGCGGTTCTGGTGGTGGCGGCTCCATCCAGCGTACTCCAAAGA TTCAGGTTTACTCACGTCATCCAGCAGAGAATGGAAAGTCAAATTTCCTGAATTGCTATGTGTCTGGGTTTCATCC ATCCGACATTGAAGTTGACTTACTGAAGAATGGAGAGAGAATTGAAAAAGTGGAGCATTCAGACTTGTCTTTCAG CAAGGACTGGTCTTTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGCGACATGGGTGGTGGCGGTTCTGGTGGTGG CGGTAGTGGCGGCGGAGGAAGCGGTGGTGGCGGTTCCGGATCTCACTCCTTGAAGTATTTCCACACTTCCGTGTC CCGGCCCGGCCGCGGGGAGCCCCGCTTCATCTCTGTGGGCTACGTGGACGACACCCAGTTCGTGCGCTTCGACA ACGACGCCGCGAGTCCGAGGATGGTGCCGCGGGCGCCGTGGATGGAGCAGGAGGGGTCAGAGTATTGGGACC GGGAGACACGGAGCGCCAGGGACACCGCACAGATTTTCCGAGTGAACCTGCGGACGCTGCGCGGCTACTACAA TCAGAGCGAGGCCGGGTCTCACACCCTGCAGTGGATGCATGGCTGCGAGCTGGGGCCCGACAGGCGCTTCCTC CGCGGGTATGAACAGTTCGCCTACGACGGCAAGGATTATCTCACCCTGAATGAGGACCTGCGCTCCTGGACCGC GGTGGACACGGCGGCTCAGATCTCCGAGCAAAAGTCAAATGATGCCTCTGAGGCGGAGCACCAGAGAGCCTAC CTGGAAGACACATGCGTGGAGTGGCTCCACAAATACCTGGAGAAGGGGAAGGAGACGCTGCTTCACCTGGAGC CCCCAAAGACACACGTGACTCACCACCCCATCTCTGACCATGAGGCCACCCTGAGGTGCTGGGCCCTGGGCTTC TACCCTGCGGAGATCACACTGACCTGGCAGCAGGATGGGGAGGGCCATACCCAGGACACGGAGCTCGTGGAGA CCAGGCCTGCAGGGGATGGAACCTTCCAGAAGTGGGCAGCTGTGGTGGTGCCTTCTGGAGAGGAGCAGAGATA CACGTGCCATGTGCAGCATGAGGGGCTACCCGAGCCCGTCACCCTGAGATGGAAGCCGGCTTCCCAGCCCACC ATCCCCATCGTGGGCATCATTGCTGGCCTGGTTCTCCTTGGATCTGTGGTCTCTGGAGCTGTGGTTGCTGCTGTGA TATGGAGGAAGAAGAGCTCAGGTGGAAAAGGAGGGAGCTACTCTAAGGCTGAGTGGAGCGACAGTGCCCAGG GGTCTGAGTCTCACAGCTTG |
| 34 | ATGGCTCTGCCTGTGACAGCTCTGCTGCTGCCTCTGGCTCTGCTTCTGCATGCCGCTAGACCTGAAGTGCAGCTG CTGGAATCTGGCGGAGGACTGGTTCAACCTGGCGGCTCTCTGAGACTGAGCTGTGCCGTGTCCGGCTTCACCTTC AACAGCTTCGCCATGAGCTGGGTCCGACAGGCCCCTGGAAAAGGCCTTGAATGGGTGTCCGCCATCTCTGGAAG CGGCGGAGGCACATATTACGCCGACTCTGTGAAGGGCAGATTCACCATCAGCCGGGACAACAGCAAGAACACC CTGTACCTGCAGATGAACTCCCTGAGAGCCGAGGATACCGCCGTGTACTTCTGCGCCAAGGACAAGATCCTTTG |
| | GTTCGGCGAGCCCGTGTTCGACTATTGGGGACAGGGAACCCTGGTCACCGTTTCTAGCGGAGGCGGAGGTTCTG GTGGTGGTGGATCAGGTGGCGGCGGATCTGAGATTGTGCTGACACAGTCTCCCGCCACACTGTCTCTTAGCCCTG GCGAAAGAGCCACACTGAGCTGCAGAGCCAGCCAGAGCGTGTCCTCTTACCTGGCCTGGTATCAGCAGAAGCC CGGCCAAGCTCCTCGGCTGCTGATCTACGATGCCAGCAATAGAGCCACAGGCATCCCTGCCAGATTTTCCGGCT CTGGCAGCGGCACCGATTTCACCCTGACCATAAGCAGCCTGGAACCTGAGGACTTCGCCGTGTATTACTGCCAG CAGCGGAGCAACTGGCCTCCTACATTTGGCCAGGGAACCAAAGTCGAGATCAAGGAGTCTAAGTACGGCCCTCC TTGTCCTCCATGTCCTATGTTCTGGGTGCTCGTGGTTGTTGGCGGAGTGCTGGCCTGTTACTCTCTGCTGGTGACC GTGGCCTTCATCATCTTTTGGGTCAAGCGGGGCAGAAAGAAGCTGCTGTACATCTTCAAGCAGCCCTTCATGCGG CCCGTGCAGACCACACAAGAGGAAGATGGCTGCTCCTGCAGATTCCCCGAGGAAGAAGAAGGCGGCTGCGAGC TGAGAGTGAAGTTCAGCAGATCCGCCGACGCTCCTGCCTATCAGCAGGGACAGAACCAGCTGTACAACGAGCTG AACCTCGGGAGAAGAGAAGAGTACGACGTGCTGGACAAGCGGAGAGGCAGAGATCCTGAGATGGGCGGAAAG CCCCAGCGGAGAAAGAATCCTCAAGAGGGCCTGTATAATGAGCTGCAGAAAGACAAGATGGCCGAGGCCTACA GCGAGATCGGAATGAAGGGCGAGCGCAGAAGAGGCAAGGGACACGATGGACTGTACCAGGGCCTGAGCACCG CCACCAAGGATACCTATGATGCCCTGCACATGCAGGCCCTGCCTCCAAGAGGAAGCGGAGCTACTAACTTCAGC CTGCTGAAGCAGGCTGGAGACGTGGAGGAGAACCCTGGGCCCATGGCACTGCCAGTTACTGCCCTGCTGCTCCC ACTTGCACTGCTGCTTCATGCTGCCAGACCTCAGGTGCAGCTGGTGCAGAGCGGCGCCGAGGTGAAGAAGCCCG GCGCCAGCGTGAAGGTCAGCTGCAAGGCCAGCGGCTACACCTTCACCAGCTACTGGATGAACTGGGTGCGGCA GGCCCCCGGCCAGGGCCTGGAGTGGATGGGCCGGATCGACCCCTACGACAGCGAGACCCACTACGCCCAGAA GCTGCAGGGCCGGGTGACCATGACCACCGACACCAGCACCAGCACCGCCTACATGGAGCTGCGGAGCCTGCGG AGCGACGACACCGCCGTGTACTACTGCGCCCGGGGCGGCTACGACTTCGACGTGGGCACCCTGTACTGGTTCTT CGACGTGTGGGGCCAGGGCACCACCGTGACCGTGAGCAGCGGTGGAGGCGGTTCAGGCGGAGGTGGTTCTGG CGGTGGCGGATCGGACATCCAGATGACCCAGAGCCCCAGCAGCCTGAGCGCCAGCGTGGGCGACCGGGTGAC CATCACCTGCCGGGCCAGCGAGAACATCTACAGCTACCTGGCCTGGTACCAGCAGAAGCCCGGCAAGGCCCCC AAGCTGCTGATCTACAACGCCAAGACCCTGGCCGAGGGCGTGCCCAGCCGGTTCAGCGGCAGCGGCAGCGGCA CCGACTTCACCCTGACCATCAGCAGCCTGCAGCCCGAGGACTTCGCCACCTACTACTGCCAGCACCACTACGGC ACCCCCCGGACCTTCGGCGGCGGCACCAAGGTGGAGATCAAGACCACCACACCAGCACCTAGGCCACCTACTC CAGCTCCTACCATTGCCTCTCAACCCCTGAGCCTCAGACCTGAGGCATGTAGACCAGCCGCAGGCGGCGCTGTT CACACAAGAGGCCTCGACTTTGCCTGTGATTTTTGGGTCCTCGTCGTCGTCGGAGGCGTGCTGGCTTGCTATAGC CTGCTTGTGACAGTGGCCTTTATTATCTTCTGGGTTAGAAGCAAGCGGAGCCGGCTGCTGCACAGCGACTACATG AACATGACCCCTAGACGGCCCGGACCTACCAGAAAGCACTACCAGCCTTACGCTCCTCCTCGGGATTTCGCCGC CTACAGATCTCGCGTGAAATTCTCCAGAAGCGCTGACGCCCCTGCTTATCAGCAAGGCCAGAATCAGCTCTATAA CGAACTCAATCTCGGCAGGCGCGAGGAATATGATGTGCTGGATAAGAGGCGCGGCAGGGACCCAGAAATGGGA GGAAAACCTCAGCGGCGGAAAAACCCACAAGAAGGCCTTTACAACGAACTGCAAAAGGATAAGATGGCAGAAG CTTACTCCGAGATTGGCATGAAGGGCGAACGTCGGAGAGGAAAAGGCCACGACGGACTCTATCAGGGACTGTC TACAGCCACAAAGGACACATACGACGCACTCCATATGCAGGCTCTCCCACCTAGA |
| 35 | ATGGCCTTACCAGTGACCGCCTTGCTCCTGCCGCTGGCCTTGCTGCTCCACGCCGCCAGGCCGGAGGTTCAGCT GCAGCAGTCTGGCGCCGAACTTGTGAAACCTGGCGCCTCTGTGAAGCTGAGCTGTAAAGCCAGCGGCTACACCT TCACCAGCTACTGGATGCAGTGGGTCAAGCAGAGGCCTGGACAGGGACTCGAGTGGATCGGAGAGATCGACCC CAGCGACAGCTACACCAACTACAACCAGAAGTTCAAGGGCAAAGCCACACTGACCGTGGACACCAGCAGCAGC ACAACCTACATGCAGCTGAGCAGCCTGACCAGCGAGGATAGCGCCGTGTACTACTGTGCCAGATGGGGCATGG CCTACGGCACCAGCTCCTACTGGTACTTCGATGTGTGGGGCACCGGCACCACCGTGACAGTTAGTTCTGGCGGA GGAAGCGGTGGCGGATCAGGTGGTGGATCTGGCGGCGGATCTGACATCCAGATGACCCAGACAACCAGCAGCC TGTCTGCCAGCCTGGGCGATAGAGTGACCATCTCCTGTAGAGCCAGCCAGGACATCAGCAACTACCTGAACTGG TATCAGCAGAAACCCGACGGCACCGTGAAGCTGCTGATCTACTACACCTCCAGACTGCACAGCGGCGTGCCCAG CAGATTTTCTGGCTCTGGCAGCGGCACCGACTACTCCCTGACAATCAGCAACCTGGAACAAGAGGATATCGCTA CCTACTTCTGCCAGCAAGGCAACACCCTGCCTTGGACATTTGGCGGAGGCACCAAGCTGGAAATCAAGACCACC ACCCCGGCGCCGCGCCCGCCGACCCCGGCGCCGACCATTGCGAGCCAGCCGCTGAGCCTGCGCCCGGAAGCG TGCCGCCCGGCGGCGGCGGTGCATACCCGCGGCCTGGATTTTGCGTGCGATGCGGCGGTGCTGGTGCTGCTGGT GATTGTGATTATTAGCCTGATTGTGCTGGTGGTGATTTGG |
| 36 | ATGGCTCTGCCTGTGACAGCTCTGCTGCTGCCTCTGGCTCTGCTTCTGCATGCCGCTAGACCTGAAGTGCAGCTG CTGGAATCTGGCGGAGGACTGGTTCAACCTGGCGGCTCTCTGAGACTGAGCTGTGCCGTGTCCGGCTTCACCTTC AACAGCTTCGCCATGAGCTGGGTCCGACAGGCCCCTGGAAAAGGCCTTGAATGGGTGTCCGCCATCTCTGGAAG CGGCGGAGGCACATATTACGCCGACTCTGTGAAGGGCAGATTCACCATCAGCCGGGACAACAGCAAGAACACC |
| | CTGTACCTGCAGATGAACTCCCTGAGAGCCGAGGATACCGCCGTGTACTTCTGCGCCAAGGACAAGATCCTTTG GTTCGGCGAGCCCGTGTTCGACTATTGGGGACAGGGAACCCTGGTCACCGTTTCTAGCGGAGGCGGAGGTTCTG GTGGTGGTGGATCAGGTGGCGGCGGATCTGAGATTGTGCTGACACAGTCTCCCGCCACACTGTCTCTTAGCCCTG GCGAAAGAGCCACACTGAGCTGCAGAGCCAGCCAGAGCGTGTCCTCTTACCTGGCCTGGTATCAGCAGAAGCC CGGCCAAGCTCCTCGGCTGCTGATCTACGATGCCAGCAATAGAGCCACAGGCATCCCTGCCAGATTTTCCGGCT CTGGCAGCGGCACCGATTTCACCCTGACCATAAGCAGCCTGGAACCTGAGGACTTCGCCGTGTATTACTGCCAG CAGCGGAGCAACTGGCCTCCTACATTTGGCCAGGGAACCAAAGTCGAGATCAAGGAGTCTAAGTACGGCCCTCC TTGTCCTCCATGTCCTATGTTCTGGGTGCTCGTGGTTGTTGGCGGAGTGCTGGCCTGTTACTCTCTGCTGGTGACC GTGGCCTTCATCATCTTTTGGGTCAAGCGGGGCAGAAAGAAGCTGCTGTACATCTTCAAGCAGCCCTTCATGCGG CCCGTGCAGACCACACAAGAGGAAGATGGCTGCTCCTGCAGATTCCCCGAGGAAGAAGAAGGCGGCTGCGAGC TGAGAGTGAAGTTCAGCAGATCCGCCGACGCTCCTGCCTATCAGCAGGGACAGAACCAGCTGTACAACGAGCTG AACCTCGGGAGAAGAGAAGAGTACGACGTGCTGGACAAGCGGAGAGGCAGAGATCCTGAGATGGGCGGAAAG CCCCAGCGGAGAAAGAATCCTCAAGAGGGCCTGTATAATGAGCTGCAGAAAGACAAGATGGCCGAGGCCTACA GCGAGATCGGAATGAAGGGCGAGCGCAGAAGAGGCAAGGGACACGATGGACTGTACCAGGGCCTGAGCACCG CCACCAAGGATACCTATGATGCCCTGCACATGCAGGCCCTGCCTCCAAGAGGAAGCGGAGCTACTAACTTCAGC CTGCTGAAGCAGGCTGGAGACGTGGAGGAGAACCCTGGGCCCATGGCACTGCCAGTTACTGCCCTGCTGCTCCC ACTTGCACTGCTGCTTCATGCTGCCAGACCTGAGGTGCAATTGCTGGAGTCTGGGGGAGGCTTGGTACAGCCTG GGGGGTCCATGAGACTCTCCTGTGCAGCCTCCGGATTCACCTTTAGCAGTTATGCCATGAGCTGGGTCCGCCAGG CTCCAGGGAAGGGGCTGGAGTGGGTCTCAGCTATTAGTGGTAGTGGTGGTAGCACATACTACGCAGACTCCGTG AAGGGCCGGTTCACCATCTCCAGAGACAATTCCAAGAACACGCTGTATCTGCAGATGAACAGCCTGAGAGCCGA GGACACGGCCGTATATTACTGTGCGAAATACGAAGGTCGTTACGTTCTGGGTGCAATGGACTACTGGGGCCAAG GAACCCTGGTCACCGTCTCGAGTGGTGGAGGCGGTTCAGGCGGAGGTGGTTCTGGCGGTGGCGGATCGGACAT CCAGATGACCCAGTCTCCTTCCACCCTGTCTGCATCTGTAGGAGACCGTGTCACCATCACTTGCCGTGCCAGTCA GAGTATTAGTAGCTGGTTGGCCTGGTATCAGCAGAAACCAGGGAAAGCCCCTAAGCTCCTGATCTATGATGCCTC CAGTTTGGAAAGTGGGGTCCCATCACGTTTCAGCGGCAGTGGATCCGGGACAGAATTCACTCTCACCATCAGCA GCTTGCAGCCTGATGATTTTGCAACTTATTACTGCCAACAGTACTTCTCTTACCCACTGACGTTTGGCCAGGGCAC CAAAGTCGAGATCAAGCGTACCACCACACCAGCACCTAGGCCACCTACTCCAGCTCCTACCATTGCCTCTCAACC CCTGAGCCTCAGACCTGAGGCATGTAGACCAGCCGCAGGCGGCGCTGTTCACACAAGAGGCCTCGACTTTGCCT GTGATTTTTGGGTCCTCGTCGTCGTCGGAGGCGTGCTGGCTTGCTATAGCCTGCTTGTGACAGTGGCCTTTATTAT CTTCTGGGTTAGAAGCAAGCGGAGCCGGCTGCTGCACAGCGACTACATGAACATGACCCCTAGACGGCCCGGA CCTACCAGAAAGCACTACCAGCCTTACGCTCCTCCTCGGGATTTCGCCGCCTACAGATCTCGCGTGAAATTCTCC AGAAGCGCTGACGCCCCTGCTTATCAGCAAGGCCAGAATCAGCTCTATAACGAACTCAATCTCGGCAGGCGCGA GGAATATGATGTGCTGGATAAGAGGCGCGGCAGGGACCCAGAAATGGGAGGAAAACCTCAGCGGCGGAAAAA CCCACAAGAAGGCCTTTACAACGAACTGCAAAAGGATAAGATGGCAGAAGCTTACTCCGAGATTGGCATGAAGG GCGAACGTCGGAGAGGAAAAGGCCACGACGGACTCTATCAGGGACTGTCTACAGCCACAAAGGACACATACGA CGCACTCCATATGCAGGCTCTCCCACCTAGA |
| 37 | ATGGCACTGCCAGTTACTGCCCTGCTGCTCCCACTTGCACTGCTGCTTCATGCTGCCAGACCTGAAATCGTGCTG ACCCAGTCTCCTGGAACTCTGTCTCTGTCCCCTGGCGAGAGGGCTACCCTGTCTTGTAGAGCCTCTCAGTCCGTGT CCTCCAGCTATCTCGCCTGGTATCAACAAAAGCCTGGGCAAGCCCCTCGGCTGCTGATATACGGGGCTTCTTCTC GCGCCACCGGCATTCCAGACAGATTCAGCGGATCTGGAAGCGGCACAGATTTCACACTGACCATCTCTCGGCTG GAACCTGAAGATTTCGCTGTCTACTACTGTCAGCAGTACAAGTCTCACCCCATCACCTTTGGCCAGGGAACAAAG GTCGAGATCAAGAGAGGTGGCGGCGGTAGTCAGGTGCAGCTGGTGCAGAGCGGCGCCGAGGTGAAGAAGCCC GGCGCCAGCGTGAAGGTCAGCTGCAAGGCCAGCGGCTACACCTTCACCAGCTACTGGATGAACTGGGTGCGGC AGGCCCCCGGCCAGGGCCTGGAGTGGATGGGCCGGATCGACCCCTACGACAGCGAGACCCACTACGCCCAGA AGCTGCAGGGCCGGGTGACCATGACCACCGACACCAGCACCAGCACCGCCTACATGGAGCTGCGGAGCCTGCG GAGCGACGACACCGCCGTGTACTACTGCGCCCGGGGCGGCTACGACTTCGACGTGGGCACCCTGTACTGGTTCT TCGACGTGTGGGGCCAGGGCACCACCGTGACCGTGAGCAGCGGTGGAGGCGGTTCAGGCGGAGGTGGTTCTGG CGGTGGCGGATCGGACATCCAGATGACCCAGAGCCCCAGCAGCCTGAGCGCCAGCGTGGGCGACCGGGTGAC CATCACCTGCCGGGCCAGCGAGAACATCTACAGCTACCTGGCCTGGTACCAGCAGAAGCCCGGCAAGGCCCCC AAGCTGCTGATCTACAACGCCAAGACCCTGGCCGAGGGCGTGCCCAGCCGGTTCAGCGGCAGCGGCAGCGGCA CCGACTTCACCCTGACCATCAGCAGCCTGCAGCCCGAGGACTTCGCCACCTACTACTGCCAGCACCACTACGGC ACCCCCCGGACCTTCGGCGGCGGCACCAAGGTGGAGATCAAGGGCGGTGGTGGAAGCGAGGTCCAGCTGTTGG AAAGTGGCGGTGGTCTTGTGCAGCCTGGCGGAAGCCTCAGACTTAGCTGTGCCGCCTCCGGCTTCACATTCAGC AGCTACGCCATGTCCTGGGTTCGACAAGCACCCGGAAAAGGATTGGAGTGGGTTTCAGCCATCAGCGGCAGCG |
| | GAGGCAGCACTTATTATGCCGATAGCGTGAAAGGCCGGTTCACCATCTCCAGAGACAACTCCAAGAATACGCTC TATCTCCAGATGAACTCTCTGCGCGCCGAAGATACAGCTGTGTACTATTGCGCCAGAGGCTGGCCCTCTCCTGTG ACCTTTGATTACTGGGGACAAGGGACCCTCGTGACCGTTTCTTCTACCACCACACCAGCACCTAGGCCACCTACT CCAGCTCCTACCATTGCCTCTCAACCCCTGAGCCTCAGACCTGAGGCATGTAGACCAGCCGCAGGCGGCGCTGT TCACACAAGAGGCCTCGACTTTGCCTGTGATATCTATATTTGGGCTCCCCTGGCCGGGACCTGCGGAGTTCTTCTG CTGTCTCTCGTGATCACACTGTATTGCAAGAGAGGCCGGAAGAAGCTCCTCTATATCTTTAAACAGCCGTTTATGC GCCCGGTCCAGACTACCCAAGAAGAGGACGGCTGTAGCTGTCGGTTCCCTGAAGAGGAAGAAGGCGGTTGCGA ACTGCGCGTGAAATTCTCCAGAAGCGCTGACGCCCCTGCTTATCAGCAAGGCCAGAATCAGCTCTATAACGAACT CAATCTCGGCAGGCGCGAGGAATATGATGTGCTGGATAAGAGGCGCGGCAGGGACCCAGAAATGGGAGGAAA ACCTCAGCGGCGGAAAAACCCACAAGAAGGCCTTTACAACGAACTGCAAAAGGATAAGATGGCAGAAGCTTAC TCCGAGATTGGCATGAAGGGCGAACGTCGGAGAGGAAAAGGCCACGACGGACTCTATCAGGGACTGTCTACAG CCACAAAGGACACATACGACGCACTCCATATGCAGGCTCTCCCACCTAGAGGAAGCGGAGCTACTAACTTCAGC CTGCTGAAGCAGGCTGGAGACGTGGAGGAGAACCCTGGGCCCATGGCTCTGCCTGTGACAGCTCTGCTGCTGCC TCTGGCTCTGCTTCTGCATGCCGCTAGACCTGAAGTGCAGCTGCTGGAATCTGGCGGAGGACTGGTTCAACCTGG CGGCTCTCTGAGACTGAGCTGTGCCGTGTCCGGCTTCACCTTCAACAGCTTCGCCATGAGCTGGGTCCGACAGGC CCCTGGAAAAGGCCTTGAATGGGTGTCCGCCATCTCTGGAAGCGGCGGAGGCACATATTACGCCGACTCTGTGA AGGGCAGATTCACCATCAGCCGGGACAACAGCAAGAACACCCTGTACCTGCAGATGAACTCCCTGAGAGCCGA GGATACCGCCGTGTACTTCTGCGCCAAGGACAAGATCCTTTGGTTCGGCGAGCCCGTGTTCGACTATTGGGGACA GGGAACCCTGGTCACCGTTTCTAGCGGAGGCGGAGGTTCTGGTGGTGGTGGATCAGGTGGCGGCGGATCTGAG ATTGTGCTGACACAGTCTCCCGCCACACTGTCTCTTAGCCCTGGCGAAAGAGCCACACTGAGCTGCAGAGCCAG CCAGAGCGTGTCCTCTTACCTGGCCTGGTATCAGCAGAAGCCCGGCCAAGCTCCTCGGCTGCTGATCTACGATGC CAGCAATAGAGCCACAGGCATCCCTGCCAGATTTTCCGGCTCTGGCAGCGGCACCGATTTCACCCTGACCATAA GCAGCCTGGAACCTGAGGACTTCGCCGTGTATTACTGCCAGCAGCGGAGCAACTGGCCTCCTACATTTGGCCAG GGAACCAAAGTCGAGATCAAGGAGTCTAAGTACGGCCCTCCTTGTCCTCCATGTCCTATGTTCTGGGTGCTCGTG GTTGTTGGCGGAGTGCTGGCCTGTTACTCTCTGCTGGTGACCGTGGCCTTCATCATCTTTTGGGTCAAGCGGGGC AGAAAGAAGCTGCTGTACATCTTCAAGCAGCCCTTCATGCGGCCCGTGCAGACCACACAAGAGGAAGATGGCTG CTCCTGCAGATTCCCCGAGGAAGAAGAAGGCGGCTGCGAGCTGAGAGTGAAGTTCAGCAGATCCGCCGACGCT CCTGCCTATCAGCAGGGACAGAACCAGCTGTACAACGAGCTGAACCTCGGGAGAAGAGAAGAGTACGACGTGC TGGACAAGCGGAGAGGCAGAGATCCTGAGATGGGCGGAAAGCCCCAGCGGAGAAAGAATCCTCAAGAGGGCC TGTATAATGAGCTGCAGAAAGACAAGATGGCCGAGGCCTACAGCGAGATCGGAATGAAGGGCGAGCGCAGAAG AGGCAAGGGACACGATGGACTGTACCAGGGCCTGAGCACCGCCACCAAGGATACCTATGATGCCCTGCACATG CAGGCCCTGCCTCCAAGA[?] |
| 38 | ATGGCACTGCCAGTTACTGCCCTGCTGCTCCCACTTGCACTGCTGCTTCATGCTGCCAGACCTGAAATCGTGTTAA CGCAGTCTCCAGGCACCCTGTCTTTGTCTCCTGGAGAAAGAGCCACCCTCTCTTGCCGCGCCAGTCAGAGTGTTA GCAGCAGCTACTTAGCCTGGTACCAGCAGAAACCTGGCCAGGCTCCCAGGCTCCTCATCTATGGAGCATCCAGC AGGGCCACTGGCATCCCAGACAGGTTCAGTGGCAGTGGATCCGGGACAGACTTCACTCTCACCATCAGCAGACT GGAGCCTGAAGATTTTGCAGTGTATTACTGTCAGCAGTACTTCAACCCACCAGAATACACGTTCGGCCAGGGGAC CAAAGTGGAAATCAAACGTGGTGGAGGCGGTTCACAGGTGCAGCTGGTGCAGAGCGGCGCCGAGGTGAAGAA GCCCGGCGCCAGCGTGAAGGTCAGCTGCAAGGCCAGCGGCTACACCTTCACCAGCTACTGGATGAACTGGGTG CGGCAGGCCCCCGGCCAGGGCCTGGAGTGGATGGGCCGGATCGACCCCTACGACAGCGAGACCCACTACGCC CAGAAGCTGCAGGGCCGGGTGACCATGACCACCGACACCAGCACCAGCACCGCCTACATGGAGCTGCGGAGCC TGCGGAGCGACGACACCGCCGTGTACTACTGCGCCCGGGGCGGCTACGACTTCGACGTGGGCACCCTGTACTG GTTCTTCGACGTGTGGGGCCAGGGCACCACCGTGACCGTGAGCAGCGGTGGAGGCGGTTCAGGCGGAGGTGGT TCTGGCGGTGGCGGATCGGACATCCAGATGACCCAGAGCCCCAGCAGCCTGAGCGCCAGCGTGGGCGACCGG GTGACCATCACCTGCCGGGCCAGCGAGAACATCTACAGCTACCTGGCCTGGTACCAGCAGAAGCCCGGCAAGG CCCCCAAGCTGCTGATCTACAACGCCAAGACCCTGGCCGAGGGCGTGCCCAGCCGGTTCAGCGGCAGCGGCAG CGGCACCGACTTCACCCTGACCATCAGCAGCCTGCAGCCCGAGGACTTCGCCACCTACTACTGCCAGCACCACT ACGGCACCCCCCGGACCTTCGGCGGCGGCACCAAGGTGGAGATCAAGGGCGGTGGCGGATCGGAGGTGCAATT GCTGGAGTCTGGGGGAGGCTTGGTACAGCCTGGGGGGTCCCTGAGACTCTCCTGTGCAGCCTCCGGATTCACCT TTGGCGGTAATGCCATGTCCTGGGTCCGCCAGGCTCCAGGGAAGGGGCTGGAGTGGGTCTCAGCAATTAGTGGT AATGGTGGTAGTACATTCTACGCAGACTCCGTGAAGGGCCGGTTCACCATCTCCAGAGACAATTCCAAGAACAC GCTGTATCTGCAGATGAACAGCCTGAGAGCCGAGGACACGGCCGTATATTACTGTGCGAAAGTTCGTCCATTCTG GGGTACTTTCGACTACTGGGGCCAAGGAACCCTGGTCACCGTCTCGAGTACCACCACACCAGCACCTAGGCCAC CTACTCCAGCTCCTACCATTGCCTCTCAACCCCTGAGCCTCAGACCTGAGGCATGTAGACCAGCCGCAGGCGGC |
| | GCTGTTCACACAAGAGGCCTCGACTTTGCCTGTGATATCTATATTTGGGCTCCCCTGGCCGGGACCTGCGGAGTT CTTCTGCTGTCTCTCGTGATCACACTGTATTGCAAGAGAGGCCGGAAGAAGCTCCTCTATATCTTTAAACAGCCGT TTATGCGCCCGGTCCAGACTACCCAAGAAGAGGACGGCTGTAGCTGTCGGTTCCCTGAAGAGGAAGAAGGCGG TTGCGAACTGCGCGTGAAATTCTCCAGAAGCGCTGACGCCCCTGCTTATCAGCAAGGCCAGAATCAGCTCTATAA CGAACTCAATCTCGGCAGGCGCGAGGAATATGATGTGCTGGATAAGAGGCGCGGCAGGGACCCAGAAATGGGA GGAAAACCTCAGCGGCGGAAAAACCCACAAGAAGGCCTTTACAACGAACTGCAAAAGGATAAGATGGCAGAAG CTTACTCCGAGATTGGCATGAAGGGCGAACGTCGGAGAGGAAAAGGTCACGACGGACTCTATCAGGGACTGTCT ACAGCCACAAAGGACACATACGACGCACTCCATATGCAGGCTCTCCCACCTAGAGGAAGCGGAGCTACTAACTT CAGCCTGCTGAAGCAGGCTGGAGACGTGGAGGAGAACCCTGGGCCCATGGCTCTGCCTGTGACAGCTCTGCTG CTGCCTCTGGCTCTGCTTCTGCATGCCGCTAGACCTGAAGTGCAGCTGCTGGAATCTGGCGGAGGACTGGTTCAA CCTGGCGGCTCTCTGAGACTGAGCTGTGCCGTGTCCGGCTTCACCTTCAACAGCTTCGCCATGAGCTGGGTCCGA CAGGCCCCTGGAAAAGGCCTTGAATGGGTGTCCGCCATCTCTGGAAGCGGCGGAGGCACATATTACGCCGACTC TGTGAAGGGCAGATTCACCATCAGCCGGGACAACAGCAAGAACACCCTGTACCTGCAGATGAACTCCCTGAGAG CCGAGGATACCGCCGTGTACTTCTGCGCCAAGGACAAGATCCTTTGGTTCGGCGAGCCCGTGTTCGACTATTGGG GACAGGGAACCCTGGTCACCGTTTCTAGCGGAGGCGGAGGTTCTGGTGGTGGTGGATCAGGTGGCGGCGGATC TGAGATTGTGCTGACACAGTCTCCCGCCACACTGTCTCTTAGCCCTGGCGAAAGAGCCACACTGAGCTGCAGAG CCAGCCAGAGCGTGTCCTCTTACCTGGCCTGGTATCAGCAGAAGCCCGGCCAAGCTCCTCGGCTGCTGATCTAC GATGCCAGCAATAGAGCCACAGGCATCCCTGCCAGATTTTCCGGCTCTGGCAGCGGCACCGATTTCACCCTGAC CATAAGCAGCCTGGAACCTGAGGACTTCGCCGTGTATTACTGCCAGCAGCGGAGCAACTGGCCTCCTACATTTG GCCAGGGAACCAAAGTCGAGATCAAGGAGTCTAAGTACGGCCCTCCTTGTCCTCCATGTCCTATGTTCTGGGTGC TCGTGGTTGTTGGCGGAGTGCTGGCCTGTTACTCTCTGCTGGTGACCGTGGCCTTCATCATCTTTTGGGTCAAGCG GGGCAGAAAGAAGCTGCTGTACATCTTCAAGCAGCCCTTCATGCGGCCCGTGCAGACCACACAAGAGGAAGAT GGCTGCTCCTGCAGATTCCCCGAGGAAGAAGAAGGCGGCTGCGAGCTGAGAGTGAAGTTCAGCAGATCCGCCG ACGCTCCTGCCTATCAGCAGGGACAGAACCAGCTGTACAACGAGCTGAACCTCGGGAGAAGAGAAGAGTACGA CGTGCTGGACAAGCGGAGAGGCAGAGATCCTGAGATGGGCGGAAAGCCCCAGCGGAGAAAGAATCCTCAAGA GGGCCTGTATAATGAGCTGCAGAAAGACAAGATGGCCGAGGCCTACAGCGAGATCGGAATGAAGGGCGAGCG CAGAAGAGGCAAGGGACACGATGGACTGTACCAGGGCCTGAGCACCGCCACCAAGGATACCTATGATGCCCTG CACATGCAGGCCCTGCCTCCAAGA |
| 39 | AGAGTCTCTCAGCTGGTACA |
| 40 | GAGTAGCGCGAGCACAGCTA |
| 41 | TGAACAGGAAATAACCTATG |
| 42 | GCGCTTTCCCGAGACCGTCC |
| 43 | SFAMS |
| 44 | AISGSGGGTYYADSVKG |
| 45 | DKILWFGEPVFDY |
| 46 | RASQSVSSYLA |
| 47 | DASNRAT |
| 48 | QQRSNWPPT |
| 49 | ↵ |
| 50 | ↵ |
| 51 | SYYMN |
| 52 | GISGDPSNTYYADSVKG |
| 53 | DLPLVYTGFAY |
| 54 | SGDNLRHYYVY |
| 55 | CDSKRPS |
| 56 | QTYTGGASLV |
| 57 | QVQLVESGGGLVQPGGSLRLSCAASGFTFSSYYMNWVRQAPGKGLEWVSGISGDPSNTYYADSVKGRFTISRDNSKN - TLYLQMNSLRAEDTAVYYCARDLPLVYTGFAYWGQGTLVTVSS↵ |
| 58 | DIELTQPPSVSVAPGQTARISCSGDNLRHYYVYWYQQKPGQAPVLVIYGDSKRPSGIPERFSGSNSGNTATLTISGTQAE DEADYYCQTYTGGASLVFGGGTKLTVLGQ↵ |
| 59 | DYWMQ |
| 60 | TIYPCDGDTGYAQKFQG |
| 61 | GDYYGSNSLDY |
| 62 | KASQDVSTVVA |
| 63 | SASYRYI |
| 61 | QQHYSPPYT |
| 65 | ↵ |
| 66 | ↵ |
| 67 | SYAIS |
| 68 | RIIRFLGIANYAQKFQG |
| 69 | EPGERDPDAVDI |
| 70 | RASQGISNYLA |
| 71 | AASSLQS |
| 72 | QQYNSYPYT |
| 73 | QVQLVQSGAEVKKPGSSVKVSCKAFGGTFSSYAISWVRQAPGQGLEWMGRIIRFLGIANYAQKFQGRVTLIADKSTNT AYMELSSLRSEDTAVYYCAGEPGERDPDAVDIWGQGTMVTVSS↵ |
| 74 | DIQMTQSPSSLSASVGDRVTITCRASQGISNYLAWFQQKPGKAPKSLIYAASSLQSGVPSRFSGSGSGTDFTLTISSLQPE DFATYYCQQYNSYPYTFGQGTKLEIK↵ |
| 75 | GTGAAACAGACTTTGAATTTTGACCTTCTGAAGTTGGCAGGAGACGTTGAGTCCAACCCTGGGCCC |
| 76 | ATCTGGAGCGCCGTGTTCCTGAACAGCCTGTTCAACCAGGAGGTGCAGATCCCCCTGACCGAGAGCTACTGCGG CCCCTGCCCCAAGAACTGGATCTGCTACAAGAACAACTGCTACCAGTTCTTCGACGAGAGCAAGAACTGGTACG AGAGCCAGGCCAGCTGCATGAGCCAGAACGCCAGCCTGCTGAAGGTGTACAGCAAGGAGGACCAGGACCTGCT GAAGCTGGTGAAGAGCTACCACTGGATGGGCCTGGTGCACATCCCCACCAACGGCAGCTGGCAGTGGGAGGAC GGCAGCATCCTGAGCCCCAACCTGCTGACCATCATCGAGATGCAGAAGGGCGACTGCGCCCTGTACGCCAGCA GCTTCAAGGGCTACATCGAGAACTGCAGCACCCCCAACACCTACATCTGCATGCAGCGGACCGTG |
| 77 | GWIRGRRSRHSWEMSEFHNYNLDLKKSDFSTRWQKQRCPVVKSKCRENASPFFFCCFIAVAMGIRFIIMVAIWSAVFLN SLFNQEVQIPLTESYCGPCPKNWICYKNNCYQFFDESKNWYESQASCMSQNASLLKVYSKEDQDLLKLVKSYHWMGL VHIPTNGSWQWEDGSILSPNLLTIIEMQKGDCALYASSFKGYIENCSTPNTYICMQRTV↵ |
| 78 | ATGAGAGTGAAGTTCAGCAGGAGCGCAGACGCCCCCGCGTACCAGCAGGGCCAGAACCAGCTCTATAACGAGC TCAATCTAGGACGAAGAGAGGAGTACGATGTTTTGGACAAGAGACGTGGCCGGGACCCTGAGATGGGGGGAAA GCCGCAGAGAAGGAAGAACCCTCAGGAAGGCCTGTACAATGAACTGCAGAAAGATAAGATGGCGGAGGCCTAC AGTGAGATTGGGATGAAAGGCGAGCGCCGGAGGGGCAAGGGGCACGATGGCCTTTACCAGGGTCTCAGTACAG CCACCAAGGACACCTACGACGCCCTTCACATGCAGGCCCTGCCCCCTCGCGGGTGGATTCGTGGTCGGAGGTCT CGACACAGCTGGGAGATGAGTGAATTTCATAATTATAACTTGGATCTGAAGAAGAGTGATTTTTCAACACGATGG CAAAAGCAAAGATGTCCAGTAGTCAAAAGCAAATGTAGAGAAAATGCATCTCCATTTTTTTTCTGCTGCTTCATCG CTGTAGCCATGGGAATCCGTTTCATTATTATGGTAGCTATATGGAGTGCTGTATTCCTAAACTCATTATTCAACCAA GAAGTTCAAATTCCCTTGACCGAAAGTTACTGTGGCCCATGTCCTAAAAACTGGATATGTTACAAAAATAACTGCT ACCAATTTTTTGATGAGAGTAAAAACTGGTATGAGAGCCAGGCTTCTTGTATGTCTCAAAATGCCAGCCTTCTGAA AGTATACAGCAAAGAGGACCAGGATTTACTTAAACTGGTGAAGTCATATCATTGGATGGGACTAGTACACATTCC AACAAATGGATCTTGGCAGTGGGAAGATGGCTCCATTCTCTCACCCAACCTACTAACAATAATTGAAATGCAGAA GGGAGACTGTGCACTCTATGCCTCGAGCTTTAAAGGCTATATAGAAAACTGTTCAACTCCAAATACATACATCTG CATGCAAAGGACTGTG |
| 79 | ATGGCGCTCCCTGTCACCGCCTTGCTCCTGCCGCTGGCCTTGCTGCTCCACGCCGCCAGGCCGATATGGAGTGCT GTATTCCTAAACTCATTATTCAACCAAGAAGTTCAAATTCCCTTGACCGAAAGTTACTGTGGCCCATGTCCTAAAA ACTGGATATGTTACAAAAATAACTGCTACCAATTTTTTGATGAGAGTAAAAACTGGTATGAGAGCCAGGCTTCTTG TATGTCTCAAAATGCCAGCCTTCTGAAAGTATACAGCAAAGAGGACCAGGATTTACTTAAACTGGTGAAGTCATA TCATTGGATGGGACTAGTACACATTCCAACAAATGGATCTTGGCAGTGGGAAGATGGCTCCATTCTCTCACCCAA CCTACTAACAATAATTGAAATGCAGAAGGGAGACTGTGCACTCTATGCCTCGAGCTTTAAAGGCTATATAGAAAA CTGTTCAACTCCAAATACATACATCTGCATGCAAAGGACTGTGACCACGACGCCAGCGCCGCGACCACCAACAC CGGCGCCCACCATCGCGTCGCAGCCCCTGTCCCTGCGCCCAGAGGCGTGCCGGCCAGCGGCGGGGGGCGCAG TGCACACGAGGGGGCTGGACTTCGCCTGTGATATCTACATCTGGGCGCCCTTGGCCGGGACTTGTGGGGTCCTTC TCCTGTCACTGGTTATCACCCTTTACTGCAAACGGGGCAGAAAGAAACTCCTGTATATATTCAAACAACCATTTAT |
| | GAGACCAGTACAAACTACTCAAGAGGAAGATGGCTGTAGCTGCCGATTTCCAGAAGAAGAAGGAGGATGT GAACTGAGAGTGAAGTTCAGCAGGAGCGCAGACGCCCCCGCGTACCAGCAGGGCCAGAACCAGCTCTATAACG AGCTCAATCTAGGACGAAGAGAGGAGTACGATGTTTTGGACAAGAGACGTGGCCGGGACCCTGAGATGGGGGG AAAGCCGCAGAGAAGGAAGAACCCTCAGGAAGGCCTGTACAATGAACTGCAGAAAGATAAGATGGCGGAGGC CTACAGTGAGATTGGGATGAAAGGCGAGCGCCGGAGGGGCAAGGGGCACGATGGCCTTTACCAGGGTCTCAGT ACAGCCACCAAGGACACCTACGACGCCCTTCACATGCAGGCCCTGCCCCCTCGC |
| 80 | ATGACCAAGGAGTATCAAGACCTTCAGCATCTGGACAATGAGGAGAGTGACCACCATCAGCTCAGAAAAGGGC CACCTCCTCCCCAGCCCCTCCTGCAGCGTCTCTGCTCCGGACCTCGCCTCCTCCTGCTCTCCCTGGGCCTCAGCCT CCTGCTGCTTGTGGTTGTCTGTGTGATCGGATCCCAAAACTCCCAGCTGCAGGAGGAGCTGCGGGGCCTGAGAG AGACGTTCAGCAACTTCACAGCGAGCACGGAGGCCCAGGTCAAGGGCTTGAGCACCCAGGGAGGCAATGTGGG AAGAAAGATGAAGTCGCTAGAGTCCCAGCTGGAGAAACAGCAGAAGGACCTGAGTGAAGATCACTCCAGCCTG CTGCTCCACGTGAAGCAGTTCGTGTCTGACCTGCGGAGCCTGAGCTGTCAGATGGCGGCGCTCCAGGGCAATGG CTCAGAAAGGACCTGCTGCCCGGTCAACTGGGTGGAGCACGAGCGCAGCTGCTACTGGTTCTCTCGCTCCGGGA AGGCCTGGGCTGACGCCGACAACTACTGCCGGCTGGAGGACGCGCACCTGGTGGTGGTCACGTCCTGGGAGGA GCAGAAATTTGTCCAGCACCACATAGGCCCTGTGAACACCTGGATGGGCCTCCACGACCAAAACGGGCCCTGGA AGTGGGTGGACGGGACGGACTACGAGACGGGCTTCAAGAACTGGAGGCCGGAGCAGCCGGACGACTGGTACG GCCACGGGCTCGGAGGAGGCGAGGACTGTGCCCACTTCACCGACGACGGCCGCTGGAACGACGACGTCTGCCA GAGGCCCTACCGCTGGGTCTGCGAGACAGAGCTGGACAAGGCCAGCCAGGAGCCACCTCTCCTT |
| 81 | ATGGAATACGCCTCTGACGCTTCACTGGACCCCGAAGCCCCGTGGCCTCCCGCGCCCCGCGCTCGCGCCTGCCG CGTACTGCCTTGGGCCCTGGTCGCGGGGCTGCTGCTGCTGCTGCTCGCTGCCGCCTGCGCCGTCTTCCTCGC CTGCCCCTGGGCCGTGTCCGGGGCTCGCGCCTCGCCCGGCTCCGCGGCCAGCCCGAGACTCCGCGAGGGTCCC GAGCTTTCGCCCGACGATCCCGCCGGCCTCTTGGACCTGCGGCAGGGCATGTTTGCGCAGCTGGTGGCCCAAAA TGTTCTGCTGATCGATGGGCCCCTGAGCTGGTACAGTGACCCAGGCCTGGCAGGCGTGTCCCTGACGGGGGGCC TGAGCTACAAAGAGGACACGAAGGAGCTGGTGGTGGCCAAGGCTGGAGTCTACTATGTCTTCTTTCAACTAGAG CTGCGGCGCGTGGTGGCCGGCGAGGGCTCAGGCTCCGTTTCACTTGCGCTGCACCTGCAGCCACTGCGCTCTGC TGCTGGGGCCGCCGCCCTGGCTTTGACCGTGGACCTGCCACCCGCCTCCTCCGAGGCTCGGAACTCGGCCTTCG GTTTCCAGGGCCGCTTGCTGCACCTGAGTGCCGGCCAGCGCCTGGGCGTCCATCTTCACACTGAGGCCAGGGCA CGCCATGCCTGGCAGCTTACCCAGGGCGCCACAGTCTTGGGACTCTTCCGGGTGACCCCCGAAATCCCAGCCGG ACTCCCTTCACCGAGGTCGGAA |
| 82 | ATGGCCTTACCAGTGACCGCCTTGCTCCTGCCGCTGGCCTTGCTGCTCCACGCCGCCAGGCCGGAGGTGCAATT GCTGGAGTCTGGGGGAGGCTTGGTACAGCCTGGGGGGTCCCTGAGACTCTCCTGTGCAGCCTCCGGATTCACCT TTAGCAGTTATGCCATGAGCTGGGTCCGCCAGGCTCCAGGGAAGGGGCTGGAGTGGGTCTCAGCTATTAGTGGT AGTGGTGGTAGCACATACTACGCAGACTCCGTGAAGGGCCGGTTCACCATCTCCAGAGACAATTCCAAGAACAC GCTGTATCTGCAGATGAACAGCCTGAGAGCCGAGGACACGGCCGTATATTACTGTGCGAAAGATGATTCTTTCTA CGGTGGTTTCGACTACTGGGGCCAAGGAACCCTGGTCACCGTCTCGAGTGGTGGAGGCGGTTCAGGCGGAGGT GGTTCTGGCGGTGGCGGATCGGACATCCAGATGACCCAGTCTCCTTCCACCCTGTCTGCATCTGTAGGAGACCGT GTCACCATCACTTGCCGTGCCAGTCAGAGTATTAGTAGCTGGTTGGCCTGGTATCAGCAGAAACCAGGGAAAGC CCCTAAGCTCCTGATCTATGATGCCTCCAGTTTGGAAAGTGGGGTCCCATCACGTTTCAGCGGCAGTGGATCCGG GACAGAATTCACTCTCACCATCAGCAGCTTGCAGCCTGATGATTTTGCAACTTATTACTGCCAACAGTACTCTTCT TACAAATACACGTTTGGCCAGGGCACCAAAGTCGAGATCAAGCGTGGACAAGATGCAAGTTCTGCTGGATCAAC CAGGAATAAAGATCCAACAAGACGTCCAGAACTAGAAGCTGGTACAAATGGAAGAAGTTCGACAACAAGTCCCT TTGTAAAACCAAATCCAGGTTCTAGCACAGACGGCAACAGCGCCGGACATTCGGGGAACAACATCCTCGGTTCC GAAGTGGCCTTATTTGCACTCATGTACGTGGCAGCGGCCGCCTTCGTGCTCCTGTTCTTTGTGGGCTGTGGGGTG CTGCTGTCCCGCAAGCGCCGGCGGATGAAGCTGCTGAGCAGCATCGAGCAGGCCTGTGACATCTGCCGGCTGA AGAAACTGAAGTGCAGCAAAGAAAAGCCCAAGTGCGCCAAGTGCCTGAAGAACAACTGGGAGTGCCGGTACAG CCCCAAGACCAAGAGAAGCCCCCTGACCAGAGCCCACCTGACCGAGGTGGAAAGCCGGCTGGAAAGACTGGA ACAGCTGTTTCTGCTGATCTTCCCACGCGAGGACCTGGACATGATCCTGAAGATGGACAGCCTGCAGGACATCA AGGCCCTGCTGACCGGCCTGTTCGTGCAGGACAACGTGAACAAGGACGCCGTGACCGACAGACTGGCCAGCGT GGAAACCGACATGCCCCTGACCCTGCGGCAGCACAGAATCAGCGCCACCAGCAGCAGCGAGGAAAGCAGCAA CAAGGGCCAGCGGCAGCTGACAGTGTCTGCTGCTGCAGGCGGAAGCGGAGGCTCTGGCGGATCTGATGCCCTG GACGACTTCGACCTGGATATGCTGGGCAGCGACGCCCTGGATGATTTTGATCTGGACATGCTGGGATCTGACGCT CTGGACGATTTCGATCTCGACATGTTGGGATCAGATGCACTGGATGACTTTGACCTGGACATGCTCGGATCA |
| 83 | MALPVTALLLPLALLLHAARPQVQLVQSGAEVKKPGASVKVSCKASGYTFTSYWMNWVRQAPGQGLEWMGRIDPYD |
| | SETHYAQKLQGRVTMTTDTSTSTAYMELRSLRSDDTAVYYCARGGYDFDVGTLYWFFDVWGQGTTVTVSSGGGGSG GGGSGGGGSDIQMTQSPSSLSASVGDRVTITCRASENIYSYLAWYQQKPGKAPKLLIYNAKTLAEGVPSRFSGSGSGTD FTLTISSLQPEDFATYYCQHHYGTPRTFGGGTKVEIKTTTPAPRPPTPAPTIASQPLSLRPEACRPAAGGAVHTRGLDFAC DFWVLVVVGGVLACYSLLVTVAFIIFWVRSKRSRLLHSDYMNMTPRRPGPTRKHYQPYAPPRDFAAYRSRVKFSRSADA PAYQQGQNQLYNELNLGRREEYDVLDKRRGRDPEMGGKPQRRKNPQEGLYNELQKDKMAEAYSEIGMKGERRRGK GHDGLYQGLSTATKDTYDALHMQALPPR↵ |
| 84 | MALPVTALLLPLALLLHAARPEIVLTQSPGTLSLSPGERATLSCRASQSVSSSYLAWYQQKPGQAPRLLIYGASSRATGIP DRFSGSGSGTDFTLTISRLEPEDFAVYYCQQYKSHPITFGQGTKVEIKRGGGGSQVQLVQSGAEVKKPGASVKVSCKASG YTFTSYWMNWVRQAPGQGLEWMGRIDPYDSETHYAQKLQGRVTMTTDTSTSTAYMELRSLRSDDTAVYYCARGGY DFDVGTLYWFFDVWGQGTTVTVSSGGGGSGGGGSGGGGSDIQMTQSPSSLSASVGDRVTITCRASENIYSYLAWYQ QKPGKAPKLLIYNAKTLAEGVPSRFSGSGSGTDFTLTISSLQPEDFATYYCQHHYGTPRTFGGGTKVEIKGGGGSEVQLLE SGGGLVQPGGSLRLSCAASGFTFSSYAMSWVRQAPGKGLEWVSAISGSGGSTYYADSVKGRFTISRDNSKNTLYLQMN SLRAEDTAVYYCARGWPSPVTFDYWGQGTLVTVSSTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEEDGCSCRFPEEEEGGCELRVKFSRSADAPAYQ QGQNQLYNELNLGRREEYDVLDKRRGRDPEMGGKPQRRKNPQEGLYNELQKDKMAEAYSEIGMKGERRRGKGHDG LYQGLSTATKDTYDALHMQALPPR |
| 85 | MALPVTALLLPLALLLHAARPEIVLTQSPGTLSLSPGERATLSCRASQSVSSSYLAWYQQKPGQAPRLLIYGASSRATGIP DRFSGSGSGTDFTLTISRLEPEDFAVYYCQQYFNPPEYTFGQGTKVEIKRGGGGSQVQLVQSGAEVKKPGASVKVSCKA SGYTFTSYWMNWVRQAPGQGLEWMGRIDPYDSETHYAQKLQGRVTMTTDTSTSTAYMELRSLRSDDTAVYYCARG GYDFDVGTLYWFFDVWGQGTTVTVSSGGGGSGGGGSGGGGSDIQMTQSPSSLSASVGDRVTITCRASENIYSYLAWY QQKPGKAPKLLIYNAKTLAEGVPSRFSGSGSGTDFTLTISSLQPEDFATYYCQHHYGTPRTFGGGTKVEIKGGGGSEVQLL ESGGGLVQPGGSLRLSCAASGFTFGGNAMSWVRQAPGKGLEWVSAISGNGGSTFYADSVKGRFTISRDNSKNTLYLQ MNSLRAEDTAVYYCAKVRPFWGTFDYWGQGTLVTVSSTTTPAPRPPTPAPTIASQPLSLRPEACRPAAGGAVHTRGLD FACDIYIWAPLAGTCGVLLLSLVITLYCKRGRKKLLYIFKQPFMRPVQTTQEEDGCSCRFPEEEEGGCELRVKFSRSADAPA YQQGQNQLYNELNLGRREEYDVLDKRRGRDPEMGGKPQRRKNPQEGLYNELQKDKMAEAYSEIGMKGERRRGKGH DGLYQGLSTATKDTYDALHMQALPPR |
| 86 | MALPVTALLLPLALLLHAARPEVQLLESGGGLVQPGGSMRLSCAASGFTFSSYAMSWVRQAPGKGLEWVSAISGSGGS TYYADSVKGRFTISRDNSKNTLYLQMNSLRAEDTAVYYCAKYEGRYVLGAMDYWGQGTLVTVSSGGGGSGGGGSGG GGSDIQMTQSPSTLSASVGDRVTITCRASQSISSWLAWYQQKPGKAPKLLIYDASSLESGVPSRFSGSGSGTEFTLTISSL QPDDFATYYCQQYFSYPLTFGQGTKVEIKRTTTPAPRPPTPAPTIASQPLSLRPEACRPAAGGAVHTRGLDFACDFWVLV VVGGVLACYSLLVTVAFIIFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYSEIGMKGERRRGKGHDGLY QGLSTATKDTYDALHMQALPPR↵ |
| 87 | ATNFSLLKQAGDVEENPGP |
| 88 | AAGCTTGATATCGAATTCGGAGCACTGTCCTCCGAACGTCGGAGCACTGTCCTCCGAACGTCGGAGCACTGTCCT CCGAACGTCGGAGCACTGTCCTCCGAACGGAGCATGTCCTCCGAACGTCGGAGCACTGTCCTCCGAACGACTAG TTAGGCGTGTACGGTGGGAGGCCTATATAAGCAGAGCTCGTTTAGTGAACCGTCAGATCGCCTGGAGACGCCAT CCACGCTGTTTTGACCTCCATAGAAGACACCGGGACCGATCCAGCCTCTCGACATTCGTTGGATC↵ |
| 89 | GGGCAGAGCGCACATCGCCCACAGTCCCCGAGAAGTTGGGGGGAGGGGTCGGCAATTGAACCGGTGCCTAGA GAAGGTGGCGCGGGGTAAACTGGGAAAGTGATGTCGTGTACTGGCTCCGCCTTTTTCCCGAGGGTGGGGGAGA ACCGTATATAAGTGCAGTAGTCGCCGTGAACGTTCTTTTTCGCAACGGGTTTGCCGCCAGAACACAGGTGTCGTG ACGCG↵ |
| 90 | ATGGCCTTACCAGTGACCGCCTTGCTCCTGCCGCTGGCCTTGCTGCTCCACGCCGCCAGGCCGGAGGTGCAATT GCTGGAGTCTGGGGGAGGCTTGGTACAGCCTGGGGGGTCCCTGAGACTCTCCTGTGCAGCCTCCGGATTCACCT TTGGCGGTAATGCCATGTCCTGGGTCCGCCAGGCTCCAGGGAAGGGGCTGGAGTGGGTCTCAGCAATTAGTGGT AATGGTGGTAGTACATTCTACGCAGACTCCGTGAAGGGCCGGTTCACCATCTCCAGAGACAATTCCAAGAACAC GCTGTATCTGCAGATGAACAGCCTGAGAGCCGAGGACACGGCCGTATATTACTGTGCGAAAGTTCGTCCATTCTG GGGTACTTTCGACTACTGGGGCCAAGGAACCCTGGTCACCGTCTCGAGTGGTGGAGGCGGTTCAGGCGGAGGT GGTTCTGGCGGTGGCGGATCGGAAATCGTGTTAACGCAGTCTCCAGGCACCCTGTCTTTGTCTCCAGGGGAAAG AGCCACCCTCTCTTGCAGGGCCAGTCAGAGTGTTAGCAGCAGCTACTTAGCCTGGTACCAGCAGAAACCTGGCC AGGCTCCCAGGCTCCTCATCTATGGAGCATCCAGCAGGGCCACTGGCATCCCAGACAGGTTCAGTGGCAGTGGA TCCGGGACAGACTTCACTCTCACCATCAGCAGACTGGAGCCTGAAGATTTTGCAGTGTATTACTGTCAGCAGTAC TTCAACCCACCAGAATACACGTTCGGCCAGGGGACCAAAGTGGAAATCAAACGTACCACGACGCCAGCGCCGC |
| | GACCACCAACACCGGCGCCCACCATCGCGTCGCAGCCCCTGTCCCTGCGCCCAGAGGCGTGCCGGCCAGCGGC GGGGGGCGCAGTGCACACGAGGGGGCTGGACTTCGCCTGTGATATCTACATCTGGGCGCCCTTGGCCGGGACTT GTGGGGTCCTTCTCCTGTCACTGGTTATCACCCTTTACTGCAAACGGGGCAGAAAGAAACTCCTGTATATATTCAA ACAACCATTTATGAGACCAGTACAAACTACTCAAGAGGAAGATGGCTGTAGCTGCCGATTTCCAGAAGAAGAAG AAGGAGGATGTGAACTGAGAGTGAAGTTCAGCAGGAGCGCAGACGCCCCCGCGTACCAGCAGGGCCAGAACC AGCTCTATAACGAGCTCAATCTAGGACGAAGAGAGGAGTACGATGTTTTGGACAAGAGACGTGGCCGGGACCCT GAGATGGGGGGAAAGCCGCAGAGAAGGAAGAACCCTCAGGAAGGCCTGTACAATGAACTGCAGAAAGATAAG ATGGCGGAGGCCTACAGTGAGATTGGGATGAAAGGCGAGCGCCGGAGGGGCAAGGGGCACGATGGCCTTTAC CAGGGTCTCAGTACAGCCACCAAGGACACCTACGACGCCCTTCACATGCAGGCCCTGCCCCCTCGCGGAAGCG GAGCTACTAACTTCAGCCTGCTGAAGCAGGCTGGAGACGTGGAGGAGAACCCTGGACCTATGGCCTTACCAGTG ACCGCCTTGCTCCTGCCGCTGGCCTTGCTGCTCCACGCCGCCAGGCCGGAGGTGCAATTGCTGGAGTCTGGGGG AGGCTTGGTACAGCCTGGGGGGTCCCTGAGACTCTCCTGTGCAGCCTCCGGATTCACCTTTAGCAGTTATGCCAT GAGCTGGGTCCGCCAGGCTCCAGGGAAGGGGCTGGAGTGGGTCTCAGCTATTAGTGGTAGTGGTGGTAGCACAT ACTACGCAGACTCCGTGAAGGGCCGGTTCACCATCTCCAGAGACAATTCCAAGAACACGCTGTATCTGCAGATG AACAGCCTGAGAGCCGAGGACACGGCCGTATATTACTGTGCGCGTGGTTGGCCATCTCCAGTTACTTTCGACTAC TGGGGCCAAGGAACCCTGGTCACCGTCTCGAGTGGTGGAGGCGGTTCAGGCGGAGGTGGTTCTGGCGGTGGCG GATCGGAAATCGTGTTAACGCAGTCTCCAGGCACCCTGTCTTTGTCTCCAGGGGAAAGAGCCACCCTCTCTTGCA GGGCCAGTCAGAGTGTTAGCAGCAGCTACTTAGCCTGGTACCAGCAGAAACCTGGCCAGGCTCCCAGGCTCCTC ATCTATGGAGCATCCAGCAGGGCCACTGGCATCCCAGACAGGTTCAGTGGCAGTGGATCCGGGACAGACTTCAC TCTCACCATCAGCAGACTGGAGCCTGAAGATTTTGCAGTGTATTACTGTCAGCAGTACAAATCTCATCCAATCAC GTTCGGCCAGGGGACCAAAGTGGAAATCAAACGTACCACGACGCCAGCGCCGCGACCACCAACACCGGCGCCC ACCATCGCGTCGCAGCCCCTGTCCCTGCGCCCAGAGGCGTGCCGGCCAGCGGCGGGGGGCGCAGTGCACACG AGGGGGCTGGACTTCGCCTGTGATATCTACATCTGGGCGCCCTTGGCCGGGACTTGTGGGGTCCTTCTCCTGTCA CTGGTTATCACCCTTTACTGCAAACGGGGCAGAAAGAAACTCCTGTATATATTCAAACAACCATTTATGAGACCA GTACAAACTACTCAAGAGGAAGATGGCTGTAGCTGCCGATTTCCAGAAGAAGAAGAAGGAGGATGTGAACTGA GAGTGAAGTTCAGCAGGAGCGCAGACGCCCCCGCGTACCAGCAGGGCCAGAACCAGCTCTATAACGAGCTCAA TCTAGGACGAAGAGAGGAGTACGATGTTTTGGACAAGAGACGTGGCCGGGACCCTGAGATGGGGGGAAAGCCG CAGAGAAGGAAGAACCCTCAGGAAGGCCTGTACAATGAACTGCAGAAAGATAAGATGGCGGAGGCCTACAGTG AGATTGGGATGAAAGGCGAGCGCCGGAGGGGCAAGGGGCACGATGGCCTTTACCAGGGTCTCAGTACAGCCAC CAAGGACACCTACGACGCCCTTCACATGCAGGCCCTGCCCCCTCGC |
| 91 | ATGGCTCTGCCTGTGACAGCTCTGCTGCTGCCTCTGGCTCTGCTTCTGCATGCCGCTAGACCTGAAGTGCAGCTG CTGGAATCTGGCGGAGGACTGGTTCAACCTGGCGGCTCTCTGAGACTGTCTTGTGCCGCCAGCGGCTTCACCTTT GGCGGCAATGCCATGAGCTGGGTCCGACAGGCTCCTGGCAAAGGCCTTGAATGGGTGTCCGCCATCTCTGGCAA TGGCGGCAGCACCTTTTACGCCGACTCTGTGAAGGGCAGATTCACCATCAGCCGGGACAACAGCAAGAACACCC TGTATCTGCAGATGAACAGCCTGAGAGCCGAGGACACCGCCGTGTACTACTGTGCTAAAGTGCGGCCTTTCTGG GGCACCTTCGATTATTGGGGCCAGGGCACACTGGTCACAGTGTCTAGCGGAGGCGGAGGTTCTGGCGGCGGAG GAAGTGGTGGCGGAGGCTCTGAAATTGTGCTGACACAGAGCCCCGGCACACTGTCTCTTAGCCCTGGCGAAAGA GCCACACTGAGCTGTAGAGCCAGCCAGAGCGTGTCCAGCTCTTACCTGGCTTGGTATCAGCAGAAGCCCGGACA GGCACCCAGACTGCTGATCTATGGCGCCTCCTCTAGAGCTACAGGCATCCCCGATAGATTTTCCGGCAGCGGCTC TGGCACCGACTTCACCCTGACAATCAGCAGACTGGAACCCGAGGACTTTGCCGTGTATTATTGCCAGCAGTACTT CAACCCTCCTGAGTACACCTTCGGACAGGGCACCAAGGTGGAAATCAAGCGCACCACAACACCCGCTCCTAGAC CTCCAACACCAGCTCCAACAATCGCCAGCCAGCCTCTGTCTCTGAGGCCAGAAGCTTGTAGACCTGCTGCTGGC GGAGCCGTGCATACAAGAGGACTGGATTTCGCCTGCGACATCTACATCTGGGCCCCTCTGGCTGGAACATGTGG CGTTCTGCTGCTGAGCCTGGTCATCACCCTGTACTGCAAGCGGGGCAGAAAGAAGCTGCTGTACATCTTCAAGCA GCCCTTCATGCGGCCCGTGCAGACCACACAAGAGGAAGATGGCTGCTCCTGCAGATTCCCCGAGGAAGAAGAA GGCGGCTGCGAGCTGAGAGTGAAGTTCAGCAGATCCGCCGACGCTCCTGCCTATCAGCAGGGACAGAACCAGC TGTACAACGAGCTGAACCTCGGGAGAAGAGAAGAGTACGACGTGCTGGACAAGCGGAGAGGCAGAGATCCTGA GATGGGCGGAAAGCCCCAGCGGAGAAAGAATCCTCAAGAGGGCCTGTATAATGAGCTGCAGAAAGACAAGATG GCCGAGGCCTACAGCGAGATCGGAATGAAGGGCGAGCGCAGAAGAGGCAAGGGACACGATGGACTGTACCAG GGCCTGAGCACCGCCACCAAGGATACCTATGATGCCCTGCACATGCAGGCCCTGCCTCCAAGAGGAAGCGGAG CTACTAACTTCAGCCTGCTGAAGCAGGCTGGAGACGTGGAGGAGAACCCTGGGCCCATGGCACTGCCAGTTACT GCCCTGCTGCTCCCACTTGCACTGCTGCTTCATGCTGCCAGACCTGAGGTCCAGCTGTTGGAAAGTGGCGGTGGT CTTGTGCAGCCTGGCGGAAGCCTCAGACTTAGCTGTGCCGCCTCCGGCTTCACATTCAGCAGCTACGCCATGTCC TGGGTTCGACAAGCACCCGGAAAAGGATTGGAGTGGGTTTCAGCCATCAGCGGCAGCGGAGGCAGCACTTATTA |
| | TGCCGATAGCGTGAAAGGCCGGTTCACCATCTCCAGAGACAACTCCAAGAATACGCTCTATCTCCAGATGAACTC TCTGCGCGCCGAAGATACAGCTGTGTACTATTGCGCCAGAGGCTGGCCCTCTCCTGTGACCTTTGATTACTGGGG ACAAGGGACCCTCGTGACCGTTTCTTCTGGTGGCGGCGGTAGTGGCGGAGGTGGAAGTGGCGGTGGTGGAAGC GAAATCGTGCTGACCCAGTCTCCTGGAACTCTGTCTCTGTCCCCTGGCGAGAGGGCTACCCTGTCTTGTAGAGCC TCTCAGTCCGTGTCCTCCAGCTATCTCGCCTGGTATCAACAAAAGCCTGGGCAAGCCCCTCGGCTGCTGATATAC GGGGCTTCTTCTCGCGCCACCGGCATTCCAGACAGATTCAGCGGATCTGGAAGCGGCACAGATTTCACACTGAC CATCTCTCGGCTGGAACCTGAAGATTTCGCTGTCTACTACTGTCAGCAGTACAAGTCTCACCCCATCACCTTTGGC CAGGGAACAAAGGTCGAGATCAAGAGAACCACCACACCAGCACCTAGGCCACCTACTCCAGCTCCTACCATTGC CTCTCAACCCCTGAGCCTCAGACCTGAGGCATGTAGACCAGCCGCAGGCGGCGCTGTTCACACAAGAGGCCTCG ACTTTGCCTGTGATATCTATATTTGGGCTCCCCTGGCCGGGACCTGCGGAGTTCTTCTGCTGTCTCTCGTGATCAC ACTGTATTGCAAGAGAGGCCGGAAGAAGCTCCTCTATATCTTTAAACAGCCGTTTATGCGCCCGGTCCAGACTAC CCAAGAAGAGGACGGCTGTAGCTGTCGGTTCCCTGAAGAGGAAGAAGGCGGTTGCGAACTGCGCGTGAAATTC TCCAGAAGCGCTGACGCCCCTGCTTATCAGCAAGGCCAGAATCAGCTCTATAACGAACTCAATCTCGGCAGGCG CGAGGAATATGATGTGCTGGATAAGAGGCGCGGCAGGGACCCAGAAATGGGAGGAAAACCTCAGCGGCGGAA AAACCCACAAGAAGGCCTTTACAACGAACTGCAAAAGGATAAGATGGCAGAAGCTTACTCCGAGATTGGCATGA AGGGCGAACGTCGGAGAGGAAAAGGCCACGACGGACTCTATCAGGGACTGTCTACAGCCACAAAGGACACATA CGACGCACTCCATATGCAGGCTCTCCCACCTAGA |
| 92 | ATGGCCTTACCAGTGACCGCCTTGCTCCTGCCGCTGGCCTTGCTGCTCCACGCCGCCAGGCCGGAGGTGCAATT GCTGGAGTCTGGGGGAGGCTTGGTACAGCCTGGGGGGTCCCTGAGACTCTCCTGTGCAGCCTCCGGATTCACCT TTAGCAGTTATGCCATGAGCTGGGTCCGCCAGGCTCCAGGGAAGGGGCTGGAGTGGGTCTCAGCTATTAGTGGT AGTGGTGGTAGCACATACTACGCAGACTCCGTGAAGGGCCGGTTCACCATCTCCAGAGACAATTCCAAGAACAC GCTGTATCTGCAGATGAACAGCCTGAGAGCCGAGGACACGGCCGTATATTACTGTGCGCGTGGTTGGCCATCTC CAGTTACTTTCGACTACTGGGGCCAAGGAACCCTGGTCACCGTCTCGAGTGGTGGAGGCGGTTCAGGCGGAGGT GGTTCTGGCGGTGGCGGATCGGAAATCGTGTTAACGCAGTCTCCAGGCACCCTGTCTTTGTCTCCAGGGGAAAG AGCCACCCTCTCTTGCAGGGCCAGTCAGAGTGTTAGCAGCAGCTACTTAGCCTGGTACCAGCAGAAACCTGGCC AGGCTCCCAGGCTCCTCATCTATGGAGCATCCAGCAGGGCCACTGGCATCCCAGACAGGTTCAGTGGCAGTGGA TCCGGGACAGACTTCACTCTCACCATCAGCAGACTGGAGCCTGAAGATTTTGCAGTGTATTACTGTCAGCAGTAC AAATCTCATCCAATCACGTTCGGCCAGGGGACCAAAGTGGAAATCAAACGTACCACGACGCCAGCGCCGCGAC CACCAACACCGGCGCCCACCATCGCGTCGCAGCCCCTGTCCCTGCGCCCAGAGGCGTGCCGGCCAGCGGCGGGG GGCGCAGTGCACACGAGGGGGCTGGACTTCGCCTGTGATATCTACATCTGGGCGCCCTTGGCCGGGACTTGTG GGGTCCTTCTCCTGTCACTGGTTATCACCCTTTACTGCAAACGGGGCAGAAAGAAACTCCTGTATATATTCAAACA ACCATTTATGAGACCAGTACAAACTACTCAAGAGGAAGATGGCTGTAGCTGCCGATTTCCAGAAGAAGAAGAAG GAGGATGTGAACTGAGAGTGAAGTTCAGCAGGAGCGCAGACGCCCCCGCGTACCAGCAGGGCCAGAACCAGCT CTATAACGAGCTCAATCTAGGACGAAGAGAGGAGTACGATGTTTTGGACAAGAGACGTGGCCGGGACCCTGAG ATGGGGGGAAAGCCGCAGAGAAGGAAGAACCCTCAGGAAGGCCTGTACAATGAACTGCAGAAAGATAAGATG GCGGAGGCCTACAGTGAGATTGGGATGAAAGGCGAGCGCCGGAGGGGCAAGGGGCACGATGGCCTTTACCAG GGTCTCAGTACAGCCACCAAGGACACCTACGACGCCCTTCACATGCAGGCCCTGCCCCCTCGCGGAAGCGGAG CTACTAACTTCAGCCTGCTGAAGCAGGCTGGAGACGTGGAGGAGAACCCTGGACCTATGGCCTTACCAGTGACC GCCTTGCTCCTGCCGCTGGCCTTGCTGCTCCACGCCGCCAGGCCGGAGGTGCAATTGCTGGAGTCTGGGGGAGG CTTGGTACAGCCTGGGGGGTCCCTGAGACTCTCCTGTGCAGCCTCCGGATTCACCTTTGGCGGTAATGCCATGTC CTGGGTCCGCCAGGCTCCAGGGAAGGGGCTGGAGTGGGTCTCAGCAATTAGTGGTAATGGTGGTAGTACATTCT ACGCAGACTCCGTGAAGGGCCGGTTCACCATCTCCAGAGACAATTCCAAGAACACGCTGTATCTGCAGATGAAC AGCCTGAGAGCCGAGGACACGGCCGTATATTACTGTGCGAAAGTTCGTCCATTCTGGGGTACTTTCGACTACTGG GGCCAAGGAACCCTGGTCACCGTCTCGAGTGGTGGAGGCGGTTCAGGCGGAGGTGGTTCTGGCGGTGGCGGAT CGGAAATCGTGTTAACGCAGTCTCCAGGCACCCTGTCTTTGTCTCCAGGGGAAAGAGCCACCCTCTCTTGCAGGG CCAGTCAGAGTGTTAGCAGCAGCTACTTAGCCTGGTACCAGCAGAAACCTGGCCAGGCTCCCAGGCTCCTCATC TATGGAGCATCCAGCAGGGCCACTGGCATCCCAGACAGGTTCAGTGGCAGTGGATCCGGGACAGACTTCACTCT CACCATCAGCAGACTGGAGCCTGAAGATTTTGCAGTGTATTACTGTCAGCAGTACTTCAACCCACCAGAATACAC GTTCGGCCAGGGGACCAAAGTGGAAATCAAACGTACCACGACGCCAGCGCCGCGACCACCAACACCGGCGCCC ACCATCGCGTCGCAGCCCCTGTCCCTGCGCCCAGAGGCGTGCCGGCCAGCGGCGGGGGGCGCAGTGCACACG AGGGGGCTGGACTTCGCCTGTGATATCTACATCTGGGCGCCCTTGGCCGGGACTTGTGGGGTCCTTCTCCTGTCA CTGGTTATCACCCTTTACTGCAAACGGGGCAGAAAGAAACTCCTGTATATATTCAAACAACCATTTATGAGACCA GTACAAACTACTCAAGAGGAAGATGGCTGTAGCTGCCGATTTCCAGAAGAAGAAGAAGGAGGATGTGAACTGA GAGTGAAGTTCAGCAGGAGCGCAGACGCCCCCGCGTACCAGCAGGGCCAGAACCAGCTCTATAACGAGCTCAA |
| | TCTAGGACGAAGAGAGGAGTACGATGTTTTGGACAAGAGACGTGGCCGGGACCCTGAGATGGGGGGAAAGCCG CAGAGAAGGAAGAACCCTCAGGAAGGCCTGTACAATGAACTGCAGAAAGATAAGATGGCGGAGGCCTACAGTG AGATTGGGATGAAAGGCGAGCGCCGGAGGGGCAAGGGGCACGATGGCCTTTACCAGGGTCTCAGTACAGCCAC CAAGGACACCTACGACGCCCTTCACATGCAGGCCCTGCCCCCTCGC |
| 93 | ATGGCTCTGCCTGTGACAGCTCTGCTGCTGCCTCTGGCTCTGCTTCTGCATGCCGCTAGACCTGAAATTGTGCTGA CACAGAGCCCCGGCACACTGTCTCTTAGCCCTGGCGAAAGAGCCACACTGAGCTGTAGAGCCAGCCAGAGCGT GTCCAGCTCTTACCTGGCTTGGTATCAGCAGAAGCCCGGACAGGCACCCAGACTGCTGATCTATGGCGCCTCCTC TAGAGCTACAGGCATCCCCGATAGATTTTCCGGCAGCGGCTCTGGCACCGACTTCACCCTGACAATCAGCAGACT GGAACCCGAGGACTTTGCCGTGTATTATTGCCAGCAGTACTTCAACCCTCCTGAGTACACCTTCGGACAGGGCAC CAAGGTGGAAATCAAGCGCGGAGGCGGAGGTTCTGAGGTCCAGCTGTTGGAAAGTGGCGGTGGTCTTGTGCAG CCTGGCGGAAGCCTCAGACTTAGCTGTGCCGCCTCCGGCTTCACATTCAGCAGCTACGCCATGTCCTGGGTTCGA CAAGCACCCGGAAAAGGATTGGAGTGGGTTTCAGCCATCAGCGGCAGCGGAGGCAGCACTTATTATGCCGATA GCGTGAAAGGCCGGTTCACCATCTCCAGAGACAACTCCAAGAATACGCTCTATCTCCAGATGAACTCTCTGCGCG CCGAAGATACAGCTGTGTACTATTGCGCCAGAGGCTGGCCCTCTCCTGTGACCTTTGATTACTGGGGACAAGGGA CCCTCGTGACCGTTTCTTCTGGTGGCGGCGGTAGTGGCGGAGGTGGAAGTGGCGGTGGTGGAAGCGAAATCGTG CTGACCCAGTCTCCTGGAACTCTGTCTCTGTCCCCTGGCGAGAGGGCTACCCTGTCTTGTAGAGCCTCTCAGTCC GTGTCCTCCAGCTATCTCGCCTGGTATCAACAAAAGCCTGGGCAAGCCCCTCGGCTGCTGATATACGGGGCTTCT TCTCGCGCCACCGGCATTCCAGACAGATTCAGCGGATCTGGAAGCGGCACAGATTTCACACTGACCATCTCTCG GCTGGAACCTGAAGATTTCGCTGTCTACTACTGTCAGCAGTACAAGTCTCACCCCATCACCTTTGGCCAGGGAAC AAAGGTCGAGATCAAGAGAGGTGGCGGAGGCTCTGAAGTGCAGCTGCTGGAATCTGGCGGAGGACTGGTTCAA CCTGGCGGCTCTCTGAGACTGTCTTGTGCCGCCAGCGGCTTCACCTTTGGCGGCAATGCCATGAGCTGGGTCCGA CAGGCTCCTGGCAAAGGCCTTGAATGGGTGTCCGCCATCTCTGGCAATGGCGGCAGCACCTTTTACGCCGACTCT GTGAAGGGCAGATTCACCATCAGCCGGGACAACAGCAAGAACACCCTGTATCTGCAGATGAACAGCCTGAGAG CCGAGGACACCGCCGTGTACTACTGTGCTAAAGTGCGGCCTTTCTGGGGCACCTTCGATTATTGGGGCCAGGGC ACACTGGTCACAGTGTCTAGCACCACAACACCCGCTCCTAGACCTCCAACACCAGCTCCAACAATCGCCAGCCA GCCTCTGTCTCTGAGGCCAGAAGCTTGTAGACCTGCTGCTGGCGGAGCCGTGCATACAAGAGGACTGGATTTCG CCTGCGACATCTACATCTGGGCCCCTCTGGCTGGAACATGTGGCGTTCTGCTGCTGAGCCTGGTCATCACCCTGT ACTGCAAGCGGGGCAGAAAGAAGCTGCTGTACATCTTCAAGCAGCCCTTCATGCGGCCCGTGCAGACCACACAA GAGGAAGATGGCTGCTCCTGCAGATTCCCCGAGGAAGAAGAAGGCGGCTGCGAGCTGAGAGTGAAGTTCAGCA GATCCGCCGACGCTCCTGCCTATCAGCAGGGACAGAACCAGCTGTACAACGAGCTGAACCTCGGGAGAAGAGA AGAGTACGACGTGCTGGACAAGCGGAGAGGCAGAGATCCTGAGATGGGCGGAAAGCCCCAGCGGAGAAAGAA TCCTCAAGAGGGCCTGTATAATGAGCTGCAGAAAGACAAGATGGCCGAGGCCTACAGCGAGATCGGAATGAAG GGCGAGCGCAGAAGAGGCAAGGGACACGATGGACTGTACCAGGGCCTGAGCACCGCCACCAAGGATACCTAT GATGCCCTGCACATGCAGGCCCTGCCTCCAAGA |
| 94 | ATGGCCTTACCAGTGACCGCCTTGCTCCTGCCGCTGGCCTTGCTGCTCCACGCCGCCAGGCCGGAAATCGTGTTA ACGCAGTCTCCAGGCACCCTGTCTTTGTCTCCAGGGGAAAGAGCCACCCTCTCTTGCAGGGCCAGTCAGAGTGTT AGCAGCAGCTACTTAGCCTGGTACCAGCAGAAACCTGGCCAGGCTCCCAGGCTCCTCATCTATGGAGCATCCAG CAGGGCCACTGGCATCCCAGACAGGTTCAGTGGCAGTGGATCCGGGACAGACTTCACTCTCACCATCAGCAGAC TGGAGCCTGAAGATTTTGCAGTGTATTACTGTCAGCAGTACTTCAACCCACCAGAATACACGTTCGGCCAGGGGA CCAAAGTGGAAATCAAACGTGGTGGCGGAGGAAGCGAGGTGCAATTGCTGGAGTCTGGGGGAGGCTTGGTACA GCCTGGGGGGTCCCTGAGACTCTCCTGTGCAGCCTCCGGATTCACCTTTAGCAGTTATGCCATGAGCTGGGTCCG CCAGGCTCCAGGGAAGGGGCTGGAGTGGGTCTCAGCTATTAGTGGTAGTGGTGGTAGCACATACTACGCAGACT CCGTGAAGGGCCGGTTCACCATCTCCAGAGACAATTCCAAGAACACGCTGTATCTGCAGATGAACAGCCTGAGA GCCGAGGACACGGCCGTATATTACTGTGCGCGTGGTTGGCCATCTCCAGTTACTTTCGACTACTGGGGCCAAGGA ACCCTGGTCACCGTCTCGAGTGGTGGAGGCGGTTCAGGCGGAGGTGGTTCTGGCGGTGGCGGATCGGAAATCGT GTTAACGCAGTCTCCAGGCACCCTGTCTTTGTCTCCAGGGGAAAGAGCCACCCTCTCTTGCAGGGCCAGTCAGA GTGTTAGCAGCAGCTACTTAGCCTGGTACCAGCAGAAACCTGGCCAGGCTCCCAGGCTCCTCATCTATGGAGCAT CCAGCAGGGCCACTGGCATCCCAGACAGGTTCAGTGGCAGTGGATCCGGGACAGACTTCACTCTCACCATCAGC AGACTGGAGCCTGAAGATTTTGCAGTGTATTACTGTCAGCAGTACAAATCTCATCCAATCACGTTCGGCCAGGGG ACCAAAGTGGAAATCAAACGTGGAGGCGGCGGAAGCGAGGTGCAATTGCTGGAGTCTGGGGGAGGCTTGGTAC AGCCTGGGGGGTCCCTGAGACTCTCCTGTGCAGCCTCCGGATTCACCTTTGGCGGTAATGCCATGTCCTGGGTCC GCCAGGCTCCAGGGAAGGGGCTGGAGTGGGTCTCAGCAATTAGTGGTAATGGTGGTAGTACATTCTACGCAGAC TCCGTGAAGGGCCGGTTCACCATCTCCAGAGACAATTCCAAGAACACGCTGTATCTGCAGATGAACAGCCTGAG |
| | AGCCGAGGACACGGCCGTATATTACTGTGCGAAAGTTCGTCCATTCTGGGGTACTTTCGACTACTGGGGCCAAGG AACCCTGGTCACCGTCTCGAGTACCACGACGCCAGCGCCGCGACCACCAACACCGGCGCCCACCATCGCGTCG CAGCCCCTGTCCCTGCGCCCAGAGGCGTGCCGGCCAGCGGCGGGGGGCGCAGTGCACACGAGGGGGCTGGAC TTCGCCTGTGATATCTACATCTGGGCGCCCTTGGCCGGGACTTGTGGGGTCCTTCTCCTGTCACTGGTTATCACCC TTTACTGCAAACGGGGCAGAAAGAAACTCCTGTATATATTCAAACAACCATTTATGAGACCAGTACAAACTACTC AAGAGGAAGATGGCTGTAGCTGCCGATTTCCAGAAGAAGAAGAAGGAGGATGTGAACTGAGAGTGAAGTTCAG CAGGAGCGCAGACGCCCCCGCGTACCAGCAGGGCCAGAACCAGCTCTATAACGAGCTCAATCTAGGACGAAGA GAGGAGTACGATGTTTTGGACAAGAGACGTGGCCGGGACCCTGAGATGGGGGGAAAGCCGCAGAGAAGGAAG AACCCTCAGGAAGGCCTGTACAATGAACTGCAGAAAGATAAGATGGCGGAGGCCTACAGTGAGATTGGGATGA AAGGCGAGCGCCGGAGGGGCAAGGGGCACGATGGCCTTTACCAGGGTCTCAGTACAGCCACCAAGGACACCTA CGACGCCCTTCACATGCAGGCCCTGCCCCCTCGC |
| 95 | ATGGCTCTGCCTGTGACAGCTCTGCTGCTGCCTCTGGCTCTGCTTCTGCATGCCGCTAGACCTGAAATCGTGCTGA CCCAGTCTCCTGGAACTCTGTCTCTGTCCCCTGGCGAGAGGGCTACCCTGTCTTGTAGAGCCTCTCAGTCCGTGTC CTCCAGCTATCTCGCCTGGTATCAACAAAAGCCTGGGCAAGCCCCTCGGCTGCTGATATACGGGGCTTCTTCTCG CGCCACCGGCATTCCAGACAGATTCAGCGGATCTGGAAGCGGCACAGATTTCACACTGACCATCTCTCGGCTGG AACCTGAAGATTTCGCTGTCTACTACTGTCAGCAGTACAAGTCTCACCCCATCACCTTTGGCCAGGGAACAAAGG TCGAGATCAAGAGAGGTGGCGGCGGTAGTGAAGTGCAGCTGCTGGAATCTGGCGGAGGACTGGTTCAACCTGG CGGCTCTCTGAGACTGTCTTGTGCCGCCAGCGGCTTCACCTTTGGCGGCAATGCCATGAGCTGGGTCCGACAGG CTCCTGGCAAAGGCCTTGAATGGGTGTCCGCCATCTCTGGCAATGGCGGCAGCACCTTTTACGCCGACTCTGTGA AGGGCAGATTCACCATCAGCCGGGACAACAGCAAGAACACCCTGTATCTGCAGATGAACAGCCTGAGAGCCGA GGACACCGCCGTGTACTACTGTGCTAAAGTGCGGCCTTTCTGGGGCACCTTCGATTATTGGGGCCAGGGCACACT GGTCACAGTGTCTAGCGGAGGCGGAGGTTCTGGCGGCGGAGGAAGTGGTGGCGGAGGCTCTGAAATTGTGCTG ACACAGAGCCCCGGCACACTGTCTCTTAGCCCTGGCGAAAGAGCCACACTGAGCTGTAGAGCCAGCCAGAGCG TGTCCAGCTCTTACCTGGCTTGGTATCAGCAGAAGCCCGGACAGGCACCCAGACTGCTGATCTATGGCGCCTCCT CTAGAGCTACAGGCATCCCCGATAGATTTTCCGGCAGCGGCTCTGGCACCGACTTCACCCTGACAATCAGCAGA CTGGAACCCGAGGACTTTGCCGTGTATTATTGCCAGCAGTACTTCAACCCTCCTGAGTACACCTTCGGACAGGGC ACCAAGGTGGAAATCAAGCGCGGCGGTGGTGGAAGCGAGGTCCAGCTGTTGGAAAGTGGCGGTGGTCTTGTGC AGCCTGGCGGAAGCCTCAGACTTAGCTGTGCCGCCTCCGGCTTCACATTCAGCAGCTACGCCATGTCCTGGGTTC GACAAGCACCCGGAAAAGGATTGGAGTGGGTTTCAGCCATCAGCGGCAGCGGAGGCAGCACTTATTATGCCGA TAGCGTGAAAGGCCGGTTCACCATCTCCAGAGACAACTCCAAGAATACGCTCTATCTCCAGATGAACTCTCTGCG CGCCGAAGATACAGCTGTGTACTATTGCGCCAGAGGCTGGCCCTCTCCTGTGACCTTTGATTACTGGGGACAAGG GACCCTCGTGACCGTTTCTTCTACCACAACACCCGCTCCTAGACCTCCAACACCAGCTCCAACAATCGCCAGCCA GCCTCTGTCTCTGAGGCCAGAAGCTTGTAGACCTGCTGCTGGCGGAGCCGTGCATACAAGAGGACTGGATTTCG CCTGCGACATCTACATCTGGGCCCCTCTGGCTGGAACATGTGGCGTTCTGCTGCTGAGCCTGGTCATCACCCTGT ACTGCAAGCGGGGCAGAAAGAAGCTGCTGTACATCTTCAAGCAGCCCTTCATGCGGCCCGTGCAGACCACACAA GAGGAAGATGGCTGCTCCTGCAGATTCCCCGAGGAAGAAGAAGGCGGCTGCGAGCTGAGAGTGAAGTTCAGCA GATCCGCCGACGCTCCTGCCTATCAGCAGGGACAGAACCAGCTGTACAACGAGCTGAACCTCGGGAGAAGAGA AGAGTACGACGTGCTGGACAAGCGGAGAGGCAGAGATCCTGAGATGGGCGGAAAGCCCCAGCGGAGAAAGAA TCCTCAAGAGGGCCTGTATAATGAGCTGCAGAAAGACAAGATGGCCGAGGCCTACAGCGAGATCGGAATGAAG GGCGAGCGCAGAAGAGGCAAGGGACACGATGGACTGTACCAGGGCCTGAGCACCGCCACCAAGGATACCTAT GATGCCCTGCACATGCAGGCCCTGCCTCCAAGA |
| 96 | EIVLTQSPGTLSLSPGERATLSCRASQSVSSSYLAWYQQKPGQAPRLLIYGASSRATGIPDRFSGSGSGTDFTLTISRLEPE DFAVYYCQQYFNPPEYTFGQGTKVEIKRGGGGSEVQLLESGGGLVQPGGSLRLSCAASGFTFSSYAMSWVRQAPGKGL EWVSAISGSGGSTYYADSVKGRFTISRDNSKNTLYLQMNSLRAEDTAVYYCARGWPSPVTFDYWGQGTLVTVSSGGG GSGGGGSGGGGSEIVLTQSPGTLSLSPGERATLSCRASQSVSSSYLAWYQQKPGQAPRLLIYGASSRATGIPDRFSGSGS GTDFTLTISRLEPEDFAVYYCQQYKSHPITFGQGTKVEIKRGGGGSEVQLLESGGGLVQPGGSLRLSCAASGFTFGGNAM SWVRQAPGKGLEWVSAISGNGGSTFYADSVKGRFTISRDNSKNTLYLQMNSLRAEDTAVYYCAKVRPFWGTFDYWG QGTLVTVSSESKYGPPCPPCPMFWVLVVVGGVLACYSLLVTVAFIIFWVKRGRKKLLYIFKQPFMRPVQTTQEEDGCSCR FPEEEEGGCELRVKFSRSADAPAYQQGQNQLYNELNLGRREEYDVLDKRRGRDPEMGGKPQRRKNPQEGLYNELQKD KMAEAYSEIGMKGERRRGKGHDGLYQGLSTATKDTYDALHMQALPPR* |
| 97 | EVQLLESGGGLVQPGGSLRLSCAASGFTFSSYAMSWVRQAPGKGLEWVSAISGSGGSTYYADSVKGRFTISRDNSKNTL YLQMNSLRAEDTAVYYCARGWPSPVTFDYWGQGTLVTVSSGGGGSGGGGSGGGGSEIVLTQSPGTLSLSPGERATLS CRASQSVSSSYLAWYQQKPGQAPRLLIYGASSRATGIPDRFSGSGSGTDFTLTISRLEPEDFAVYYCQQYKSHPITFGQGT |
| | KVEIKRTTTPAPRPPTPAPTIASQPLSLRPEACRPAAGGAVHTRGLDFACDIYIWAPLAGTCGVLLLSLVITLYCKRGRKKLL YIFKQPFMRPVQTTQEEDGCSCRFPEEEEGGCELRVKFSRSADAPAYQQGQNQLYNELNLGRREEYDVLDKRRGRDPE MGGKPQRRKNPQEGLYNELQKDKMAEAYSEIGMKGERRRGKGHDGLYQGLSTATKDTYDALHMQALPPR↵ |
| 98 | EVQLLESGGGLVQPGGSLRLSCAASGFTFGGNAMSWVRQAPGKGLEWVSAISGNGGSTFYADSVKGRFTISRDNSKN TLYLQMNSLRAEDTAVYYCAKVRPFWGTFDYWGQGTLVTVSSGGGGSGGGGSGGGGSEIVLTQSPGTLSLSPGERATL SCRASQSVSSSYLAWYQQKPGQAPRLLIYGASSRATGIPDRFSGSGSGTDFTLTISRLEPEDFAVYYCQQYFNPPEYTFG QGTKVEIKRTTTPAPRPPTPAPTIASQPLSLRPEACRPAAGGAVHTRGLDFACDIYIWAPLAGTCGVLLLSLVITLYCKRGR KKLLYIFKQPFMRPVQTTQEEDGCSCRFPEEEEGGCELRVKFSRSADAPAYQQGQNQLYNELNLGRREEYDVLDKRRGR DPEMGGKPQRRKNPQEGLYNELQKDKMAEAYSEIGMKGERRRGKGHDGLYQGLSTATKDTYDALHMQALPPR |
| 99 | EVQLLESGGGLVQPGGSLRLSCAASGFTFSSYAMSWVRQAPGKGLEWVSAISGSGGSTYYADSVKGRFTISRDNSKNTL YLQMNSLRAEDTAVYYCARGWPSPVTFDYWGQGTLVTVSS |
| 100 | EVQLLESGGGLVQPGGSLRLSCAASGFTFGGNAMSWVRQAPGKGLEWVSAISGNGGSTFYADSVKGRFTISRDNSKN TLYLQMNSLRAEDTAVYYCAKVRPFWGTFDYWGQGTLVTVSS |
| 101 | EIVLTQSPGTLSLSPGERATLSCRASQSVSSSYLAWYQQKPGQAPRLLIYGASSRATGIPDRFSGSGSGTDFTLTISRLEPE DFAVYYCQQYFNPPEYTFGQGTKVEIKR↵ |
| 102 | EIVLTQSPGTLSLSPGERATLSCRASQSVSSSYLAWYQQKPGQAPRLLIYGASSRATGIPDRFSGSGSGTDFTLTISRLEPE DFAVYYCQQYKSHPITFGQGTKVEIKR↵ |
| 103 | EVQLLESGGGLVQPGGSLRLSCAASGFTFSSYAMSWVRQAPGKGLEWVSAISGSGGSTYYADSVKGRFTISRDNSKNTL YLQMNSLRAEDTAVYYCARGWPSPVTFDYWGQGTLVTVSSGGGGSGGGGSGGGGSEIVLTQSPGTLSLSPGERATLS CRASQSVSSSYLAWYQQKPGQAPRLLIYGASSRATGIPDRFSGSGSGTDFTLTISRLEPEDFAVYYCQQYKSHPITFGQGT KVEIKR↵ |
| 104 | SYAMS |
| 105 | AISGSGGSTYYADSVKG |
| 106 | GWPSPVTFDY |
| 107 | RASQSVSSSYLA |
| 108 | GASSRAT |
| 109 | QQYKSHPIT |
| 110 | GNAMS |
| 111 | AISGNGGSTFYADSVKG |
| 112 | VRPFWGTFDY |
| 113 | RASQSVSSSYLA |
| 114 | GASSRAT |
| 115 | QQYFNPPEYT |
| 116 | QVQLVQSGAEVKKPGASVKVSCKASGYTFTSYWMNWVRQAPGQGLEWMGRIDPYDSETHYAQKLQGRVTMTTDTS TSTAYMELRSLRSDDTAVYYCARGGYDFDVGTLYWFFDVWGQGTTVTVSS↵ |
| 117 | DIQMTQSPSSLSASVGDRVTITCRASENIYSYLAWYQQKPGKAPKLLIYNAKTLAEGVPSRFSGSGSGTDFTLTISSLQPE DFATYYCQHHYGTPRTFGGGTKVEIK |

## Claims

1. A chimeric receptor, wherein the chimeric receptor comprises an antigen binding domain that binds to CD38, a transmembrane domain and an intracellular domain, wherein the antigen binding domain is connected to the transmembrane domain via an IgG4 hinge region or a fragment thereof.

2. The chimeric receptor according to claim 1, wherein the IgG4 hinge region or a fragment thereof is a truncated IgG4 hinge or a modified IgG4 hinge;
preferably, the IgG4 hinge region or a fragment thereof comprises an amino acid sequence shown in SEQ ID NO:5 or 6, or an amino acid sequence having at least 80%, 85%, 90%, 95%, 96%, 97%, 98% or 99% sequence identity to the amino acid sequence shown in SEQ ID NO:5 or 6.

3. The chimeric receptor according to claim 1 or 2, wherein the antigen binding domain comprises: an extracellular segment of a natural CD38 ligand or a fragment or variant thereof that binds to CD38, an anti-CD38 antibody or a fragment thereof, or a synthetic binding domain that binds to CD38; and preferably, the anti-CD38 antibody or a fragment thereof is selected from: a full antibody, a scFv, a single domain antibody, a Fab fragment, a Fab' fragment, a Fv fragment, a F(ab')2 fragment, a Fd fragment, a sdAb, a multifunctional antibody, a DDPP antibody, a scFv-Fc antibody or an IgG4 antibody.

4. The chimeric receptor according to any one of claims 1 to 3, wherein the antigen binding domain is an anti-CD38 antibody selected from any one of the following (1)-(3):
(1) an anti-CD38 antibody, comprising an scFv comprising an amino acid sequence having at least 80%, 85%, 90%, 95% or 100% identity to the amino acid sequence shown in SEQ ID NO: 19, 20, 21 or 22;
(2) an anti-CD38 antibody comprising a VH and a VL, wherein the VH and the VL respectively comprise the amino acid sequences shown in SEQ ID NOs: 49, and 50; or the amino acid sequences shown in SEQ ID NOs: 57, and 58; or the amino acid sequences shown in SEQ ID NOs: 65, and 66; or the amino acid sequences shown in SEQ ID NOs: 73, and 74, or amino acid sequences having at least 80%, 85%, 90%, 95%, 96%, 97%, 98% or 99% identity to the above sequences;
(3) an anti-CD38 antibody, comprising VH and VL, wherein the VH comprises HCDR1, HCDR2, and HCDR3, and the VL comprises LCDR1, LCDR2, and LCDR3; wherein the HCDR1, HCDR2, HCDR3, LCDR1, LCDR2, and LCDR3 have the amino acid sequences shown in SEQ ID NOs: 43, 44, 45, 46, 47, and 48, respectively; or the amino acid sequences shown in SEQ ID NOs: 51, 52, 53, 54, 55, and 56, respectively; or the amino acid sequences shown in SEQ ID NOs: 59, 60, 61, 62, 63, and 64, respectively; or the amino acid sequences shown in SEQ ID NOs: 67, 68, 69, 70, 71, and 72, respectively.

5. The chimeric receptor according to any one of claims 1 to 4, wherein the transmembrane domain is selected from: the transmembrane domain of CD3ε, CD3δ, CD3ζ, CD8, CD28, CD134, CD137, CD150, CD152, DAP10, FcRα, FcRβ, FcRγ, or Zap70; and preferably, the transmembrane domain comprises the amino acid sequence shown in SEQ ID NO:7, 8 or 9.

6. The chimeric receptor according to any one of claims 1 to 5, wherein the intracellular domain is selected from: the intracellular signaling domain of CD3, CD28, CD137, OX40, DAP10 or ICOS or a combination thereof; and preferably, the intracellular domain comprises the amino acid sequence shown in SEQ ID NO: 10, 11, 12, or 13 or a combination thereof.

7. The chimeric receptor according to any one of claims 1 to 6, wherein the chimeric receptor expressed by the nucleic acid sequence having at least 85%, 90%, 95%, 96%, 97%, 98%, 99% or 100% identity to the nucleic acid sequence shown in SEQ ID NO:4, 18, 23, 25, 29, 34, 36, 37, or 38; or comprises an amino acid sequence having at least 85%, 90%, 95%, 96%, 97%, 98%, 99% or 100% identity to the amino acid sequence shown in SEQ ID NO:24, 26, 27, or 28.

8. The chimeric receptor according to any one of claims 1 to 7, wherein the antigen binding domain also binds to another antigen; preferably, the another antigen is a pathological cell antigen or an immune cell antigen; and preferably, the pathological cell is selected from a solid tumor cell, a hematological tumor cell, or a pathological cell of autoimmune diseases; and the immune cell antigen is selected from the antigen of NK cell or T cell.

9. An engineered cell comprising the chimeric receptor of any one of claims 1 to 8.

10. The engineered cell according to claim 9, wherein the engineered cell further comprises another polypeptide, the another polypeptide comprises: a second chimeric receptor that binds to another NK cell marker, a target antigen on a pathological cell, or a combination thereof, or an HLA-E polypeptide or a fragment thereof, or CD300A-PDGFR.

11. The engineered cell according to claim 10, wherein the pathological cell is selected from a malignant cell or an infected cell; and preferably, the pathological cell is selected from a solid tumor cell, a hematological tumor cells, or a pathological cells of autoimmune diseases;
preferably, the solid tumor is selected from the group consisting of esophageal cancer, gastric cancer, liver cancer, biliary tract tumors, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, and sarcoma; the hematological tumor is selected from the group consisting of leukemia, lymphoma, and myeloma; the autoimmune disease is selected from the group consisting of multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis, and dermatomyositis.

12. The engineered cell according to claim 10 or 11, wherein the target antigen is selected from the group consisting of CD19, CD20, CD22, CD38, BCMA, GPRC5D, B7H3, GPC3, Claudin 6, Claudin18.2, FAP, Mesothelin, NKG2D ligand, NKG2A, CD94, FCRH5, EGFR and its mutants, ASGPR1, IL13RA2, WT1, CLL1 or a combination thereof; the another NK cell marker is selected from the group consisting of CD2, CD3, CD4, CD5, CD7, CD8, CD16a, CD16b, CD25, CD27, CD28, CD30, CD38, CD45, CD48, CD50, CD52, CD56, CD57, CD62L, CD69, CD94, CD100, CD102, CD122, CD127, CD132, CD137, CD138, CD160, CD161, CD178, CD218, CD226, CD244, CD159a (NKG2A), CD159c (NKG2C), FasL, NKG2E, CD279, CD300A, CD314 (NKG2D), CD305, CD335 (NKP46), CD337, CD319 (CS1), TCRα, TCRβ, TIGIT, TRAIL, SLAMF7, NKG2F, NKG2H, NKp30, NKp44, NKp46, NKp80, SLAM family members, L-selectin, natural cytotoxicity receptor NCR1, NCR2, NCR3, and a combination thereof.

13. The engineered cell according to any one of claims 10 to 12, wherein the another polypeptide comprises an amino acid sequence having at least 80%, 85%, 90%, 95%, 96%, 97%, 98%, 99% or 100% identity to the amino acid sequence shown in any one of SEQ ID NO: 83, 84, 85 or 86, or an amino acid sequence having at least 80%, 85%, 90%, 95%, 96%, 97%, 98%, 99% or 100% identity to the amino acid sequence encoded by the nucleic acid sequence shown in any one of SEQ ID NO: 30, 31, 32, 33 or 35.

14. The engineered cell according to any one of claims 9 to 13, wherein the engineered cell has low or no expression of endogenous TCR/B2M, TCR/B2M/FAS, TCR/B2M/CD38, TCR/B2M/NKG2A, TCR/B2M/CD38/NKG2A or TCR/B2M/FAS/NKG2A; preferably, the endogenous TCR/B2M, TCR/B2M/FAS, TCR/B2M/CD38, TCR/B2M/NKG2A, TCR/B2M/CD38/NKG2A or TCR/B2M/FAS/NKG2A of the engineered cell are knocked out or knocked down using CRISPR technology; more preferably, when knocked out or knocked down using CRISPR technology, the gRNA sequences used for targeting TCR, B2M, NKG2A, and CD38 are shown in SEQ ID NOs: 39, 40, 41, and 42, respectively.

15. The engineered cell according to any one of claims 9 to 14, wherein the engineered cell is an autologous or allogeneic cell, selected from: an immune cell, a neuron, an epithelial cell, an endothelial cell, a stem cell, or a combination thereof;
preferably, the engineered cell is selected from: a B cell, a monocyte, a natural killer cell, a basophil, an eosinophil, a neutrophil, a dendritic cell, a macrophage, a T cell, a NKT cell, a stem cell-derived immune effector cell, or a combination thereof; and preferably, the engineered cell is an allogeneic T cell.

16. The engineered cell according to any one of claims 9 to 15, wherein the engineered cell significantly blocks the CD38 antigen on its own cell.

17. The engineered cell according to any one of claims 9 to 16, wherein the engineered cell has prolonged survival time or enhanced proliferation capacity in the presence of host immune cells; or the engineered cell can enhance the survival, proliferation, and killing of pathological cells of another engineered cell introduced into the subject before, concurrently with, or after the engineered cell;
preferably, the host immune cell is an NK cell.

18. A polynucleotide, which is a nucleic acid molecule encoding the chimeric receptor according to any one of claims 1 to 8.

19. The polynucleotide of claim 18, wherein the polynucleotide is connected to a polynucleotide encoding another polypeptide comprised in the engineered cell according to any one of claims 10 to 17 via a 2A fragment; the polynucleotide encoding the another polypeptide comprises a nucleic acid sequence having at least 80%, 85%, 90%, 95%, 96%, 97%, 98%, 99% or 100% identity to the nucleic acid sequence shown in any one of SEQ ID NO: 30, 31, 32, 33 or 35, or comprises a nucleic acid sequence having at least 80%, 85%, 90%, 95%, 96%, 97%, 98%, 99% or 100% identity to the nucleic acid sequence encoding the amino acid sequence shown in any one of SEQ ID NO: 83, 84, 85 or 86.

20. The polynucleotide of claim 19, wherein the polynucleotide comprises a nucleic acid sequence having at least 80%, 85%, 90%, 95%, 96%, 97%, 98%, 99% or 100% identity to the nucleic acid sequence shown in any one of SEQ ID NOs: 34, 36, 37 or 38.

21. A vector comprising the polynucleotide according to any one of claims 18 to 20.

22. A virus comprising the vector of claim 21.

23. A pharmaceutical composition comprising an effective amount of the chimeric receptor according to any one of claims 1 to 8, the engineered cell according to any one of claims 9 to 17, the polynucleotide according to any one of claims 18 to 20, the vector according to claim 21, or the virus according to claim 22, and a pharmaceutically acceptable carrier.

24. The chimeric receptor according to any one of claims 1 to 8, or the engineered cell according to any one of claims 9 to 17, for use in preparing a medicament for preventing or resisting transplant immune rejection, or for preventing or resisting transplant immune rejection, or for preparing a medicament for treating an autoimmune disease or an inflammatory disease, or for treating, preventing or ameliorating an autoimmune disease or an inflammatory disease in a subject in need thereof, or for preparing a medicament for treating a tumor, or for treating, preventing or ameliorating a tumor in a subject in need thereof.

25. The use according to claim 24, wherein the autoimmune disease or inflammatory disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis or dermatomyositis, Waldenström's macroglobulinemia, primary systemic amyloidosis, membranous glomerulonephritis, idiopathic thrombocytopenic purpura or myasthenia gravis; the tumor is selected from: esophageal cancer, gastric cancer, liver cancer, biliary tract tumors, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, prostate cancer, triple-negative breast tumors, sarcoma, leukemia, lymphoma, myeloma, chronic lymphatic leukemia (CLL), mantle cell lymphoma (MCL), acute myeloid leukemia (AML), acute lymphoblastic leukemia (ALL), NK cell leukemia, NK/T-cell lymphoma (NKTCL), or myelodysplastic syndrome.

26. A method for preventing or resisting transplant immune rejection, or a method for treating an autoimmune disease or an inflammatory disease, or a method for treating a tumor, comprising administering to a subject the chimeric receptor according to any one of claims 1 to 8, the engineered cell according to any one of claims 9 to 17, the polynucleotide according to any one of claims 18 to 20, the vector according to claim 21, a virus according to claim 22, or a pharmaceutical composition according to claim 23, preferably, the autoimmune disease or inflammatory disease is selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis or dermatomyositis, Waldenström's macroglobulinemia, primary systemic amyloidosis, membranous glomerulonephritis, idiopathic thrombocytopenic purpura or myasthenia gravis; the tumor is selected from: esophageal cancer, gastric cancer, liver cancer, biliary tract tumors, pancreatic cancer, intestinal cancer, laryngeal cancer, lung cancer, breast cancer, head and neck cancer, glioma, thyroid cancer, kidney cancer, bladder cancer, ovarian cancer, cervical cancer, melanoma, prostate cancer, triple-negative breast tumors, sarcoma, leukemia, lymphoma, myeloma, chronic lymphatic leukemia (CLL), mantle cell lymphoma (MCL), acute myeloid leukemia (AML), acute lymphoblastic leukemia (ALL), NK cell leukemia, NK/T-cell lymphoma (NKTCL), or myelodysplastic syndrome.
